# EUROPEAN PATENT SPECIFICATION

(11) **EP 2 952 505 B1**
(45) Date of publication and mention of the grant of the patent: **23.05.2018**
(21) Application number: 14171325.5
(22) Date of filing: 05.06.2014
(51) Int. Cl.: C07D 219/08, C07C 319/18, C07C 323/20, C07C 323/25, C07C 323/41, C07C 329/10, C07D 279/20, C07D 513/04, C10M 135/30

(54) **A HINDERED PHENOL COMPOUND, PREPARATION THEREOF AND USE THEREOF AS AN ANTIOXIDANT**
GEHINDERTE PHENOLVERBINDUNG, HERSTELLUNG DAVON UND VERWENDUNG DAVON ALS ANTIOXIDATIONSMITTEL
COMPOSÉ DE PHÉNOL ENCOMBRÉ, SA PRÉPARATION ET SON UTILISATION EN TANT QU'ANTIOXYDANT

(43) Date of publication of application: 09.12.2015
(73) Proprietor: China Petroleum & Chemical Corporation, Beijing 100728 (CN); RESEARCH INSTITUTE OF PETROLEUM PROCESSING, SINOPEC, Beijng 100083 (CN)
(72) Inventor: Su, Shuo, 100083 Beijing (CN); Long, Jun, 100083 Beijing (CN); Duan, Qinghua, 100083 Beijing (CN); Zhou, Han, 100083 Beijing (CN); Wu, Zhiqiang, 100083 Beijing (CN); Li, Xinhua, 100083 Beijing (CN); Zhao, Yi, 100083 Beijing (CN); Zhao, Xiaoguang, 100083 Beijing (CN)
(74) Representative: Hoffmann Eitle

(56) References cited:
- EP-A2- 0 811 631
- CA-A1- 1 219 584
- US-A- 4 551 259
- US-A- 4 772 405
- US-A- 5 304 314
- US-A1- 2006 189 824
- US-A1- 2006 217 274
- DATABASE REGISTRY [Online] CHEMICAL ABSTRACTS SERVICE, COLUMBUS, OHIO, US; 12 August 2012 (2012-08-12), XP002732884, retrieved from STN Database accession no. 1389055-22-4 -& DATABASE CHEMCATS [Online] CHEMICAL ABSTRACTS SERVICE, COLUMBUS, OHIO, US; 24 October 2013 (2013-10-24), XP002732885, retrieved from STN Database accession no. 1946301708

## Description

### Technical Field

This invention relates to a novel hindered phenol compound. Specifically, this invention relates to a hindered phenol compound containing sulfur and an aromatic amine moiety, preparation thereof and use thereof as an antioxidant.

### Background Art

Oxidation stability has been identified as a very important performance for an oil product. For example, to nearly all types of lubricant oil products, an antioxidant is added as an essential component, so as to eniongate its serve life. Further, with the rapid development of the modern automobile industry and machinery industry, the power output from engines and that input into machenical machines have been dramatically increased, and as a result, the temperature at which lubricant oils work has been significantly increased, which exacerbates the oxidation problem. At the same time, as the quality grade of lubricant oils improves, it is needed to further lengthen the change-oil cycle, which sets an even higher demand on the oxidation stability (at elevated temperatures) of lubricant oil products.

In order to met these requirements set by the prior art, the technisans have been working on developing novel compounds having improved performance in oxidation resistance, so as to improve the oxidation stability of oil products. US patent No. 5304314 discloses a phenol compound containing S and an aromatic amine moiety to be used as an antioxidant. US patent application No. 2006/0189824A1 discloses a hindered phenol compound containing a secondary aromatic amine moiety to be used as an antioxidant. Canada patent No. 1219584 discloses a hindered phenol compound containing S and a tertiary aromatic amine moiety to be used as an antioxidant. However, there still remains much room for the prior art compounds to be improved in oxidation resistance at elevated temperatures.

Therefore, there still remains a need for a novel compound, which exhibits significantly improved oxidation resistance at elevated temperatures as compared with prior art ones.

### Invention Summary

The present inventors, on the basis of the prior art, found a novel hindered phenol compound, and further found that, by using the hindered phenol compound as an antioxidant, the aforesaid problems encountered by the prior art can be solved, and then this invention is achieved.

Specifically, this invention relates to the following aspects.
1. A hindered phenol compound represented by Formula (I), 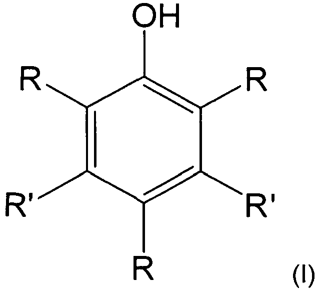
   In Formula (I), the plural Rs may be identical to or different from each other, each independently selected from the group consisting of a C₁₋₃₀₀ linear or branched alkyl (preferably a C₁₋₁₀ linear or branched alkyl or a polyolefin group having a number-average molecular weight (Mn) of from 300 to 3000), a group represented by Formula (II) and a group represented by Formula (III), with the proviso that at least one R is a group represented by Formula (II); the plural R's may be identical to or different from each other, each independently selected from the group consisting of hydrogen and a C₁₋₂₀ linear or branched alkyl (preferably each independently selected from the group consisting of hydrogen and a C₁₋₄ linear or branched alkyl),

   -S-L(̵A)ₘ (II)

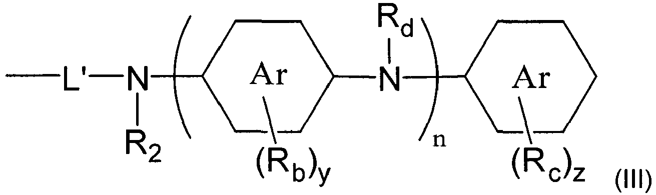

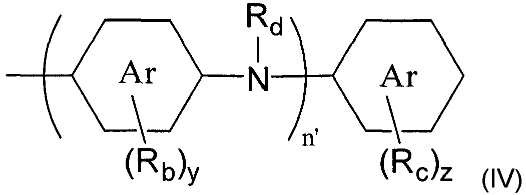

   In each Formula, the group L is selected from the group consisting of a m+1 valent C₁₋₂₀ linear or branched alkyl optionally substituted by one or more substituent selected from the group consisting of oxo, a C₁₋₂₀ hydrocarbyl (preferably a C₁₋₂₀ linear or branched alkyl, a C₆₋₂₀ aryl or a combination group thereof) and a C₃₋₂₀ linear or branched hetero-alkyl and a m+1 valent C₃₋₂₀ linear or branched hetero-alkyl optionally substituted by one or more substituent selected from the group consisting of oxo, a C₁₋₂₀ hydrocarbyl (preferably a C₁₋₂₀ linear or branched alkyl, a C₆₋₂₀ aryl or a combination group thereof) and a C₃₋₂₀ linear or branched hetero-alkyl; the group L' is a group represented by 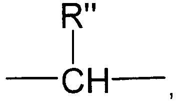 wherein the group R" is selected from the group consisting of hydrogen, a C₁₋₂₀ hydrocarbyl (preferably a C₁₋₂₀ linear or branched alkyl) and a C₃₋₂₀ linear or branched hetero-alkyl, preferably selected from the group consisting of hydrogen, a C₁₋₁₀ linear or branched alkyl and a C₃₋₁₀ linear or branched hetero-alkyl; the plural As may be identical to or different from each other, each independently selected from the group consisting of 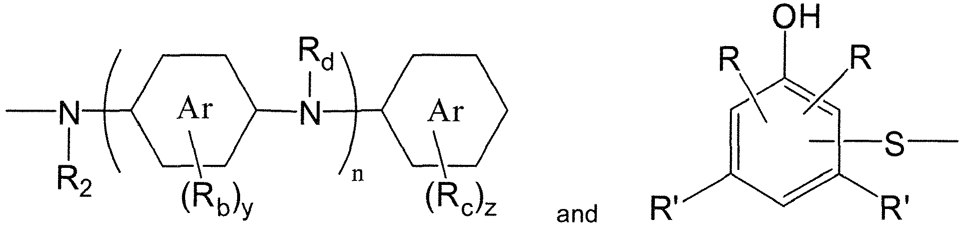 (wherein, the plural Rs may be identical to or different from each other, each independently selected from the group consisting of hydrogen, a C₁₋₃₀₀ linear or branched alkyl (preferably a C₁₋₁₀ linear or branched alkyl or a polyolefin group having a number-average molecular weight (Mn) of from 300 to 3000), a group represented by Formula (II) and a group represented by Formula (III) (preferably each independently selected from the group consisting of hydrogen and a C₁₋₃₀₀ linear or branched alkyl); the plural R's may be identical to or different from each other, each independently selected from the group consisting of hydrogen and a C₁₋₂₀ linear or branched alkyl (preferably each independently selected from the group consisting of hydrogen and a C₁₋₄ linear or branched alkyl); two Rs and one group -S- occupy the rest three positions on the benzene ring respectively), with the proviso that at least one A is 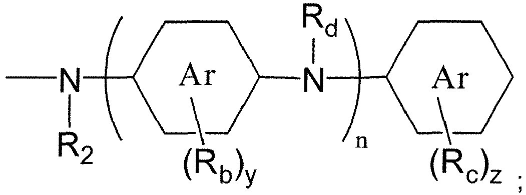 m is an integer in the range of from 1 to 4 (preferably 1); the plural R₂s may be identical to or different from each other, each independently selected from the group consisting of hydrogen, a C₁₋₂₀ linear or branched alkyl, a group represented by Formula (IV) and a group represented by Formula (V) (preferably each independently selected from the group consisting of hydrogen, a C₁₋₁₀ linear or branched alkyl and a group represented by Formula (IV)); the plural R_{b}s may be identical to or different from each other, each independently selected from the group consisting of hydrogen and a C₁₋₂₀ linear or branched alkyl (preferably each independently selected from the group consisting of hydrogen and a C₁₋₁₀ linear or branched alkyl); the plural R_{c}s may be identical to or different from each other, each independently selected from the group consisting of hydrogen, a C₁₋₂₀ linear or branched alkyl and a C₁₋₂₀ linear or branched alkyloxy (preferably each independently selected from the group consisting of hydrogen, a C₁₋₁₀ linear or branched alkyl and a C₁₋₁₀ linear or branched alkyloxy, more preferably locates at the position opposite to a N atom on the ring 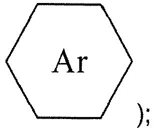 y is an integer in the range of from 0 to 3 (preferably 0 or 1); z is an integer in the range of from 0 to 3 (preferably 0 or 1); n is an integer in the range of from 1 to 8 (preferably 1 or 2); n' is an integer in the range of from 0 to 7 (preferably 0, 1 or 2), with the proviso that n'+n≤8 (preferably n'+n=1 or n'+n=2); the plural R_{d}s may be identical to or different from each other, each independently selected from the group consisting of hydrogen and a group represented by Formula (V) (preferably hydrogen); the plural rings 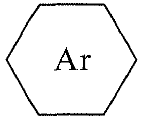 may be identical to or different from each other, each independently selected from the group consisting of benzene ring and naphthalene ring (preferably benzene ring), wherein two adjacent rings 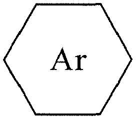 may optionally form a phenothiazine ring with the N atom bridging these two rings and an additional S atom, and/or, two adjacent rings 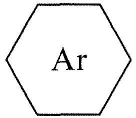 may optionally form a 9,10-dihydroacridine ring with the N atom bridging these two rings and an additional group 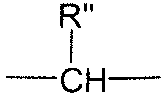 (wherein the group R" is selected from the group consisting of hydrogen, a C₁₋₂₀ hydrocarbyl (preferably a C₁₋₂₀ linear or branched alkyl) and a C₃₋₂₀ linear or branched hetero-alkyl, preferably selected from the group consisting of hydrogen and a C₁₋₁₀ linear or branched alkyl) a 9,10-dihydroacridine ring,

   -L(̵A)ₘ (V)

   In Formula (V), the plural As may be identical to or different from each other, each independently selected from the group consisting of 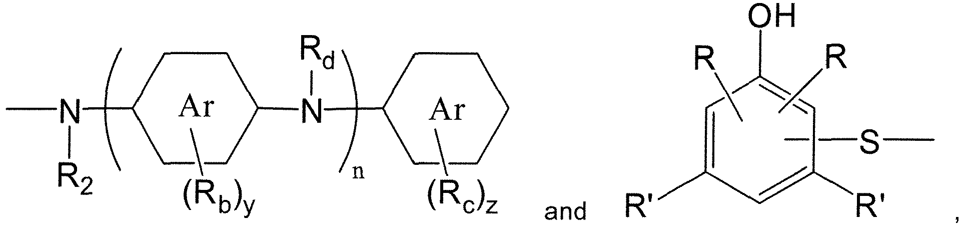 wherein the plural Rs may be identical to or different from each other, each independently selected from the group consisting of hydrogen, a C₁₋₃₀₀ linear or branched alkyl (preferably a C₁₋₁₀ linear or branched alkyl or a polyolefin group having a number-average molecular weight (Mn) of from 300 to 3000), a group represented by Formula (II) and a group represented by Formula (III) (preferably each independently selected from the group consisting of hydrogen and a C₁₋₃₀₀ linear or branched alkyl); the plural R's may be identical to or different from each other, each independently selected from the group consisting of hydrogen and a C₁₋₂₀ linear or branched alkyl (preferably each independently selected from the group consisting of hydrogen and a C₁₋₄ linear or branched alkyl); two Rs and one group -S- occupy the rest three positions on the benzene ring respectively; the group L, the group R₂, the group R_{b}, the group R_{c}, the group R_{d}, the ring 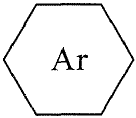 and y, n, z and m are as defined in Formula (II) respectively, wherein the linear or branched hetero-alkyl is selected from the group consisting of a group obtained by directly replacing one or more group -CH₂- locating inside of a linear or branched alkyl by a corresponding number of replacing group selected from -O-, -Sand -NR'- (R' is H or a C₁₋₄ linear or branched alkyl) and a group obtained by directly replacing one or more group -CH< locating inside of a linear or branched alkyl by a corresponding number of replacing group -N<, wherein throughout the molecular structure of the hindered phenol compound, at least one R_{d} is hydrogen.
2. The hindered phenol compound according to any one of the preceding aspects, wherein the group L is selected from the group consisting of a group 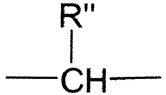 (herein m=1), a m+1 valent C₂₋₂₀ linear or branched alkyl optionally substituted by one or more substituent selected from the group consisting of a C₁₋₂₀ hydrocarbyl (preferably a C₁₋₂₀ linear or branched alkyl, a C₆₋₂₀ aryl or a combination group thereof) and a C₃₋₂₀ linear or branched hetero-alkyl, a m+1 valent C₃₋₂₀ linear or branched hetero-alkyl optionally substituted by one or more substituent selected from the group consisting of a C₁₋₂₀ hydrocarbyl (preferably a C₁₋₂₀ linear or branched alkyl, a C₆₋₂₀ aryl or a combination group thereof) and a C₃₋₂₀ linear or branched hetero-alkyl and a group 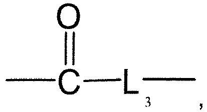 wherein the group R" is selected from the group consisting of hydrogen, a C₁₋₂₀ hydrocarbyl (preferably a C₁₋₂₀ linear or branched alkyl) and a C₃₋₂₀ linear or branched hetero-alkyl, preferably selected from the group consisting of hydrogen, a C₁₋₁₀ linear or branched alkyl and a C₃₋₁₀ linear or branched hetero-alkyl, the group L₃ is selected from the group consisting of a m+1 valent C₂₋₁₉ linear or branched alkyl optionally substituted by one or more substituent selected from the group consisting of oxo, a C₁₋₂₀ hydrocarbyl (preferably a C₁₋₂₀ linear or branched alkyl, a C₆₋₂₀ aryl or a combination group thereof) and a C₃₋₂₀ linear or branched hetero-alkyl and a m+1 valent C₃₋₁₉ linear or branched hetero-alkyl optionally substituted by one or more substituent selected from the group consisting of oxo, a C₁₋₂₀ hydrocarbyl (preferably a C₁₋₂₀ linear or branched alkyl, a C₆₋₂₀ aryl or a combination group thereof) and a C₃₋₂₀ linear or branched hetero-alkyl.
3. The hindered phenol compound according to any one of the preceding aspects, wherein the Formula (II) is selected from one of the following Formula (II_{X}), Formula (II_{XX}) and Formula (II_{XXX}), 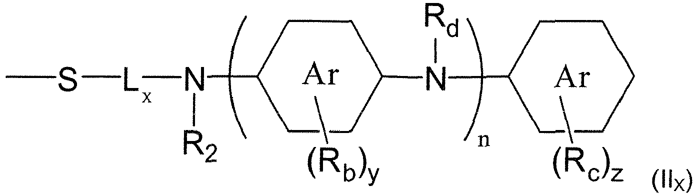
   In Formula (II_{X}), the group L_{X} is a group represented by 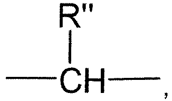 wherein the group R" is selected from the group consisting of hydrogen, a C₁₋₂₀ hydrocarbyl (preferably a C₁₋₂₀ linear or branched alkyl) and a C₃₋₂₀ linear or branched hetero-alkyl, preferably selected from the group consisting of hydrogen, a C₁₋₁₀ linear or branched alkyl and a C₃₋₁₀ linear or branched hetero-alkyl, 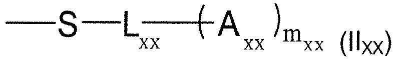
   In Formula (II_{XX}), the group L_{XX} is selected from the group consisting of a m_{XX}+1 valent C₂₋₂₀ linear or branched alkyl optionally substituted by one or more substituent selected from the group consisting of a C₁₋₂₀ hydrocarbyl (preferably a C₁₋₂₀ linear or branched alkyl, a C₆₋₂₀ aryl or a combination group thereof) and a C₃₋₂₀ linear or branched hetero-alkyl and a m_{XX}+1 valent C₃₋₂₀ linear or branched hetero-alkyl optionally substituted by one or more substituent selected from the group consisting of a C₁₋₂₀ hydrocarbyl (preferably a C₁₋₂₀ linear or branched alkyl, a C₆₋₂₀ aryl or a combination group thereof) and a C₃₋₂₀ linear or branched hetero-alkyl; the plural A_{XX}s may be identical to or different from each other, each independently selected from the group consisting of 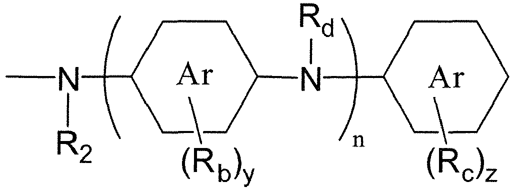 and 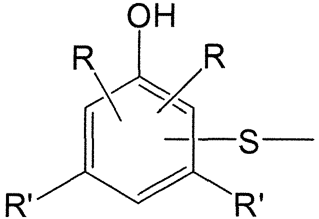 (wherein, the plural Rs may be identical to or different from each other, each independently selected from the group consisting of hydrogen, a C₁₋₃₀₀ linear or branched alkyl (preferably a C₁₋₁₀ linear or branched alkyl or a polyolefin group having a number-average molecular weight (Mn) of from 300 to 3000), a group represented by Formula (II_{XX}) and a group represented by Formula (III) (preferably each independently selected from the group consisting of hydrogen and a C₁₋₃₀₀ linear or branched alkyl); the plural R's may be identical to or different from each other, each independently selected from the group consisting of hydrogen and a C₁₋₂₀ linear or branched alkyl (preferably each independently selected from the group consisting of hydrogen and a C₁₋₄ linear or branched alkyl); two Rs and one group -S- occupy the rest three positions on the benzene ring respectively), with the proviso that at least one A_{XX} is 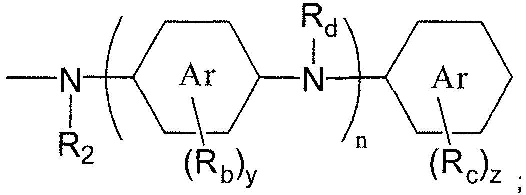 mxx is an integer in the range of from 1 to 4 (preferably 1), 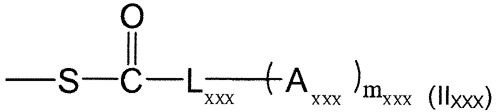
   In Formula (II_{XXX}), the group L_{XXX} is selected from the group consisting of a m_{XXX}+1 valent C₂ to C19-m_{XXX} linear or branched alkyl optionally substituted by one or more substituent selected from the group consisting of a C₁₋₂₀ hydrocarbyl (preferably a C₁₋₂₀ linear or branched alkyl, a C₆₋₂₀ aryl or a combination group thereof) and a C₃₋₂₀ linear or branched hetero-alkyl, a m_{XXX}+1 valent C₃ to C19-m_{XXX} linear or branched hetero-alkyl optionally substituted by one or more substituent selected from the group consisting of a C₁₋₂₀ hydrocarbyl (preferably a C₁₋₂₀ linear or branched alkyl, a C₆₋₂₀ aryl or a combination group thereof) and a C₃₋₂₀ linear or branched hetero-alkyl and a group represented by 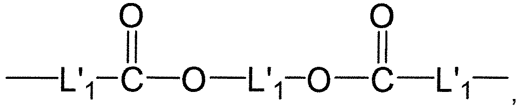 wherein the plural L'₁s may be identical to or different from each other, each independently selected from the group consisting of a C₁₋₁₀ linear or branched alkyl and a C₃₋₁₀ linear or branched hetero-alkyl, with the proviso that (1) the plural L'₁s each independently represents a 2 valent to m_{XXX}+1 valent group, so as to make the group 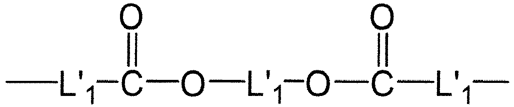 to present as, as a whole, m_{XXX}+1 valent, and (2) the total atom number of all groups L'₁ is no more than 14, wherein the plural L'₁ may each independently be optionally substituted by one or more substituent selected from the group consisting of a C₁₋₂₀ hydrocarbyl (preferably a C₁₋₂₀ linear or branched alkyl, a C₆₋₂₀ aryl or a combination group thereof) and a C₃₋₂₀ linear or branched hetero-alkyl; the plural A_{XXX}s may be identical to or different from each other, each independently selected from the group consisting of 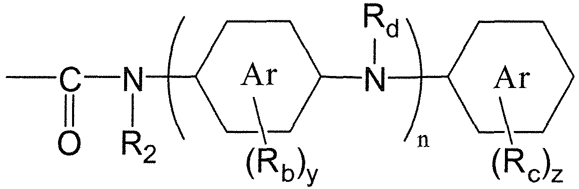 and 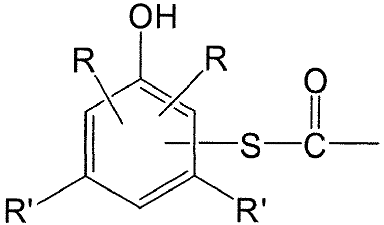 (wherein, the plural Rs may be identical to or different from each other, each independently selected from the group consisting of hydrogen, a C₁₋₃₀₀ linear or branched alkyl (preferably a C₁₋₁₀ linear or branched alkyl or a polyolefin group having a number-average molecular weight (Mn) of from 300 to 3000), a group represented by Formula (II_{XXX}) and a group represented by Formula (III) (preferably each independently selected from the group consisting of hydrogen and a C₁₋₃₀₀ linear or branched alkyl); the plural R's may be identical to or different from each other, each independently selected from the group consisting of hydrogen and a C₁₋₂₀ linear or branched alkyl (preferably each independently selected from the group consisting of hydrogen and a C₁₋₄ linear or branched alkyl); two Rs and one group 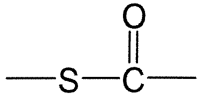 occupy the rest three positions on the benzene ring respectively), with the proviso that at least one A_{XXX} is 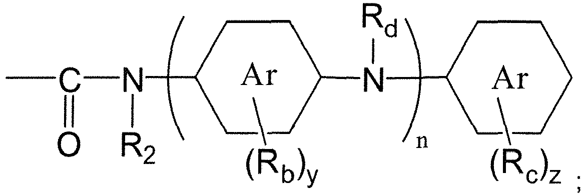 m_{XXX} is an integer in the range of from 1 to 4 (preferably 1), and/or,
   the Formula (V) is selected from one of the following Formula (V_{X}), Formula (V_{XX}) and Formula (V_{XXX}), 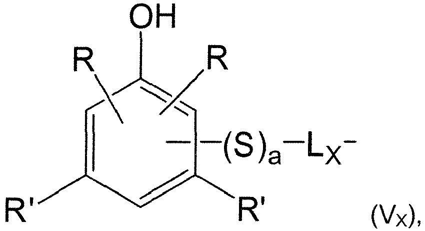
   In Formula (V_{X}), the plural Rs may be identical to or different from each other, each independently selected from the group consisting of hydrogen, a C₁₋₃₀₀ linear or branched alkyl (preferably a C₁₋₁₀ linear or branched alkyl or a polyolefin group having a number-average molecular weight (Mn) of from 300 to 3000), a group represented by Formula (II_{X}) and a group represented by Formula (III) (preferably each independently selected from the group consisting of hydrogen and a C₁₋₃₀₀ linear or branched alkyl); the plural R's may be identical to or different from each other, each independently selected from the group consisting of hydrogen and a C₁₋₂₀ linear or branched alkyl (preferably each independently selected from the group consisting of hydrogen and a C₁₋₄ linear or branched alkyl); the group L_{X} is a group represented by 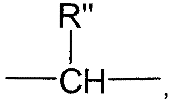 wherein the group R" is selected from the group consisting of hydrogen, a C₁₋₂₀ hydrocarbyl (preferably a C₁₋₂₀ linear or branched alkyl) and a C₃₋₂₀ linear or branched hetero-alkyl, preferably selected from the group consisting of hydrogen, a C₁₋₁₀ linear or branched alkyl and a C₃₋₁₀ linear or branched hetero-alkyl; a is 1, and two Rs and one group - (S)ₐ-L_{X}- occupy the rest three positions on the benzene ring respectively, 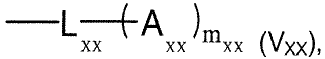
   In Formula (V_{XX}), the plural A_{XX}s may be identical to or different from each other, each independently selected from the group consisting of 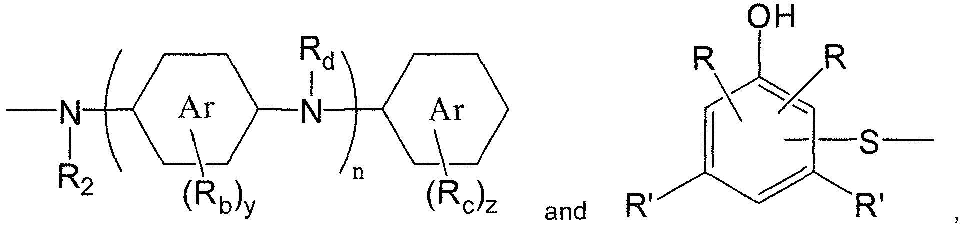 wherein the plural Rs may be identical to or different from each other, each independently selected from the group consisting of hydrogen, a C₁₋₃₀₀ linear or branched alkyl (preferably a C₁₋₁₀ linear or branched alkyl or a polyolefin group having a number-average molecular weight (Mn) of from 300 to 3000), a group represented by Formula (II_{XX}) and a group represented by Formula (III) (preferably each independently selected from the group consisting of hydrogen and a C₁₋₃₀₀ linear or branched alkyl); the plural R's may be identical to or different from each other, each independently selected from the group consisting of hydrogen and a C₁₋₂₀ linear or branched alkyl (preferably each independently selected from the group consisting of hydrogen and a C₁₋₄ linear or branched alkyl); two Rs and one group -S- occupy the rest three positions on the benzene ring respectively; the group L_{XX} and m_{XX} are as defined in Formula (II_{XX}) respectively, 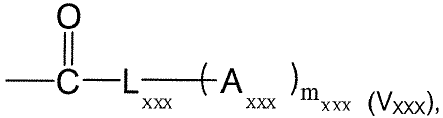
   In Formula (V_{XXX}), the plural A_{XXX}s may be identical to or different from each other, each independently selected from the group consisting of 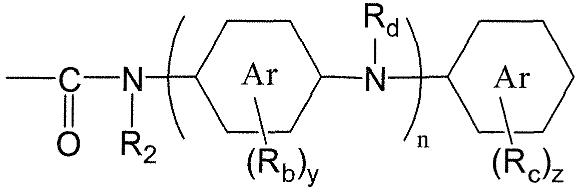 and 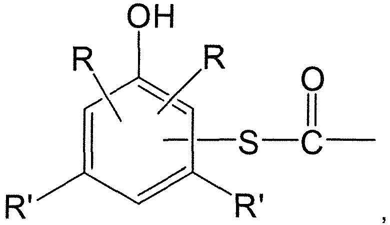 wherein the plural Rs may be identical to or different from each other, each independently selected from the group consisting of hydrogen, a C₁₋₃₀₀ linear or branched alkyl (preferably a C₁₋₁₀ linear or branched alkyl or a polyolefin group having a number-average molecular weight (Mn) of from 300 to 3000), a group represented by Formula (II_{XXX}) and a group represented by Formula (III) (preferably each independently selected from the group consisting of hydrogen and a C₁₋₃₀₀ linear or branched alkyl); the plural R's may be identical to or different from each other, each independently selected from the group consisting of hydrogen and a C₁₋₂₀ linear or branched alkyl (preferably each independently selected from the group consisting of hydrogen and a C₁₋₄ linear or branched alkyl); two Rs and one group 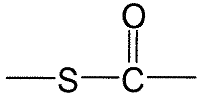 occupy the rest three positions on the benzene ring respectively; the group L_{XXX} and m_{XXX} are as defined in Formula (II_{XXX}) respectively, other groups and numerical values are as defined in aspect 1.
4. The hindered phenol compound according to any one of the preceding aspects, selected from the group consisting of the following specific compounds or their mixture at any ratio therebetween : 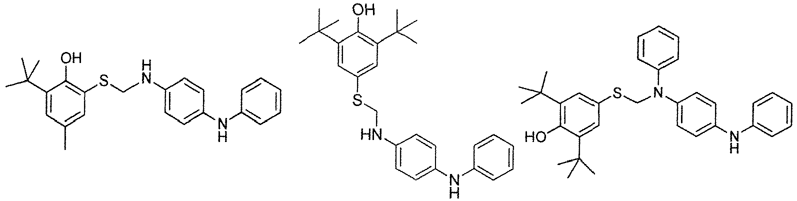 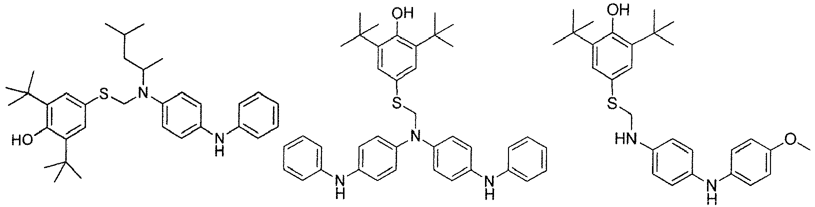 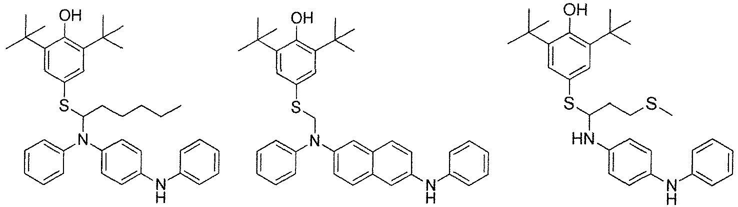 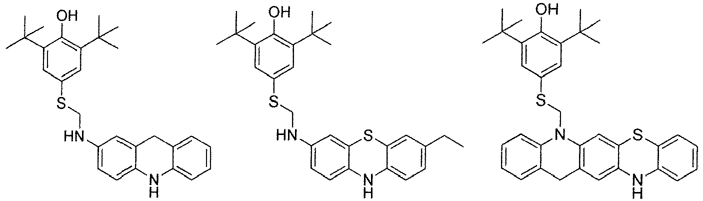 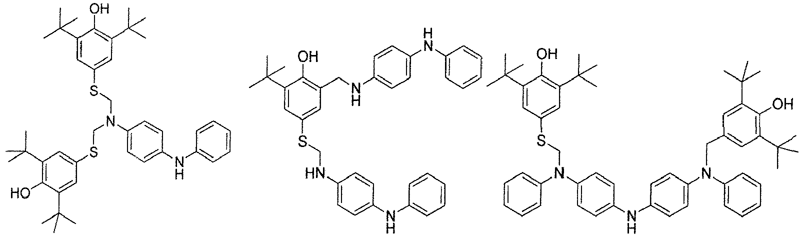 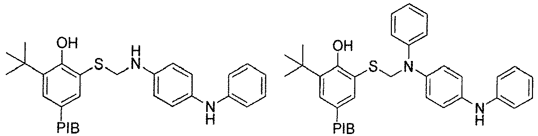 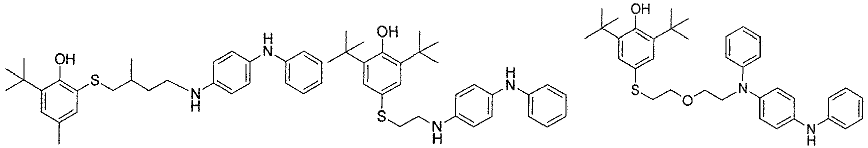 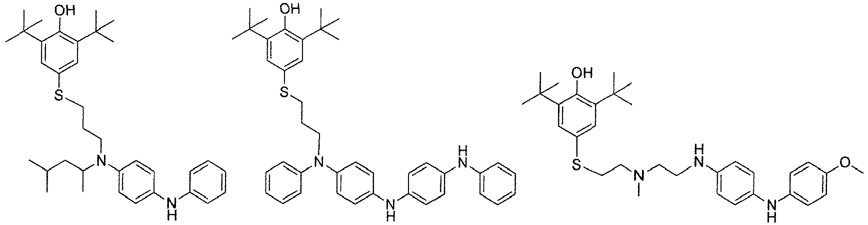 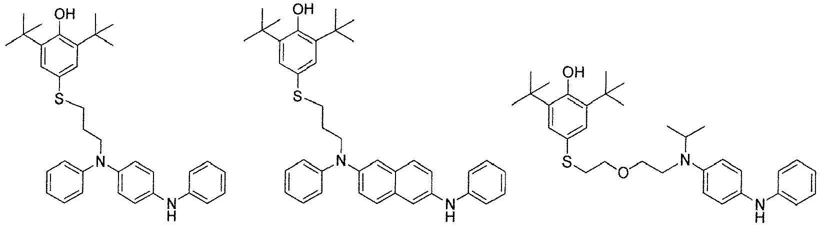 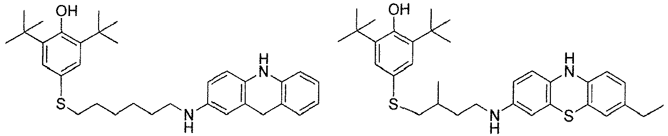 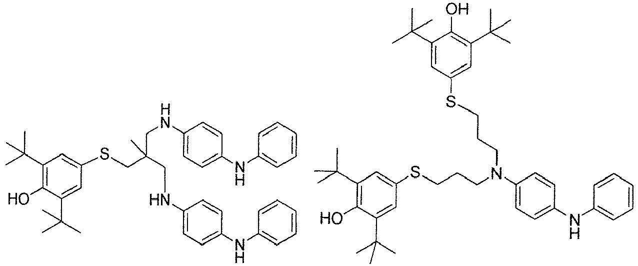 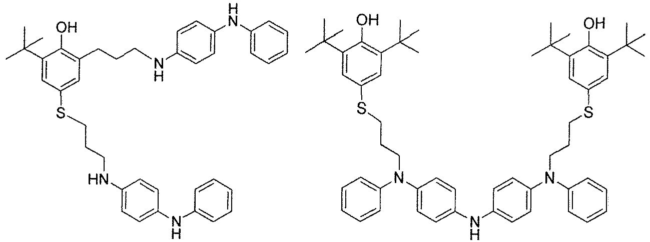 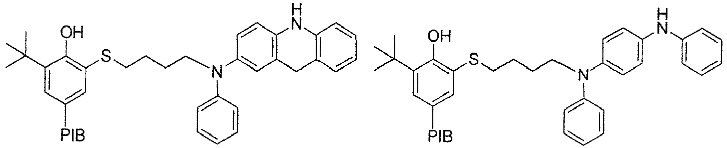 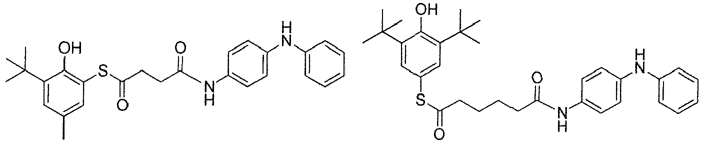 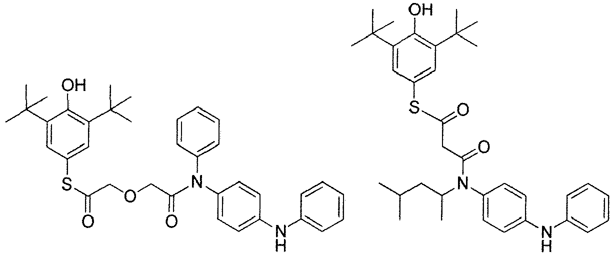 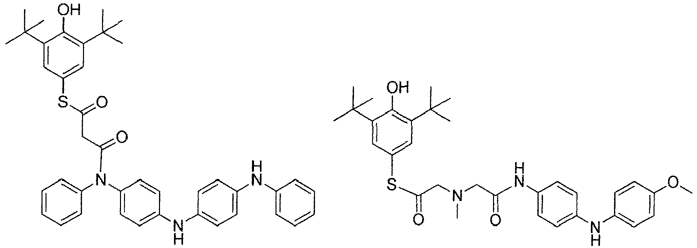 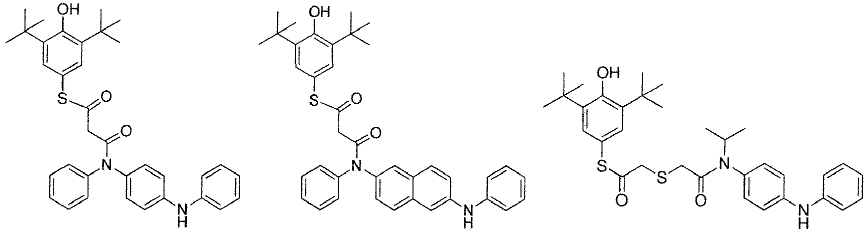 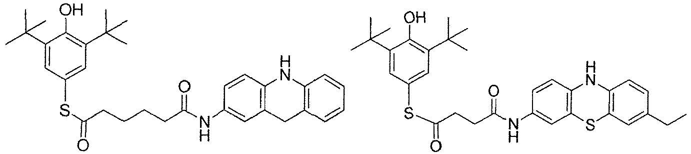 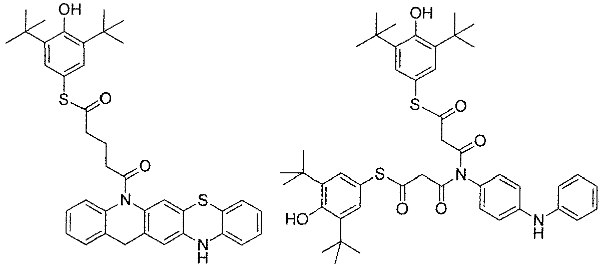 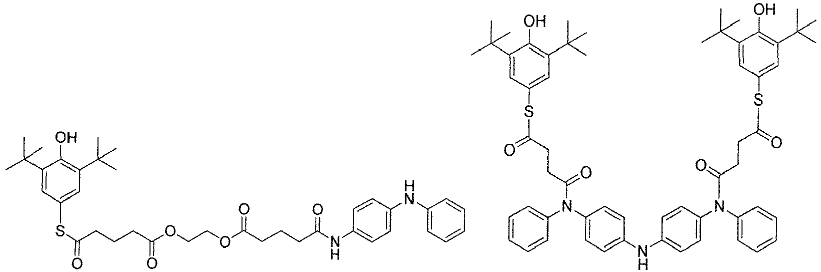 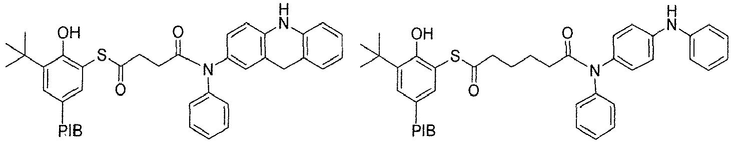 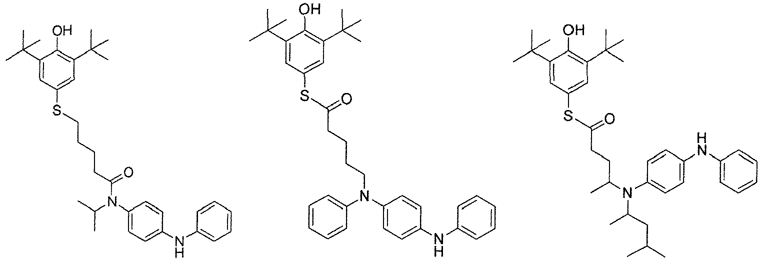 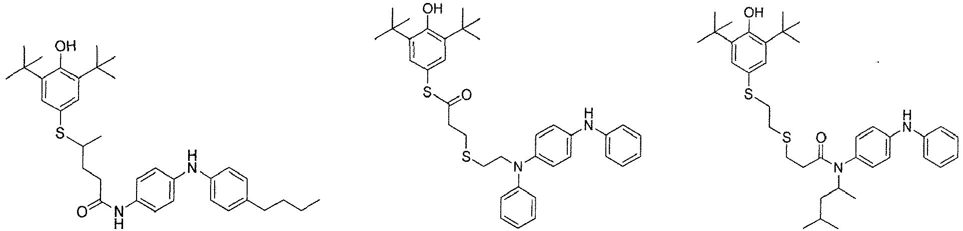 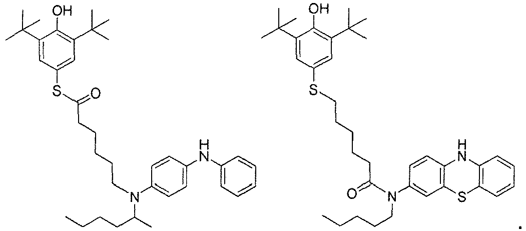
5. A process for producing a hindered phenol compound, including a first step of having a phenol compound represented by Formula (X) to react with an amine compound represented by Formula (Y) in the presence of at least one bridging compound selected from the group consisting of a compound represented by Formula (A) and formaldehyde, optionally further including an additional step of having the resultant of the first step to react with a sulfuring agent (preferably sulfur) and/or to react with an aldehyde compound represented by Formula (Z) (preferably formaldehyde), 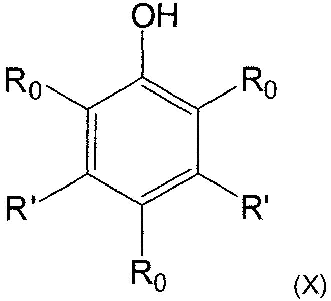
   In Formula (X), the plural R₀s may be identical to or different from each other, each independently selected from the group consisting of hydrogen, -SH and a C₁₋₃₀₀ linear or branched alkyl (preferably a C₁₋₁₀ linear or branched alkyl or a polyolefin group having a number-average molecular weight (Mn) of from 300 to 3000), with the proviso that at least one R₀ is a group represented by -SH; the plural R's may be identical to or different from each other, each independently selected from the group consisting of hydrogen and a C₁₋₂₀ linear or branched alkyl (preferably each independently selected from the group consisting of hydrogen and a C₁₋₄ linear or branched alkyl), 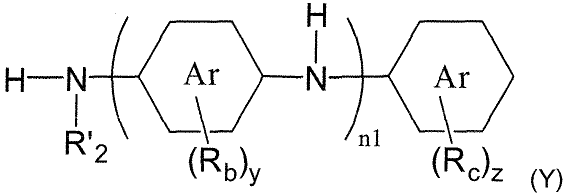
   In Formula (Y), the group R'₂ is selected from the group consisting of hydrogen, a C₁₋₂₀ linear or branched alkyl and a group represented by 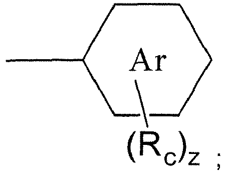 the plural R_{b}s may be identical to or different from each other, each independently selected from the group consisting of hydrogen and a C₁₋₂₀ linear or branched alkyl (preferably each independently selected from the group consisting of hydrogen and a C₁₋₁₀ linear or branched alkyl); the plural R_{c}s may be identical to or different from each other, each independently selected from the group consisting of hydrogen, a C₁₋₂₀ linear or branched alkyl and a C₁₋₂₀ linear or branched alkyloxy (preferably each independently selected from the group consisting of hydrogen, a C₁₋₁₀ linear or branched alkyl and a C₁₋₁₀ linear or branched alkyloxy, more preferably locates at the position opposite to a N atom on the ring 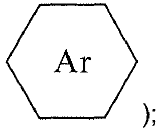 y is an integer in the range of from 0 to 3 (preferably 0 or 1); z is an integer in the range of from 0 to 3 (preferably 0 or 1); n1 is an integer in the range of from 1 to 8 (preferably 1 or 2); the plural rings 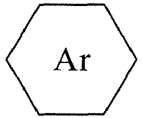 may be identical to or different from each other, each independently selected from the group consisting of benzene ring and naphthalene ring (preferably benzene ring),

   R_{f}(̵Fun)ₘ₁ (A)

   In Formula (A), the group R_{f} is selected from the group consisting of a m1 valent C₁₋₂₀ hydrocarbyl (preferably C₁₋₂₀ linear or branched alkyl) optionally substituted by one or more substituent selected from the group consisting of oxo, a C₁₋₂₀ hydrocarbyl (preferably a C₁₋₂₀ linear or branched alkyl, a C₆₋₂₀ aryl or a combination group thereof) and a C₃₋₂₀ linear or branched hetero-alkyl and a m1 valent C₃₋₂₀ linear or branched hetero-alkyl optionally substituted by one or more substituent selected from the group consisting of oxo, a C₁₋₂₀ hydrocarbyl (preferably a C₁₋₂₀ linear or branched alkyl, a C₆₋₂₀ aryl or a combination group thereof) and a C₃₋₂₀ linear or branched hetero-alkyl; m1 is an integer in the range of from 2 to 5 (preferably 2), the plural Funs may be identical to or different from each other, each independently selected from the group consisting of a halogen atom, a carboxylic acid residue group, an anhydride residue group and an aldehyde residue group, while in case that m1 is 1, the group Fun is an aldehyde residue group, wherein by "carboxylic acid residue group " it refers to a group obtained by removing one carbonyl (i.e. 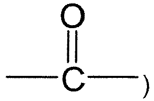 from carboxyl (i.e. -COOH), with the proviso that it is necessary for the carbon atom directly bonding to the group to take the form of carbonyl (i.e. 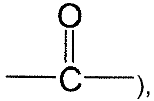 by "anhydride residue group " it refers to a group obtained by removing two carbonyls (i.e. 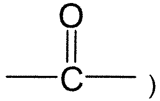 from anhydride (i.e. 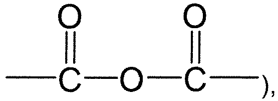 with the proviso that it is necessary for the two carbon atoms directly bonding to the group to take the form of carbonyl (i.e. 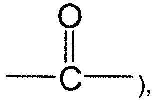 by "aldehyde residue group " it refers to a group obtained by removing one carbonyl (i.e. 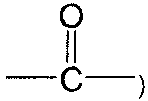 from formyl (i.e. 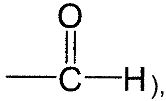 with the proviso that it is necessary for the carbon atom directly bonding to the group to take the form of carbonyl (i.e. 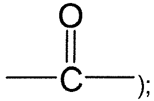 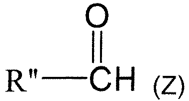
   In Formula (Z), the group R" is selected from the group consisting of hydrogen, a C₁₋₂₀ hydrocarbyl (preferably a C₁₋₂₀ linear or branched alkyl) and a C₃₋₂₀ linear or branched hetero-alkyl, preferably selected from the group consisting of hydrogen, a C₁₋₁₀ linear or branched alkyl and a C₃₋₁₀ linear or branched hetero-alkyl,
   wherein, the linear or branched hetero-alkyl is selected from the group consisting of a group obtained by directly replacing one or more group -CH₂- locating inside of a linear or branched alkyl by a corresponding number of replacing group selected from -O-, -S- or -NR'- (R' is H or a C₁₋₄ linear or branched alkyl) and a group obtained by directly replacing one or more group -CH< locating inside of a linear or branched alkyl by a corresponding number of replacing group -N<.
6. The process according to any one of the preceding aspects, wherein the bridging compound is one or more selected from the group consisting of an aldehyde compound represented by Formula (A_{Y}), a polyhalo-compound represented by Formula (A_{YY}) and a polybasic carboxylic acid represented by Formula (A_{YYY}) or derivatives thereof (selected from the group consisting of an anhydride of the polybasic carboxylic acid and an acyl halide of the polybasic carboxylic acid), 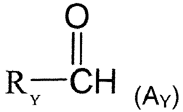
   In Formula (A_{Y}), the group R_{Y} is selected from the group consisting of hydrogen, a C₁₋₂₀ hydrocarbyl (preferably a C₁₋₂₀ linear or branched alkyl) and a C₃₋₂₀ linear or branched hetero-alkyl, preferably selected from the group consisting of hydrogen, a C₁₋₁₀ linear or branched alkyl and a C₃₋₁₀ linear or branched hetero-alkyl,

   Rₕₐₗₒ(̵Halo)_{m_{YY}} (A_{YY})

   In Formula (A_{YY}), the group Rₕₐₗₒ is selected from the group consisting of a m_{YY} valent C₂₋₂₀ linear or branched alkyl optionally substituted by one or more substituent selected from the group consisting of a C₁₋₂₀ hydrocarbyl (preferably a C₁₋₂₀ linear or branched alkyl, a C₆₋₂₀ aryl or a combination group thereof) and a C₃₋₂₀ linear or branched hetero-alkyl and a m_{YY} valent C₃₋₂₀ linear or branched hetero-alkyl optionally substituted by one or more substituent selected from the group consisting of a C₁₋₂₀ hydrocarbyl (preferably a C₁₋₂₀ linear or branched alkyl, a C₆₋₂₀ aryl or a combination group thereof) and a C₃₋₂₀ linear or branched hetero-alkyl, the group Halo is a halogen atom, m_{YY} is an integer in the range of from 2 to 5 (preferably 2),

   R_{L}(̵COOH)_{m_{YYY}} (A_{YYY})

   In Formula (A_{YYY}), the group R_{L} is selected from the group consisting of a m_{YYY} valent C₂ to C_{20-m_{YYY}} linear or branched alkyl optionally substituted by one or more substituent selected from the group consisting of a C₁₋₂₀ hydrocarbyl (preferably a C₁₋₂₀ linear or branched alkyl, a C₆₋₂₀ aryl or a combination group thereof) and a C₃₋₂₀ linear or branched hetero-alkyl, a m_{YYY} valent C₃ to C_{20-m_{YYY}} linear or branched hetero-alkyl optionally substituted by one or more substituent selected from the group consisting of a C₁₋₂₀ hydrocarbyl (preferably a C₁₋₂₀ linear or branched alkyl, a C₆₋₂₀ aryl or a combination group thereof) and a C₃₋₂₀ linear or branched hetero-alkyl and a group represented by 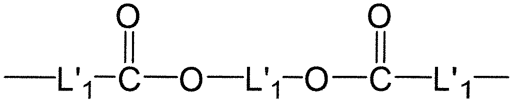 (wherein, the plural L'₁s may be identical to or different from each other, each independently selected from the group consisting of a C₁₋₁₀ linear or branched alkyl and a C₃₋₁₀ linear or branched hetero-alkyl, with the proviso that (1) the plural L'₁s each independently represents a 2 valent to m_{YYY} valent group, so as to make the group 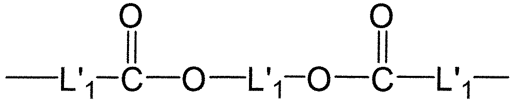 to present as, as a whole, m_{YYY} valent, and (2) the total atom number of all groups L'₁ is no more than 14, wherein the plural L'₁ may each independently be optionally substituted by one or more substituent selected from the group consisting of a C₁₋₂₀ hydrocarbyl (preferably a C₁₋₂₀ linear or branched alkyl, a C₆₋₂₀ aryl or a combination group thereof) and a C₃₋₂₀ linear or branched hetero-alkyl), m_{YYY} is an integer in the range of from 2 to 5 (preferably 2).
7. The process according to any one of the preceding aspects, wherein in the first step, molar ratio of the phenol compound represented by Formula (X) to the amine compound represented by Formula (Y) is 1 : 0.1-10, preferably 1 : 0.5-5.0, more preferably 1 : 0.8-2.0, molar ratio of the phenol compound represented by Formula (X) to the bridging compound is 1 : 0.1-10, preferably 1 : 0.2-5.0, more preferably 1 : 0.3-3.0, in the additional step, molar ratio of the amine compound represented by Formula (Y) to the sulfuring agent is 1 : 1-10, preferably 1 : 1.2-6.0, more preferably 1 : 1.5-3.0, molar ratio of the amine compound represented by Formula (Y) to the aldehyde compound represented by Formula (Z) is 1 : 0.1-10, preferably 1 : 0.5-5.0, more preferably 1 : 0.8-2.0.
8. Use of the hindered phenol compound according to any one of the preceding aspects or the hindered phenol compound produced in line with the process according to any one of the preceding aspects as an antioxidant.
9. A lubricant oil composition, comprising a lubricant base oil and a hindered phenol compound according to any one of the preceding aspects or the hindered phenol compound produced in line with the process according to any one of the preceding aspects as an antioxidant, wherein the antioxidant represents 0.001-30wt%, preferably 0.1-10wt%, of the total weight of the lubricant oil composition.
10. The lubricant oil composition according to any one of the preceding aspects, produced by mixing the hindered phenol compound with the lubricant base oil.

### Technical effects

The hindered phenol compound according to this invention, contains no phosphorus and metals, generates little ash, and is thus identified as an environment friendly antioxidant.

The hindered phenol compound according to this invention, as compared with a prior art compound, exhibits significantly improved oxidation resistance at elevated temperatures (thermal stability), and is thus capable of effectively improving the oxidation stability (at elevated temperatures) of e.g. a lubricant oil.

The hindered phenol compound according to this invention, in a preferred embodiment, in addition to the excellent oxidation resistance at elevated temperatures, further exhibits excellent anticorrosion performance. This performance is hardly found with a prior art compound.

The hindered phenol compound according to this invention, in a preferred embodiment, in addition to the excellent oxidation resistance at elevated temperatures, further exhibits excellent cleansing performance (i.e. deposite formation inhibition performance). This performance is hardly found with a prior art compound.

The hindered phenol compound according to this invention, in a preferred embodiment, in addition to the excellent oxidation resistance at elevated temperatures, further exhibits excellent performance of inhibiting inrease in viscosity. This performance is hardly found with a prior art compound.

The hindered phenol compound according to this invention, in a preferred embodiment, in addition to the excellent oxidation resistance at elevated temperatures, further exhibits excellent performance of inhibiting inrease in acid value. This performance is hardly found with a prior art compound.

### Specific Mode to Carry Out This Invention

This invention will be described in details hereinafter with reference to the following specific embodiments.

In the context of this specification, the term "halo" or the like refers to fluoro, chloro, bromo or iodo.

In the context of this specification, the term " hydrocarbyl " should be interpreted as commenly known in this field, and intends to include a linear or branched alkyl, a linear or branched alkenyl, a linear or branched alkynyl, a cycloalkyl, a cycloalkenyl, a cycloalkynyl, an aryl or a combination group thereof, preferably a linear or branched alkyl, a linear or branched alkenyl, an aryl or a combination group thereof. As the hydrocarbyl, further exemplified could be a C₁₋₂₀ hydrocarbyl, including a C₁₋₂₀ linear or branched alkyl, a C₂₋₂₀ linear or branched alkenyl, a C₂₋₂₀ linear or branched alkynyl, a C₃₋₂₀ cycloalkyl, a C₃₋₂₀ cycloalkenyl, a C₃₋₂₀ cycloalkynyl, a C₆₋₂₀ aryl or a combination group thereof, preferably a C₁₋₂₀ linear or branched alkyl, a C₆₋₂₀ aryl or a combination group thereof. As the combination group, exemplified could be a group obtained by directly bonding one or more C₁₋₂₀ linear or branched alkyl (preferably one or more C₁₋₁₀ linear or branched alkyl) with one or more C₆₋₂₀ aryl (preferably one or more phenyl or naphthyl). As the combination group, for example, further exemplified could be (one or more) C₁₋₁₀ linear or branched alkyl phenyl, phenyl C₁₋₁₀ linear or branched alkyl or (one or more) C₁₋₁₀ linear or branched alkyl phenyl C₁₋₁₀ linear or branched alkyl etc., more preferably C₁₋₁₀ linear or branched alkyl phenyl (for example, tert-butyl phenyl), phenyl C₁₋₁₀ linear or branched alkyl (for example, benzyl) or C₁₋₁₀ linear or branched alkyl phenyl C₁₋₁₀ linear or branched alkyl (for example, tert-butyl benzyl).

In the context of this specification, by " linear or branched hetero-alkyl ", it refers to a group obtained by directly replacing one or more (for example, from 1 to 4, from 1 to 3, from 1 to 2, or 1) group -CH₂- locating inside (not including that at the terminal of the main chain or any side chain in the molecular structure) of a linear or branched alkyl by a corresponding number of replacing group selected from -O-, -S- or -NR'- (R' is H or a C₁₋₄ linear or branched alkyl), and a group obtained by directly replacing one or more (for example, from 1 to 3, from 1 to 2, or 1) group -CH< locating inside (not including that at the terminal of the main chain or any side chain in the molecular structure) of a linear or branched alkyl by a corresponding number of replacing group -N<. As the replacing group, preferably -O- or -S-, more preferably -S-. It is obvious that, from the standpoint of structure stability, when plural exist, these replacing groups do not directly bond to each other. Further, the carbon atom number of the linear or branched alkyl is reduced accordingly due to the replacement of the group -CH₂- or -CH< by the replacing group, however, to simplify the description, the carbon atom number of the linear or branched alkyl before the replacement is still used to refer to the carbon atom number of the linear or branched hetero-alkyl (obtained by the replacement). As the linear or branched hetero-alkyl, if specifically exemplified, a C₄ linear alkyl, for example, 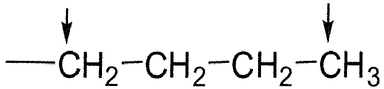 (In this figure, the groups indicated by the arrow marks do not locate inside of the linear alkyl, but rather at the terminal of the main chain) if directly replaced by one replacing group -O-, CH₂-O-CH₂-CH₃ or CH₂-CH₂-O-CH₃ will be obtained, called as a C₄ linear hetero-alkyl. Or, a C₄ branched alkyl, for example, 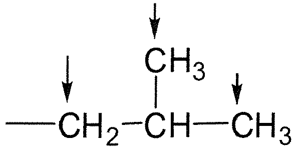 (In this figure, the groups indicated by the arrow marks do not locate inside of the branched alkyl, but rather at the terminal of the main chain and that of the side chain) if directly replaced by one replacing group -N<, 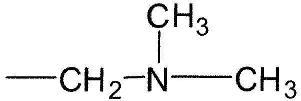 will be obtained, called as a C₄ branched hetero-alkyl. According to this invention, as the linear or branched hetero-alkyl, exemplified could be a C₃₋₂₀ linear or branched hetero-alkyl, preferably a C₃₋₁₀ linear or branched hetero-alkyl or a C₃₋₆ linear or branched hetero-alkyl. The term " linear or branched hetero-alkane " or the like can be interpreted similarly.

In the context of this specification, if a group is defined or described in the form of "*numerical value* + valent + *group*" or the like, it refers to a group obtained by removing a number of hydrogen atom (wherein the number of the hydrogen atom corresponds to the numerical value) from the corresponding basic structure (for example, a chain, a ring or a combination thereof) of the group, preferably refers to a group obtained by removing a number of hydrogen atom (wherein the number of the hydrogen atom corresponds to the numerical value) from a carbon atom (preferably from a saturated carbon atom and/or if the numerical value is two or more, from different carbon atoms) in the basic structure. For example, "3 valent linear or branched alkyl " refers to a group obtained by removing 3 (three) hydrogen atoms from a linear or branched alkane (i.e. the corresponding basic structure (chain) of the linear or branched alkyl), while "2 valent linear or branched hetero-alkyl " refers to a group obtained by removing 2 (two) hydrogen atoms from a linear or branched hetero-alkane (preferably from a carbon atom of the hetero-alkane, or, from two different carbon atoms in the hetero-alkane). Unless otherwise specified, percents, parts or ratios or the like mentioned in this specification are all on a weight basis.

In the context of this specification, unless otherwise specified, the number-average molecular weight (Mn) is determined by gel permeation chromatography (GPC).

In the context of this specification, unless otherwise specified, the gel permeation chromatography is performed on Waters 2695 Gel Permeation Chromatograph (from Waters, USA), with a mobile phase of tetrafuran, a flow rate of 1 mL/min, a column temperature of 35 degrees Celsius, an elution time of 40min, and a mass fraction of the sample of from 0.16% to 0.20%.

According to this invention, concerned is a hindered phenol compound represented by Formula (I).

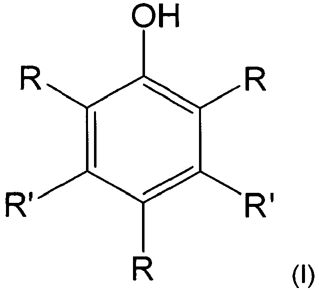

According to this invention, in Formula (I), when plural exist, the plural Rs may be identical to or different from each other, each independently selected from the group consisting of a C₁₋₃₀₀ linear or branched alkyl, a group represented by Formula (II) and a group represented by Formula (III), with the proviso that at least one R is a group represented by Formula (II).

According to this invention, in Formula (I), as the C₁₋₃₀₀ linear or branched alkyl, exemplified could be a C₁₋₂₀ linear or branched alkyl (preferably a C₁₋₁₀ linear or branched alkyl, more preferably a C₁₋₄ linear or branched alkyl) or a polyolefin group. As the polyolefin group, further exemplified could be a polyolefin group having a number-average molecular weight (Mn) of from 300 to 3000. Herein, the number-average molecular weight (Mn) of the polyolefin group is preferably in the range of from 500 to 2000, more preferably in the range of from 500 to 1500. According to this invention, the polyolefin group has a (substantially) saturated structure (in the form of a linear long-chain alkyl), however, depending on the nature of the starting polyolefin or the process for producing the starting polyolefin, there may exist some (only a small amount of) ethylenic double bond (for example, remained from or introduced during production of the polyolefin) along the molecular chain of the polyolefin group, which will not adversely interfere with the effects anticipated by this invention. For this reason, this invention does not intend to specify at what level this amount of ethylenic double bond could be, but rather, still identifies a polyolefin group of this kind as an "alkyl".

According to an embodiment of this invention, in Formula (I), there exist plural Rs, wherein one of the Rs is a group represented by Formula (II), one of the other two Rs is the polyolefin group, the rest one is selected from the group consisting of hydrogen, a C₁₋₂₀ linear or branched alkyl (preferably a C₁₋₁₀ linear or branched alkyl) or a group represented by Formula (III).

According to an embodiment of this invention, in Formula (I), when the group R is the polyolefin group, the polyolefin group preferably locates at the position opposite to the phenol hydroxyl in Formula (I)

According to this invention, in Formula (I), when plural exist, the plural R's may be identical to or different from each other, each independently selected from the group consisting of a C₁₋₂₀ linear or branched alkyl.

According to an embodiment of this invention, in Formula (I), when plural exist, the plural R's may be identical to or different from each other, each independently selected from the group consisting of a C₁₋₄ linear or branched alkyl.

-S-L(̵A)ₘ (II)

According to this invention, in Formula (II), the group L is selected from the group consisting of an optionally substituted m+1 valent C₁₋₂₀ linear or branched alkyl and an optionally substituted m+1 valent C₃₋₂₀ linear or branched hetero-alkyl, preferably selected from the group consisting of an optionally substituted m+1 valent C₁₋₁₀ linear or branched alkyl and an optionally substituted m+1 valent C₃₋₁₀ linear or branched hetero-alkyl, more preferably selected from the group consisting of an optionally substituted m+1 valent C₁₋₆ linear or branched alkyl and an optionally substituted m+1 valent C₃₋₆ linear or branched hetero-alkyl.

According to an embodiment of this invention, in Formula (II), m=1, the group L is a group represented by 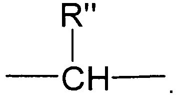 In the definition of the group L, the group R" is selected from the group consisting of hydrogen, a C₁₋₂₀ hydrocarbyl (preferably a C₁₋₂₀ linear or branched alkyl) and a C₃₋₂₀ linear or branched hetero-alkyl, preferably selected from the group consisting of hydrogen, a C₁₋₁₀ linear or branched alkyl and a C₃₋₁₀ linear or branched hetero-alkyl, preferably selected from the group consisting of hydrogen and a C₁₋₁₀ linear or branched alkyl.

According to an embodiment of this invention, in Formula (II), the group L is selected from the group consisting of an optionally substituted m+1 valent C₂₋₂₀ linear or branched alkyl and an optionally substituted m+1 valent C₃₋₂₀ linear or branched hetero-alkyl, preferably selected from the group consisting of an optionally substituted m+1 valent C₂₋₁₀ linear or branched alkyl and an optionally substituted m+1 valent C₃₋₁₀ linear or branched hetero-alkyl, more preferably selected from the group consisting of an optionally substituted m+1 valent C₂₋₆ linear or branched alkyl and an optionally substituted m+1 valent C₃₋₆ linear or branched hetero-alkyl.

According to an embodiment of this invention, in Formula (II), the group L is a group represented by 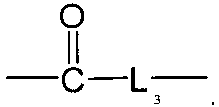 In the definition of the group L, the group L₃ is selected from the group consisting of an optionally substituted m+1 valent C₂₋₁₉ linear or branched alkyl and an optionally substituted m+1 valent C₃₋₁₉ linear or branched hetero-alkyl, preferably selected from the group consisting of an optionally substituted m+1 valent C₂₋₉ linear or branched alkyl and an optionally substituted m+1 valent C₃₋₉ linear or branched hetero-alkyl, more preferably selected from the group consisting of an optionally substituted m+1 valent C₂₋₆ linear or branched alkyl and an optionally substituted m+1 valent C₃₋₆ linear or branched hetero-alkyl. Preferably, the group 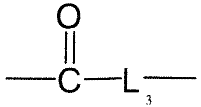 directly bonds to the S atom through its carbonyl. According to this invention, in Formula (II), in the definitions of the group L and the group L₃, by "optionally substituted", it refers to optionally substituted by one or more (for example, from 1 to 5, from 1 to 4, from 1 to 3, from 1 to 2, or 1) substituent selected from the group consisting of oxo (i.e. 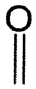 ), a C₁₋₂₀ hydrocarbyl and a C₃₋₂₀ linear or branched hetero-alkyl. As the substituent, preferably oxo, a C₁₋₂₀ linear or branched alkyl, a C₆₋₂₀ aryl or a combination group thereof. As the C₁₋₂₀ linear or branched alkyl, preferably a C₁₋₁₀ linear or branched alkyl, more preferably a C₁₋₆ linear or branched alkyl, for example, methyl or ethyl.

According to this invention, in Formula (II), in the definitions of the group L and the group L₃, when oxo exists as the substituent, preferably at least one oxo exists on the carbon atom directly bonding to the S atom or to the group A as the substituent, or in the group L or the group L₃, at least one or m+1 of the carbon atoms directly bonding to the S atom and to the group A has oxo as the substituent, so as to render the carbon atom present in the form of carbonyl (i.e. 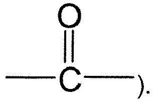 If specifically exemplified, the group L is assumed to be CH₂-CH₂-CH₂- (m is 1) substituted by two oxo as the substituent, if the two oxo exist on the carbon atoms directly bonding to the S atom and to the group A, the group L would be in the form of

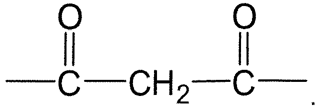

According to this invention, in Formula (II), m is an integer in the range of from 1 to 4, preferably 1.

According to this invention, in Formula (II), the plural As may be identical to or different from each other, each independently selected from the group consisting of a group represented by Formula (II-1) and a group represented by Formula (II-2), with the proviso that at least one A is a group represented by Formula (II-1).

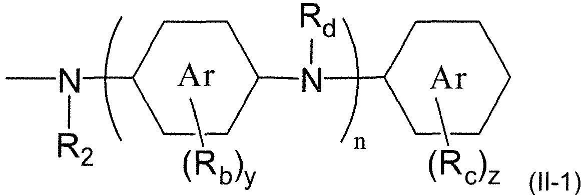

According to this invention, in Formula (II-1), the group R₂ is selected from the group consisting of hydrogen, a C₁₋₂₀ linear or branched alkyl, a group represented by Formula (IV) and a group represented by Formula (V).

According to an embodiment of this invention, in Formula (II-1), the group R₂ is selected from the group consisting of hydrogen, a C₁₋₁₀ linear or branched alkyl and a group represented by Formula (IV).

According to this invention, in Formula (II-1), when plural exist, the plural R_{b}s may be identical to or different from each other, each independently selected from the group consisting of hydrogen and a C₁₋₂₀ linear or branched alkyl, preferably each independently selected from the group consisting of hydrogen and a C₁₋₁₀ linear or branched alkyl.

According to an embodiment of this invention, in Formula (II-1), when plural exist, the plural R_{b}s may be identical to or different from each other, each independently selected from the group consisting of hydrogen and a C₁₋₆ linear or branched alkyl, preferably hydrogen.

According to this invention, in Formula (II-1), when plural exist, the plural R_{c}s may be identical to or different from each other, each independently selected from the group consisting of hydrogen, a C₁₋₂₀ linear or branched alkyl and a C₁₋₂₀ linear or branched alkyloxy.

According to an embodiment of this invention, in Formula (II-1), when plural exist, the plural R_{c}s may be identical to or different from each other, each independently selected from the group consisting of hydrogen, a C₁₋₁₀ linear or branched alkyl and a C₁₋₁₀ linear or branched alkyloxy.

According to an embodiment of this invention, in Formula (II-1), preferably one of the Relocates at the position opposite to a N atom (i.e. the N atom bonding to the group R_{d}) on the ring

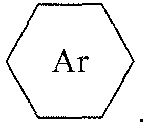

According to this invention, in Formula (II-1), y is an integer in the range of from 0 to 3, preferably 0 or 1.

According to this invention, in Formula (II-1), z is an integer in the range of from 0 to 3, preferably 0 or 1.

According to this invention, in Formula (II-1), n is an integer in the range of from 1 to 8, preferably 1 or 2.

According to this invention, in Formula (II-1), when plural exist, the plural R_{d}s may be identical to or different from each other, each independently selected from the group consisting of hydrogen and a group represented by Formula (V), preferably hydrogen.

According to this invention, in Formula (II-1), when plural exist, the plural rings 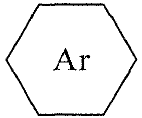 (as a bivalent group when locating inside of Formula (II-1)) may be identical to or different from each other, each independently selected from the group consisting of benzene ring (preferably in Formula (II-1) as 1,4-phenylene when locating inside thereof) and naphthalene ring (preferably in Formula (II-1) as 1,4- or 2,6-naphthylene when locating inside thereof), preferably benzene ring.

According to an embodiment of this invention, in Formula (II-1), two adjacent rings 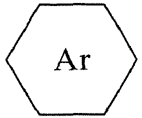 may optionally with an additional S atom (not shown in the formula) and the N atom bridging these two rings (i.e. the N atom bonding to the group R_{d}) to form a phenothiazine ring, i.e.

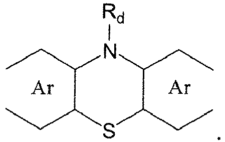

According to this embodiment of this invention, in Formula (II-1), when exist two or more rings 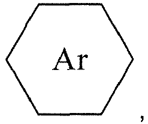 it is acceptable as long as at least two (adjacent) rings 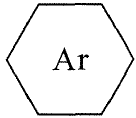 to form the phenothiazine ring, it is not necessary for each of the rings 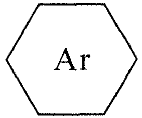 to form the phenothiazine ring with its adjacent ring

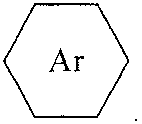

According to an embodiment of this invention, in Formula (II-1), two adjacent rings 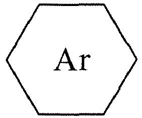 may optionally with an additional group represented by 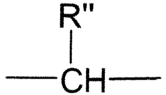 (not shown in the formula) and the N atom bridging these two rings (i.e. the N atom bonding to the group R_{d}) to form a 9,10-dihydroacridine ring, i.e.

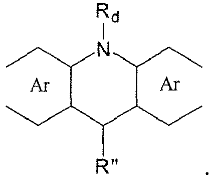

According to this invention, in the definition of the group 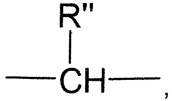 the group R" is selected from the group consisting of hydrogen, a C₁₋₂₀ hydrocarbyl (preferably a C₁₋₂₀ linear or branched alkyl) and a C₃₋₂₀ linear or branched hetero-alkyl.

According to an embodiment of this invention, in the definition of the group 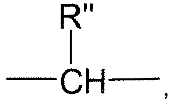 the group R" is selected from the group consisting of hydrogen and a C₁₋₁₀ linear or branched alkyl.

According to this embodiment of this invention, in Formula (II-1), when exist two or more rings 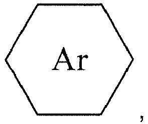 it is acceptable as long as at least two (adjacent) rings 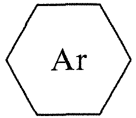 form the 9,10-dihydroacridine ring, it is not necessary for each of the rings 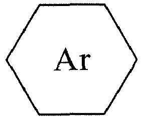 to form the 9,10-dihydroacridine ring with its adjacent ring

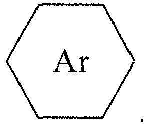

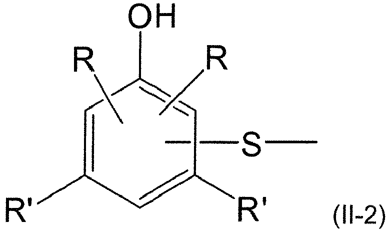

According to this invention, in Formula (II-2), when plural exist, the plural Rs may be identical to or different from each other, each independently selected from the group consisting of hydrogen, a C₁₋₃₀₀ linear or branched alkyl, a group represented by Formula (II) and a group represented by Formula (III).

According to this invention, in Formula (II-2), two Rs and one group -S- occupy the rest three positions on the benzene ring respectively.

According to an embodiment of this invention, in Formula (II-2), when plural exist, the plural Rs may be identical to or different from each other, each independently selected from the group consisting of hydrogen and a C₁₋₃₀₀ linear or branched alkyl.

According to this invention, in Formula (II-2), as the C₁₋₃₀₀ linear or branched alkyl, exemplified could be a C₁₋₂₀ linear or branched alkyl (preferably a C₁₋₁₀ linear or branched alkyl, more preferably a C₁₋₄ linear or branched alkyl) or a polyolefin group. As the polyolefin group, further exemplified could be a polyolefin group having a number-average molecular weight (Mn) of from 300 to 3000. Herein, the number-average molecular weight (Mn) of the polyolefin group is preferably in the range of from 500 to 2000, more preferably in the range of from 500 to 1500. According to this invention, the polyolefin group has a (substantially) saturated structure (in the form of a linear long-chain alkyl), however, depending on the nature of the starting polyolefin or the process for producing the starting polyolefin, there may exist some (only a small amount of) ethylenic double bond (for example, remained from or introduced during production of the polyolefin) along the molecular chain of the polyolefin group, which will not adversely interfere with the effects anticipated by this invention. For this reason, this invention does not intend to specify at what level this amount of ethylenic double bond could be, but rather, still identifies a polyolefin group of this kind as an "alkyl".

According to an embodiment of this invention, in Formula (II-2), there exist plural Rs, wherein one of the Rs is the polyolefin group, the rest one is selected from the group consisting of hydrogen or a C₁₋₂₀ linear or branched alkyl (preferably a C₁₋₁₀ linear or branched alkyl, more preferably a C₁₋₄ linear or branched alkyl).

According to an embodiment of this invention, in Formula (II-2), when the group R is the polyolefin group, the polyolefin group preferably locates at the position opposite to the phenol hydroxyl in Formula (II-2).

According to this invention, in Formula (II-2), when plural exist, the plural R's may be identical to or different from each other, each independently selected from the group consisting of hydrogen and a C₁₋₂₀ linear or branched alkyl.

According to an embodiment of this invention, in Formula (II-2), when plural exist, the plural R's may be identical to or different from each other, each independently selected from the group consisting of hydrogen and a C₁₋₄ linear or branched alkyl.

According to an embodiment of this invention, the Formula (II) is selected from one of the following Formula (II_{X}), Formula (II_{XX}) and Formula (II_{XXX}). In this embodiment, unless otherwise specified, any group or numerical value is as defined in Formula (II).

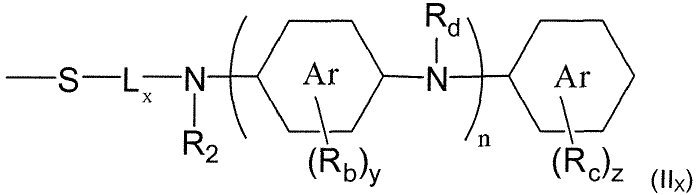

According to this invention, in Formula (II_{X}), the group L_{X} is a group represented by

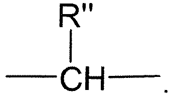

According to this invention, in Formula (II_{X}), in the definition of the group L_{X}, the group R" is selected from the group consisting of hydrogen, a C₁₋₂₀ hydrocarbyl (preferably a C₁₋₂₀ linear or branched alkyl) and a C₃₋₂₀ linear or branched hetero-alkyl.

According to an embodiment of this invention, in Formula (II_{X}), in the definition of the group L_{X}, the group R" is selected from the group consisting of hydrogen, a C₁₋₁₀ linear or branched alkyl and a C₃₋₁₀ linear or branched hetero-alkyl.

According to this invention, in Formula (II_{X}), the group R₂ is selected from the group consisting of hydrogen, a C₁₋₂₀ linear or branched alkyl, a group represented by Formula (IV) and a group represented by Formula (V) (especially a group represented by Formula (V_{X})).

According to an embodiment of this invention, in Formula (II_{X}), the group R₂ is selected from the group consisting of hydrogen, a C₁₋₁₀ linear or branched alkyl and a group represented by Formula (IV).

According to this invention, in Formula (II_{X}), when plural exist, the plural R_{b}s may be identical to or different from each other, each independently selected from the group consisting of hydrogen and a C₁₋₂₀ linear or branched alkyl, preferably each independently selected from the group consisting of hydrogen and a C₁₋₁₀ linear or branched alkyl.

According to an embodiment of this invention, in Formula (II_{X}), when plural exist, the plural R_{b}s may be identical to or different from each other, each independently selected from the group consisting of hydrogen and a C₁₋₆ linear or branched alkyl, preferably hydrogen.

According to this invention, in Formula (II_{X}), when plural exist, the plural R_{c}s may be identical to or different from each other, each independently selected from the group consisting of hydrogen, a C₁₋₂₀ linear or branched alkyl and a C₁₋₂₀ linear or branched alkyloxy.

According to an embodiment of this invention, in Formula (II_{X}), when plural exist, the plural R_{c}s may be identical to or different from each other, each independently selected from the group consisting of hydrogen, a C₁₋₁₀ linear or branched alkyl and a C₁₋₁₀ linear or branched alkyloxy.

According to an embodiment of this invention, in Formula (II_{X}), preferably one of the R_{c} locates at the position opposite to a N atom (i.e. the N atom bonding to the group R_{d}) on the ring

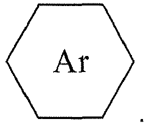

According to this invention, in Formula (II_{X}), y is an integer in the range of from 0 to 3, preferably 0 or 1.

According to this invention, in Formula (II_{X}), z is an integer in the range of from 0 to 3, preferably 0 or 1.

According to this invention, in Formula (II_{X}), n is an integer in the range of from 1 to 8, preferably 1 or 2.

According to this invention, in Formula (II_{X}), when plural exist, the plural R_{d}s may be identical to or different from each other, each independently selected from the group consisting of hydrogen and a group represented by Formula (V) (especially a group represented by Formula (V_{X})), preferably hydrogen.

According to this invention, in Formula (II_{X}), when plural exist, the plural rings 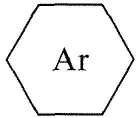 (in Formula (II_{X}) as a bivalent group when locating inside thereof) may be identical to or different from each other, each independently selected from the group consisting of benzene ring (preferably in Formula (II_{X}) as 1,4-phenylene when locating inside thereof) and naphthalene ring (preferably in Formula (II_{X}) as 1,4- or 2,6-naphthylene when locating inside thereof), preferably benzene ring.

According to an embodiment of this invention, in Formula (II_{X}), two adjacent rings 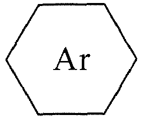 may optionally with an additional S atom (not shown in the formula) and the N atom bridging these two rings (i.e. the N atom bonding to the group R_{d}) to form a phenothiazine ring, i.e.

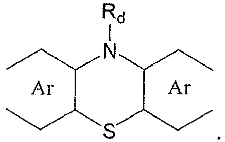

According to this embodiment of this invention, in Formula (II_{X}), when exist two or more rings 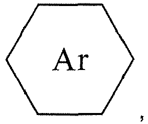 it is acceptable as long as at least two (adjacent) rings 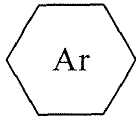 to form the phenothiazine ring, it is not necessary for each of the rings 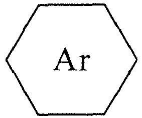 to form the phenothiazine ring with its adjacent ring

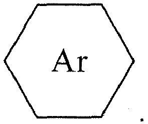

According to an embodiment of this invention, in Formula (II_{X}), two adjacent rings 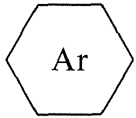 may optionally with an additional group represented by 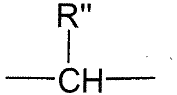 (not shown in the formula) and the N atom bridging these two rings (i.e. the N atom bonding to the group R_{d}) to form a 9,10-dihydroacridine ring, i.e.

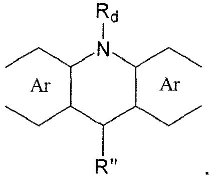

According to this invention, in the definition of the group 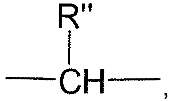 the group R" is selected from the group consisting of hydrogen, a C₁₋₂₀ hydrocarbyl (preferably a C₁₋₂₀ linear or branched alkyl) and a C₃₋₂₀ linear or branched hetero-alkyl.

According to an embodiment of this invention, in the definition of the group 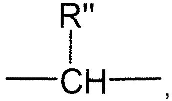 the group R" is selected from the group consisting of hydrogen and a C₁₋₁₀ linear or branched alkyl.

According to this embodiment of this invention, in Formula (II_{X}), when exist two or more rings 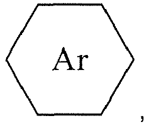 it is acceptable as long as at least two (adjacent) rings 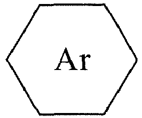 form the 9,10-dihydroacridine ring, it is not necessary for each of the rings 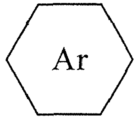 to form the 9,10-dihydroacridine ring with its adjacent ring

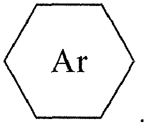

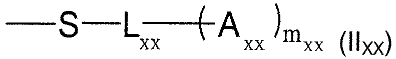

According to this invention, in Formula (II_{XX}), the group L_{XX} is selected from the group consisting of an optionally substituted m_{XX}+1 valent C₂₋₂₀ linear or branched alkyl and an optionally substituted m_{XX}+1 valent C₃₋₂₀ linear or branched hetero-alkyl, preferably selected from the group consisting of an optionally substituted m_{XX}+1 valent C₂₋₁₀ linear or branched alkyl and an optionally substituted m_{XX}+1 valent C₃₋₁₀ linear or branched hetero-alkyl, more preferably selected from the group consisting of an optionally substituted m_{XX}+1 valent C₂₋₆ linear or branched alkyl and an optionally substituted m_{XX}+1 valent C₃₋₆ linear or branched hetero-alkyl, more preferably selected from the group consisting of an optionally substituted m_{XX}+1 valent C₂₋₆ linear or branched alkyl.

According to this invention, in Formula (II_{XX}), in the definition of the group L_{XX}, by "optionally substituted", it refers to optionally substituted by one or more (for example, from 1 to 4, from 1 to 3, from 1 to 2, or 1) substituent selected from the group consisting of a C₁₋₂₀ hydrocarbyl and a C₃₋₂₀ linear or branched hetero-alkyl. As the substituent, preferably a C₁₋₂₀ linear or branched alkyl, a C₆₋₂₀ aryl or a combination group thereof. As the C₁₋₂₀ linear or branched alkyl, preferably a C₁₋₁₀ linear or branched alkyl, more preferably a C₁₋₆ linear or branched alkyl, for example, methyl or ethyl.

According to this invention, in Formula (II_{XX}), m_{XX} is an integer in the range of from 1 to 4, preferably 1.

According to this invention, in Formula (II_{XX}), the plural A_{XX}s may be identical to or different from each other, each independently selected from the group consisting of a group represented by Formula (II_{XX}-1) and a group represented by Formula (II_{XX}-2), with the proviso that at least one A_{XX} is a group represented by Formula (II_{XX}-1).

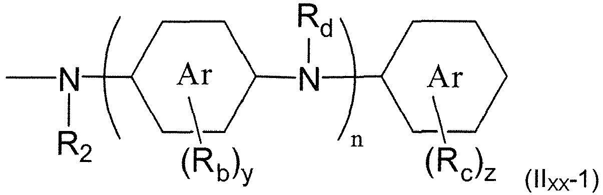

According to this invention, in Formula (II_{XX}-1), the group R₂ is selected from the group consisting of hydrogen, a C₁₋₂₀ linear or branched alkyl, a group represented by Formula (IV) and a group represented by Formula (V) (especially a group represented by Formula (V_{XX})).

According to an embodiment of this invention, in Formula (II_{XX}-1), the group R₂ is selected from the group consisting of hydrogen, a C₁₋₁₀ linear or branched alkyl and a group represented by Formula (IV).

According to this invention, in Formula (II_{XX}-1), when plural exist, the plural R_{b}s may be identical to or different from each other, each independently selected from the group consisting of hydrogen and a C₁₋₂₀ linear or branched alkyl, preferably each independently selected from the group consisting of hydrogen and a C₁₋₁₀ linear or branched alkyl.

According to an embodiment of this invention, in Formula (II_{XX}-1), when plural exist, the plural R_{b}s may be identical to or different from each other, each independently selected from the group consisting of hydrogen and a C₁₋₆ linear or branched alkyl, preferably hydrogen.

According to this invention, in Formula (II_{XX}-1), when plural exist, the plural R_{c}s may be identical to or different from each other, each independently selected from the group consisting of hydrogen, a C₁₋₂₀ linear or branched alkyl and a C₁₋₂₀ linear or branched alkyloxy.

According to an embodiment of this invention, in Formula (II_{XX}-1), when plural exist, the plural R_{c}s may be identical to or different from each other, each independently selected from the group consisting of hydrogen, a C₁₋₁₀ linear or branched alkyl and a C₁₋₁₀ linear or branched alkyloxy.

According to an embodiment of this invention, in Formula (II_{XX}-1), preferably one of the R_{c} locates at the position opposite to a N atom (i.e. the N atom bonding to the group R_{d}) on the ring

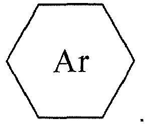

According to this invention, in Formula (II_{XX}-1), y is an integer in the range of from 0 to 3, preferably 0 or 1.

According to this invention, in Formula (II_{XX}-1), z is an integer in the range of from 0 to 3, preferably 0 or 1.

According to this invention, in Formula (II_{XX}-1), n is an integer in the range of from 1 to 8, preferably 1 or 2.

According to this invention, in Formula (II_{XX}-1), when plural exist, the plural R_{d}s may be identical to or different from each other, each independently selected from the group consisting of hydrogen and a group represented by Formula (V) (especially a group represented by Formula (V_{XX})), preferably hydrogen.

According to this invention, in Formula (II_{XX}-1), when plural exist, the plural rings 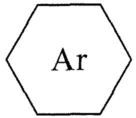 (in Formula (II_{XX}-1) as a bivalent group when locating inside thereof) may be identical to or different from each other, each independently selected from the group consisting of benzene ring (preferably in Formula (II_{XX}-1) as 1,4-phenylene when locating inside thereof) and naphthalene ring (preferably in Formula (II_{XX}-1) as 1,4- or 2,6-naphthylene when locating inside thereof), preferably benzene ring.

According to an embodiment of this invention, in Formula (II_{XX}-1), two adjacent rings 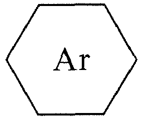 may optionally with an additional S atom (not shown in the formula) and the N atom bridging these two rings (i.e. the N atom bonding to the group R_{d}) to form a phenothiazine ring, i.e.

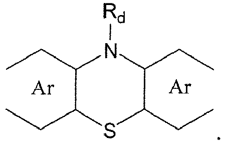

According to this embodiment of this invention, in Formula (II_{XX}-1), when exist two or more rings 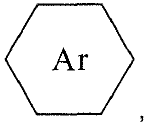 it is acceptable as long as at least two (adjacent) rings 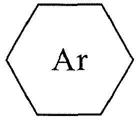 to form the phenothiazine ring, it is not necessary for each of the rings 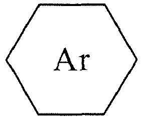 to form the phenothiazine ring with its adjacent ring

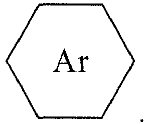

According to an embodiment of this invention, in Formula (II_{XX}-1), two adjacent rings 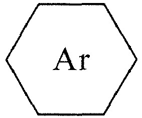 may optionally with an additional group represented by 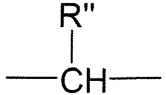 (not shown in the formula) and the N atom bridging these two rings (i.e. the N atom bonding to the group R_{d}) to form a 9,10-dihydroacridine ring, i.e.

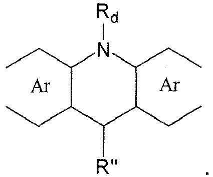

According to this invention, in the definition of the group 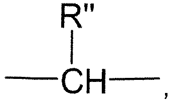 the group R" is selected from the group consisting of hydrogen, a C₁₋₂₀ hydrocarbyl (preferably a C₁₋₂₀ linear or branched alkyl) and a C₃₋₂₀ linear or branched hetero-alkyl.

According to an embodiment of this invention, in the definition of the group 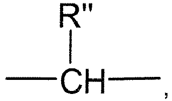 the group R" is selected from the group consisting of hydrogen and a C₁₋₁₀ linear or branched alkyl.

According to this embodiment of this invention, in Formula (II_{XX}-1), when exist two or more rings 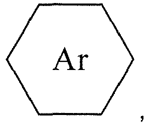 it is acceptable as long as at least two (adjacent) rings 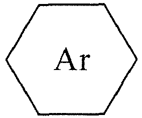 form the 9,10-dihydroacridine ring, it is not necessary for each of the rings 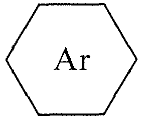 to form the 9,10-dihydroacridine ring with its adjacent ring

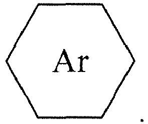

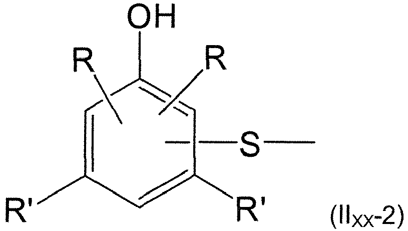

According to this invention, in Formula (II_{XX}-2), when plural exist, the plural Rs may be identical to or different from each other, each independently selected from the group consisting of hydrogen, a C₁₋₃₀₀ linear or branched alkyl, a group represented by Formula (II) (especially a group represented by Formula (II_{XX})) and a group represented by Formula (III).

According to this invention, in Formula (II_{XX}-2), two Rs and one group -S- occupy the rest three positions on the benzene ring respectively.

According to an embodiment of this invention, in Formula (II_{XX}-2), when plural exist, the plural Rs may be identical to or different from each other, each independently selected from the group consisting of hydrogen and a C₁₋₃₀₀ linear or branched alkyl.

According to this invention, in Formula (II_{XX}-2), as the C₁₋₃₀₀ linear or branched alkyl, exemplified could be a C₁₋₂₀ linear or branched alkyl (preferably a C₁₋₁₀ linear or branched alkyl, more preferably a C₁₋₄ linear or branched alkyl) or a polyolefin group. As the polyolefin group, further exemplified could be a polyolefin group having a number-average molecular weight (Mn) of from 300 to 3000. Herein, the number-average molecular weight (Mn) of the polyolefin group is preferably in the range of from 500 to 2000, more preferably in the range of from 500 to 1500. According to this invention, the polyolefin group has a (substantially) saturated structure (in the form of a linear long-chain alkyl), however, depending on the nature of the starting polyolefin or the process for producing the starting polyolefin, there may exist some (only a small amount of) ethylenic double bond (for example, remained from or introduced during production of the polyolefin) along the molecular chain of the polyolefin group, which will not adversely interfere with the effects anticipated by this invention. For this reason, this invention does not intend to specify at what level this amount of ethylenic double bond could be, but rather, still identifies a polyolefin group of this kind as an "alkyl".

According to an embodiment of this invention, in Formula (II_{XX}-2), there exist plural Rs, wherein one of the Rs is the polyolefin group, the rest one is selected from the group consisting of hydrogen or a C₁₋₂₀ linear or branched alkyl (preferably a C₁₋₁₀ linear or branched alkyl, more preferably a C₁₋₄ linear or branched alkyl).

According to an embodiment of this invention, in Formula (II_{XX}-2), when the group R is the polyolefin group, the polyolefin group preferably locates at the position opposite to the phenol hydroxyl in Formula (II_{XX}-2).

According to this invention, in Formula (II_{XX}-2), when plural exist, the plural R's may be identical to or different from each other, each independently selected from the group consisting of hydrogen and a C₁₋₂₀ linear or branched alkyl.

According to an embodiment of this invention, in Formula (II_{XX}-2), when plural exist, the plural R's may be identical to or different from each other, each independently selected from the group consisting of hydrogen and a C₁₋₄ linear or branched alkyl.

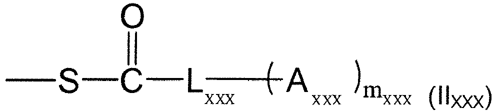

According to this invention, in Formula (II_{XXX}), m_{XXX} is an integer in the range of from 1 to 4, preferably 1.

According to this invention, in Formula (II_{XXX}), the group L_{XXX} is selected from the group consisting of an optionally substituted m_{XXX}+1 valent C₂ to C19-m_{XXX} linear or branched alkyl, an optionally substituted m_{XXX}+1 valent C₃ to C19-m_{XXX} linear or branched hetero-alkyl and a group represented by 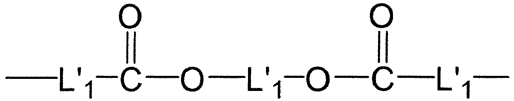 (hereinafter referred to as Formula (II_{XXX}-A)), preferably selected from the group consisting of an optionally substituted m_{XXX}+1 valent C₂ to C19-m_{XXX} linear or branched alkyl and a group represented by Formula (II_{XXX}-A), more preferably selected from the group consisting of an optionally substituted m_{XXX}+1 valent C₂ to C19-m_{XXX} linear or branched alkyl.

According to this invention, in Formula (II_{XXX}), in the definition of the group L_{XXX}, as the optionally substituted m_{XXX}+1 valent C₂ to C19-m_{XXX} linear or branched alkyl, preferably an optionally substituted m_{XXX}+1 valent C₂ to C9-m_{XXX} linear or branched alkyl, more preferably an optionally substituted m_{XXX}+1 valent C₂₋₆ linear or branched alkyl.

According to this invention, in Formula (II_{XXX}), in the definition of the group L_{XXX}, as the optionally substituted m_{XXX}+1 valent C₃ to C19-m_{XXX} linear or branched hetero-alkyl, preferably an optionally substituted m_{XXX}+1 valent C₃ to C9-m_{XXX} linear or branched hetero-alkyl, more preferably an optionally substituted m_{XXX}+1 valent C₃₋₆ linear or branched hetero-alkyl.

According to this invention, in Formula (II_{XXX}), in the definition of the group L_{XXX}, by "optionally substituted", it refers to optionally substituted by one or more (for example, from 1 to 4, from 1 to 3, from 1 to 2, or 1) substituent selected from the group consisting of a C₁₋₂₀ hydrocarbyl and a C₃₋₂₀ linear or branched hetero-alkyl. As the substituent, preferably a C₁₋₂₀ linear or branched alkyl, a C₆₋₂₀ aryl or a combination group thereof. As the C₁₋₂₀ linear or branched alkyl, preferably a C₁₋₁₀ linear or branched alkyl, more preferably a C₁₋₆ linear or branched alkyl, for example, methyl or ethyl.

According to this invention, in Formula (II_{XXX}), in the group represented by Formula (II_{XXX}-A), the plural L'₁s may be identical to or different from each other, each independently selected from the group consisting of a C₁₋₁₀ linear or branched alkyl and a C₃₋₁₀ linear or branched hetero-alkyl, preferably each independently selected from the group consisting of a C₁₋₆ linear or branched alkyl.

According to this invention, in Formula (II_{XXX}), as for the group represented by Formula (II_{XXX}-A), the requirement (1) is that: the plural L'₁s each independently represents a 2 valent to m_{XXX}+1 valent group, so as to make the group represented by Formula (II_{XXX}-A) to present as m_{XXX}+1 valent, as a whole. In this context, in the group represented by Formula (II_{XXX}-A), the valency between different L'₁ may vary and may be different from each other. In view of this, in the group represented by Formula (II_{XXX}-A), the group L'₁ is not specified for its valency. In fact, the valency of each L'₁ could be determined by the requirement (1). Specifically, assuming that each L'₁ is ethyl (with no valency specified), m_{XXX} is 2, then, the group represented by Formula (II_{XXX}-A) could be any one of the followings.

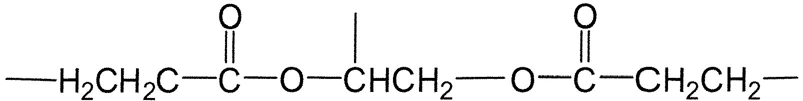

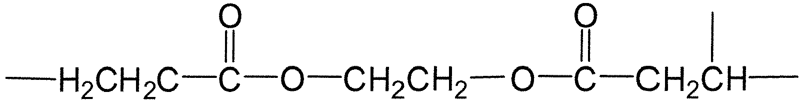

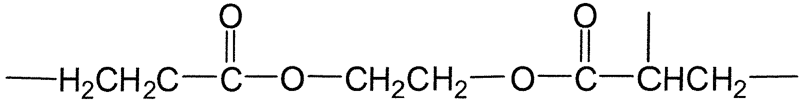

According to this invention, in Formula (II_{XXX}), in the group represented by Formula (II_{XXX}-A), the requirement (2) is that: the total atom number of all groups L'₁ (not figuring in any atom of the substituent that may exist on the group L'₁ as specified hereafter) is not more than 14, so as to make the group represented by Formula (II_{XXX}-A) to have a total atom number (not figuring in any atom of the substituent that may exist on the group L'₁ as specified hereafter) of no more than 18.

According to this invention, in Formula (II_{XXX}), in the group represented by Formula (II_{XXX}-A), after the aforesaid two requirements are met, the plural L'₁ may be each independently further optionally substituted by one or more (for example, from 1 to 4, from 1 to 3, from 1 to 2, or 1) substituent selected from the group consisting of a C₁₋₂₀ hydrocarbyl and a C₃₋₂₀ linear or branched hetero-alkyl. As the substituent, preferably a C₁₋₂₀ linear or branched alkyl, a C₆₋₂₀ aryl or a combination group thereof. As the C₁₋₂₀ linear or branched alkyl, preferably a C₁₋₁₀ linear or branched alkyl, more preferably a C₁₋₆ linear or branched alkyl, for example, methyl or ethyl.

According to this invention, in Formula (II_{XXX}), the plural A_{XXX}s may be identical to or different from each other, each independently selected from the group consisting of a group represented by Formula (II_{XXX}-1) and a group represented by Formula (II_{XXX}-2), with the proviso that at least one A_{XXX} is a group represented by Formula (II_{XXX}-1).

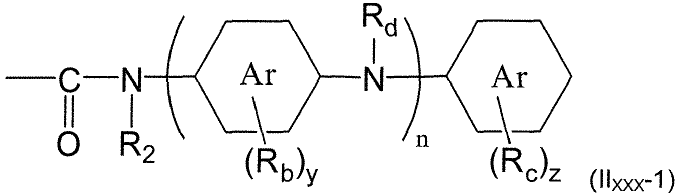

According to this invention, in Formula (II_{XXX}-1), the group R₂ is selected from the group consisting of hydrogen, a C₁₋₂₀ linear or branched alkyl, a group represented by Formula (IV) and a group represented by Formula (V) (especially a group represented by Formula (V_{XXX})).

According to an embodiment of this invention, in Formula (II_{XXX}-1), the group R₂ is selected from the group consisting of hydrogen, a C₁₋₁₀ linear or branched alkyl and a group represented by Formula (IV).

According to this invention, in Formula (II_{XXX}-1), when plural exist, the plural R_{b}s may be identical to or different from each other, each independently selected from the group consisting of hydrogen and a C₁₋₂₀ linear or branched alkyl, preferably each independently selected from the group consisting of hydrogen and a C₁₋₁₀ linear or branched alkyl.

According to an embodiment of this invention, in Formula (II_{XXX}-1), when plural exist, the plural R_{b}s may be identical to or different from each other, each independently selected from the group consisting of hydrogen and a C₁₋₆ linear or branched alkyl, preferably hydrogen.

According to this invention, in Formula (II_{XXX}-1), when plural exist, the plural R_{c}s may be identical to or different from each other, each independently selected from the group consisting of hydrogen, a C₁₋₂₀ linear or branched alkyl and a C₁₋₂₀ linear or branched alkyloxy.

According to an embodiment of this invention, in Formula (II_{XXX}-1), when plural exist, the plural R_{c}s may be identical to or different from each other, each independently selected from the group consisting of hydrogen, a C₁₋₁₀ linear or branched alkyl and a C₁₋₁₀ linear or branched alkyloxy.

According to an embodiment of this invention, in Formula (II_{XXX}-1), preferably one of the Relocates at the position opposite to a N atom (i.e. the N atom bonding to the group R_{d}) on the ring

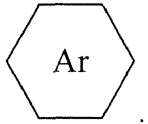

According to this invention, in Formula (II_{XXX}-1), y is an integer in the range of from 0 to 3, preferably 0 or 1.

According to this invention, in Formula (II_{XXX}-1), z is an integer in the range of from 0 to 3, preferably 0 or 1.

According to this invention, in Formula (II_{XXX}-1), n is an integer in the range of from 1 to 8, preferably 1 or 2.

According to this invention, in Formula (II_{XXX}-1), when plural exist, the plural R_{d}s may be identical to or different from each other, each independently selected from the group consisting of hydrogen and a group represented by Formula (V) (especially a group represented by Formula (V_{XXX})), preferably hydrogen.

According to this invention, in Formula (II_{XXX}-1), when plural exist, the plural rings 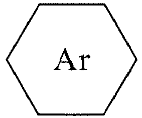 (in Formula (II_{XXX}-1) as a bivalent group when locating inside thereof) may be identical to or different from each other, each independently selected from the group consisting of benzene ring (preferably in Formula (II_{XXX}-1) as 1,4-phenylene when locating inside thereof) and naphthalene ring (preferably in Formula (II_{XXX}-1) as 1,4- or 2,6-naphthylene when locating inside thereof), preferably benzene ring.

According to an embodiment of this invention, in Formula (II_{XXX}-1), two adjacent rings 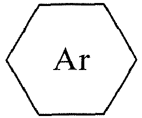 may optionally with an additional S atom (not shown in the formula) and the N atom bridging these two rings (i.e. the N atom bonding to the group R_{d}) to form a phenothiazine ring, i.e.

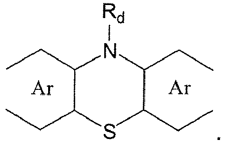

According to this embodiment of this invention, in Formula (II_{XXX}-1), when exist two or more rings 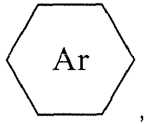 it is acceptable as long as at least two (adjacent) rings 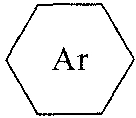 to form the phenothiazine ring, it is not necessary for each of the rings 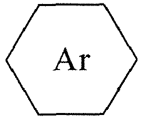 to form the phenothiazine ring with its adjacent ring

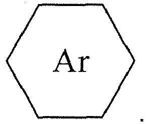

According to an embodiment of this invention, in Formula (II_{XXX}-1), two adjacent rings 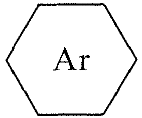 may optionally with an additional group represented by 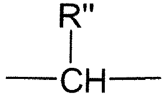 (not shown in the formula) and the N atom bridging these two rings (i.e. the N atom bonding to the group R_{d}) to form a 9,10-dihydroacridine ring, i.e.

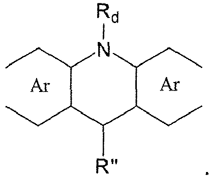

According to this invention, in the definition of the group 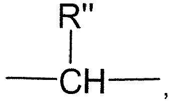 the group R" is selected from the group consisting of hydrogen, a C₁₋₂₀ hydrocarbyl (preferably a C₁₋₂₀ linear or branched alkyl) and a C₃₋₂₀ linear or branched hetero-alkyl.

According to an embodiment of this invention, in the definition of the group 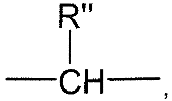 the group R" is selected from the group consisting of hydrogen and a C₁₋₁₀ linear or branched alkyl.

According to this embodiment of this invention, in Formula (II_{XXX}-1), when exist two or more rings 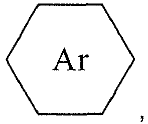 it is acceptable as long as at least two (adjacent) rings 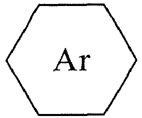 form the 9,10-dihydroacridine ring, it is not necessary for each of the rings 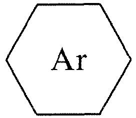 to form the 9,10-dihydroacridine ring with its adjacent ring

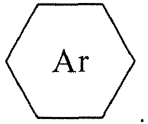

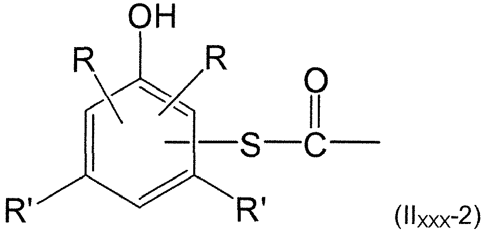

According to this invention, in Formula (II_{XXX}-2), when plural exist, the plural Rs may be identical to or different from each other, each independently selected from the group consisting of hydrogen, a C₁₋₃₀₀ linear or branched alkyl, a group represented by Formula (II) (especially a group represented by Formula (II_{XXX})) and a group represented by Formula (III).

According to this invention, in Formula (II_{XXX}-2), two Rs and one group 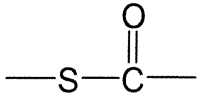 occupy the rest three positions on the benzene ring respectively.

According to an embodiment of this invention, in Formula (II_{XXX}-2), when plural exist, the plural Rs may be identical to or different from each other, each independently selected from the group consisting of hydrogen and a C₁₋₃₀₀ linear or branched alkyl.

According to this invention, in Formula (II_{XXX}-2), as the C₁₋₃₀₀ linear or branched alkyl, exemplified could be a C₁₋₂₀ linear or branched alkyl (preferably a C₁₋₁₀ linear or branched alkyl, more preferably a C₁₋₄ linear or branched alkyl) or a polyolefin group. As the polyolefin group, further exemplified could be a polyolefin group having a number-average molecular weight (Mn) of from 300 to 3000. Herein, the number-average molecular weight (Mn) of the polyolefin group is preferably in the range of from 500 to 2000, more preferably in the range of from 500 to 1500. According to this invention, the polyolefin group has a (substantially) saturated structure (in the form of a linear long-chain alkyl), however, depending on the nature of the starting polyolefin or the process for producing the starting polyolefin, there may exist some (only a small amount of) ethylenic double bond (for example, remained from or introduced during production of the polyolefin) along the molecular chain of the polyolefin group, which will not adversely interfere with the effects anticipated by this invention. For this reason, this invention does not intend to specify at what level this amount of ethylenic double bond could be, but rather, still identifies a polyolefin group of this kind as an "alkyl".

According to an embodiment of this invention, in Formula (II_{XXX}-2), there exist plural Rs, wherein one of the Rs is the polyolefin group, the rest one is selected from the group consisting of hydrogen or a C₁₋₂₀ linear or branched alkyl (preferably a C₁₋₁₀ linear or branched alkyl, more preferably a C₁₋₄ linear or branched alkyl).

According to an embodiment of this invention, in Formula (II_{XXX}-2), when the group R is the polyolefin group, the polyolefin group preferably locates at the position opposite to the phenol hydroxyl in Formula (II_{XXX}-2)

According to this invention, in Formula (II_{XXX}-2), when plural exist, the plural R's may be identical to or different from each other, each independently selected from the group consisting of hydrogen and a C₁₋₂₀ linear or branched alkyl.

According to an embodiment of this invention, in Formula (II_{XXX}-2), when plural exist, the plural R's may be identical to or different from each other, each independently selected from the group consisting of hydrogen and a C₁₋₄ linear or branched alkyl.

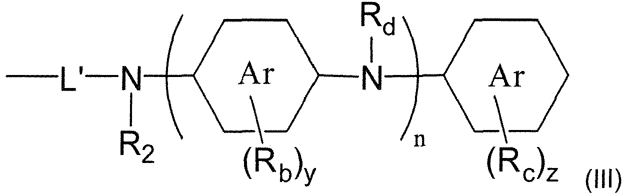

According to this invention, in Formula (III), the group L' is a group represented by

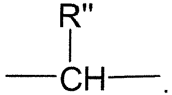

According to this invention, in Formula (III), in the definition of the group L', the group R" is selected from the group consisting of hydrogen, a C₁₋₂₀ hydrocarbyl (preferably a C₁₋₂₀ linear or branched alkyl) and a C₃₋₂₀ linear or branched hetero-alkyl.

According to an embodiment of this invention, in Formula (III), in the definition of the group L', the group R" is selected from the group consisting of hydrogen, a C₁₋₁₀ linear or branched alkyl and a C₃₋₁₀ linear or branched hetero-alkyl, preferably selected from the group consisting of hydrogen and a C₁₋₁₀ linear or branched alkyl.

According to this invention, in Formula (III), the group R₂ is selected from the group consisting of hydrogen, a C₁₋₂₀ linear or branched alkyl, a group represented by Formula (IV) and a group represented by Formula (V).

According to an embodiment of this invention, in Formula (III), the group R₂ is selected from the group consisting of hydrogen, a C₁₋₁₀ linear or branched alkyl and a group represented by Formula (IV).

According to this invention, in Formula (III), when plural exist, the plural R_{b}s may be identical to or different from each other, each independently selected from the group consisting of hydrogen and a C₁₋₂₀ linear or branched alkyl, preferably each independently selected from the group consisting of hydrogen and a C₁₋₁₀ linear or branched alkyl.

According to an embodiment of this invention, in Formula (III), when plural exist, the plural R_{b}s may be identical to or different from each other, each independently selected from the group consisting of hydrogen and a C₁₋₆ linear or branched alkyl, preferably hydrogen.

According to this invention, in Formula (III), when plural exist, the plural R_{c}s may be identical to or different from each other, each independently selected from the group consisting of hydrogen, a C₁₋₂₀ linear or branched alkyl and a C₁₋₂₀ linear or branched alkyloxy.

According to an embodiment of this invention, in Formula (III), when plural exist, the plural R_{c}s may be identical to or different from each other, each independently selected from the group consisting of hydrogen, a C₁₋₁₀ linear or branched alkyl and a C₁₋₁₀ linear or branched alkyloxy.

According to an embodiment of this invention, in Formula (III), preferably one of the R_{c} locates at the position opposite to a N atom (i.e. the N atom bonding to the group R_{d}) on the ring

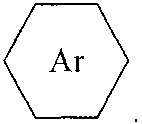

According to this invention, in Formula (III), y is an integer in the range of from 0 to 3, preferably 0 or 1.

According to this invention, in Formula (III), z is an integer in the range of from 0 to 3, preferably 0 or 1.

According to this invention, in Formula (III), n is an integer in the range of from 1 to 8, preferably 1 or 2.

According to this invention, in Formula (III), when plural exist, the plural R_{d}s may be identical to or different from each other, each independently selected from the group consisting of hydrogen and a group represented by Formula (V), preferably hydrogen. According to this invention, in Formula (III), when plural exist, the plural rings 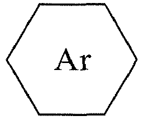 (in Formula (III) as a bivalent group when locating inside thereof) may be identical to or different from each other, each independently selected from the group consisting of benzene ring (preferably in Formula (III) as 1,4-phenylene when locating inside thereof) and naphthalene ring (preferably in Formula (III) as 1,4- or 2,6-naphthylene when locating inside thereof), preferably benzene ring.

According to an embodiment of this invention, in Formula (III), two adjacent rings 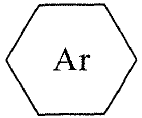 may optionally with an additional S atom (not shown in the formula) and the N atom bridging these two rings (i.e. the N atom bonding to the group R_{d}) to form a phenothiazine ring, i.e.

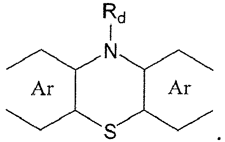

According to this embodiment of this invention, in Formula (III), when exist two or more rings 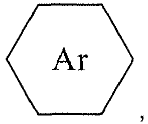 it is acceptable as long as at least two (adjacent) rings 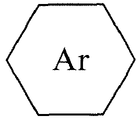 to form the phenothiazine ring, it is not necessary for each of the rings 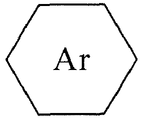 to form the phenothiazine ring with its adjacent ring

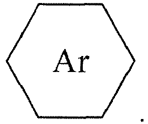

According to an embodiment of this invention, in Formula (III), two adjacent rings 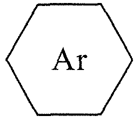 may optionally with an additional group represented by 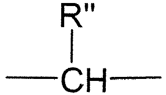 (not shown in the formula) and the N atom bridging these two rings (i.e. the N atom bonding to the group R_{d}) to form a 9,10-dihydroacridine ring, i.e.

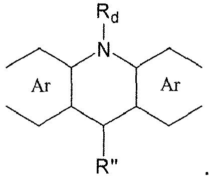

According to this invention, in the definition of the group 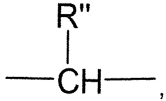 the group R" is selected from the group consisting of hydrogen, a C₁₋₂₀ hydrocarbyl (preferably a C₁₋₂₀ linear or branched alkyl) and a C₃₋₂₀ linear or branched hetero-alkyl.

According to an embodiment of this invention, in the definition of the group 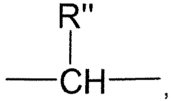 the group R" is selected from the group consisting of hydrogen and a C₁₋₁₀ linear or branched alkyl.

According to this embodiment of this invention, in Formula (III), when exist two or more rings 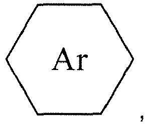 it is acceptable as long as at least two (adjacent) rings 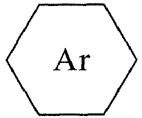 form the 9,10-dihydroacridine ring, it is not necessary for each of the rings 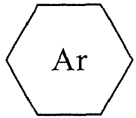 to form the 9,10-dihydroacridine ring with its adjacent ring

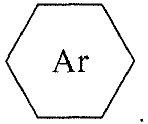

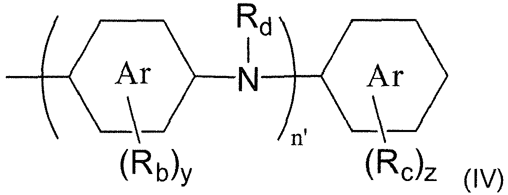

According to this invention, in Formula (IV), when plural exist, the plural R_{b}s may be identical to or different from each other, each independently selected from the group consisting of hydrogen and a C₁₋₂₀ linear or branched alkyl, preferably each independently selected from the group consisting of hydrogen and a C₁₋₁₀ linear or branched alkyl.

According to an embodiment of this invention, in Formula (IV), when plural exist, the plural R_{b}s may be identical to or different from each other, each independently selected from the group consisting of hydrogen and a C₁₋₆ linear or branched alkyl, preferably hydrogen.

According to this invention, in Formula (IV), when plural exist, the plural R_{c}s may be identical to or different from each other, each independently selected from the group consisting of hydrogen, a C₁₋₂₀ linear or branched alkyl and a C₁₋₂₀ linear or branched alkyloxy.

According to an embodiment of this invention, in Formula (IV), when plural exist, the plural R_{c}s may be identical to or different from each other, each independently selected from the group consisting of hydrogen, a C₁₋₁₀ linear or branched alkyl and a C₁₋₁₀ linear or branched alkyloxy.

According to an embodiment of this invention, in Formula (IV), preferably one of the R_{c} locates at the position opposite to a N atom (i.e. the N atom bonding to the group R_{d}) on the ring

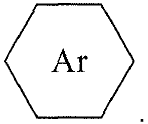

According to this invention, in Formula (IV), y is an integer in the range of from 0 to 3, preferably 0 or 1.

According to this invention, in Formula (IV), z is an integer in the range of from 0 to 3, preferably 0 or 1.

According to this invention, in Formula (IV), n' is an integer in the range of from 0 to 7, preferably 0, 1 or 2, with the proviso that n'+n≤8. According to an embodiment of this invention, n'+n=1 or n'+n=2. Herein, n is as defined in the aforesaid Formula (II-1).

According to this invention, in Formula (IV), when plural exist, the plural R_{d}s may be identical to or different from each other, each independently selected from the group consisting of hydrogen and a group represented by Formula (V), preferably hydrogen.

According to this invention, in Formula (IV), when plural exist, the plural rings 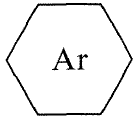 (in Formula (IV) as a bivalent group when locating inside thereof) may be identical to or different from each other, each independently selected from the group consisting of benzene ring (preferably in Formula (IV) as 1,4-phenylene when locating inside thereof) and naphthalene ring (preferably in Formula (IV) as 1,4- or 2,6-naphthylene when locating inside thereof), preferably benzene ring.

According to an embodiment of this invention, in Formula (IV), two adjacent rings 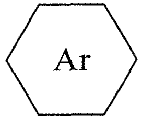 may optionally with an additional S atom (not shown in the formula) and the N atom bridging these two rings (i.e. the N atom bonding to the group R_{d}) to form a phenothiazine ring, i.e.

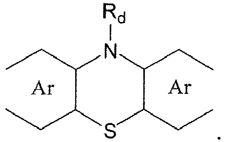

According to this embodiment of this invention, in Formula (IV), when exist two or more rings 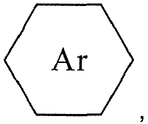 it is acceptable as long as at least two (adjacent) rings 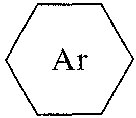 to form the phenothiazine ring, it is not necessary for each of the rings 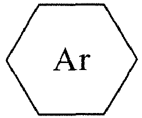 to form the phenothiazine ring with its adjacent ring

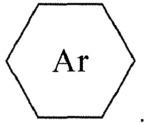

According to an embodiment of this invention, in Formula (IV), two adjacent rings 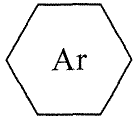 may optionally with an additional group represented by 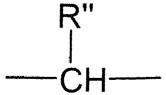 (not shown in the formula) and the N atom bridging these two rings (i.e. the N atom bonding to the group R_{d}) to form a 9,10-dihydroacridine ring, i.e.

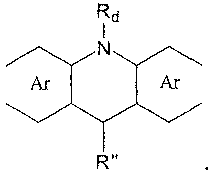

According to this invention, in the definition of the group 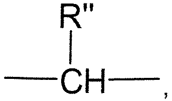 the group R" is selected from the group consisting of hydrogen, a C₁₋₂₀ hydrocarbyl (preferably a C₁₋₂₀ linear or branched alkyl) and a C₃₋₂₀ linear or branched hetero-alkyl.

According to an embodiment of this invention, in the definition of the group 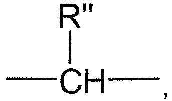 the group R" is selected from the group consisting of hydrogen and a C₁₋₁₀ linear or branched alkyl.

According to this embodiment of this invention, in Formula (IV), when exist two or more rings 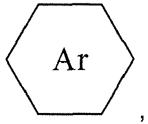 it is acceptable as long as at least two (adjacent) rings 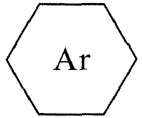 form the 9,10-dihydroacridine ring, it is not necessary for each of the rings 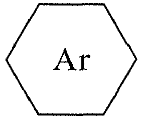 to form the 9,10-dihydroacridine ring with its adjacent ring

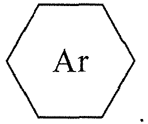

-L(̵A)ₘ (V)

According to this invention, in Formula (V), the plural As may be identical to or different from each other, each independently selected from the group consisting of a group represented by Formula (V-1) and a group represented by Formula (V-2).

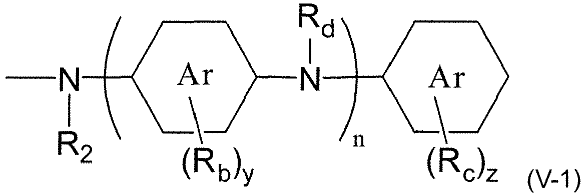

According to this invention, in Formula (V-1), the group R₂ is selected from the group consisting of hydrogen, a C₁₋₂₀ linear or branched alkyl, a group represented by Formula (IV) and a group represented by Formula (V).

According to an embodiment of this invention, in Formula (V-1), the group R₂ is selected from the group consisting of hydrogen, a C₁₋₁₀ linear or branched alkyl and a group represented by Formula (IV).

According to this invention, in Formula (V-1), when plural exist, the plural R_{b}s may be identical to or different from each other, each independently selected from the group consisting of hydrogen and a C₁₋₂₀ linear or branched alkyl, preferably each independently selected from the group consisting of hydrogen and a C₁₋₁₀ linear or branched alkyl.

According to an embodiment of this invention, in Formula (V-1), when plural exist, the plural R_{b}s may be identical to or different from each other, each independently selected from the group consisting of hydrogen and a C₁₋₆ linear or branched alkyl, preferably hydrogen.

According to this invention, in Formula (V-1), when plural exist, the plural R_{c}s may be identical to or different from each other, each independently selected from the group consisting of hydrogen, a C₁₋₂₀ linear or branched alkyl and a C₁₋₂₀ linear or branched alkyloxy.

According to an embodiment of this invention, in Formula (V-1), when plural exist, the plural R_{c}s may be identical to or different from each other, each independently selected from the group consisting of hydrogen, a C₁₋₁₀ linear or branched alkyl and a C₁₋₁₀ linear or branched alkyloxy.

According to an embodiment of this invention, in Formula (V-1), preferably one of the R_{c} locates at the position opposite to a N atom (i.e. the N atom bonding to the group R_{d}) on the ring

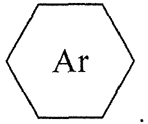

According to this invention, in Formula (V-1), y is an integer in the range of from 0 to 3, preferably 0 or 1.

According to this invention, in Formula (V-1), z is an integer in the range of from 0 to 3, preferably 0 or 1.

According to this invention, in Formula (V-1), n is an integer in the range of from 1 to 8, preferably 1 or 2.

According to this invention, in Formula (V-1), when plural exist, the plural R_{d}s may be identical to or different from each other, each independently selected from the group consisting of hydrogen and a group represented by Formula (V), preferably hydrogen.

According to this invention, in Formula (V-1), when plural exist, the plural rings 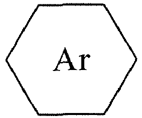 (in Formula (V-1) as a bivalent group when locating inside thereof) may be identical to or different from each other, each independently selected from the group consisting of benzene ring (preferably in Formula (V-1) as 1,4-phenylene when locating inside thereof) and naphthalene ring (preferably in Formula (V-1) as 1,4- or 2,6-naphthylene when locating inside thereof), preferably benzene ring.

According to an embodiment of this invention, in Formula (V-1), two adjacent rings 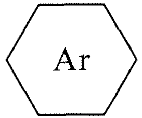 may optionally with an additional S atom (not shown in the formula) and the N atom bridging these two rings (i.e. the N atom bonding to the group R_{d}) to form a phenothiazine ring, i.e.

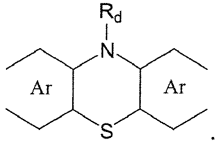

According to this embodiment of this invention, in Formula (V-1), when exist two or more rings 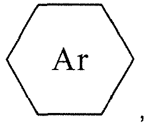 it is acceptable as long as at least two (adjacent) rings 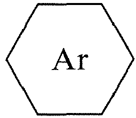 to form the phenothiazine ring, it is not necessary for each of the rings 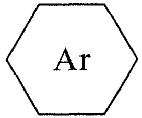 to form the phenothiazine ring with its adjacent ring

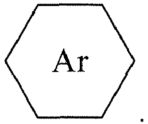

According to an embodiment of this invention, in Formula (V-1), two adjacent rings 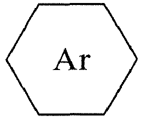 may optionally with an additional group represented by 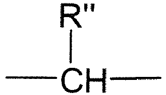 (not shown in the formula) and the N atom bridging these two rings (i.e. the N atom bonding to the group R_{d}) to form a 9,10-dihydroacridine ring, i.e.

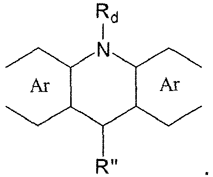

According to this invention, in the definition of the group 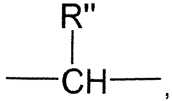 the group R" is selected from the group consisting of hydrogen, a C₁₋₂₀ hydrocarbyl (preferably a C₁₋₂₀ linear or branched alkyl) and a C₃₋₂₀ linear or branched hetero-alkyl.

According to an embodiment of this invention, in the definition of the group 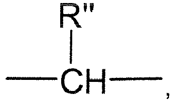 the group R" is selected from the group consisting of hydrogen and a C₁₋₁₀ linear or branched alkyl.

According to this embodiment of this invention, in Formula (V-1), when exist two or more rings 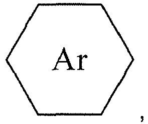 it is acceptable as long as at least two (adjacent) rings 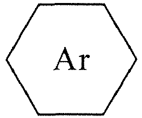 form the 9,10-dihydroacridine ring, it is not necessary for each of the rings 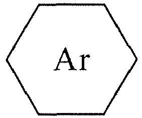 to form the 9,10-dihydroacridine ring with its adjacent ring

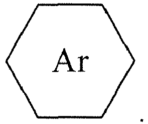

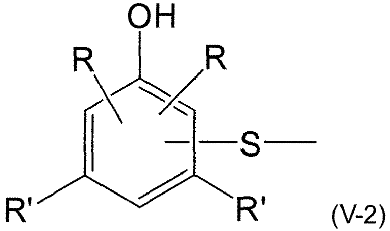

According to this invention, in Formula (V-2), when plural exist, the plural Rs may be identical to or different from each other, each independently selected from the group consisting of hydrogen, a C₁₋₃₀₀ linear or branched alkyl, a group represented by Formula (II) and a group represented by Formula (III).

According to this invention, in Formula (V-2), two Rs and one group -S- occupy the rest three positions on the benzene ring respectively.

According to an embodiment of this invention, in Formula (V-2), when plural exist, the plural Rs may be identical to or different from each other, each independently selected from the group consisting of hydrogen and a C₁₋₃₀₀ linear or branched alkyl.

According to this invention, in Formula (V-2), as the C₁₋₃₀₀ linear or branched alkyl, exemplified could be a C₁₋₂₀ linear or branched alkyl (preferably a C₁₋₁₀ linear or branched alkyl, more preferably a C₁₋₄ linear or branched alkyl) or a polyolefin group. As the polyolefin group, further exemplified could be a polyolefin group having a number-average molecular weight (Mn) of from 300 to 3000. Herein, the number-average molecular weight (Mn) of the polyolefin group is preferably in the range of from 500 to 2000, more preferably in the range of from 500 to 1500. According to this invention, the polyolefin group has a (substantially) saturated structure (in the form of a linear long-chain alkyl), however, depending on the nature of the starting polyolefin or the process for producing the starting polyolefin, there may exist some (only a small amount of) ethylenic double bond (for example, remained from or introduced during production of the polyolefin) along the molecular chain of the polyolefin group, which will not adversely interfere with the effects anticipated by this invention. For this reason, this invention does not intend to specify at what level this amount of ethylenic double bond could be, but rather, still identifies a polyolefin group of this kind as an "alkyl".

According to an embodiment of this invention, in Formula (V-2), there exist plural Rs, wherein one of the Rs is the polyolefin group, the rest one is selected from the group consisting of hydrogen or a C₁₋₂₀ linear or branched alkyl (preferably a C₁₋₁₀ linear or branched alkyl, more preferably a C₁₋₄ linear or branched alkyl).

According to an embodiment of this invention, in Formula (V-2), when the group R is the polyolefin group, the polyolefin group preferably locates at the position opposite to the phenol hydroxyl in Formula (V-2)

According to this invention, in Formula (V-2), when plural exist, the plural R's may be identical to or different from each other, each independently selected from the group consisting of hydrogen and a C₁₋₂₀ linear or branched alkyl.

According to an embodiment of this invention, in Formula (V-2), when plural exist, the plural R's may be identical to or different from each other, each independently selected from the group consisting of hydrogen and a C₁₋₄ linear or branched alkyl.

According to this invention, in Formula (V), other groups including the group L, the group R₂, the group R_{b}, the group R_{c}, the group R_{d} and the ring 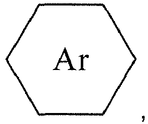 and other numerical values including y, n, z and m, are as defined in Formula (II) respectively.

According to an embodiment of this invention, the Formula (V) is selected from one of the following Formula (V_{X}), Formula (V_{XX}) and Formula (V_{XXX}). In this embodiment, unless otherwise specified, any group or numerical value is as defined in Formula (V).

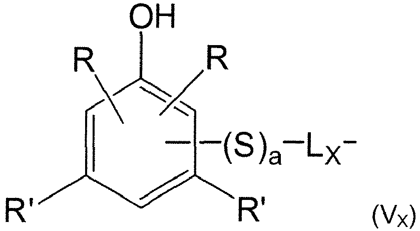

According to this invention, in Formula (V_{X}), when plural exist, the plural Rs may be identical to or different from each other, each independently selected from the group consisting of hydrogen, a C₁₋₃₀₀ linear or branched alkyl, a group represented by Formula (II) (especially a group represented by Formula (II_{X})) and a group represented by Formula (III).

According to an embodiment of this invention, in Formula (V_{X}), when plural exist, the plural Rs may be identical to or different from each other, each independently selected from the group consisting of hydrogen and a C₁₋₃₀₀ linear or branched alkyl.

According to this invention, in Formula (V_{X}), as the C₁₋₃₀₀ linear or branched alkyl, exemplified could be a C₁₋₂₀ linear or branched alkyl (preferably a C₁₋₁₀ linear or branched alkyl, more preferably a C₁₋₄ linear or branched alkyl) or a polyolefin group. As the polyolefin group, further exemplified could be a polyolefin group having a number-average molecular weight (Mn) of from 300 to 3000. Herein, the number-average molecular weight (Mn) of the polyolefin group is preferably in the range of from 500 to 2000, more preferably in the range of from 500 to 1500. According to this invention, the polyolefin group has a (substantially) saturated structure (in the form of a linear long-chain alkyl), however, depending on the nature of the starting polyolefin or the process for producing the starting polyolefin, there may exist some (only a small amount of) ethylenic double bond (for example, remained from or introduced during production of the polyolefin) along the molecular chain of the polyolefin group, which will not adversely interfere with the effects anticipated by this invention. For this reason, this invention does not intend to specify at what level this amount of ethylenic double bond could be, but rather, still identifies a polyolefin group of this kind as an "alkyl".

According to an embodiment of this invention, in Formula (V_{X}), there exist plural Rs, wherein one of the Rs is the polyolefin group, the rest one is selected from the group consisting of hydrogen or a C₁₋₂₀ linear or branched alkyl (preferably a C₁₋₁₀ linear or branched alkyl, more preferably a C₁₋₄ linear or branched alkyl).

According to an embodiment of this invention, in Formula (V_{X}), when the group R is the polyolefin group, the polyolefin group preferably locates at the position opposite to the phenol hydroxyl in Formula (V_{X})

According to this invention, in Formula (V_{X}), when plural exist, the plural R's may be identical to or different from each other, each independently selected from the group consisting of hydrogen and a C₁₋₂₀ linear or branched alkyl.

According to an embodiment of this invention, in Formula (V_{X}), when plural exist, the plural R's may be identical to or different from each other, each independently selected from the group consisting of hydrogen and a C₁₋₄ linear or branched alkyl.

According to this invention, in Formula (V_{X}), the group L_{X} is a group represented by

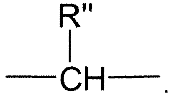

According to this invention, in Formula (V_{X}), in the definition of the group L_{X}, the group R" is selected from the group consisting of hydrogen, a C₁₋₂₀ hydrocarbyl (preferably a C₁₋₂₀ linear or branched alkyl) and a C₃₋₂₀ linear or branched hetero-alkyl.

According to an embodiment of this invention, in Formula (V_{X}), in the definition of the group L_{X}, the group R" is selected from the group consisting of hydrogen, a C₁₋₁₀ linear or branched alkyl and a C₃₋₁₀ linear or branched hetero-alkyl.

According to a preferred embodiment of this invention, in Formula (V_{X}), in the definition of the group L_{X}, the group R" is selected from the group consisting of hydrogen and a C₁₋₁₀ linear or branched alkyl.

According to this invention, in Formula (V_{X}), two Rs and one group -(S)ₐ-L- occupy the rest three positions on the benzene ring in Formula (V_{X}) respectively.

According to this invention, in Formula (V_{X}), a is 1.

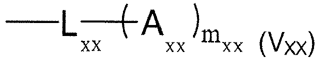

According to this invention, in Formula (V_{XX}), the group L_{XX} is selected from the group consisting of an optionally substituted m_{XX}+1 valent C₂₋₂₀ linear or branched alkyl and an optionally substituted m_{XX}+1 valent C₃₋₂₀ linear or branched hetero-alkyl, preferably selected from the group consisting of an optionally substituted m_{XX}+1 valent C₂₋₁₀ linear or branched alkyl and an optionally substituted m_{XX}+1 valent C₃₋₁₀ linear or branched hetero-alkyl, more preferably selected from the group consisting of an optionally substituted m_{XX}+1 valent C₂₋₆ linear or branched alkyl and an optionally substituted m_{XX}+1 valent C₃₋₆ linear or branched hetero-alkyl, more preferably selected from the group consisting of an optionally substituted m_{XX}+1 valent C₂₋₆ linear or branched alkyl.

According to this invention, in Formula (V_{XX}), in the definition of the group L_{XX}, by optionally substituted, it refers to optionally substituted by one or more (for example, from 1 to 4, from 1 to 3, from 1 to 2, or 1) substituent selected from the group consisting of a C₁₋₂₀ hydrocarbyl and a C₃₋₂₀ linear or branched hetero-alkyl. As the substituent, preferably a C₁₋₂₀ linear or branched alkyl, a C₆₋₂₀ aryl or a combination group thereof. As the C₁₋₂₀ linear or branched alkyl, preferably a C₁₋₁₀ linear or branched alkyl, more preferably a C₁₋₆ linear or branched alkyl, for example, methyl or ethyl.

According to this invention, in Formula (V_{XX}), m_{XX} is an integer in the range of from 1 to 4, preferably 1.

According to this invention, in Formula (V_{XX}), the plural A_{XX}s may be identical to or different from each other, each independently selected from the group consisting of a group represented by Formula (V_{XX}-1) and a group represented by Formula (V_{XX}-2).

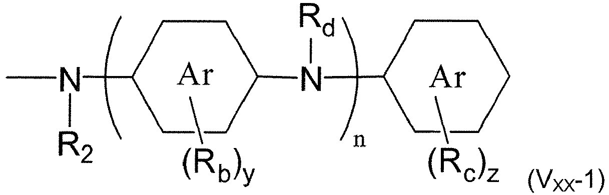

According to this invention, in Formula (V_{XX}-1), the group R₂ is selected from the group consisting of hydrogen, a C₁₋₂₀ linear or branched alkyl, a group represented by Formula (IV) and a group represented by Formula (V) (especially a group represented by Formula (V_{XX})).

According to an embodiment of this invention, in Formula (V_{XX}-1), the group R₂ is selected from the group consisting of hydrogen, a C₁₋₁₀ linear or branched alkyl and a group represented by Formula (IV).

According to this invention, in Formula (V_{XX}-1), when plural exist, the plural R_{b}s may be identical to or different from each other, each independently selected from the group consisting of hydrogen and a C₁₋₂₀ linear or branched alkyl, preferably each independently selected from the group consisting of hydrogen and a C₁₋₁₀ linear or branched alkyl.

According to an embodiment of this invention, in Formula (V_{XX}-1), when plural exist, the plural R_{b}s may be identical to or different from each other, each independently selected from the group consisting of hydrogen and a C₁₋₆ linear or branched alkyl, preferably hydrogen.

According to this invention, in Formula (V_{XX}-1), when plural exist, the plural R_{c}s may be identical to or different from each other, each independently selected from the group consisting of hydrogen, a C₁₋₂₀ linear or branched alkyl and a C₁₋₂₀ linear or branched alkyloxy.

According to an embodiment of this invention, in Formula (V_{XX}-1), when plural exist, the plural R_{c}s may be identical to or different from each other, each independently selected from the group consisting of hydrogen, a C₁₋₁₀ linear or branched alkyl and a C₁₋₁₀ linear or branched alkyloxy.

According to an embodiment of this invention, in Formula (V_{XX}-1), preferably one of the Relocates at the position opposite to a N atom (i.e. the N atom bonding to the group R_{d}) on the ring

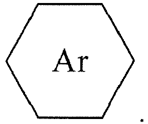

According to this invention, in Formula (V_{XX}-1), y is an integer in the range of from 0 to 3, preferably 0 or 1.

According to this invention, in Formula (V_{XX}-1), z is an integer in the range of from 0 to 3, preferably 0 or 1.

According to this invention, in Formula (V_{XX}-1), n is an integer in the range of from 1 to 8, preferably 1 or 2.

According to this invention, in Formula (V_{XX}-1), when plural exist, the plural R_{d}s may be identical to or different from each other, each independently selected from the group consisting of hydrogen and a group represented by Formula (V) (especially a group represented by Formula (V_{XX})), preferably hydrogen.

According to this invention, in Formula (V_{XX}-1), when plural exist, the plural rings 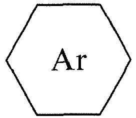 (in Formula (V_{XX}-1) as a bivalent group when locating inside thereof) may be identical to or different from each other, each independently selected from the group consisting of benzene ring (preferably in Formula (V_{XX}-1) as 1,4-phenylene when locating inside thereof) and naphthalene ring (preferably in Formula (V_{XX}-1) as 1,4- or 2,6-naphthylene when locating inside thereof), preferably benzene ring.

According to an embodiment of this invention, in Formula (V_{XX}-1), two adjacent rings 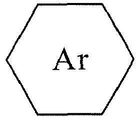 may optionally with an additional S atom (not shown in the formula) and the N atom bridging these two rings (i.e. the N atom bonding to the group R_{d}) to form a phenothiazine ring, i.e.

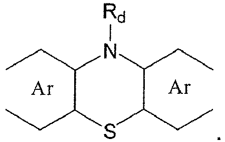

According to this embodiment of this invention, in Formula (V_{XX}-1), when exist two or more rings 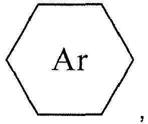 it is acceptable as long as at least two (adjacent) rings 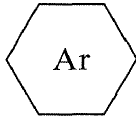 to form the phenothiazine ring, it is not necessary for each of the rings 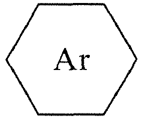 to form the phenothiazine ring with its adjacent ring

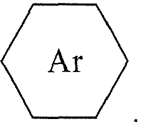

According to an embodiment of this invention, in Formula (V_{XX}-1), two adjacent rings 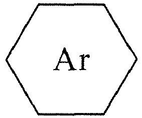 may optionally with an additional group represented by 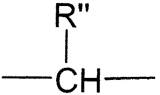 (not shown in the formula) and the N atom bridging these two rings (i.e. the N atom bonding to the group R_{d}) to form a 9,10-dihydroacridine ring, i.e.

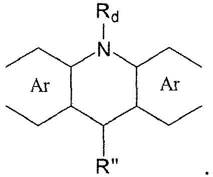

According to this invention, in the definition of the group 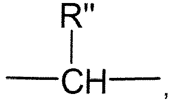 the group R" is selected from the group consisting of hydrogen, a C₁₋₂₀ hydrocarbyl (preferably a C₁₋₂₀ linear or branched alkyl) and a C₃₋₂₀ linear or branched hetero-alkyl.

According to an embodiment of this invention, in the definition of the group 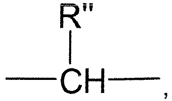 the group R" is selected from the group consisting of hydrogen and a C₁₋₁₀ linear or branched alkyl.

According to this embodiment of this invention, in Formula (V_{XX}-1), when exist two or more rings 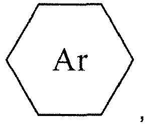 it is acceptable as long as at least two (adjacent) rings 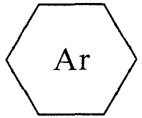 form the 9,10-dihydroacridine ring, it is not necessary for each of the rings 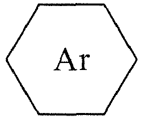 to form the 9,10-dihydroacridine ring with its adjacent ring

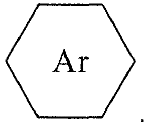

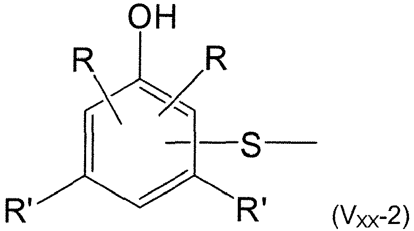

According to this invention, in Formula (V_{XX}-2), when plural exist, the plural Rs may be identical to or different from each other, each independently selected from the group consisting of hydrogen, a C₁₋₃₀₀ linear or branched alkyl, a group represented by Formula (II) (especially a group represented by Formula (II_{XX})) and a group represented by Formula (III).

According to this invention, in Formula (V_{XX}-2), two Rs and one group -S- occupy the rest three positions on the benzene ring respectively.

According to an embodiment of this invention, in Formula (V_{XX}-2), when plural exist, the plural Rs may be identical to or different from each other, each independently selected from the group consisting of hydrogen and a C₁₋₃₀₀ linear or branched alkyl.

According to this invention, in Formula (V_{XX}-2), as the C₁₋₃₀₀ linear or branched alkyl, exemplified could be a C₁₋₂₀ linear or branched alkyl (preferably a C₁₋₁₀ linear or branched alkyl, more preferably a C₁₋₄ linear or branched alkyl) or a polyolefin group. As the polyolefin group, further exemplified could be a polyolefin group having a number-average molecular weight (Mn) of from 300 to 3000. Herein, the number-average molecular weight (Mn) of the polyolefin group is preferably in the range of from 500 to 2000, more preferably in the range of from 500 to 1500. According to this invention, the polyolefin group has a (substantially) saturated structure (in the form of a linear long-chain alkyl), however, depending on the nature of the starting polyolefin or the process for producing the starting polyolefin, there may exist some (only a small amount of) ethylenic double bond (for example, remained from or introduced during production of the polyolefin) along the molecular chain of the polyolefin group, which will not adversely interfere with the effects anticipated by this invention. For this reason, this invention does not intend to specify at what level this amount of ethylenic double bond could be, but rather, still identifies a polyolefin group of this kind as an "alkyl".

According to an embodiment of this invention, in Formula (V_{XX}-2), there exist plural Rs, wherein one of the Rs is the polyolefin group, the rest one is selected from the group consisting of hydrogen or a C₁₋₂₀ linear or branched alkyl (preferably a C₁₋₁₀ linear or branched alkyl, more preferably a C₁₋₄ linear or branched alkyl).

According to an embodiment of this invention, in Formula (V_{XX}-2), when the group R is the polyolefin group, the polyolefin group preferably locates at the position opposite to the phenol hydroxyl in Formula (V_{XX}-2)

According to this invention, in Formula (V_{XX}-2), when plural exist, the plural R's may be identical to or different from each other, each independently selected from the group consisting of hydrogen and a C₁₋₂₀ linear or branched alkyl.

According to an embodiment of this invention, in Formula (V_{XX}-2), when plural exist, the plural R's may be identical to or different from each other, each independently selected from the group consisting of hydrogen and a C₁₋₄ linear or branched alkyl.

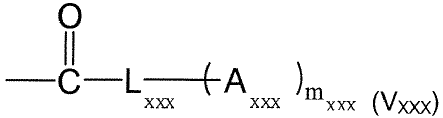

According to this invention, in Formula (V_{XXX}), m_{XXX} is an integer in the range of from 1 to 4, preferably 1.

According to this invention, in Formula (V_{XXX}), the group L_{XXX} is selected from the group consisting of an optionally substituted m_{XXX}+1 valent C₂ to C19-m_{XXX} linear or branched alkyl, an optionally substituted m_{XXX}+1 valent C₃ to C19-m_{XXX} linear or branched hetero-alkyl and a group represented by 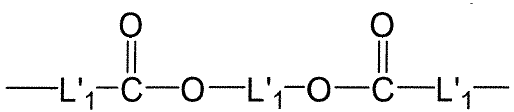 (hereinafter referred to as Formula (V_{XXX}-A)), preferably selected from the group consisting of an optionally substituted m_{XXX}+1 valent C₂ to C19-m_{XXX} linear or branched alkyl and a group represented by Formula (V_{XXX}-A), more preferably selected from the group consisting of an optionally substituted m_{XXX}+1 valent C₂ to C19-m_{XXX} linear or branched alkyl.

According to this invention, in Formula (V_{XXX}), in the definition of the group L_{XXX}, as the optionally substituted m_{XXX}+1 valent C₂ to C19-m_{XXX} linear or branched alkyl, preferably an optionally substituted m_{XXX}+1 valent C₂ to C9-m_{XXX} linear or branched alkyl, more preferably an optionally substituted m_{XXX}+1 valent C₂₋₆ linear or branched alkyl.

According to this invention, in Formula (V_{XXX}), in the definition of the group L_{XXX}, as the optionally substituted L_{XXX}+1 valent C₃ to C19-m_{XXX} linear or branched hetero-alkyl, preferably an optionally substituted m_{XXX}+1 valent C₃ to C9-m_{XXX} linear or branched hetero-alkyl, more preferably an optionally substituted m_{XXX}+1 valent C₃₋₆ linear or branched hetero-alkyl.

According to this invention, in Formula (V_{XXX}), in the definition of the group L_{XXX}, by "optionally substituted", it refers to optionally substituted by one or more (for example, from 1 to 4, from 1 to 3, from 1 to 2, or 1) substituent selected from the group consisting of a C₁₋₂₀ hydrocarbyl and a C₃₋₂₀ linear or branched hetero-alkyl. As the substituent, preferably a C₁₋₂₀ linear or branched alkyl, a C₆₋₂₀ aryl or a combination group thereof. As the C₁₋₂₀ linear or branched alkyl, preferably a C₁₋₁₀ linear or branched alkyl, more preferably a C₁₋₆ linear or branched alkyl, for example, methyl or ethyl.

According to this invention, in Formula (V_{XXX}), in the group represented by Formula (V_{XXX}-A), the plural L'₁s may be identical to or different from each other, each independently selected from the group consisting of a C₁₋₁₀ linear or branched alkyl and a C₃₋₁₀ linear or branched hetero-alkyl, preferably each independently selected from the group consisting of a C₁₋₆ linear or branched alkyl.

According to this invention, in Formula (V_{XXX}), as for the group represented by Formula (V_{XXX}-A), the requirement (1) is that: the plural L'₁s each independently represents a 2 valent to m_{XXX}+1 valent group, so as to make the group represented by Formula (V_{XXX}-A) to present as m_{XXX}+1 valent, as a whole. In this context, in the group represented by Formula (V_{XXX}-A), the valency between different L'₁ may vary and may be different from each other. In view of this, in the group represented by Formula (V_{XXX}-A), the group L'₁ is not specified for its valency. In fact, the valency of each L'₁ could be determined by the requirement (1). Specifically, assuming that each L'₁ is ethyl (with no valency specified), m_{XXX} is 2, then, the group represented by Formula (V_{XXX}-A) could be any one of the followings.

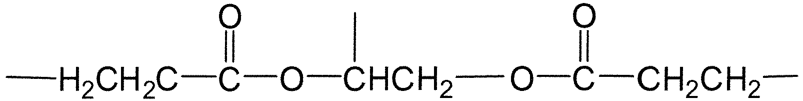

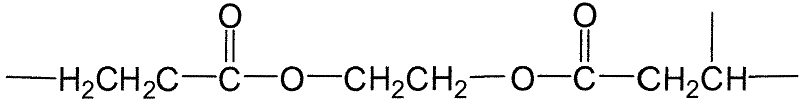

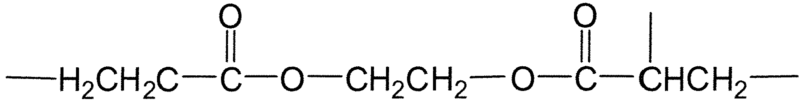

According to this invention, in Formula (V_{XXX}), in the group represented by Formula (V_{XXX}-A), the requirement (2) is that: the total atom number of all groups L'₁ (not figuring in any atom of the substituent that may exist on the group L'₁ as specified hereafter) is not more than 14, so as to make the group represented by Formula (V_{XXX}-A) to have a total atom number (not figuring in any atom of the substituent that may exist on the group L'₁ as specified hereafter) of no more than 18.

According to this invention, in Formula (V_{XXX}), in the group represented by Formula (V_{XXX}-A), after the aforesaid two requirements are met, the plural L'₁ may be each independently further optionally substituted by one or more (for example, from 1 to 4, from 1 to 3, from 1 to 2, or 1) substituent selected from the group consisting of a C₁₋₂₀ hydrocarbyl and a C₃₋₂₀ linear or branched hetero-alkyl. As the substituent, preferably a C₁₋₂₀ linear or branched alkyl, a C₆₋₂₀ aryl or a combination group thereof. As the C₁₋₂₀ linear or branched alkyl, preferably a C₁₋₁₀ linear or branched alkyl, more preferably a C₁₋₆ linear or branched alkyl, for example, methyl or ethyl.

According to this invention, in Formula (V_{XXX}), the plural A_{XXX}s may be identical to or different from each other, each independently selected from the group consisting of a group represented by Formula (V_{XXX}-1) and a group represented by Formula (V_{XXX}-2).

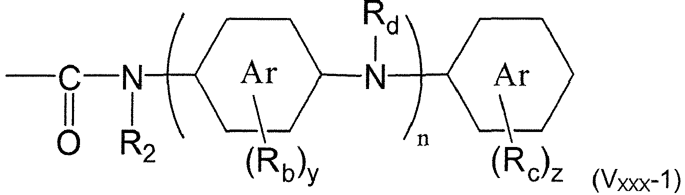

According to this invention, in Formula (V_{XXX}-1), the group R₂ is selected from the group consisting of hydrogen, a C₁₋₂₀ linear or branched alkyl, a group represented by Formula (IV) and a group represented by Formula (V) (especially a group represented by Formula (V_{XXX})).

According to an embodiment of this invention, in Formula (V_{XXX}-1), the group R₂ is selected from the group consisting of hydrogen, a C₁₋₁₀ linear or branched alkyl and a group represented by Formula (IV).

According to this invention, in Formula (V_{XXX}-1), when plural exist, the plural R_{b}s may be identical to or different from each other, each independently selected from the group consisting of hydrogen and a C₁₋₂₀ linear or branched alkyl, preferably each independently selected from the group consisting of hydrogen and a C₁₋₁₀ linear or branched alkyl.

According to an embodiment of this invention, in Formula (V_{XXX}-1), when plural exist, the plural R_{b}s may be identical to or different from each other, each independently selected from the group consisting of hydrogen and a C₁₋₆ linear or branched alkyl, preferably hydrogen.

According to this invention, in Formula (V_{XXX}-1), when plural exist, the plural R_{c}s may be identical to or different from each other, each independently selected from the group consisting of hydrogen, a C₁₋₂₀ linear or branched alkyl and a C₁₋₂₀ linear or branched alkyloxy.

According to an embodiment of this invention, in Formula (V_{XXX}-1), when plural exist, the plural R_{c}s may be identical to or different from each other, each independently selected from the group consisting of hydrogen, a C₁₋₁₀ linear or branched alkyl and a C₁₋₁₀ linear or branched alkyloxy.

According to an embodiment of this invention, in Formula (V_{XXX}-1), preferably one of the R_{c} locates at the position opposite to a N atom (i.e. the N atom bonding to the group R_{d}) on the ring

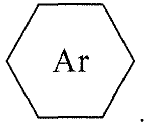

According to this invention, in Formula (V_{XXX}-1), y is an integer in the range of from 0 to 3, preferably 0 or 1.

According to this invention, in Formula (V_{XXX}-1), z is an integer in the range of from 0 to 3, preferably 0 or 1.

According to this invention, in Formula (V_{XXX}-1), n is an integer in the range of from 1 to 8, preferably 1 or 2.

According to this invention, in Formula (V_{XXX}-1), when plural exist, the plural R_{d}s may be identical to or different from each other, each independently selected from the group consisting of hydrogen and a group represented by Formula (V) (especially a group represented by Formula (V_{XXX})), preferably hydrogen.

According to this invention, in Formula (V_{XXX}-1), when plural exist, the plural rings 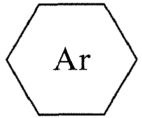 (in Formula (V_{XXX}-1) as a bivalent group when locating inside thereof) may be identical to or different from each other, each independently selected from the group consisting of benzene ring (preferably in Formula (V_{XXX}-1) as 1,4-phenylene when locating inside thereof) and naphthalene ring (preferably in Formula (V_{XXX}-1) as 1,4- or 2,6-naphthylene when locating inside thereof), preferably benzene ring.

According to an embodiment of this invention, in Formula (V_{XXX}-1), two adjacent rings 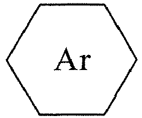 may optionally with an additional S atom (not shown in the formula) and the N atom bridging these two rings (i.e. the N atom bonding to the group R_{d}) to form a phenothiazine ring, i.e.

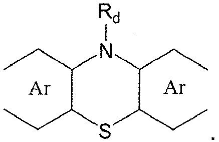

According to this embodiment of this invention, in Formula (V_{XXX}-1), when exist two or more rings 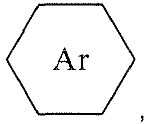 it is acceptable as long as at least two (adjacent) rings 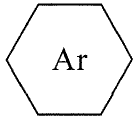 to form the phenothiazine ring, it is not necessary for each of the rings 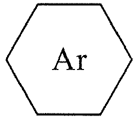 to form the phenothiazine ring with its adjacent ring

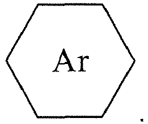

According to an embodiment of this invention, in Formula (V_{XXX}-1), two adjacent rings 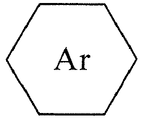 may optionally with an additional group represented by 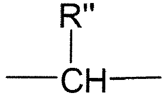 (not shown in the formula) and the N atom bridging these two rings (i.e. the N atom bonding to the group R_{d}) to form a 9,10-dihydroacridine ring, i.e.

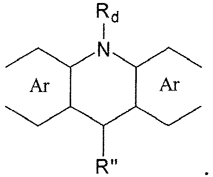

According to this invention, in the definition of the group 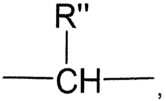 the group R" is selected from the group consisting of hydrogen, a C₁₋₂₀ hydrocarbyl (preferably a C₁₋₂₀ linear or branched alkyl) and a C₃₋₂₀ linear or branched hetero-alkyl.

According to an embodiment of this invention, in the definition of the group 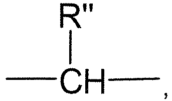 the group R" is selected from the group consisting of hydrogen and a C₁₋₁₀ linear or branched alkyl.

According to this embodiment of this invention, in Formula (V_{XXX}-1), when exist two or more rings 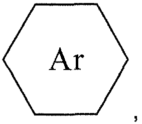 it is acceptable as long as at least two (adjacent) rings 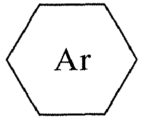 form the 9,10-dihydroacridine ring, it is not necessary for each of the rings 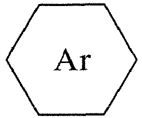 to form the 9,10-dihydroacridine ring with its adjacent ring

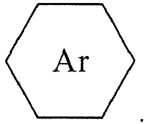

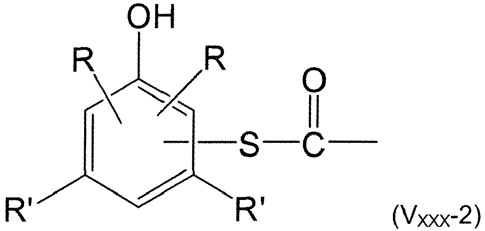

According to this invention, in Formula (V_{XXX}-2), when plural exist, the plural Rs may be identical to or different from each other, each independently selected from the group consisting of hydrogen, a C₁₋₃₀₀ linear or branched alkyl, a group represented by Formula (II) (especially a group represented by Formula (II_{XXX})) and a group represented by Formula (III).

According to this invention, in Formula (V_{XXX}-2), two Rs and one group 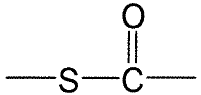 occupy the rest three positions on the benzene ring respectively.

According to an embodiment of this invention, in Formula (V_{XXX}-2), when plural exist, the plural Rs may be identical to or different from each other, each independently selected from the group consisting of hydrogen and a C₁₋₃₀₀ linear or branched alkyl.

According to this invention, in Formula (V_{XXX}-2), as the C₁₋₃₀₀ linear or branched alkyl, exemplified could be a C₁₋₂₀ linear or branched alkyl (preferably a C₁₋₁₀ linear or branched alkyl, more preferably a C₁₋₄ linear or branched alkyl) or a polyolefin group. As the polyolefin group, further exemplified could be a polyolefin group having a number-average molecular weight (Mn) of from 300 to 3000. Herein, the number-average molecular weight (Mn) of the polyolefin group is preferably in the range of from 500 to 2000, more preferably in the range of from 500 to 1500. According to this invention, the polyolefin group has a (substantially) saturated structure (in the form of a linear long-chain alkyl), however, depending on the nature of the starting polyolefin or the process for producing the starting polyolefin, there may exist some (only a small amount of) ethylenic double bond (for example, remained from or introduced during production of the polyolefin) along the molecular chain of the polyolefin group, which will not adversely interfere with the effects anticipated by this invention. For this reason, this invention does not intend to specify at what level this amount of ethylenic double bond could be, but rather, still identifies a polyolefin group of this kind as an "alkyl".

According to an embodiment of this invention, in Formula (V_{XXX}-2), there exist plural Rs, wherein one of the Rs is the polyolefin group, the rest one is selected from the group consisting of hydrogen or a C₁₋₂₀ linear or branched alkyl (preferably a C₁₋₁₀ linear or branched alkyl, more preferably a C₁₋₄ linear or branched alkyl).

According to an embodiment of this invention, in Formula (V_{XXX}-2), when the group R is the polyolefin group, the polyolefin group preferably locates at the position opposite to the phenol hydroxyl in Formula (V_{XXX}-2)

According to this invention, in Formula (V_{XXX}-2), when plural exist, the plural R's may be identical to or different from each other, each independently selected from the group consisting of hydrogen and a C₁₋₂₀ linear or branched alkyl.

According to an embodiment of this invention, in Formula (V_{XXX}-2), when plural exist, the plural R's may be identical to or different from each other, each independently selected from the group consisting of hydrogen and a C₁₋₄ linear or branched alkyl.

According to this invention, the hindered phenol compound, as aforesaid defined (for example, illustrated by Formula (I) or further by Formula (II-2) or further by Formula (V-2) etc.), necessarily comprises the structure 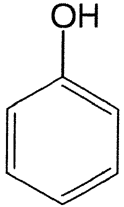 as the phenol unit.

According to a preferred embodiment of this invention, the hindered phenol compound, throughout its molecular structure, comprises at most 5, at most 3, at most 2 or at most 1 of the phenol unit.

According to this invention, the hindered phenol compound, as aforesaid defined (for example, illustrated by Formula (II-1) or further by Formula (III), Formula (IV) or Formula (V-1) etc.), necessarily comprises the structure 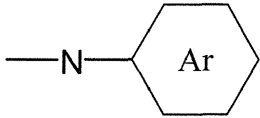 as the amine unit. According to a preferred embodiment of this invention, the hindered phenol compound, throughout its molecular structure, comprises at most 15, at most 10, at most 8, at most 6, at most 4, at most 3 or at most 2 of the amine unit. According to this invention, the hindered phenol compound, throughout its molecular structure, preferably at least one (preferably 2, 3 or 4 or more) group R_{d} is hydrogen. Specifically, the hindered phenol compound preferably, throughout its molecular structure, comprise at least one selected from the group consisting of the following hydroamine unit (1), the following hydroamine unit (2) and the following hydroamine unit (3).

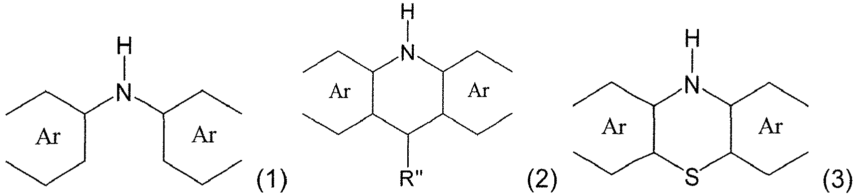

According to this invention, in these hydroamine units, as aforesaid, the group R" is selected from the group consisting of hydrogen, a C₁₋₂₀ hydrocarbyl (preferably a C₁₋₂₀ linear or branched alkyl) and a C₃₋₂₀ linear or branched hetero-alkyl, preferably selected from the group consisting of hydrogen, a C₁₋₁₀ linear or branched alkyl and a C₃₋₁₀ linear or branched hetero-alkyl, more preferably selected from the group consisting of hydrogen and a C₁₋₁₀ linear or branched alkyl, while the ring 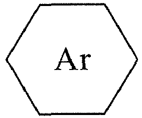 is selected from the group consisting of benzene ring and naphthalene ring, preferably benzene ring.

According to a preferred embodiment of this invention, the hindered phenol compound, throughout its molecular structure, comprises at least one hydroamine unit (1). Specifically, taking the hindered phenol compound 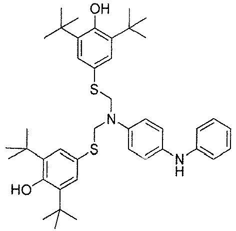 as an example, this compound, throughout its molecular structure, comprises 2 phenol units and 2 amine units, and at the same time, comprises one hydroamine unit (1). Or, taking the hindered phenol compound 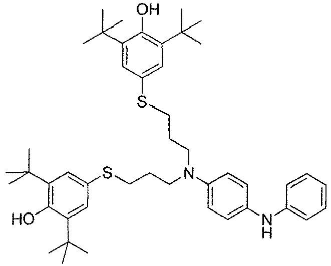 as an example, this compound, throughout its molecular structure, comprises 2 phenol units and 2 amine units, and at the same time, comprises one hydroamine unit (1). Or, taking the hindered phenol compound 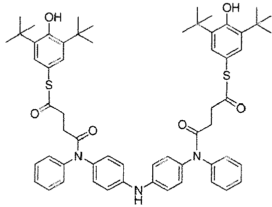 as an example, this compound, throughout its molecular structure, comprises 2 phenol units and 3 amine units, and at the same time, comprises one hydroamine unit (1).

According to this invention, as the hindered phenol compound, exemplified could be any of the following specific compounds or their mixture at any ratio therebetween, but this invention is not limited thereto.

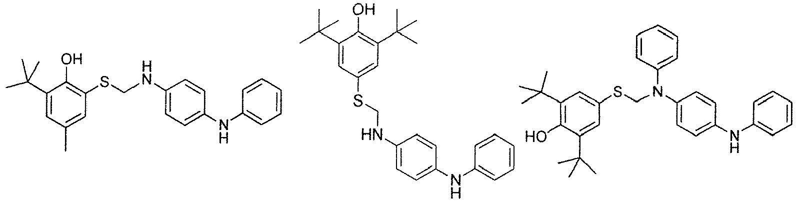

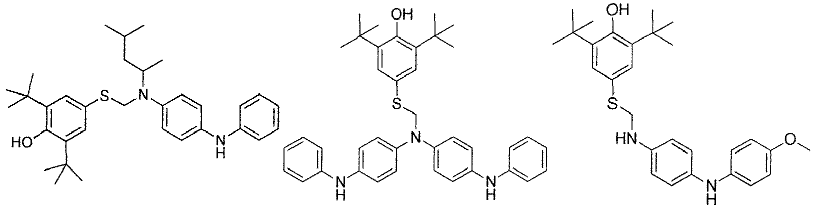

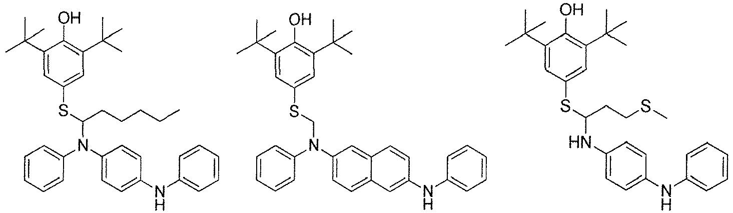

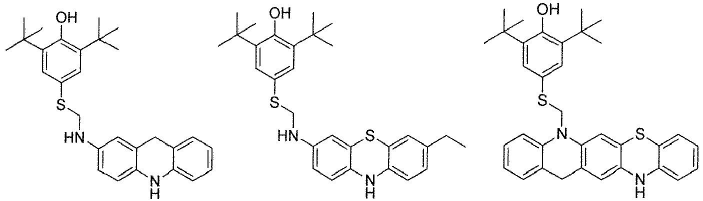

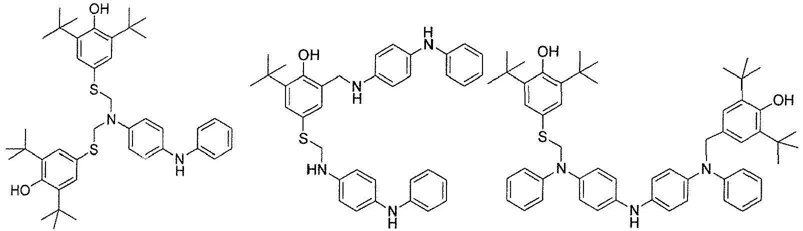

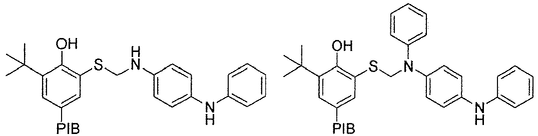

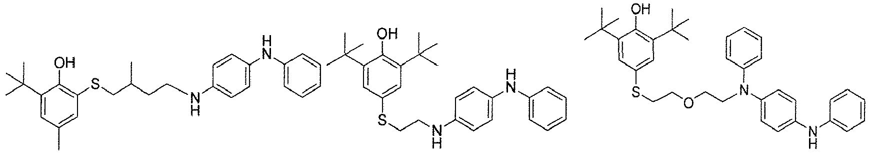

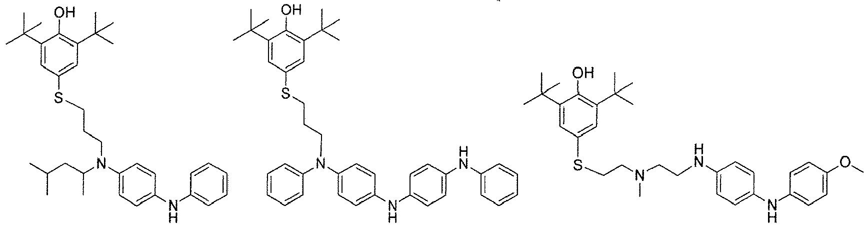

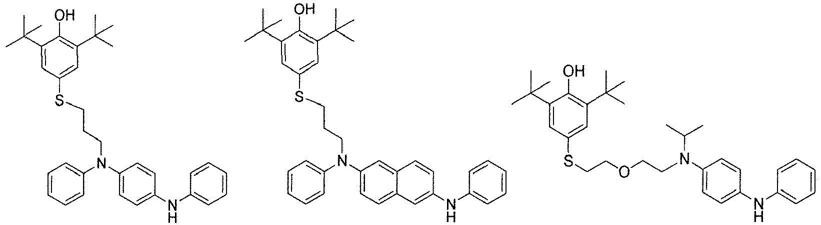

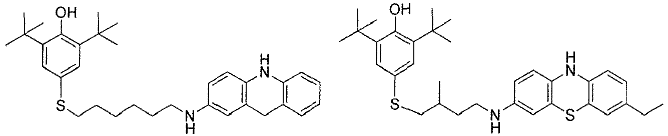

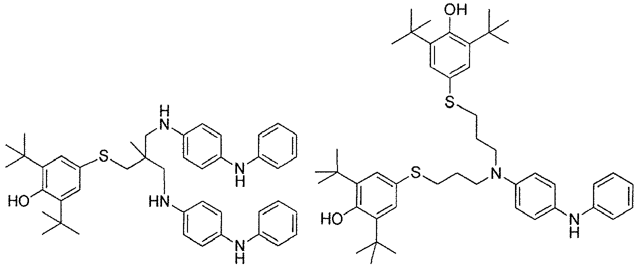

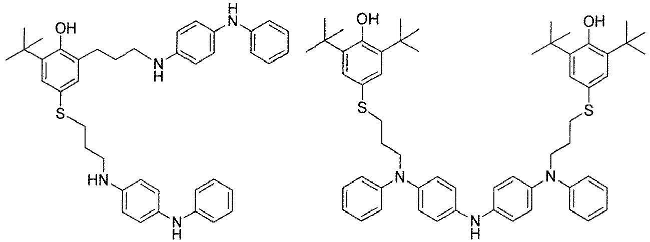

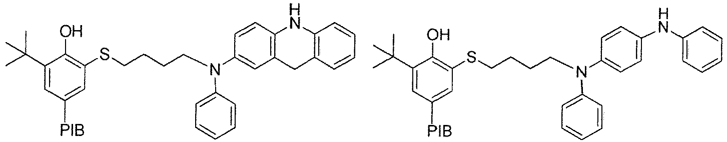

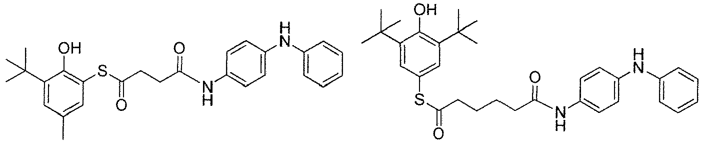

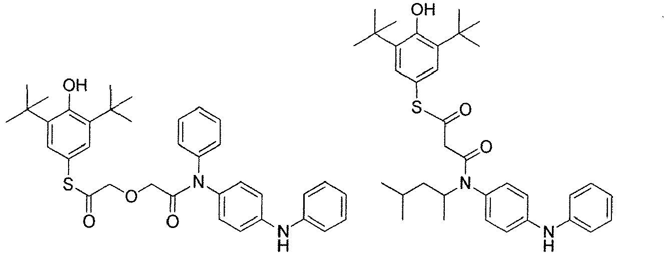

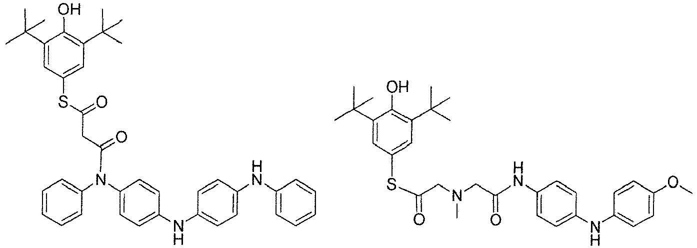

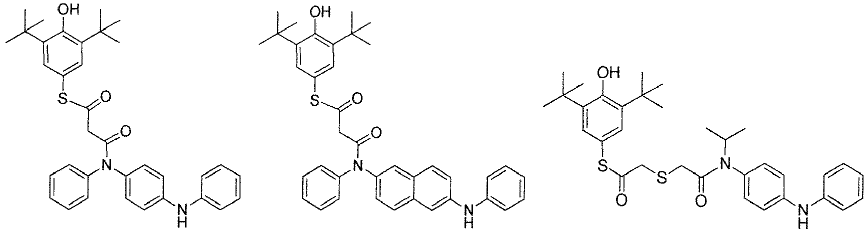

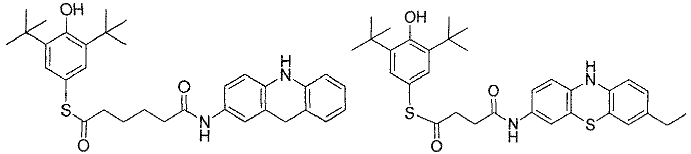

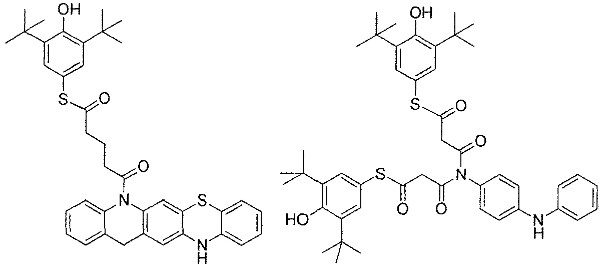

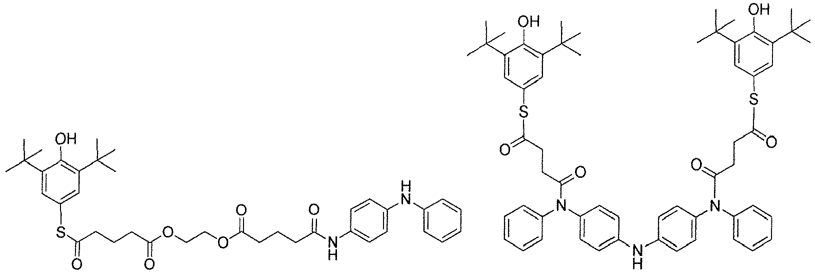

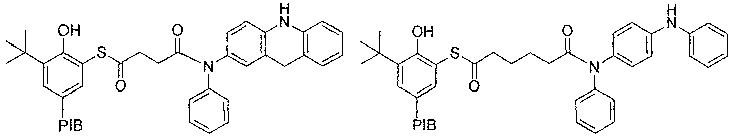

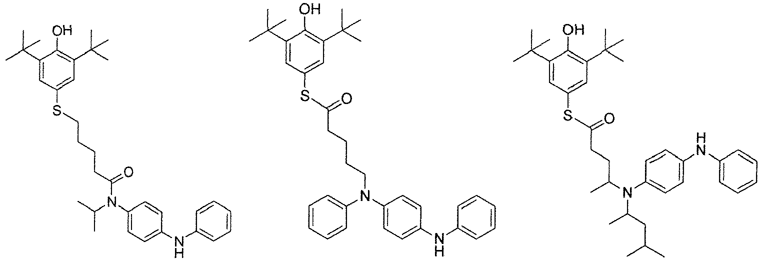

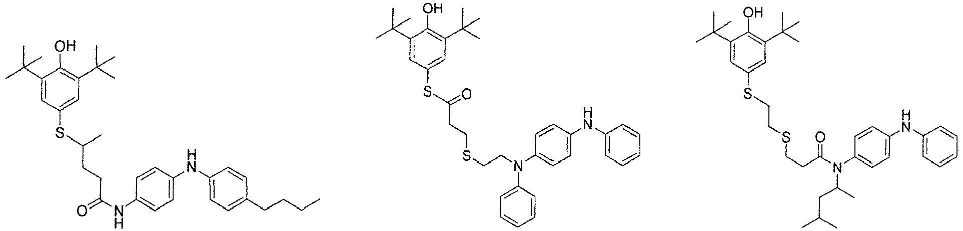

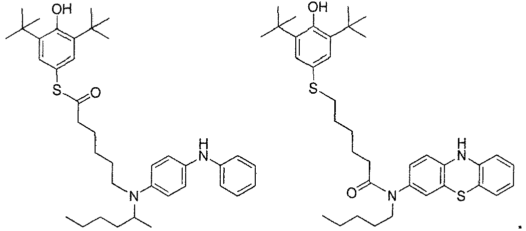

According to this invention, the aforesaid hindered phenol compound represented by Formula (I) could be present, produced or used as individual (pure) compounds, or as a mixture (at any ratio therebetween) thereof.

According to this invention, the hindered phenol compound represented by Formula (I) could be produced by the following process, but not limited thereto.

According to this invention, the process includes a first step of having a phenol compound represented by Formula (X) to react with an amine compound represented by Formula (Y) in the presence of at least one bridging compound selected from the group consisting of a compound represented by Formula (A) and formaldehyde, hereinafter referred to as bridging reaction.

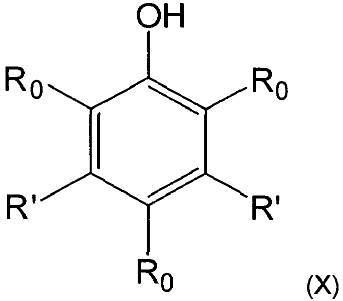

According to this invention, in Formula (X), when plural exist, the plural R₀s may be identical to or different from each other, each independently selected from the group consisting of -SH and a C₁₋₃₀₀ linear or branched alkyl, with the proviso that at least one R₀ is -SH.

According to this invention, in Formula (X), as the C₁₋₃₀₀ linear or branched alkyl, exemplified could be a C₁₋₂₀ linear or branched alkyl (preferably a C₁₋₁₀ linear or branched alkyl, more preferably a C₁₋₄ linear or branched alkyl) or a polyolefin group. As the polyolefin group, further exemplified could be a polyolefin group having a number-average molecular weight (Mn) of from 300 to 3000. Herein, the number-average molecular weight (Mn) of the polyolefin group is preferably in the range of from 500 to 2000, more preferably in the range of from 500 to 1500. According to this invention, the polyolefin group has a (substantially) saturated structure (in the form of a linear long-chain alkyl), however, depending on the nature of the starting polyolefin or the process for producing the starting polyolefin, there may exist some (only a small amount of) ethylenic double bond (for example, remained from or introduced during production of the polyolefin) along the molecular chain of the polyolefin group, which will not adversely interfere with the effects anticipated by this invention. For this reason, this invention does not intend to specify at what level this amount of ethylenic double bond could be, but rather, still identifies a polyolefin group of this kind as an "alkyl".

According to an embodiment of this invention, in Formula (X), there exist plural R₀s, wherein one of the R₀s is -SH, one of the other two R₀s is the polyolefin group, the rest one is a C₁₋₂₀ linear or branched alkyl (preferably a C₁₋₁₀ linear or branched alkyl).

According to an embodiment of this invention, in Formula (X), the group R₀ is the polyolefin group, and the polyolefin group preferably locates at the position opposite to the phenol hydroxyl in Formula (X).

According to this invention, in Formula (X), when plural exist, the plural R's may be identical to or different from each other, each independently selected from the group consisting of hydrogen and a C₁₋₂₀ linear or branched alkyl.

According to an embodiment of this invention, in Formula (X), when plural exist, the plural R's may be identical to or different from each other, each independently selected from the group consisting of hydrogen and a C₁₋₄ linear or branched alkyl.

According to this invention, the phenol compound represented by Formula (X) could be commercially available, or could be produced in line with any process known in this field, without any specific limitation thereto. Further, as the phenol compound represented by Formula (X), one kind or a mixture of two or more kinds at any ratio therebetween could be used.

According to this invention, as a process for producing the phenol compound represented by Formula (X), exemplified could be a process including a step of in the presence of an alkylation catalyst, reacting a phenol compound represented by Formula (X') with a polyolefin (having a number-average molecular weight (Mn) in the range of from 300 to 3000, preferably in the range of from 500 to 2000, more preferably in the range of from 500 to 1500), hereinafter referred to as alkylation reaction.

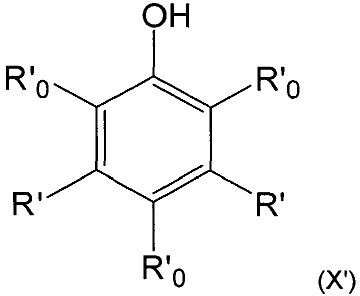

According to this invention, in Formula (X'), the group R' is as defined in Formula (X).

According to this invention, there are three R'₀s in Formula (X'), wherein one of the R'₀ is a group represented by -SH, the rest of R'₀ is -SH or a C₁₋₂₀ linear or branched alkyl (preferably a C₁₋₁₀ linear or branched alkyl).

According to this invention, the polyolefin may be a polyolefin obtained by the homopolymerization of ethylene, propylene or a C₄-C₁₀ α-olefin or that obtained by the copolymerization between two or more of these olefins. As the C₄-C₁₀ α-olefin, exemplified could be n-butene, iso-butene, n-pentene, n-hexene, n-octene and n-decene.

According to this invention, in the polyolefin, at least 20wt% (preferably at least 50wt%, more preferably at least 70wt%) of the polymer chain have an ethylenic double bond at its terminal. The ethylenic double bond generally takes the form of (highly reactive) vinylidene or vinyl.

According to this invention, as the polyolefin, more preferably polybutene. Unless otherwise specified, the term "polybutene" herein includes any polymer obtained by the homopolymerization of 1-butene or isobutene, or that obtained by the copolymerization of two or more selected from the group consisting of 1-butene, 2-butene, and isobute The commercial available polymer of this kind may further contain some amount of other olefin units, which is acceptable to this invention.

According to this invention, as the polyolefin, more preferably polyisobutene (PIB), also referred to as highly reactive polyisobutene. In the polyisobutene of this kind, at least 20wt% (preferably at least 50wt%, more preferably at least 70wt%) of the total terminal ethylenic double bond is provided by methyl vinylidene.

According to this invention, as the alkylation catalyst, exemplified could be a Lewis acid catalyst, for example, one or more selected from the group consisting of aluminim trichloride, boron trifluoride, tin tetrachloride, titanium tetrabromide, boron trifluoride ·phenol, boron trifluoride · alcohol complex and boron trifluoride · ether complex, preferably boron trifluoride · diethyl ether complex and/or boron trifluoride · methanol complex. These alkylation catalysts are commercially available.

According to this invention, in the alkylation reaction, molar ratio between the polyolefin, the phenol compound represented by Formula (X') and the alkylation catalyst could be for example, 1 : 1-3 : 0.1-0.5, preferably 1 : 1.5-3 : 0.1-0.4, more preferably 1 : 1.5-3 : 0.2-0.4, but not limited thereto.

According to this invention, the duration of the alkylation reaction could be for example, 0.5h-10h, preferably 1h-8h, more preferably 3h-5h, but not limited thereto.

According to this invention, the reaction temperature of the alkylation reaction could be for example, 0 degrees Celsius to 200 degrees Celsius, preferably 10 degrees Celsius to 150 degrees Celsius, more preferably 20 degrees Celsius to 100 degrees Celsius, but not limited thereto.

According to this invention, the alkylation reaction could be carried out in the presence of a solvent. As the solvent, exemplified could be a C₆₋₁₀ alkane (for example, hexane, heptane, octane, nonane or decane), preferably hexane and heptane, more preferably hexane.

According to this invention, upon completion of the alkylation reaction, after removing the alkylation catalyst, unreacted reactants and any solvent by a conventional way from the resultant reaction mixture, a phenol compound represented by Formula (X) will be obtained.

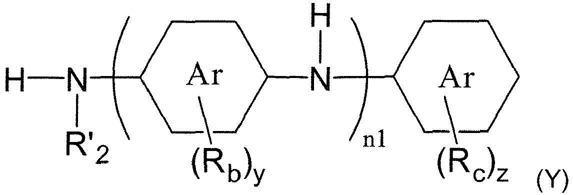

According to this invention, in Formula (Y), the group R'₂ is selected from the group consisting of hydrogen, a C₁₋₂₀ linear or branched alkyl and a group represented by

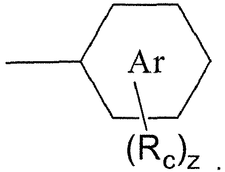

According to this invention, in Formula (Y), when plural exist, the plural R_{b}s may be identical to or different from each other, each independently selected from the group consisting of hydrogen and a C₁₋₂₀ linear or branched alkyl, preferably each independently selected from the group consisting of hydrogen and a C₁₋₁₀ linear or branched alkyl.

According to an embodiment of this invention, in Formula (Y), when plural exist, the plural R_{b}s may be identical to or different from each other, each independently selected from the group consisting of hydrogen and a C₁₋₆ linear or branched alkyl, preferably hydrogen.

According to this invention, in Formula (Y), when plural exist, the plural R_{c}s may be identical to or different from each other, each independently selected from the group consisting of hydrogen, a C₁₋₂₀ linear or branched alkyl and a C₁₋₂₀ linear or branched alkyloxy.

According to an embodiment of this invention, in Formula (Y), when plural exist, the plural R_{c}s may be identical to or different from each other, each independently selected from the group consisting of hydrogen, a C₁₋₁₀ linear or branched alkyl and a C₁₋₁₀ linear or branched alkyloxy.

According to an embodiment of this invention, in Formula (Y), preferably one of the R_{c} locates at the position opposite to a N atom (i.e. the N atom bonding to the group R_{d}) on the ring

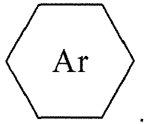

According to this invention, in Formula (Y), y is an integer in the range of from 0 to 3, preferably 0 or 1.

According to this invention, in Formula (Y), z is an integer in the range of from 0 to 3, preferably 0 or 1.

According to this invention, in Formula (Y), n1 is an integer in the range of from 1 to 8, preferably 1 or 2.

According to this invention, in Formula (Y), when plural exist, the plural rings 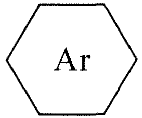 (in Formula (Y) as a bivalent group when locating inside thereof) may be identical to or different from each other, each independently selected from the group consisting of benzene ring (preferably in Formula (Y) as 1,4-phenylene when locating inside thereof) and naphthalene ring (preferably in Formula (Y) as 1,4- or 2,6-naphthylene when locating inside thereof), preferably benzene ring.

According to this invention, as the amine compound represented by Formula (Y), any commercial available one could be used, without any specific limitation thereto. Further, as the amine compound represented by Formula (Y), one kind or a mixture of two or more kinds at any ratio therebetween could be used.

According to an embodiment of this invention, a compound represented by Formula (A) is used as the bridging compound.

R_{f}(̵Fun)ₘ₁ (A)

According to an embodiment of this invention, in Formula (A), when m1 is an integer in the range of from 2 to 5 (preferably 2), the plural Funs may be identical to or different from each other, each independently selected from the group consisting of a halogen atom, a carboxylic acid residue group, an anhydride residue group and an aldehyde residue group.

According to an embodiment of this invention, in Formula (A), when m1 is 1, the group Fun is an aldehyde residue group.

According to this invention, in Formula (A), the group R_{f} is selected from the group consisting of an optionally substituted m1 valent C₁₋₂₀ hydrocarbyl (preferably a C₁₋₂₀ linear or branched alkyl) and an optionally substituted m1 valent C₃₋₂₀ linear or branched hetero-alkyl.

According to this invention, in Formula (A), in the definition of the group R_{f}, by optionally substituted, it refers to optionally substituted by one or more (for example, from 1 to 5, from 1 to 4, from 1 to 3, from 1 to 2, or 1) substituent selected from the group consisting of oxo (i.e. 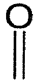 ), a C₁₋₂₀ hydrocarbyl and a C₃₋₂₀ linear or branched hetero-alkyl. As the substituent, preferably oxo, a C₁₋₂₀ linear or branched alkyl, a C₆₋₂₀ aryl or a combination group thereof. As the C₁₋₂₀ linear or branched alkyl, preferably a C₁₋₁₀ linear or branched alkyl, more preferably a C₁₋₆ linear or branched alkyl, for example, methyl or ethyl.

According to this invention, in Formula (A), in the definition of the group R_{f}, when oxo exists as the substituent, it is preferred that at least one oxo exists on the carbon atom directly bonding to the group Fun, or, in the definition of the group R_{f}, at least one or m1 carbon atom(s) directly bonding to the group Fun has oxo as the substituent, whereby making the carbon atom present in the form of carbonyl (i.e. 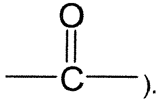 For example, assuming that R_{f} is -CH₂-CH₂-CH₂- (m1=2), both groups Fun are Cl, when one oxo exists as the substituent, the comound represented by Formula (A) could be 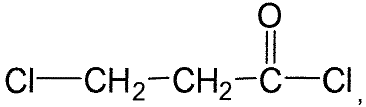 when two oxo exist as the substituent, the compound represented by Formula (A) could be 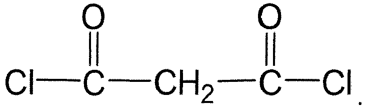 In the context of this specification, by "carboxylic acid residue group " it refers to a group (i.e. -OH) obtained by removing one carbonyl (i.e. 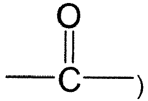 from carboxyl (i.e. -COOH), with the proviso that it is necessary for the carbon atom directly bonding to the group to take the form of carbonyl (i.e. 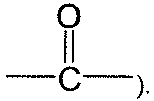 Specifically, assuming that R_{f} is -CH₂-CH₂-CH₂- (m1=2), one Fun is Cl, one Fun is a carboxylic acid residue group, the compound represented by Formula (A) could be

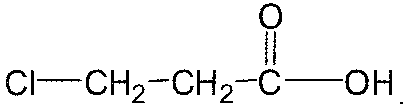

In the context of this specification, by "aldehyde residue group " it refers to a group (i.e. -H) obtained by removing one carbonyl (i.e. 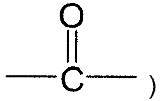 from formyl (i.e. 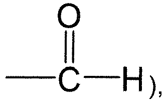 with the proviso that it is necessary for the carbon atom directly bonding to the group to take the form of carbonyl (i.e. 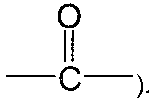 Specifically, assuming that R_{f} is -CH₂-CH₂-CH₂- (m1=2), one Fun is Cl, one Fun is an aldehyde residue group, the compound represented by Formula (A) could be

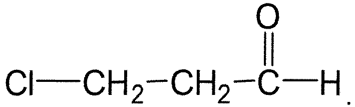

In the context of this specification, by "anhydride residue group" it refers to a group (i.e. -O-) obtained by removing two carbonyls (i.e. 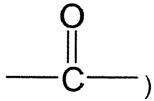 from anhydride (i.e. 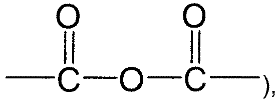 with the proviso that it is necessary for the two carbon atoms directly bonding to the group to take the form of carbonyl (i.e. 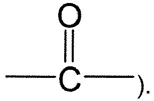 Herein, the two carbon atoms could originate from one single compound, or from different compounds. Specifically, assuming that R_{f} is -CH₂-CH₂-CH₂- (m1=2), one Fun is Cl, one Fun is an anhydride residue group, the compound represented by Formula (A) could be

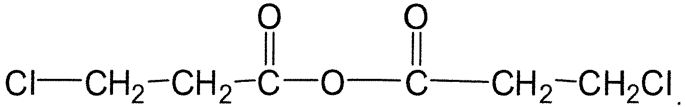

According to an embodiment of this invention, formaldehyde is used as the bridging compound. As the formaldehyde, for example, an aqueous solution thereof could be used, or in the form of paraformaldehyde or polyformaldehyde, without any specific limitation thereto.

According to this invention, as the bridging compound, any commercial available one could be used, without any specific limitation thereto. Further, as the bridging compound, one kind or a mixture of two or more kinds at any ratio therebetween could be used.

According to an embodiment of this invention, the bridging compound is selected from the group consisting of an aldehyde compound represented by Formula (A_{Y}), a polyhalo-compound represented by Formula (A_{YY}) and a polybasic carboxylic acid represented by Formula (A_{YYY}) or derivatives thereof (selected from the group consisting of an anhydride of the polybasic carboxylic acid and an acyl halide of the polybasic carboxylic acid). As the bridging compound, any commercial available one could be used, without any specific limitation thereto. Further, as the bridging compound, one kind or a mixture of two or more kinds at any ratio therebetween could be used.

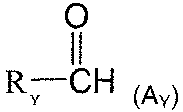

According to this invention, in Formula (A_{Y}), the group R_{Y} is selected from the group consisting of hydrogen, a C₁₋₂₀ hydrocarbyl (preferably a C₁₋₂₀ linear or branched alkyl) and a C₃₋₂₀ linear or branched hetero-alkyl.

According to an embodiment of this invention, in Formula (A_{Y}), the group R_{Y} is selected from the group consisting of hydrogen, a C₁₋₁₀ linear or branched alkyl and a C₃₋₁₀ linear or branched hetero-alkyl.

According to a preferred embodiment of this invention, in Formula (A_{Y}), the group R_{Y} is selected from the group consisting of hydrogen and a C₁₋₁₀ linear or branched alkyl. According to this invention, as the aldehyde compound represented by Formula (A_{Y}), exemplified could be a C₁₋₆ aliphatic aldehyde and benzaldehyde.

According to an embodiment of this invention, as the C₁₋₆ aliphatic aldehyde, exemplified could be a C₁₋₆ linear or branched saturated aliphatic aldehyde, specifically, acetaldehyde or formaldehyde, more preferably formaldehyde. As the formaldehyde, for example, an aqueous solution thereof could be used, or in the form of paraformaldehyde or polyformaldehyde, without any specific limitation thereto. According to this invention, as the aldehyde compound represented by Formula (A_{Y}), one kind or a mixture of two or more kinds at any ratio therebetween could be used.

Rₕₐₗₒ(̵Halo)_{m_{YY}} (A_{YY})

According to this invention, in Formula (A_{YY}), the group Rₕₐₗₒ is selected from the group consisting of an optionally substituted m_{YY} valent C₂₋₂₀ linear or branched alkyl and an optionally substituted m_{YY} valent C₃₋₂₀ linear or branched hetero-alkyl, preferably selected from the group consisting of an optionally substituted m_{YY} valent C₂₋₁₀ linear or branched alkyl and an optionally substituted m_{YY} valent C₃₋₁₀ linear or branched hetero-alkyl, more preferably selected from the group consisting of an optionally substituted m_{YY} valent C₂₋₆ linear or branched alkyl and an optionally substituted m_{YY} valent C₃₋₆ linear or branched hetero-alkyl, more preferably selected from the group consisting of an optionally substituted m_{YY} valent C₂₋₆ linear or branched alkyl.

According to this invention, in Formula (A_{YY}), in the definition of the group Rₕₐₗₒ, by optionally substituted, it refers to optionally substituted by one or more (for example, from 1 to 4, from 1 to 3, from 1 to 2, or 1) substituent selected from the group consisting of a C₁₋₂₀ hydrocarbyl and a C₃₋₂₀ linear or branched hetero-alkyl. As the substituent, preferably a C₁₋₂₀ linear or branched alkyl, a C₆₋₂₀ aryl or a combination group thereof. As the C₁₋₂₀ linear or branched alkyl, preferably a C₁₋₁₀ linear or branched alkyl, more preferably a C₁₋₆ linear or branched alkyl, for example, methyl or ethyl.

According to this invention, in Formula (A_{YY}), the group Halo is halo, including fluoro, chloro, bromo and iodo, preferably chloro.

According to this invention, in Formula (A_{YY}), m_{YY} is an integer in the range of from 2 to 5, preferably 2.

According to this invention, as the polyhalo-compound represented by Formula (A_{YY}), one kind or a mixture of two or more kinds at any ratio therebetween could be used.

R_{L}(̵COOH)_{m_{YYY}} (A_{YYY})

According to this invention, in Formula (A_{YYY}), m_{YYY} is an integer in the range of from 2 to 5, preferably 2.

According to this invention, in Formula (A_{YYY}), the group R_{L} is selected from the group consisting of an optionally substituted m_{YYY} valent C₂ to C20-m_{YYY} linear or branched alkyl, an optionally substituted m_{YYY} valent C₃ to C20-m_{YYY} linear or branched hetero-alkyl and a group represented by 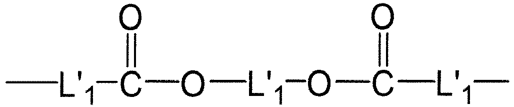 (hereinafter referred to as Formula (A_{YYY}-A)), preferably selected from the group consisting of an optionally substituted m_{YYY} valent C₂ to C20-m_{YYY} linear or branched alkyl and a group represented by Formula (A_{YYY}-A), more preferably selected from the group consisting of an optionally substituted m_{YYY} valent C₂ to C20-m_{YYY} linear or branched alkyl.

According to this invention, in Formula (A_{YYY}), in the definition of the group R_{L}, as the optionally substituted m_{YYY} valent C₂ to C20-m_{YYY} linear or branched alkyl, preferably an optionally substituted m_{YYY} valent C₂ to C10-m_{YYY} linear or branched alkyl, more preferably an optionally substituted m_{YYY} valent C₂₋₆ linear or branched alkyl.

According to this invention, in Formula (A_{YYY}), in the definition of the group R_{L}, as the optionally substituted m_{YYY} valent C₃ to C20-m_{YYY} linear or branched hetero-alkyl, preferably an optionally substituted m_{YYY} valent C₃ to C10-m_{YYY} linear or branched hetero-alkyl, more preferably an optionally substituted m_{YYY} valent C₃₋₆ linear or branched hetero-alkyl.

According to this invention, in Formula (A_{YYY}), in the definition of the group R_{L}, by "optionally substituted", it refers to optionally substituted by one or more (for example, from 1 to 4, from 1 to 3, from 1 to 2, or 1) substituent selected from the group consisting of a C₁₋₂₀ hydrocarbyl and a C₃₋₂₀ linear or branched hetero-alkyl. As the substituent, preferably a C₁₋₂₀ linear or branched alkyl, a C₆₋₂₀ aryl or a combination group thereof. As the C₁₋₂₀ linear or branched alkyl, preferably a C₁₋₁₀ linear or branched alkyl, more preferably a C₁₋₆ linear or branched alkyl, for example, methyl or ethyl.

According to this invention, in Formula (A_{YYY}), in the group represented by Formula (A_{YYY}-A), the plural L'₁s may be identical to or different from each other, each independently selected from the group consisting of a C₁₋₁₀ linear or branched alkyl and a C₃₋₁₀ linear or branched hetero-alkyl, preferably each independently selected from the group consisting of a C₁₋₆ linear or branched alkyl.

According to this invention, in Formula (A_{YYY}), as for the group represented by Formula (A_{YYY}-A), the requirement (1) is that: the plural L'₁s each independently represents a 2 valent to m_{YYY} valent group, so as to make the group represented by Formula (A_{YYY}-A) to present as m_{YYY} valent, as a whole. In this context, in the group represented by Formula (A_{YYY}-A), the valency between different L'₁ may vary and may be different from each other. In view of this, in the group represented by Formula (A_{YYY}-A), the group L'₁ is not specified for its valency. In fact, the valency of each L'₁ could be determined by the requirement (1). Specifically, assuming that each L'₁ is ethyl (with no valency specified), m_{YYY} is 3, then, the group represented by Formula (A_{YYY}-A) could be any one of the followings.

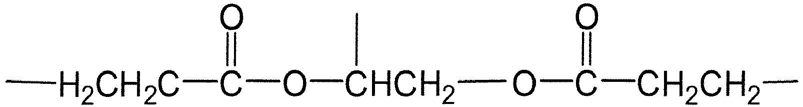

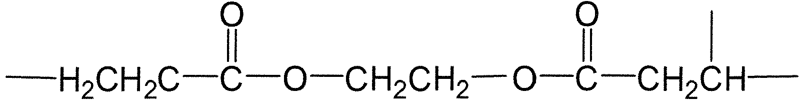

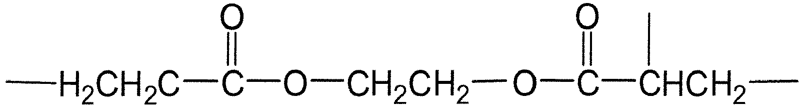

According to this invention, in Formula (A_{YYY}), in the group represented by Formula (A_{YYY}-A), the requirement (2) is that: the total atom number of all groups L'₁ (not figuring in any atom of the substituent that may exist on the group L'₁ as specified hereafter) is not more than 14, so as to make the group represented by Formula (A_{YYY}-A) to have a total atom number (not figuring in any atom of the substituent that may exist on the group L'₁ as specified hereafter) of no more than 18.

According to this invention, in Formula (A_{YYY}), in the group represented by Formula (A_{YYY}-A), after the aforesaid two requirements are met, the plural L'₁ may be each independently further optionally substituted by one or more (for example, from 1 to 4, from 1 to 3, from 1 to 2, or 1) substituent selected from the group consisting of a C₁₋₂₀ hydrocarbyl and a C₃₋₂₀ linear or branched hetero-alkyl. As the substituent, preferably a C₁₋₂₀ linear or branched alkyl, a C₆₋₂₀ aryl or a combination group thereof. As the C₁₋₂₀ linear or branched alkyl, preferably a C₁₋₁₀ linear or branched alkyl, more preferably a C₁₋₆ linear or branched alkyl, for example, methyl or ethyl.

According to this invention, as the derivative of the polybasic carboxylic acid represented by Formula (A_{YYY}), exemplified could be an anhydride or acyl halide of the polybasic carboxylic acid. As the acyl halide, exemplified could be acyl fluorides, acyl chlorides and acyl bromides, preferably acyl chlorides.

According to this invention, as the polybasic carboxylic acid represented by Formula (A_{YYY}) or derivatives thereof, one kind or a mixture of two or more kinds at any ratio therebetween could be used. Further, as the polybasic carboxylic acid represented by Formula (A_{YYY}) or derivatives thereof, any commercial available one could be used, without any specific limitation thereto.

According to this invention, in the process for producing the hindered phenol compound, in the first step, molar ratio of the phenol compound represented by Formula (X) to the amine compound represented by Formula (Y) is generally 1 : 0.1-10, preferably 1 : 0.5-5.0, more preferably 1 : 0.8-2.0.

According to this invention, in the process for producing the hindered phenol compound, in the first step, as a whole, molar ratio of the phenol compound represented by Formula (X) to the bridging compound is generally 1 : 0.1-10, preferably 1 : 0.2-5.0, more preferably 1 : 0.3-3.0.

According to this invention, there is no specific limitation as to how to conduct the bridging reaction in the first step, as long as the reaction between the phenol compound represented by Formula (X), the amine compound represented by Formula (Y) and the bridging compound will occur. To simplify description, in this specification, the aldehyde compound represented by Formula (A_{Y}) (First embodiment), the polyhalo-compound represented by Formula (A_{YY}) (Second embodiment) and the polybasic carboxylic acid represented by Formula (A_{YYY}) or derivatives thereof (Third embodiment) are exemplified as the bridging compound, to illustrate how to conduct the bridging reaction, however, this invention is not limited thereto. It is easy for a person skilled in the art to learn how to conduct the bridging reaction when using other compounds represented by Formula (A) as the bridging compound if enlightend by the various reaction procedures illustrated in the following embodiments, for example, by combining the reaction procedures specified in the following embodiments or the reaction steps involved therein in a suitable order.

### First embodiment

According to the first embodiment, to conduct the first step, a phenol compound represented by Formula (X) reacts with an amine compound represented by Formula (Y) in the presence of an aldehyde compound represented by Formula (A_{Y}) (hereinafter referred to as bridging reaction).

According to this invention, in the process for producing the hindered phenol compound, in the first step, molar ratio of the phenol compound represented by Formula (X) to the aldehyde compound represented by Formula (A_{Y}) is generally 1 : 0.1-10, preferably 1 : 0.5-5.0, more preferably 1 : 0.8-2.0.

According to this invention, in the process for producing the hindered phenol compound, the fist step could be conducted in the presence of a solvent. As the solvent, exemplified could be a C₂₋₁₀ aliphatic nitrile (for example, acetonitrile, etc.), a C₆₋₂₀ aromatic hydrocarbon (for example, benzene, toluene, xylene and cumene), a C₆₋₁₀ alkane (for example, n-hexane, cyclohexane and petroleum ether), a C₁₋₆ aliphatic alcohol (for example, methanol, ethanol, n-propanol, isopropanol, n-butanol and ethylene glycol), a C₂₋₂₀ halogenated hydrocarbon (for example, dichloromethane, carbon tetrachloride, chlorobenzene and 1,2-dichlorobenzene), a C₃₋₁₀ ketone (for example, acetone, butanone and methyl isobutyl ketone) or a C₃₋₁₀ amide (for example, dimethylfomamide, dimethylacetamide and N-methyl pyrrolidone), etc. As the solvent, one kind or a mixture of two or more kinds at any ratio therebetween could be used.

According to this invention, in the process for producing the hindered phenol compound, in the first step, a catalyst could be used, if needed. As the catalyst, exemplified could be an inorganic acid catalyst and an organic acid catalyst. As the inorganic acid catalyst, exemplified could be hydrochloric acid, sulphuric acid and phosphoric acid, etc. As the organic acid catalyst, exemplified could be methyl sulfonic acid, ethyl sulfonic acid, aminosulfonic acid and p-toluene sulfonic acid, etc.

As the amount of the catalyst to be used, any amount that conventionally used is acceptable, without any specific limitation thereto.

According to this invention, in the process for producing the hindered phenol compound, there is no specific limitation as to the reaction duration of the first step, but generally in the range of 0.1h-24h, preferably 0.2h-12h, more preferably 0.5h-6h.

According to this invention, in the process for producing the hindered phenol compound, there is no specific limitation as to the reaction temperature of the first step, but generally in the range of 0 degrees Celsius to 250 degrees Celsius, preferably 20 degrees Celsius to 180 degrees Celsius, more preferably 60 degrees Celsius to 120 degrees Celsius.

According to this invention, in the process for producing the hindered phenol compound, upon completion of the first step, by a separation method commonly known (for example, by evaporation, etc.), any volatile like solvent that may exist is removed from the reaction mixture obtained from the first step (hereinafter referred to as reaction mixture of the first step), then the resultant of the first step can be obtained.

### Second embodiment

According to the Second embodiment, to conduct the first step, a phenol compound represented by Formula (X) reacts with an amine compound represented by Formula (Y) in the presence of the polyhalo-compound represented by Formula (A_{YY}) (hereinafter referred to as bridging reaction).

According to this invention, in the process for producing the hindered phenol compound, in the first step, molar ratio of the phenol compound represented by Formula (X) to the polyhalo-compound represented by Formula (A_{YY}) is generally 1 : 0.1-10, preferably 1 : 0.2-5.0, more preferably 1 : 0.3-3.0.

According to this invention, in the process for producing the hindered phenol compound, the fist step could be conducted in the presence of a solvent. As the solvent, exemplified could be a C₂₋₁₀ aliphatic nitrile (for example, acetonitrile, etc.), a C₆₋₂₀ aromatic hydrocarbon (for example, benzene, toluene, xylene and cumene), a C₆₋₁₀ alkane (for example, n-hexane, cyclohexane and petroleum ether), a C₁₋₆ aliphatic alcohol (for example, methanol, ethanol, n-propanol, isopropanol, n-butanol and ethylene glycol), a C₂₋₂₀ halogenated hydrocarbon (for example, dichloromethane, carbon tetrachloride, chlorobenzene and 1,2-dichlorobenzene), a C₃₋₁₀ ketone (for example, acetone, butanone and methyl isobutyl ketone) or a C₃₋₁₀ amide (for example, dimethylfomamide, dimethylacetamide and N-methyl pyrrolidone), etc. As the solvent, preferably a C₆₋₂₀ aromatic hydrocarbon (for example, benzene, toluene, xylene and cumene), a C₃₋₁₀ ketone (for example, acetone, butanone and methyl isobutyl ketone) or a C₃₋₁₀ amide (for example, dimethylfomamide, dimethylacetamide and N-methyl pyrrolidone), etc. As the solvent, one kind or a mixture of two or more kinds at any ratio therebetween could be used.

According to this invention, in the process for producing the hindered phenol compound, in the first step, a catalyst could be used, if needed. As the catalyst, exemplified could be an alkaline catalyst. As the alkaline catalyst, exemplified could be a hydroxide of alkali metal, for example, NaOH and KOH, etc., or an alkali metal salt of weak acid, for example, sodium acetate, potassium acetate, sodium carbonate, potassium carbonate, sodium hydrogen carbonate and potassium hydrogen carbonate, etc. As the catalyst, one kind or a mixture of two or more kinds at any ratio therebetween could be used. As the amount of the catalyst to be used, any amount that conventionally used is acceptable, without any specific limitation thereto.

According to this invention, in the process for producing the hindered phenol compound, there is no specific limitation as to the reaction duration of the first step, but generally in the range of 0.1h-48h, preferably 0.2-12h, more preferably 0.5-6h.

According to this invention, in the process for producing the hindered phenol compound, there is no specific limitation as to the reaction temperature of the first step, but generally in the range of 0-200 degrees Celsius, preferably 20-180 degrees Celsius, more preferably 60-120 degrees Celsius.

According to this invention, in the process for producing the hindered phenol compound, upon completion of the first step, by a separation method commonly known (for example, by evaporation, etc.), any volatile like solvent that may exist is removed from the reaction mixture obtained from the first step (hereinafter referred to as reaction mixture of the first step), then the resultant of the first step can be obtained.

### Third embodiment

According to the Third embodiment, to conduct the first step, a phenol compound represented by Formula (X) reacts with an amine compound represented by Formula (Y) in the presence of a polybasic carboxylic acid represented by Formula (A_{YYY}) or derivatives thereof (hereinafter referred to as bridging reaction).

According to this invention, in the process for producing the hindered phenol compound, in the first step, molar ratio of the phenol compound represented by Formula (X) to the polybasic carboxylic acid represented by Formula (A_{YYY}) or derivatives thereof is generally 1 : 0.1-10, preferably 1 : 0.2-5.0, more preferably 1 : 0.3-3.0.

According to this invention, in the process for producing the hindered phenol compound, the fist step could be conducted in the presence of a solvent. As the solvent, exemplified could be a C₂₋₁₀ aliphatic nitrile (for example, acetonitrile, etc.) and a C₆₋₂₀ aromatic hydrocarbon (for example, benzene, toluene, xylene and cumene), etc. As the solvent, one kind or a mixture of two or more kinds at any ratio therebetween could be used.

According to this invention, in the process for producing the hindered phenol compound, in the first step, a catalyst could be used, if needed. Specifically, for a polybasic carboxylic acid represented by Formula (A_{YYY}), as the catalyst, exemplified could be an alkaline catalyst. As the alkaline catalyst, exemplified could be a hydroxide of alkali metal, for example, NaOH and KOH, etc., or an alkali metal salt of weak acid, for example, sodium acetate, potassium acetate, sodium carbonate, potassium carbonate, sodium hydrogen carbonate and potassium hydrogen carbonate, etc. As the catalyst, one kind or a mixture of two or more kinds at any ratio therebetween could be used. As the amount of the catalyst to be used, any amount that conventionally used is acceptable, without any specific limitation thereto. Further, for an anhydride of the polybasic carboxylic acid represented by Formula (A_{YYY}), as the catalyst, exemplified could be a transition metal salt, further exemplified could be cobalt dichloride and ruthenium trichloride, etc. As the catalyst, one kind or a mixture of two or more kinds at any ratio therebetween could be used. As the amount of the catalyst to be used, any amount that conventionally used is acceptable, without any specific limitation thereto.

According to this invention, in the process for producing the hindered phenol compound, there is no specific limitation as to the reaction duration of the first step, but generally in the range of 0.2h-12h, preferably 0.2-12h, more preferably 0.5-6h.

According to this invention, in the process for producing the hindered phenol compound, there is no specific limitation as to the reaction temperature of the first step, but generally in the range of 0-200 degrees Celsius, preferably 10-150 degrees Celsius, more preferably 20-120 degrees Celsius.

According to this invention, in the process for producing the hindered phenol compound, upon completion of the first step, by a separation method commonly known (for example, by evaporation, etc.), any volatile like solvent that may exist is removed from the reaction mixture obtained from the first step (hereinafter referred to as reaction mixture of the first step), then the resultant of the first step can be obtained.

According to an embodiment of this invention, the process for producing the hindered phenol compound optionally further includes an additional step of having the resultant of the first step to react with a sulfuring agent (to form a phenothiazine ring) (hereinafter referred to as additional step A).

According to an embodiment of this invention, the process for producing the hindered phenol compound optionally further includes an additional step of having the resultant of the first step to react with an aldehyde compound represented by Formula (Z) (to form a 9,10-dihydroacridine ring) (hereinafter referred to as additional step B).

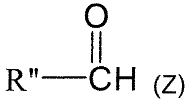

According to this invention, in Formula (Z), the group R" is selected from the group consisting of hydrogen, a C₁₋₂₀ hydrocarbyl (preferably a C₁₋₂₀ linear or branched alkyl) and a C₃₋₂₀ linear or branched hetero-alkyl.

According to an embodiment of this invention, in Formula (Z), the group R" is selected from the group consisting of hydrogen, a C₁₋₁₀ linear or branched alkyl and a C₃₋₁₀ linear or branched hetero-alkyl.

According to a preferred embodiment of this invention, in Formula (Z), the group R" is selected from the group consisting of hydrogen and a C₁₋₁₀ linear or branched alkyl. According to this invention, as the aldehyde compound represented by Formula (Z), exemplified could be a C₁₋₆ aliphatic aldehyde and benzaldehyde.

According to an embodiment of this invention, as the C₁₋₆ aliphatic aldehyde, exemplified could be a C₁₋₆ linear or branched saturated aliphatic aldehyde, specifically, acetaldehyde or formaldehyde, more preferably formaldehyde. As the formaldehyde, for example, an aqueous solution thereof could be used, or in the form of paraformaldehyde or polyformaldehyde, without any specific limitation thereto.

According to this invention, in the process for producing the hindered phenol compound, it is acceptable to conduct only the additional step A, or to conduct only the additional step B, or to conduct both the additional step A and the additional step B. In case that both the additional step A and the additional step B are conducted, there is no specific limitation as to the sequence or order in which the additional step A and the additional step B are conducted. It is acceptable to firstly conduct the additional step A, and after completion of the additional step A, to conduct the additional step B, or to firstly conduct the additional step B, and after completion of the additional step B, to conduct the additional step A. Further, there may be, or may not be, a product (i.e. the resultant obtained from the preceding additional step) isolation operation between two additional steps, without any specific limitation thereto.

According to an embodiment of this invention, in the process for producing the hindered phenol compound, the additional step A could be conducted after completion of the first step. When the additional step A is conducted after completion of the first step, it is acceptable for the resultant of the first step to have been isolated out of the reaction mixture of the first step as herein described, or without any isolation, to be directly used to conduct the additional step A in the form of the reaction mixture of the first step. And/or, the additional step A could be conducted after completion of the additional step B. When the additional step A is conducted after completion of the additional step B, it is acceptable for the resultant of the additional step B to have been isolated out of the reaction mixture of the additional step B as herein described, or without any isolation, to be directly used to conduct the additional step A in the form of the reaction mixture of the additional step B.

According to an embodiment of this invention, in the process for producing the hindered phenol compound, in the additional step A, as the sulfuring agent, exemplified could be sulfur and sulfur dichloride, etc., preferably sulfur. As the sulfur, exemplified could be powdered sulfur or sublimed sulfur.

According to this invention, in the process for producing the hindered phenol compound, in the additional step A, molar ratio of the amine compound represented by Formula (Y) to the sulfuring agent is generally 1 : 1-10, preferably 1 : 1.2-6.0, more preferably 1 : 1.5-3.0.

According to this invention, in the process for producing the hindered phenol compound, the additional step A could be conducted in the presence of a solvent. As the solvent, exemplified could be a C₂₋₁₀ aliphatic nitrile (for example, acetonitrile, etc.), a C₆₋₂₀ aromatic hydrocarbon (for example, benzene, toluene, xylene and cumene), a C₆₋₁₀ alkane (for example, n-hexane, cyclohexane and petroleum ether), a C₁₋₆ aliphatic alcohol (for example, methanol, ethanol, n-propanol, isopropanol, n-butanol and ethylene glycol), a C₂₋₂₀ halogenated hydrocarbon (for example, dichloromethane, carbon tetrachloride, chlorobenzene and 1,2-dichlorobenzene), a C₃₋₁₀ ketone (for example, acetone, butanone and methyl isobutyl ketone) or a C₃₋₁₀ amide (for example, dimethylfomamide, dimethylacetamide and N-methyl pyrrolidone), etc. As the solvent, one kind or a mixture of two or more kinds at any ratio therebetween could be used.

According to this invention, in the process for producing the hindered phenol compound, the additional step A may be conducted in the presence of a catalyst. As the catalyst, exemplified could be a catalyst commonly known for this purpose, specifically, iodine. As the amount of the catalyst to be used, any amount that conventionally used is acceptable, without any specific limitation thereto.

According to this invention, in the process for producing the hindered phenol compound, there is no specific limitation as to the reaction duration of the additional step A, but generally in the range of 0.1h-24h, preferably 0.2h-12h, more preferably 0.5h-4h.

According to this invention, in the process for producing the hindered phenol compound, there is no specific limitation as to the reaction temperature of the additional step A, but generally in the range of 60 degrees Celsius to 300 degrees Celsius, preferably 120 degrees Celsius to 240 degrees Celsius, more preferably 150 degrees Celsius to 200 degrees Celsius.

According to this invention, in the process for producing the hindered phenol compound, upon completion of the additional step A, by a separation method commonly known (for example, by evaporation, etc.), any volatile like solvent that may exist is removed from the reaction mixture obtained from the additional step A, so as to obtain a resultant of the additional step A, or alternatively, without any separation, this reaction mixture is directly used as the resultant of the additional step A to conduct the subsequent reaction step (for example, the additional step B).

According to an embodiment of this invention, in the process for producing the hindered phenol compound, the additional step B could be conducted after completion of the first step. When the additional step B is conducted after completion of the first step, it is acceptable for the resultant of the first step to have been isolated out of the reaction mixture of the first step as herein described, or without any isolation, to be directly used to conduct the additional step B in the form of the reaction mixture of the first step. And/or, the additional step B could be conducted after completion of the additional step A. When the additional step B is conducted after completion of the additional step A, it is acceptable for the resultant of the additional step A to have been isolated out of the reaction mixture of the additional step A as herein described, or without any isolation, to be directly used to conduct the additional step B in the form of the reaction mixture of the additional step A.

According to this invention, in the process for producing the hindered phenol compound, in the additional step B, molar ratio of the amine compound represented by Formula (Y) to the aldehyde compound represented by Formula (Z) is generally 1 : 0.1-10, preferably 1 : 0.5-5.0, more preferably 1 : 0.8-2.0.

According to this invention, in the process for producing the hindered phenol compound, the additional step B could be conducted in the presence of a solvent. As the solvent, exemplified could be a C₂₋₁₀ aliphatic nitrile (for example, acetonitrile, etc.), a C₆₋₂₀ aromatic hydrocarbon (for example, benzene, toluene, xylene and cumene), a C₆₋₁₀ alkane (for example, n-hexane, cyclohexane and petroleum ether), a C₁₋₆ aliphatic alcohol (for example, methanol, ethanol, n-propanol, isopropanol, n-butanol and ethylene glycol), a C₂₋₂₀ halogenated hydrocarbon (for example, dichloromethane, carbon tetrachloride, chlorobenzene and 1,2-dichlorobenzene), a C₃₋₁₀ ketone (for example, acetone, butanone and methyl isobutyl ketone) or a C₃₋₁₀ amide (for example, dimethylfomamide, dimethylacetamide and N-methyl pyrrolidone), etc. As the solvent, one kind or a mixture of two or more kinds at any ratio therebetween could be used.

According to this invention, in the process for producing the hindered phenol compound, in the additional step B, a catalyst could be used, if needed. As the catalyst, exemplified could be an inorganic acid catalyst and an organic acid catalyst. As the inorganic acid catalyst, exemplified could be a hydrochloric acid, sulphuric acid and phosphoric acid, etc. As the organic acid catalyst, exemplified could be a methyl sulfonic acid, ethyl sulfonic acid, aminosulfonic acid and p-toluene sulfonic acid, etc. As the amount of the catalyst to be used, any amount that conventionally used is acceptable, without any specific limitation thereto.

According to this invention, in the process for producing the hindered phenol compound, at the begining of or during the additional step B, if needed, it is acceptable to further add an amine compound represented by Formula (Y), whereby forming both the 9,10-dihydroacridine ring and the group represented by Formula (III) in a single step. Herein, molar ratio of the amine compound represented by Formula (Y) to the aldehyde compound represented by Formula (Z) is generally 1 : 0.1-10, preferably 1 : 0.5-5.0, more preferably 1 : 0.8-2.0.

According to this invention, in the process for producing the hindered phenol compound, there is no specific limitation as to the reaction duration of the additional step B, but generally in the range of 0.1h-24h, preferably 0.2h-12h, more preferably 0.5h-6h.

According to this invention, in the process for producing the hindered phenol compound, there is no specific limitation as to the reaction temperature of the additional step B, but generally in the range of 0 degrees Celsius to 250 degrees Celsius, preferably 20 degrees Celsius to 180 degrees Celsius, more preferably 60 degrees Celsius to 120 degrees Celsius.

According to this invention, in the process for producing the hindered phenol compound, after completion of the additional step B, by a separation method commonly known (for example, by evaporation, etc.), any volatile like solvent that may exist is removed from the reaction mixture obtained from the additional step B, so as to obtain a resultant of the additional step B, or alternatively, without any separation, this reaction mixture is directly used as the resultant of the additional step B to conduct the subsequent reaction step (for example, the additional step A).

According to this invention, obviously, each of the aforesaid reactions or steps (including the first step, the additional step A and the additional step B) is generally conducted under the protection of an inert gas atmosphere. As the inert gas, exemplified could be N₂ gas and Ar gas, etc., without any specific limitation thereto.

According to this invention, in the process for producing the hindered phenol compound, the resultant of the first step, the resultant of the additional step A or the resultant of the additional step B, could be isolated into individual hindered phenol compound(s) (for example, one single hindered phenol compound represented by Formula (I)), or could be produced as a mixture of two or more hindered phenol compounds. All these resultants are covered by this invention and identified as being effective and desirable in this invention. In view of this, in the context of this specification, these resultants are all accepted or referred to as the hindered phenol compound of this invention without any discrimination therebetween. In this context, according to this invention, it is not absolutely necessary to further purify these resultants, or to further isolate one or more specific hindered phenol compound(s) from these resultants. Of course, this purification or isolation may be preferred in some cases by this invention, however, is not absolutely necessary to this invention. Nevertheless, as the purification or isolation method, exemplified could be column chromatography or preparative chromatography, etc.

According to an embodiment of this invention, further related to is a hindered phenol compound produced in line with the aforesaid process.

According to this invention, the hindered phenol compound exhibits excellent performance in oxidation resistance at elevated temperatures, and therefore is especially suitable to be used as an antioxidant, especially in a lubricant oil composition for which improved oxidation stability (at elevated temperatures) is anticipated. In view of this, according to an embodiment of this invention, related to is a lubricant oil composition, which comprises a lubricant base oil and any aforesaid hindered phenol compound (or their mixture at any ratio therebetween) of this invention as an antioxidant.

According to this invention, any lubricant base oil commonly used in this field could be used. As the lubricant base oil, exemplified could be one or more selected from mineral base oils, animal oils, vegetable oils, synthetic base oils and etc. As the mineral base oil, exemplified could be one having a viscosity index of above 80, or one having a saturated hydrocarbon content of above 90wt% and a sulfur content of less than 0.03wt%. As the synthetic base oil, exemplified could be one or more selected from polyolefins, synthetic esters, silicone oils and polyethers, etc. As the lubricant base oil, one kind or a mixture of two or more kinds at any ratio therebetween could be used.

According to an embodiment of this invention, in the lubricant oil composition, the antioxidant represents 0.001-30wt%, preferably 0.1-10wt%, of the total weight of the lubricant oil composition.

According to this invention, the lubricant oil composition optionally further comprises other lubricant oil additive selected from the group consisting of metal cleansing agents, ashless dispersants, antifriction agents, anti-scuff agents, extreme pressure agents, viscosity index modifiers, metal corrosion inhibitors, antirust additives, pour-point depressants and anti-foaming agents. As the other lubricant oil additive, one kind or a mixture of two or more kinds at any ratio therebetween, at an amount commonly used in this field, could be used, without any specific limitation thereto.

According to this invention, the aforesaid hindered phenol compound (or their mixture at any ratio therebetween) and the lubricant base oil (and other lubricant oil additive, if any) are mixed together till homogenous (under heat if needed) at a predetermined ratio or respective amount, so as to produce the lubricant oil composition.

In a preferred aspect of this invention, the lubricant oil composition comprises the hindered phenol compound of this invention as an antioxidant, and therefore exhibits excellent performance in oxidation resistance at elevated temperatures, and at the same time, in at least one performance selected from anticorrosion, cleansing (deposite formation inhibition), inhibiting inrease in viscosity and inhibiting inrease in acid value. These performces can not be found at the same time with a prior art antioxidant. Further, the hindered phenol compound according to this invention, contains no phosphorus and metals, generates little ash when burning, leading to less particulate emission of engines (which is greatly responsible for smog), and is thus identified as an environment friendly antioxidant.

### Example

The present invention is further illustrated by using the following examples, but not limiting to same.

Performances mentioned in Examples and Comparative Examples are determined as follows.

### (1) oxidation resistance at elevated temperatures

The lubricant oil composition produced in Example or Comparative Example was taken as the test sample, evaluated for its oxidation resistance by Pressured differential Scanning Calorimetry (PDSC), with a PDSC temperature of 210 degrees Celsius, a pressure of 0.5MPa and an oxygen flow rate of 100mL/min, expressed as oxidative induction period (unit: min).

### (2) deposite formation inhibition

The lubricant oil composition produced in Example or Comparative Example was taken as the test sample to conduct the engine crankcase coking simulation test, which simulates depositing on the piston. During this test, 300ml of the test sample was added to a coke-forming plate simulator, heated up to 150 degrees Celsius, continuously splashed onto an aluminium plate having a temperature of 310 degrees Celsius. After 6 hours, coke deposited on the aluminium plate was weighted, expressed as the deposit amount (unit : mg). The more the deposit amount is, the worse the piston cleansing performance of the test sample exhibits.

### (3) anticorrosion

The lubricant oil composition produced in Example or Comparative Example was taken as the test sample to conduct the ball rust test (BRT). Throughout the bench scale test lasting for 18 hours, a metal ball protected by the test sample was made to continuously contact an acid liquid and air, with an acid liquid injection speed of 0.19ml/h, an air flow rate of 40ml/min and an oil temperature of 48 degrees Celsius. Upon completion of the test, the spherical reflection intensity of the metal ball was determined to obtain a gray value, which was used to evaluate the corrosion degree. The acid liquid was an acetic acid/HBr/hydrochloric acid /deionized water solution. The greater the gray value is, the more serious the corrosion degree is.

### (4) inhibiting inrease in viscosity

The lubricant oil composition produced in Example or Comparative Example was taken as the test sample to conduct the IIIE simulation experiment (VIT) at a temperature of 180 degrees Celsius, with a duration of 72h and an oxygen flow rate of 5L/h, expressed as the increase (by %) in viscosity of the test sample after and before the experiment.

### (5) inhibiting inrease in acid value

The lubricant oil composition produced in Example or Comparative Example was taken as the test sample to conduct the IIIE simulation experiment (VIT) at a temperature of 180 degrees Celsius, with a duration of 72h and an oxygen flow rate of 5L/h, expressed as the increase (by mgKOH·g⁻¹) in acid value of the test sample after and before the experiment.

### Example I-1

To a 500ml four-necked flask equiped with a stirrer, a thermometer, a condensing tube and a dropping funnel, there were added 58.79g (0.323mol) 2-tert-butyl-6-mercaptophenol, 6.88g (0.048mol) boron trifluoride · diethyl ether (as the alkylation catalyst), 100ml n-hexane (as the solvent) and 161.61g (0.162mol) polyisobutene (Mn=1000, from Jilin Chemical Group Fine Chemicals Co., Ltd.), reacted at 80 degrees Celsius for 2h. Upon completion of the reaction, the reaction mixture was washed once with a 5wt% KOH aqueous solution, and then washed with hot water till neutral to remove any catalyst, and then vacuum distillated to remove any solvent and unreacted phenols, to obtain a polyisobutenyl thiophenol, having a hydroxyl value of 53.49mgKOH/g. The hydroxyl value was determined by referring to the acetic anhydride method in GB/T7383-2007.

The reaction procedure can be illustrated as follows.

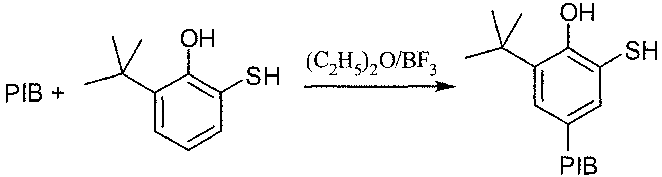

On the ¹H-NMR spectra of the produced polyisobutenyl thiophenol, the peak at the chemical shift of 1.40 was identified as the characteristic peak of the hydrogen of the tert-butyl on the benzene ring; the single peak at the chemical shift of 3.58 was identified as the characteristic peak of the hydrogen of the mercapto on the benzene ring; the single peak at the chemical shift of 4.84 was identified as the characteristic peak of the hydrogen of the hydroxyl on the benzene ring; the single peak at the chemical shift of 7.12 and that at 7.20 were respectively identified as the characteristic peak of the two hydrogens on the benzene ring. If the integral of the hydrogen of the hydroxyl on the benzene ring is defined as 1, the value of the integral ratio between the hydrogen on the benzene ring, the hydrogen of the mercapto and the hydrogen of the hydroxyl were caculated as 0.95 : 0.97 : 1.05 : 0.94, approximating to the theoretical value of 1 : 1 : 1 : 1 : 1. As can be seen from this NMR spectra analysis, the polyisobutenyl thiophenol was obtained as anticipated.

### Example I-2

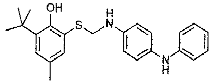

Under the protective atomosphere of nitrogen gas, to a 250ml four-necked flask equiped with a stirrer, a thermometer, a condensing tube and a dropping funnel, there were added 30.58g (156mmol) 2-mercapto-4-methyl-6-methyl phenol, 9.29g (112mmol) formaldehyde, 32.38g (176mmol) N-phenyl p-phenylene diamine and 100mL toluene, rapidly stirred, reacted at 100 degrees Celsius for 2h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ1.40 (9H), 2.37 (3H), 4.02 (1H), 4.83 (2H), 6.95-7.00 (12H), 7.26 (2H), 7.55 (1H);
¹³C NMR (75MHz, CDCl₃) : δ 21.2, 30.1, 34.1,49.9, 114.3, 118.9, 119.4, 121.8, 122.2, 125.8, 126.5, 129.5, 131.8, 132.3, 133.5, 143.3, 150.8;
C₂₄H₂₈N₂OS (calculated) : C 73.43, H 7.19, N 7.14, O 4.08, S 8.17; (measured) : C 73.31, H 7.23, N 7.21, O 3.99, S 8.28.

### Example I-3

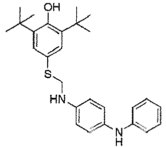

Under the protective atomosphere of nitrogen gas, to a 250ml four-necked flask equiped with a stirrer, a thermometer, a condensing tube and a dropping funnel, there were added 31.65g (133mmol) 2,6-di-tert-butyl-4-mercaptophenol, 1.86g (62mmol) formaldehyde, 28.15g (153mmol) N-phenyl p-phenylene diamine, 0.75g (7.5mmol) hydrochloric acid and 150mL isopropanol, rapidly stirred, reacted at 25 degrees Celsius for 24h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ1.36-1.54 (18H), 3.75 (1H), 4.80 (2H), 5.32 (1H), 6.80 (2H), 6.97 (5H), 7.17 (2H), 7.26 (2H), 7.55 (1H);
¹³C NMR (75MHz, CDCl₃) : δ 29.6, 34.6, 51.2, 119.4, 121.8, 126.5, 129.5, 131.4, 136.6, 144.5, 153.4;
C₂₇H₃₄N₂OS (calculated) : C 74.61, H 7.88, N 6.45, O 3.68, S 7.38; (measured) : C 74.52, H 7.84, N 6.51, O 3.73, S 7.40.

### Example I-4

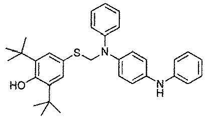

Under the protective atomosphere of nitrogen gas, to a 250ml four-necked flask equiped with a stirrer, a thermometer, a condensing tube and a dropping funnel, there were added 8.57g (36mmol) 2,6-di-tert-butyl-4-mercaptophenol, 1.24g (15mmol) formaldehyde, 10.14g (39mmol) N,N'-diphenyl-1,4-phenylene diamine and 150mL toluene, rapidly stirred, reacted at 90 degrees Celsius for 4h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ1.36 (18H), 5.23 (2H), 6.80-7.02 (10H), 7.17 (2H), 7.26 (4H), 7.55 (1H);
¹³C NMR (75MHz, CDCl₃): δ 29.6, 34.6, 55.2, 117.1, 121.8, 126.2, 129.5, 136.6, 143.6, 153.4;
C₃₃H₃₈N₂OS (calculated) : C 77.60, H 7.50, N 5.48, O 3.13, S 6.28; (measured) : C 77.71, H 7.52, N 5.53, O 3.10, S 6.23.

### Example I-5

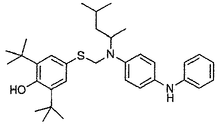

Under the protective atomosphere of nitrogen gas, to a 250ml four-necked flask equiped with a stirrer, a thermometer, a condensing tube and a dropping funnel, there were added 20.23g (85mmol) 2,6-di-tert-butyl-4-mercaptophenol, 9.54g (115mmol) formaldehyde, 18.49g (69mmol) N-(1,3-dimethylbutyl)-N'-phenyl p-phenylene diamine and 150mL benzene, rapidly stirred, reacted at 85 degrees Celsius for 3h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 0.80 (6H), 1.11-1.21 (4H), 1.36 (18H), 1.67 (2H), 3.47
(1H), 4.80 (2H), 5.32 (1H), 6.80 (2H), 6.97 (5H), 7.17 (2H), 7.26 (2H), 7.55 (1H);
¹³C NMR (75MHz, CDCl₃) : δ 17.9, 22.4, 29.6, 34.6, 45.2, 52.7, 58.6, 119.4, 121.8, 126.2, 129.5, 131.2, 136.6, 144.1, 146.1, 153.4;
C₃₃H₄₆N₂OS (calculated): : C 76.40, H 8.94, N 5.40, O 3.08, S 6.18; (measured): C 76.48, H 8.96, N 5.35, O 3.09, S 6.12.

### Example I-6

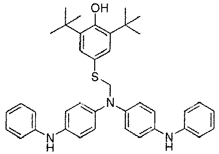

Under the protective atomosphere of nitrogen gas, to a 250ml four-necked flask equiped with a stirrer, a thermometer, a condensing tube and a dropping funnel, there were added 3.57g (15mmol) 2,6-di-tert-butyl-4-mercaptophenol, 5.14g (62mmol) formaldehyde, 21.76g (103mmol) N-phenyl-N'-[4-(phenylamino)phenyl]-1,4-phenylene diamine and 150mL toluene, rapidly stirred, reacted at 110 degrees Celsius for 2h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ1.36 (18H), 5.20 (2H), 5.32 (1H), 6.80-7.02 (14H), 7.17 (2H), 7.20 (1H), 7.26 (4H), 7.55 (1H);
¹³C NMR (75MHz, CDCl₃) : δ 29.6, 34.6, 55.5, 117.1, 120.4, 126.2, 129.5, 132.3, 136.6, 142.8, 146.1, 153.4;
C₃₆H₄₃N₃OS (calculated) : C 77.83, H 7.20, N 6.98, O 2.66, S 5.33; (measured) : C 77.69, H 7.19, N 7.08, O 2.65, S 5.38.

### Example I-7

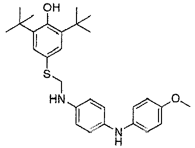

Under the protective atomosphere of nitrogen gas, to a 250ml four-necked flask equiped with a stirrer, a thermometer, a condensing tube and a dropping funnel, there were added 34.51g (145mmol) 2,6-di-tert-butyl-4-mercaptophenol, 2.91g (35mmol) formaldehyde, 4.49g (21 mmol) 4-amino-4'-methoxy diphenyl amine, 0.26g (2.61 mmol) hydrochloric acid and 150mL benzene, rapidly stirred, reacted at 90 degrees Celsius for 2h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ1.36 (18H), 3.72-3.76 (4H), 4.80 (2H), 5.32 (1H), 6.97-7.06 (8H), 7.17 (2H), 7.20 (1H);
¹³C NMR (75MHz, CDCl₃) : δ 29.6, 34.6, 51.1, 55.5, 114.6, 118.9, 121.8, 124.7, 126.5, 132.3, 136.6, 144.5, 153.4, 154.5;
C₂₈H₃₆N₂O₂S (calculated) : C 72.38, H 7.81, N 6.03, O 6.89, S 6.90; (measured) : C 72.37, H 7.69, N 6.12, O 6.80, S 6.83.

### Example I-8

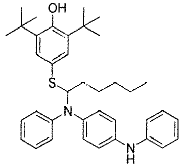

Under the protective atomosphere of nitrogen gas, to a 250ml four-necked flask equiped with a stirrer, a thermometer, a condensing tube and a dropping funnel, there were added 21.66g (91 mmol) 2,6-di-tert-butyl-4-mercaptophenol, 1.35g (9.51 mmol) nonanal, 6.51g (25mmol) N,N'-diphenyl-1,4-phenylene diamine and 150mL benzene, rapidly stirred, reacted at 70 degrees Celsius for 6h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 0.88 (3H), 1.08-1.85 (24H), 5.23 (1H), 5.32 (1H), 6.80 (1H), 6.97 (8H), 7.17 (2H), 7.26 (4H), 7.55 (1H);
¹³C NMR (75MHz, CDCl₃) : δ 14.1,22.6, 29.6, 31.7, 34.6, 41.6, 60.8, 116.6, 119.4, 121.8, 126.5, 129.5, 131.4, 136.6, 142.8, 144.5, 146.1, 153.4;
C₃₈H₄₈N₂OS (calculated) : C 78.57, H 8.33, N 4.82, O 2.75, S 5.52; (measured) : C 78.62, H 8.25, N 4.75, O 2.81, S 5.42.

### Example I-9

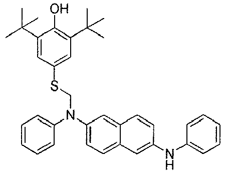

Under the protective atomosphere of nitrogen gas, to a 250ml four-necked flask equiped with a stirrer, a thermometer, a condensing tube and a dropping funnel, there were added 8.33g (35mmol) 2,6-di-tert-butyl-4-mercaptophenol, 4.23g (51 mmol) formaldehyde, 9.61g (31mmol) N,N'-diphenyl-2,6-naphthalene diamine and 150mL toluene, rapidly stirred, reacted at 110 degrees Celsius for 4h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.36 (18H), 5.25 (2H), 5.32 (1H), 5.80 (1H), 6.99-7.06 (7H), 7.17 (3H), 7.26 (4H), 7.40 (2H), 7.84 (2H);
¹³C NMR (75MHz, CDCl₃) : δ 29.6, 34.6, 54.8, 106.9, 118.6, 123.3, 126.2, 129.2, 136.6, 142.8, 148.3, 153.5;
C₃₇H₄₁N₂OS (calculated) : C 79.24, H 7.19, N 5.00, O 2.85, S 5.72; (measured) : C 79.20, H 7.23, N 5.14, O 2.72, S 5.65.

### Example I-10

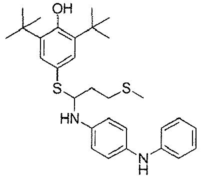

Under the protective atomosphere of nitrogen gas, to a 250ml four-necked flask equiped with a stirrer, a thermometer, a condensing tube and a dropping funnel, there were added 6.19g (26mmol) 2,6-di-tert-butyl-4-mercaptophenol, 9.57g (92mmol) 3-methylthiopropionic aldehyde, 3.13g (17mmol) N-phenyl p-phenylene diamine and 150mL toluene, rapidly stirred, reacted at 80 degrees Celsius for 2h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ1.36-1.54 (18H), 2.12-2.80 (7H), 3.47 (1H), 4.97 (1H), 5.32 (1H), 6.97 (7H), 7.17 (2H), 7.26 (2H), 7.55 (1H);
¹³C NMR (75MHz, CDCl₃) : δ 15.2,29.6, 31.9, 34.6, 41.7, 60.2, 114.3, 119.4, 121.8, 126.2, 129.5, 132.4, 136.6, 144.5, 146.1, 153.5;
C₃₀H₄₀N₂OS₂ (calculated) : C 70.82, H 7.92, N 5.51, O 3.14, S 12.60; (measured) : C 70.75, H 7.83, N 5.46, O 3.20, S 12.71.

### Example I-11

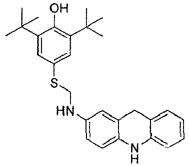

Under the protective atomosphere of nitrogen gas, to a 250ml four-necked flask equiped with a stirrer, a thermometer, a condensing tube and a dropping funnel, there were added 4.99g (21 mmol) 2,6-di-tert-butyl-4-mercaptophenol, 4.4g (53mmol) formaldehyde, 5.70g (31mmol) N-phenyl p-phenylene diamine and 150mL ethanol, rapidly stirred, after reacted at 60 degrees Celsius for 2.5h, cooled to the room temperature, there were added 7.05g (85mmol) formaldehyde, heated to 85 degrees Celsius and reacted for 2.5h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ1.36 (18H), 3.74 (1H), 4.12 (2H), 4.59 (1H), 4.83 (2H), 5.32 (1H), 6.97-7.11 (5H), 7.17 (2H), 7.24 (2H);
¹³C NMR (75MHz, CDCl₃) : δ 29.6, 33.1, 34.6, 50.6, 119.4, 123.3, 126.2, 126.7, 127.9, 136.6, 140.5, 142.9, 153.4;
C₂₆H₃₄N₂OS (calculated) : C 75.29, H 7.67, N 6.27, O 3.58, S 7.18; (measured) : C 75.20, H 7.59, N 6.29, O 3.65, S 7.22.

### Example I-12

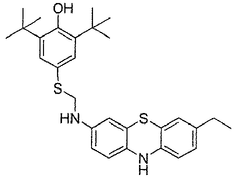

Under the protective atomosphere of nitrogen gas, to a 250ml four-necked flask equiped with a stirrer, a thermometer, a condensing tube and a dropping funnel, there were added 19.52g (82mmol) 2,6-di-tert-butyl-4-mercaptophenol, 10.04g (121mmol) formaldehyde, 19.93g (94mmol) 4-amino-4'-ethyl diphenyl amine and 150mL xylene, rapidly stirred, after reacted at 90 degrees Celsius for 3h, cooled to the room temperature, there were added 10.11g (316mmol) sulfur and 0.04g (0.35mmol) iodine, heated to 150 degrees Celsius and reacted for 8h. Upon completion of the reaction, the solvent and water (generated during the reaction) were removed by vacuum distillation, the titled hindered phenol compound was obtained by using column chromatography. The compound was characterized as follows :
¹H NMR (300MHz, CDCl₃) : δ 1.23 (1H), 1.36 (18H), 2.61 (2H), 3.77 (1H), 4.84 (2H), 5.32 (1H), 5.70 (1H), 6.98 (5H), 7.17 (2H), 7.49 (1H);
¹³C NMR (75MHz, CDCl₃): δ 15.60, 29.6, 34.6, 50.7, 118.9, 121.8, 126.2, 129.7, 136.6, 141.2, 146.1, 153.4;
C₂₉H₃₆N₂OS₂ (calculated) : C 70.69, H 7.36, N 5.69, O 3.25, S 13.02; (measured) : C 70.77, H 7.41, N 5.67, O 3.17, S 12.93.

### Example I-13

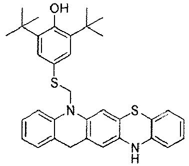

Under the protective atomosphere of nitrogen gas, to a 250ml four-necked flask equiped with a stirrer, a thermometer, a condensing tube and a dropping funnel, there were added 10.95g (46mmol) 2,6-di-tert-butyl-4-mercaptophenol, 3.98g (48mmol) formaldehyde, 13.78g (53mmol) N,N'-diphenyl-1,4-phenylene diamine and 150mL trimethylbenzene, rapidly stirred, after reacted at 85 degrees Celsius for 1h, there were added 3.82g (46mmol) formaldehyde, after reacted at 85 degrees Celsius for 1h, cooled to the room temperature, there were added 10.11g (316mmol) sulfur and 0.27g (2.11mmol) iodine, heated to 180 degrees Celsius and reacted for 1h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ1.36 (18H), 4.26 (2H), 5.18 (2H), 5.32 (1H), 6.40 (1H), 6.97 (8H), 7.17 (2H), 7.37 (2H);
¹³C NMR (75MHz, CDCl₃) : δ 29.6, 33.6, 34.6, 54.3, 106.5, 114.4, 119.4, 121.8, 126.2, 129.5, 136.6, 141.9, 144.5, 153.4;
C₃₄H₃₆N₂OS₂ (calculated) : C 73.87, H 6.56, N 5.07, O 2.89, S 11.60; (measured) : C 73.91, H 6.49, N 5.02, O 2.94, S 11.49.

### Example I-14

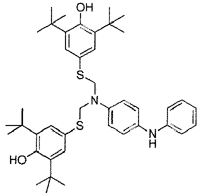

Under the protective atomosphere of nitrogen gas, to a 250ml four-necked flask equiped with a stirrer, a thermometer, a condensing tube and a dropping funnel, there were added 21.66g (91 mmol) 2,6-di-tert-butyl-4-mercaptophenol, 7.39g (89mmol) formaldehyde, 6.62g (36mmol) N-phenyl p-phenylene diamine, 0.45g (4.51mmol) NaOH and 150mL isopropanol, rapidly stirred, reacted at 80 degrees Celsius for 3h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ1.36-1.54 (36H), 4.82 (4H), 5.32 (2H), 6.97 (7H), 7.17 (4H), 7.26 (2H), 7.55 (1H);
¹³C NMR (75MHz, CDCl₃) : δ 29.6, 34.6, 56.5, 106.5, 119.4, 121.8, 126.5, 129.5, 131.4, 136.6, 144.5, 146.1, 153.4;
C₄₂H₅₆N₂O₂S₂ (calculated) : C 73.64, H 8.24, N 4.09, O 4.67, S 9.36; (measured) : C 73.59, H 8.21, N 4.15, O 4.62, S 9.34.

### Example I-15

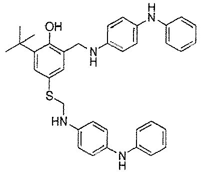

Under the protective atomosphere of nitrogen gas, to a 250ml four-necked flask equiped with a stirrer, a thermometer, a condensing tube and a dropping funnel, there were added 6.55g (36mmol) 2-tert-butyl-4-mercaptophenol, 7.64g (92mmol) formaldehyde, 13.06g (71 mmol) N-phenyl p-phenylene diamine and 150mL toluene, rapidly stirred, reacted at 90 degrees Celsius for 4h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ1.36 (9H), 3.81 (1H), 4.38 (2H), 4.69 (2H), 5.29 (1H), 6.95-7.00 (15H), 7.17 (1H), 7.26 (4H), 7.55 (1H), 8.62 (1H);
¹³C NMR (75MHz, CDCl₃) : δ 29.6, 34.6, 43.5, 50.8, 114.3, 119.4, 121.8, 125.4, 129.5, 132.3, 136.6, 145.2, 146.1, 149.0, 153.4;
C₃₆H₃₈N₄OS (calculated) : C 75.23, H 6.66, N 9.75, O 2.78, S 5.58; (measured) : C 75.27, H 6.69, N 9.68, O 2.79, S 5.51.

### Example I-16

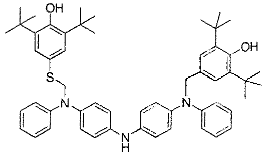

Under the protective atomosphere of nitrogen gas, to a 250ml four-necked flask equiped with a stirrer, a thermometer, a condensing tube and a dropping funnel, there were added 11.66g (49mmol) 2,6-di-tert-butyl-4-mercaptophenol, 10.92g (53mmol) 2,6-ditert-butyl phenol, 7.89g (95mmol) formaldehyde, 11.23g (32mmol) N-phenyl-N'-[4-(phenylamino)phenyl]-1,4-phenylene diamine and 150mL toluene, rapidly stirred, reacted at 100 degrees Celsius for 3h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ1.36-1.54 (36H), 4.91 (2H), 5.22 (2H), 5.32 (2H), 6.97-7.07 (16H), 7.17 (2H), 7.20 (1H), 7.27 (4H);
¹³C NMR (75MHz, CDCl₃) : δ 29.6, 34.4, 55.6, 56.9, 93.6, 119.4, 123.3, 126.5, 129.2, 131.4, 136.6, 142.8, 146.1, 153.4;
C₅₄H₆₅N₃O₂S (calculated) : C 79.08, H 7.99, N 5.12, O 3.90, S 3.91; (measured) : C 79.15, H 8.03, N 5.01, O 3.87, S 3.85.

### Example I-17

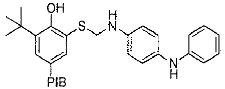

Under the protective atomosphere of nitrogen gas, to a 250ml four-necked flask equiped with a stirrer, a thermometer, a condensing tube and a dropping funnel, there were added 47.16g (45mmol) polyisobutenyl thiophenol produced by Example I-1, 4.23g (51mmol) formaldehyde, 9.75g (53mmol) N-phenyl p-phenylene diamine and 150mL benzene, rapidly stirred, reacted at 80 degrees Celsius for 2.5h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 0.88, 0.98, 1.02, 1.24, 1.40, 2.42, 4.86, 6.97, 7.02, 7.14, 7.26, 7.55;
¹³C NMR (75MHz, CDCl₃) : δ 28.2, 30.1, 32.3, 34.5, 38.1,49.8, 59.1, 114.3, 119.4, 121.8, 122.0, 127.3, 129.5, 132.3, 143.3, 146.1, 151.3.

### Example I-18

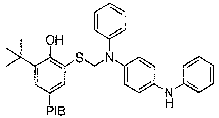

Under the protective atomosphere of nitrogen gas, to a 250ml four-necked flask equiped with a stirrer, a thermometer, a condensing tube and a dropping funnel, there were added 29.34g (28mmol) polyisobutenyl thiophenol produced by Example I-1, 2.91g (35mmol) formaldehyde, 6.24g (24mmol) N,N'-diphenyl-1,4-phenylene diamine and 150mL toluene, rapidly stirred, reacted at 90 degrees Celsius for 2h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 0.88, 0.98, 1.02, 1.24, 1.40, 2.42, 4.00, 4.86, 5.56, 6.97, 7.02, 7.14, 7.26, 7.55;
¹³C NMR (75MHz, CDCl₃): δ 28.2, 30.1, 32.3, 33.1, 34.5, 38.1,49.8, 59.1, 114.3, 119.4, 121.8, 125.2, 122.0, 127.3, 129.5, 132.3, 143.3, 146.1, 151.3.

### Comparative Example I-1

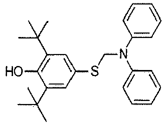

Under the protective atomosphere of nitrogen gas, to a 250ml four-necked flask equiped with a stirrer, a thermometer, a condensing tube and a dropping funnel, there were added 20.23g (85mmol) 2,6-di-tert-butyl-4-mercaptophenol, 7.22g (87mmol) formaldehyde, 14.37g (85mmol) diphenyl amine and 150mL methanol, rapidly stirred, reacted at 60 degrees Celsius for 2h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : 51.36 (18H), 5.21 (2H), 5.32 (1H), 6.99 (6H), 7.17 (2H), 7.27 (4H);
¹³C NMR (75MHz, CDCl₃) : δ 29.6, 34.6, 55.2, 120.4, 123.3, 125.9, 126.2, 129.2, 136.6, 150.0, 153.5;
C₂₇H₃₃NOS (calculated) : C 77.28, H 7.93, N 3.34, O 3.81, S 7.64; (measured) : C 77.22, H 7.89, N 3.31, O 3.83, S 7.67.

### Comparative Example I-2

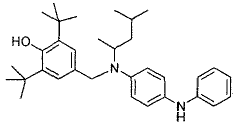

Under the protective atomosphere of nitrogen gas, to a 250ml four-necked flask equiped with a stirrer, a thermometer, a condensing tube and a dropping funnel, there were added 10.71g (52mmol) 2,6-di-tert-butyl-4-mercaptophenol, 1.89g (63mmol) formaldehyde, 17.42g (65mmol) N-(1,3-dimethyl butyl)-N'-phenyl p-phenylene diamine and 150mL methanol, rapidly stirred, reacted at 70 degrees Celsius for 4h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 0.80 (6H), 1.11 (3H), 1.29 (1H), 1.36 (18H), 3.45 (1H), 4.53 (2H), 5.32 (2H), 6.97-7.07 (9H), 7.26 (2H), 7.55 (1H);
¹³C NMR (75MHz, CDCl₃) : δ17.9, 22.40, 24.6, 30.4, 34.3, 45.7, 54.5, 56.7, 116.6, 120.4, 121.8, 125.9, 128.9, 129.5, 135.6, 146.1, 153.5, 154.8;
C₃₃H₄₆N₂O (calculated) : C 81.43, H 9.53, N 5.76, O 3.29; (measured): C 81.38, H 9.51, N 5.79, O 3.31.

### Comparative Example I-3

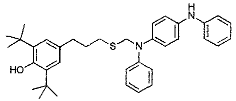

Under the protective atomosphere of nitrogen gas, to a 250ml four-necked flask equiped with a stirrer, a thermometer, a condensing tube and a dropping funnel, there were added 9.24g (33mmol) 2,6-ditert-butyl-4-(3-mercaptopropyl) phenol, 3.07g (37mmol) formaldehyde, 11.71g (45mmol) N,N'-diphenyl-1,4-phenylene diamine and 150mL benzene, rapidly stirred, reacted at 80 degrees Celsius for 4h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.40 (18H), 1.99 (1H), 2.61-2.80 (6H), 4.63 (2H), 5.32 (1H), 6.97-7.02 (12H), 7.26 (4H), 7.55 (1H);
¹³C NMR (75MHz, CDCl₃) : δ 28.7, 31.1, 34.3, 35.7, 54.1, 117.1, 119.4, 123.3, 124.8, 129.2, 131.8, 136.0, 142.8, 144.9, 146.1, 151.9;
C₃₆H₄₄N₂OS (calculated) : C 78.22, H 8.02, N 5.07, O 2.89, S 5.80; (measured) : C 78.27, H 7.96, N 4.98, O 2.92, S 5.83.

### Example 1-19 to Example I-35 and Comparative Example I-4 to Comparative Example I-8

Each of the hindered phenol compounds of Example I-2 to Example I-18 and Comparative Example I-1 to Comparative Example I-3 was respectively mixed with a lubricant base oil in line with the composition and ratio specified in Table I-1 at 40 degrees Celsius for 2h, to obtain the lubricant oil compositions of Example I-19 to Example I-35 and the lubricant oil compositions of Comparative Example I-4 to Comparative Example I-6. Further, in line with the composition and ratio specified in Table I-2, the antioxidant was respectively mixed with a lubricant base oil at 40 degrees Celsius for 2h, to obtain the lubricant oil compositions of Comparative Example I-7 to Comparative Example I-10. Herein, based on the total weight of each lubricant oil composition, the amount of the antioxidant to be used in each case was 0.5wt%, the lubricant base oil used was a Group II hydrogenated base oil (produced by Sinopec Group,Gaoqiao Shanghai Branch). Further, the base oil without the antioxidant was taken as Control.

**Table I-1**

| lubricant oil composition | antioxidant | lubricant base oil | antioxidant amount |
|---|---|---|---|
| Example I-19 | Example I-2 | II | 0.50% |
| Example I-20 | Example I-3 | II | 0.50% |
| Example I-21 | Example I-4 | II | 0.50% |
| Example I-22 | Example I-5 | II | 0.50% |
| Example I-23 | Example I-6 | II | 0.50% |
| Example I-24 | Example I-7 | II | 0.50% |
| Example I-25 | Example I-8 | II | 0.50% |
| Example I-26 | Example I-9 | II | 0.50% |
| Example I-27 | Example I-10 | II | 0.50% |
| Example I-28 | Example I-11 | I | 0.50% |
| Example I-29 | Example I-12 | II | 0.50% |
| Example I-30 | Example I-13 | II | 0.50% |
| Example I-31 | Example I-14 | II | 0.50% |
| Example I-32 | Example I-15 | II | 0.50% |
| Example I-33 | Example I-16 | II | 0.50% |
| Example I-34 | Example I-17 | II | 0.50% |
| Example I-35 | Example I-18 | II | 0.50% |
| Comparative Example I-4 | Comparative Example I-1 | II | 0.50% |
| Comparative Example I-5 | Comparative Example I-2 | II | 0.50% |
| Comparative Example I-6 | Comparative Example I-3 | II | 0.50% |
| Control | - | II | - |

**Table I-2**

| lubricant oil composition | hindered phenol compound | aromatic amine | hindered phenol compoun d amount | aromati c amine amount | lubrican t base oil |
|---|---|---|---|---|---|
| Comparative Example I-7 | 2,2'-thiobis[3-(3,5-ditert-butyl-4-hydroxyl phenyl)propionic acid ethyl ester] | - | 0.50% | - | II |
| Comparative Example I-8 | - | N-(4-tert-octyl phenyl)-1-naphthylamin e | - | 0.50% | II |
| Comparative Example I-9 | 2,6-ditert-butyl-4-amylthiophenol | 4,4'-ditert-octyl diphenyl amine | 0.25% | 0.25 % | II |
| Comparative Example I-10 | 4,4'-[thiobismethylene]bis[2,6-ditert-but yl phenol] | 3,7-ditert-octyl phenothiazine | 0.30% | 0.20% | II |

The lubricant oil compositions of Example I-19 to Example I-35, the lubricant oil compositions of Comparative Example I-4 to Comparative Example I-10 and the Control were taken as test samples, to evaluate the oxidation resistance at elevated temperatures, with a PDSC temperature of 210 degrees Celsius, the results of were shown in Table I-3.

**Table I-3**

| lubricant oil composition | PDSC oxidative induction period (min) | lubricant oil composition | PDSC oxidative induction period (min) |
|---|---|---|---|
| Example I-19 | 18.5 | Example I-32 | 23.9 |
| Example I-20 | 23.6 | Example I-33 | 27.1 |
| Example I-21 | 21.1 | Example I-34 | 16.9 |
| Example I-22 | 20.3 | Example I-35 | 18.3 |
| Example I-23 | 28.6 | Comparative Example I-4 | 4.9 |
| Example I-24 | 20.9 | Comparative Example I-5 | 7.5 |
| Example I-25 | 21.5 | Comparative Example I-6 | 16.1 |
| Example I-26 | 29.1 | Comparative Example I-7 | 8.6 |
| Example I-27 | 23.2 | Comparative Example I-8 | 15.2 |
| Example I-28 | 24.1 | Comparative Example I-9 | 5.3 |
| Example I-29 | 26.4 | Comparative Example I-10 | 7.1 |
| Example I-30 | 27.8 | Control | 0.2 |
| Example I-31 | 24.3 | | |

The lubricant oil compositions of Example I-19 to Example I-35, the lubricant oil compositions of Comparative Example I-4 to Comparative Example I-10 and the Control were taken as test samples, to evaluate the deposite formation inhibition performance, the results were shown in Table I-4.

**Table I-4**

| lubricant oil composition | deposit amount (mg) |
|---|---|
| Example I-22 | 15.5 |
| Example I-25 | 13.4 |
| Example I-29 | 10.5 |
| Example I-31 | 12.1 |
| Example I-34 | 4.2 |
| Example I-35 | 5.3 |
| Comparative Example I-4 | 19.5 |
| Comparative Example I-5 | 23.4 |
| Comparative Example I-6 | 20.8 |
| Comparative Example I-7 | 21.6 |
| Comparative Example I-8 | 25.6 |
| Comparative Example I-9 | 19.2 |
| Comparative Example I-10 | 18.5 |
| Control | 32.8 |

The lubricant oil compositions of Example I-19 to Example I-35, the lubricant oil compositions of Comparative Example I-4 to Comparative Example I-10 and the Control were taken as test samples, to evaluate the anticorrosion performance. The results of BRT (ball rust test) were shown in Table I-5.

**Table I-5**

| lubricant oil composition | corrosion degree |
|---|---|
| Example I-22 | slight |
| Example I-25 | slight |
| Example I-29 | slight |
| Example I-31 | moderate |
| Example I-34 | slight |
| Example I-35 | slight |
| Comparative Example I-4 | moderate |
| Comparative Example I-5 | moderate |
| Comparative Example I-6 | moderate |
| Comparative Example I-7 | moderate |
| Comparative Example I-8 | moderate |
| Comparative Example I-9 | moderate |
| Comparative Example I-10 | moderate |
| Control | severe |

The lubricant oil compositions of Example I-19 to Example I-35, the lubricant oil compositions of Comparative Example I-4 to Comparative Example I-10 and the Control were taken as test samples, to evaluate the performances of inhibiting inrease in viscosity and inhibiting inrease in acid value, the results of were shown in Table I-6.

**Table I-6**

| lubricant oil composition | increase in viscosity (%) | increase in acid value (mgKOH·g⁻¹) |
|---|---|---|
| Example I-19 | 24.1 | 2.811 |
| Example I-20 | 20.5 | 1.254 |
| Example I-21 | 21.3 | 1.139 |
| Example I-22 | 19.9 | 1.034 |
| Example I-23 | 17.3 | 0.810 |
| Example I-24 | 22.3 | 1.034 |
| Example I-25 | 20.9 | 0.937 |
| Example I-26 | 16.5 | 0.897 |
| Example I-27 | 18.4 | 2.138 |
| Example I-28 | 22.3 | 1.276 |
| Example I-29 | 17.2 | 1.531 |
| Example I-30 | 18.1 | 1.482 |
| Example I-31 | 19.7 | 1.734 |
| Example I-32 | 22.6 | 1.115 |
| Example I-33 | 19.8 | 1.065 |
| Example I-34 | 20.3 | 0.973 |
| Example I-35 | 18.5 | 1.341 |
| Comparative Example I-4 | 33.5 | 4.621 |
| Comparative Example I-5 | 27.5 | 3.482 |
| Comparative Example I-6 | 25.8 | 5.168 |
| Comparative Example I-7 | 27.6 | 2.953 |
| Comparative Example I-8 | 30.5 | 3.846 |
| Comparative Example I-9 | 28.1 | 5.429 |
| Comparative Example I-10 | 24.7 | 6.517 |
| Control | 67.2 | 24.525 |

### Example II-1

To a 500ml four-necked flask equiped with a stirrer, a thermometer, a condensing tube and a dropping funnel, there were added 58.79g (0.323mol) 2-tert-butyl-6-mercaptophenol, 6.88g (0.048mol) boron trifluoride ·diethyl ether (an alkylation catalyst), 100ml n-hexane (as the solvent) and 161.61g (0.162mol) polyisobutene (Mn=1000, from Jilin Chemical Group Fine Chemicals Co., Ltd.), reacted at 80 degrees Celsius for 2h. Upon completion of the reaction, the reaction mixture was washed once with a 5wt% KOH aqueous solution, and then washed with hot water till neutral to remove any catalyst, and then vacuum distillated to remove any solvent and unreacted phenols, to obtain a polyisobutenyl thiophenol, having a hydroxyl value of 53.49mgKOH/g. The hydroxyl value was determined by referring to the acetic anhydride method in GB/T7383-2007.

The reaction procedure can be illustrated as follows.

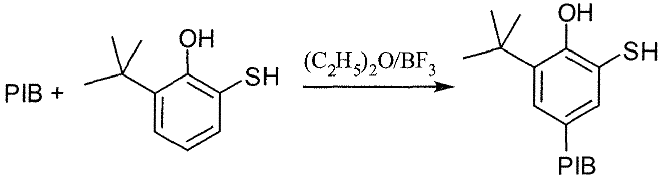

On the ¹H-NMR spectra of the produced polyisobutenyl thiophenol, the peak at the chemical shift of 1.40 was identified as the characteristic peak of the hydrogen of the tert-butyl on the benzene ring; the single peak at the chemical shift of 3.58 was identified as the characteristic peak of the hydrogen of the mercapto on the benzene ring; the single peak at the chemical shift of 4.84 was identified as the characteristic peak of the hydrogen of the hydroxyl on the benzene ring; the single peak at the chemical shift of 7.12 and that at 7.20 were respectively identified as the characteristic peak of the two hydrogens on the benzene ring. If the integral of the hydrogen of the hydroxyl on the benzene ring is defined as 1, the value of the integral ratio between the hydrogen on the benzene ring, the hydrogen of the mercapto and the hydrogen of the hydroxyl were caculated as 0.95 : 0.97 : 1.05 : 0.94, approximating to the theoretical value of 1 : 1 : 1 : 1 : 1. As can be seen from this NMR spectra analysis, the polyisobutenyl thiophenol was obtained as anticipated.

### Example II-2

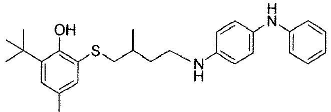

Under the protective atomosphere of nitrogen gas, to a 250ml four-necked flask equiped with a stirrer, a thermometer, a condensing tube and a dropping funnel, there were added 30.58g (156mmol) 2-mercapto-4-methyl-6-methyl phenol, 15.79g (112mmol) 1,4-dichloro-2-methyl butane, 32.38g (176mmol) N-phenyl p-phenylene diamine, 3.45g (25mmol) potassium carbonate and 100mL toluene, rapidly stirred, reacted at 90 degrees Celsius for 1h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.00 (3H), 1.31 (1H), 1.40 (9H), 1.78 (1H), 1.98 (1H), 2.37 (3H), 2.99 (2H), 3.33 (2H), 4.83 (1H), 6.95-7.26 (12H), 7.55 (1H);
¹³C NMR (75MHz, CDCl₃) : δ 20.4, 21.2, 30.1, 33.7, 34.5, 43.8, 114.3, 118.9, 119.4, 121.8, 125.8, 129.5, 132.3, 133.5, 146.1, 149.0, 150.8;
C₂₈H₃₆N₂OS (calculated) : C 74.96, H 8.09, N 6.24, O 3.57, S 7.15; (measured) : C 74.88, H 8.13, N 6.21, O 3.54, S 7.09.

### Example II-3

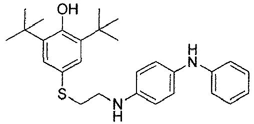

Under the protective atomosphere of nitrogen gas, to a 250ml four-necked flask equiped with a stirrer, a thermometer, a condensing tube and a dropping funnel, there were added 31.65g (133mmol) 2,6-di-tert-butyl-4-mercaptophenol, 11.66g (62mmol) dibromoethane, 28.15g (153mmol) N-phenyl p-phenylene diamine, 0.79g (7.5mmol) sodium carbonate and 100mL benzene, rapidly stirred, reacted at 25 degrees Celsius for 24h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.36 (18H), 3.15 (2H), 3.63 (2H), 5.17 (1H), 5.32 (1H), 6.97-7.26 (11H), 7.55 (1H);
¹³C NMR (75MHz, CDCl₃) : δ 29.6, 32.7, 34.6, 47.5, 114.3, 118.9, 119.4, 121.8, 126.2, 127.5, 129.5, 132.3, 136.5, 146.1, 149.0, 153.5;
C₂₈H₃₆N₂OS (calculated) : C 74.96, H 8.09, N 6.24, O 3.57, S 7.15; (measured) : C 75.06, H 8.13, N 6.11, O 3.61, S 7.09.

### Example II-4

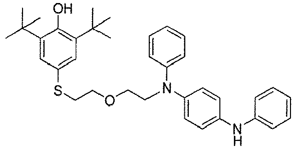

Under the protective atomosphere of nitrogen gas, to a 250ml four-necked flask equiped with a stirrer, a thermometer, a condensing tube and a dropping funnel, there were added 8.57g (36mmol) 2,6-di-tert-butyl-4-mercaptophenol, 2.15g (15mmol) dichlorodiethyl ether, 10.14g (39mmol) N,N'-diphenyl-1,4-phenylene diamine, 0.55g (5.1mmol) sodium carbonate and 100mL toluene, rapidly stirred, reacted at 90 degrees Celsius for 4h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.36 (18H), 2.98 (2H), 3.34 (2H), 3.63 (2H), 3.76 (2H), 5.32 (1H), 6.80-7.27 (16H), 7.55 (1H);
¹³C NMR (75MHz, CDCl₃) : δ 29.6, 32.1, 34.6, 46.9, 69.4, 69.6, 114.0, 116.6, 117.1, 119.3, 121.8, 126.2, 129.2, 136.5, 142.8, 146.1, 148.7, 153.5;
C₃₆H₄₄N₂O₂S (calculated) : C 76.02, H 7.80, N 4.92, O 5.63, S 5.64; (measured) : C 76.09, H 7.85, N 4.83, O 5.65, S 5.60.

### Example II-5

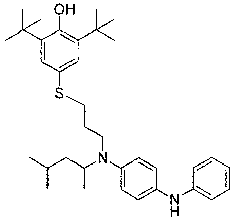

Under the protective atomosphere of nitrogen gas, to a 250ml four-necked flask equiped with a stirrer, a thermometer, a condensing tube and a dropping funnel, there were added 20.23g (85mmol) 2,6-di-tert-butyl-4-mercaptophenol, 12.99g (115mmol) 1,3-dichloropropane, 18.49g (69mmol) N-(1,3-dimethyl butyl)-N'-phenyl p-phenylene diamine, 0.76g (7.2mmol) sodium carbonate and 150mL benzene, rapidly stirred, reacted at 85 degrees Celsius for 3h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 0.80 (6H), 1.11-1.21 (5H), 1.36 (18H), 1.67 (1H), 2.16-3.47 (6H), 5.32 (1H), 6.80-7.17 (7H), 7.55 (1H);
¹³C NMR (75MHz, CDCl₃) : δ 22.4, 24.6, 34.6, 45.2, 52.7, 58.6, 115.4, 116.6, 119.4, 121.8, 126.2, 129.5, 136.6, 144.1, 153.5;
C₃₅H₅₀N₂OS (calculated) : : C 76.87, H 9.22, N 5.12, O 2.93, S 5.86; (measured) : C 76.95, H 9.28, N 5.08, O 2.87, S 5.80.

### Example II-6

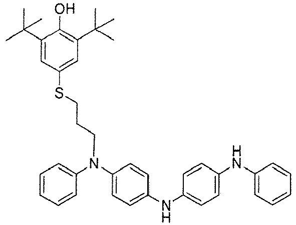

Under the protective atomosphere of nitrogen gas, to a 250ml four-necked flask equiped with a stirrer, a thermometer, a condensing tube and a dropping funnel, there were added 3.57g (15mmol) 2,6-di-tert-butyl-4-mercaptophenol, 20.81g (103mmol) 1,3-dibromopropane, 21.76g (103mmol) N-phenyl-N'-[4-(phenylamino) phenyl]-1,4-phenylene diamine, 0.29g (2.1 mmol) sodium carbonate and 150mL benzene, rapidly stirred, reacted at 110 degrees Celsius for 4h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.36 (18H), 1.94 (2H), 2.95 (2H), 3.43 (2H), 5.32 (1H), 6.80-7.17 (16H), 7.20 (1H), 7.26 (4H), 7.55 (1H);
¹³C NMR (75MHz, CDCl₃) : δ 28.5, 29.6, 29.9, 34.6, 47.2, 114.0, 116.6, 117.7, 118.4, 121.8, 126.2, 129.2, 132.3, 136.5, 142.8, 146.1, 148.7, 153.5;
C₄₁H₄₇N₃OS (calculated) : C 78.18, H 7.52, N 6.67, O 2.54, S 5.09; (measured) : C 78.29, H 7.57, N 6.55, O 2.51, S 5.10.

### Example II-7

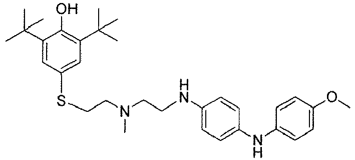

Under the protective atomosphere of nitrogen gas, to a 250ml four-necked flask equiped with a stirrer, a thermometer, a condensing tube and a dropping funnel, there were added 34.51g (145mmol) 2,6-di-tert-butyl-4-mercaptophenol, 5.95g (35mmol) 2-chloroethyl-3-chloropropylmethylamine, 4.49g (21 mmol) 4-amino-4'-methoxy diphenyl amine, 0.28g (2.6mmol) sodium carbonate and 150mL benzene, rapidly stirred, reacted at 90 degrees Celsius for 2h. Upon completion of the reaction, the solvent and water (generated during the reaction) were removed by vacuum distillation, the titled hindered phenol compound was obtained by using column chromatography. The compound was characterized as follows :
¹H NMR (300MHz, CDCl₃) : δ 1.36 (18H), 2.27 (3H), 2.65 (2H), 2.99 (2H), 3.09 (2H), 3.46 (2H), 3.76 (3H), 4.10 (1H), 5.32 (1H), 6.97-7.17 (10H), 7.20 (1H);
¹³C NMR (75MHz, CDCl₃) : δ 29.6, 34.5, 35.1,40.6, 45.2, 53.4, 54.3, 55.5, 114.3, 118.9, 120.1, 126.2, 132.3, 136.5, 149.0, 153.4, 154.5;
C₃₂H₄₅N₃O₂S (calculated) : C 71.73, H 8.47, N 7.84, O 5.97, S 5.98; (measured) : C 71.69, H 8.43, N 7.91, O 5.92, S 5.95.

### Example II-8

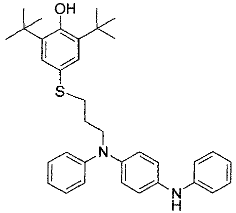

Under the protective atomosphere of nitrogen gas, to a 250ml four-necked flask equiped with a stirrer, a thermometer, a condensing tube and a dropping funnel, there were added 21.66g (91 mmol) 2,6-di-tert-butyl-4-mercaptophenol, 1.07g (9.51 mmol) 1,3-dichloropropane, 6.51g (25mmol) N,N'-diphenyl-1,4-phenylene diamine, 0.65g (6.5mmol) potassium hydrogen carbonate and 150mL benzene, rapidly stirred, reacted at 70 degrees Celsius for 6h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.36 (18H), 2.26 (2H), 3.05 (2H), 3.48 (2H), 5.32 (1H), 6.80-7.17 (12H), 7.27 (2H), 7.55 (1H);
¹³C NMR (75MHz, CDCl₃) : δ 28.5, 29.6, 33.4, 34.6, 44.2, 81.5, 114.0, 117.1, 119.4, 121.8, 126.2, 129.5, 136.6, 142.8, 146.1, 148.7, 153.4;
C₃₅H₄₂N₂OS (calculated) : C 78.02, H 7.86, N 5.20, O 2.97, S 5.95; (measured) : C 78.09, H 7.91, N 5.15, O 2.93, S 5.87.

### Example II-9

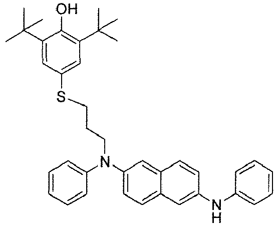

Under the protective atomosphere of nitrogen gas, to a 250ml four-necked flask equiped with a stirrer, a thermometer, a condensing tube and a dropping funnel, there were added 8.33g (35mmol) 2,6-di-tert-butyl-4-mercaptophenol, 10.30g (51 mmol) 1,3-dibromopropane, 9.61g (31mmol) N,N'-diphenyl-2,6-naphthalene diamine, 0.24g (1.71 mmol) potassium carbonate and 150mL toluene, rapidly stirred, reacted at 110 degrees Celsius for 4h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.36 (18H), 1.99 (2H), 3.06 (2H), 3.52 (2H), 5.32 (1H), 5.80 (1H), 6.99-7.27 (15H), 7.40 (1H), 7.84 (2H);
¹³C NMR (75MHz, CDCl₃) : δ 28.5, 29.6, 29.9, 34.6, 43.0, 106.9, 111.1, 114.0, 116.5, 119.9, 121.4, 126.2, 128.9, 129.1, 129.4, 136.5, 140.7, 142.8, 147.5, 148.7, 153.5;
C₃₉H₄₄N₂OS (calculated) : C 79.55, H 7.53, N 4.76, O 2.72, S 5.45; (measured) : C 79.49, H 7.51, N 4.79, O 2.70, S 5.41.

### Example II-10

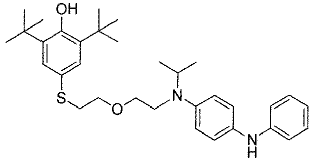

Under the protective atomosphere of nitrogen gas, to a 250ml four-necked flask equiped with a stirrer, a thermometer, a condensing tube and a dropping funnel, there were added 6.19g (26mmol) 2,6-di-tert-butyl-4-mercaptophenol, 13.16g (92mmol) dichlorodiethyl ether, 3.84g (17mmol) N-isopropyl-N'-phenyl p-phenylene diamine, 0.43g (3.1mmol) potassium carbonate and 150mL ethanol, rapidly stirred, reacted at 80 degrees Celsius for 2h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.19 (6H), 1.36 (18H), 3.04 (2H), 3.25 (2H), 3.35 (2H), 3.43 (1H), 3.49 (2H), 5.32 (1H), 6.80-7.26 (11H), 7.55 (1H);
¹³C NMR (75MHz, CDCl₃) : δ 19.9, 29.6, 31.9, 34.6, 44.5, 54.3, 69.24, 116.6, 117.2, 119.3, 121.8, 126.2, 129.5, 136.5, 142.8, 146.1, 153.4, 154.8;
C₃₃H₄₆N₂O₂S (calculated) : C 74.11, H 8.67, N 5.24, O 5.98, S 6.00; (measured) : C 74.05, H 8.61, N 5.29, O 5.93, S 6.07.

### Example II-11

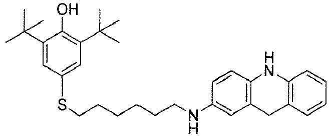

Under the protective atomosphere of nitrogen gas, to a 250ml four-necked flask equiped with a stirrer, a thermometer, a condensing tube and a dropping funnel, there were added 4.99g (21mmol) 2,6-di-tert-butyl-4-mercaptophenol, 7.56g (31 mmol) 1,6-dibromohexane, 5.70g (31mmol) N-phenyl p-phenylene diamine, 0.12g (0.85mmol) potassium carbonate and 150mL ethanol, rapidly stirred, after reacted at 60 degrees Celsius for 2.5h, cooled to the room temperature, there were added 7.05g (85mmol) formaldehyde, heated to 85 degrees Celsius and reacted for 2.5h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.32 (2H), 1.36 (18H), 1.41 (2H), 1.64-1.81 (4H), 2.93-3.28 (4H), 4.14 (2H), 4.43 (1H), 4.59 (1H), 5.32 (1H), 6.82-7.21 (9H);
¹³C NMR (75MHz, CDCl₃) : δ 26.8, 28.3, 28.6, 29.6, 31.8, 33.1, 34.3, 44.56, 113.0, 118.9, 119.6, 122.4, 123.3, 125.1, 126.4, 127.9, 132.2, 140.5, 148.5, 153.4;
C₃₃H₄₄N₂OS (calculated) : C 76.70, H 8.58, N 5.42, O 3.10, S 6.20; (measured) : C 76.69, H 8.51, N 5.49, O 3.12, S 6.18.

### Example II-12

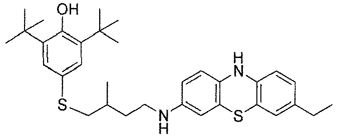

Under the protective atomosphere of nitrogen gas, to a 250ml four-necked flask equiped with a stirrer, a thermometer, a condensing tube and a dropping funnel, there were added 19.52g (82mmol) 2,6-di-tert-butyl-4-mercaptophenol, 39.20g (121 mmol) 1,4-diiodo-2-methyl butane, 19.93g (94mmol) 4-amino-4'-ethyl diphenyl amine, 2.22g (16.1mmol) potassium carbonate and 100mL toluene, rapidly stirred, after reacted at 90 degrees Celsius for 3h, cooled to the room temperature, to the toluene layer, there were added 10.11g (316mmol) sulfur and 0.04g (0.35mmol) iodine, heated to 150 degrees Celsius and reacted for 8h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.00 (3H), 1.23 (3H), 1.36 (18H), 1.87 (2H), 2.06 (1H), 2.65 (2H), 2.82 (2H), 3.29 (2H), 4.25 (1H), 5.32 (1H), 5.70 (1H), 6.72 (1H), 6.98-7.08 (4H), 7.17 (2H), 7.49 (1H);
¹³C NMR (75MHz, CDCl₃) : δ 15.60, 20.3, 29.0, 29.6, 34.6, 44.2, 113.7, 116.1, 118.9, 126.2, 127.9, 129.7, 136.6, 141.2, 146.1, 153.4;
C₃₃H₄₄N₂OS₂ (calculated) : C 72.22, H 8.08, N 5.10, O 2.92, S 11.68; (measured) : C 72.29, H 8.14, N 5.08, O 2.86, S 11.57.

### Example II-13

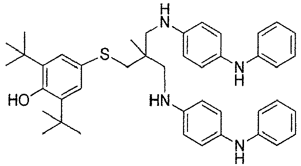

Under the protective atomosphere of nitrogen gas, to a 250ml four-necked flask equiped with a stirrer, a thermometer, a condensing tube and a dropping funnel, there were added 10.95g (46mmol) 2,6-di-tert-butyl-4-mercaptophenol, 8.42g (48mmol) 1,1,1-trichloromethyl ethane, 18.95g (103mmol) N-phenyl p-phenylene diamine, 0.71g (5.12mmol) potassium carbonate and 150mL benzene, rapidly stirred, reacted at 85 degrees Celsius for 1h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.01 (3H), 1.36 (18H), 2.89 (2H), 3.03 (4H), 4.25 (2H), 5.32 (1H), 69.7-7.26 (20H), 7.55 (2H);
¹³C NMR (75MHz, CDCl₃) : δ 21.4, 29.6, 34.6, 38.0, 43.2, 54.0, 114.3, 118.7, 119.3, 121.8, 126.2, 129.5, 132.3, 136.5, 146.1, 149.0, 153.4;
C₄₃H₅₂N₄OS (calculated) : C 76.74, H 7.79, N 8.33, O 2.38, S 4.76; (measured) : C 76.69, H 7.73, N 8.45, O 2.32, S 4.71.

### Example II-14

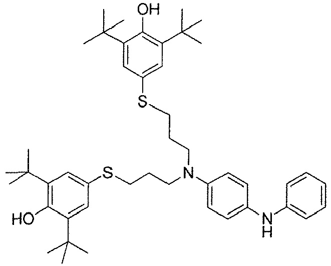

Under the protective atomosphere of nitrogen gas, to a 250ml four-necked flask equiped with a stirrer, a thermometer, a condensing tube and a dropping funnel, there were added 21.66g (91 mmol) 2,6-di-tert-butyl-4-mercaptophenol, 17.98g (89mmol) 1,2-dibromopropane, 6.62g (36mmol) N-phenyl p-phenylene diamine, 0.62g (5.12mmol) potassium carbonate and 150mLisopropanol, rapidly stirred, reacted at 80 degrees Celsius for 3h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.36 (36H), 1.95 (4H), 3.04 (4H), 3.12 (4H), 5.32 (2H), 6.80-7.26 (13H), 7.55 (1H);
¹³C NMR (75MHz, CDCl₃) : δ 28.5, 29.6, 34.6, 48.4, 101.3, 116.6, 117.1, 119.3, 121.8, 126.2, 129.5, 136.5, 142.8, 146.1, 149.1, 153.4;
C₄₆H₆₄N₂O₂S₂ (calculated) : C 74.55, H 8.70, N 3.78, O 4.32, S 8.65; (measured) : C 74.59, H 8.73, N 3.67, O 4.35, S 8.64.

### Example II-15

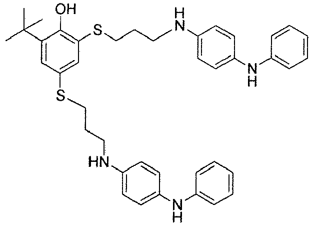

Under the protective atomosphere of nitrogen gas, to a 250ml four-necked flask equiped with a stirrer, a thermometer, a condensing tube and a dropping funnel, there were added 7.70g (36mmol) 2-tert-butyl-4,6-dimercaptophenol, 10.40g (92mmol) 1,2-dichloropropane, 13.06g (71mmol) N-phenyl p-phenylene diamine, 0.43g (3.15mmol) potassium carbonate and 150mL toluene, rapidly stirred, reacted at 90 degrees Celsius for 4h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.36 (9H), 1.88 (4H), 2.85 (4H), 3.17 (4H), 3.55 (2H), 6.97-7.26 (19H), 7.28 (1H), 7.55 (2H), 8.30 (1H);
¹³C NMR (75MHz, CDCl₃) : δ 28.8, 29.2, 30.0, 31.4, 34.5, 44.8, 45.4, 113.5, 114.3, 118.9, 121.8, 122.7, 125.3, 126.2, 129.5, 132.3, 146.4, 149.0, 154.9;
C₄₀H₄₆N₄OS₂ (calculated) : C 72.47, H 6.99, N 8.45, O 2.41, S 9.67; (measured) : C 72.52, H 7.03, N 8.37, O 2.43, S 9.64.

### Example II-16

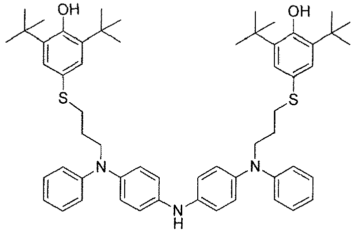

Under the protective atomosphere of nitrogen gas, to a 250ml four-necked flask equiped with a stirrer, a thermometer, a condensing tube and a dropping funnel, there were added 18.56g (78mmol) 2-tert-butyl-4-mercaptophenol, 10.74g (95mmol) 1,2-dichloropropane, 11.23g (32mmol) N-phenyl-N'-[4-(phenylamino)phenyl]-1,4-phenylene diamine, 0.79g (5.71 mmol) potassium carbonate and 150mL toluene, rapidly stirred, reacted at 100 degrees Celsius for 3h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.36 (36H), 2.02 (4H), 3.03 (4H), 3.50 (4H), 5.32 (2H), 6.80-7.17 (18H), 7.20 (1H), 7.27 (4H);
¹³C NMR (75MHz, CDCl₃) : δ 28.5, 29.6, 30.1, 31.8, 34.6, 49.9, 92.4, 114.0, 116.6, 117.1, 122.0, 126.2, 129.2, 136.5, 142.8, 148.7, 153.4;
C₅₈H₇₃N₃O₂S₂ (calculated) : C 76.69, H 8.10, N 4.63, O 3.52, S 7.06; (measured) : C 76.61, H 8.05, N 4.71, O 3.49, S 7.03.

### Example II-17

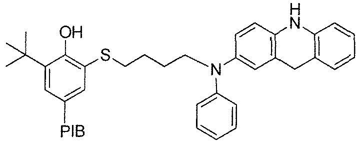

Under the protective atomosphere of nitrogen gas, to a 250ml four-necked flask equiped with a stirrer, a thermometer, a condensing tube and a dropping funnel, there were added 47.16g (45mmol) polyisobutenyl thiophenol produced by Example II-1, 6.48g (51 mmol) 1,4-dichlorobutane, 13.78g (53mmol) N,N'-diphenyl-1,4-phenylene diamine, 0.86g (8.15mmol) sodium carbonate and 150mL benzene, rapidly stirred, reacted at 80 degrees Celsius for 2.5h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 0.88, 0.98, 1.33, 1.40, 1.59, 1.64, 1.73, 1.82, 1.93, 3.25, 3.38, 3.71,4.86, 6.97, 7.02, 7.19, 7.26, 7.55;
¹³C NMR (75MHz, CDCl₃) : δ 27.1,28.9, 30.1, 31.6, 32.3, 34.5, 37.9, 43.9, 57.1, 114.0, 117.7, 119.4, 121.8, 129.5, 131.6, 144.5, 146.1, 148.7, 155.0.

### Example II-18

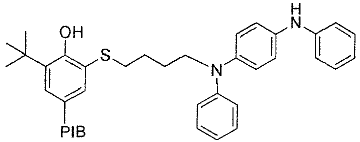

Under the protective atomosphere of nitrogen gas, to a 250ml four-necked flask equiped with a stirrer, a thermometer, a condensing tube and a dropping funnel, there were added 29.34g (28mmol) polyisobutenyl thiophenol produced by Example II-1, 2.91g (35mmol) formaldehyde, 6.24g (24mmol) N,N'-diphenyl-1,4-phenylene diamine, 0.34g (3.2mmol) sodium carbonate and 150mL toluene, rapidly stirred, reacted at 90 degrees Celsius for 2h. Upon completion of the reaction, the solvent and water

(generated during the reaction) were removed by vacuum distillation, the titled hindered phenol compound was obtained by using column chromatography.

The compound was characterized as follows :
¹H NMR (300MHz, CDCl₃) : δ 0.86, 0.98, 1.02, 1.40, 1.60, 1.83, 2.59, 3.25, 3.72, 4.86, 6.80, 6.97, 7.02, 7.17, 7.26, 7.55;
¹³C NMR (75MHz, CDCl₃) : δ 28.2, 30.1, 32.3, 33.1, 34.5, 38.1,49.8, 59.1, 114.3, 119.4, 121.8, 125.2, 122.0, 127.3, 129.5, 132.3, 143.3, 146.1, 151.3.

### Comparative Example II-1

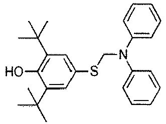

Under the protective atomosphere of nitrogen gas, to a 250ml four-necked flask equiped with a stirrer, a thermometer, a condensing tube and a dropping funnel, there were added 20.23g (85mmol) 2,6-di-tert-butyl-4-mercaptophenol, 7.22g (87mmol) formaldehyde, 14.37g (85mmol) diphenyl amine and 150mL methanol, rapidly stirred, reacted at 60 degrees Celsius for 2h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ1.36 (18H), 5.21 (2H), 5.32 (1H), 6.99 (6H), 7.17 (2H), 7.27 (4H);
¹³C NMR (75MHz, CDCl₃): δ 29.6, 34.6, 55.2, 120.4, 123.3, 125.9, 126.2, 129.2, 136.6, 150.0, 153.5;
C₂₇H₃₃NOS (calculated) : C 77.28, H 7.93, N 3.34, O 3.81, S 7.64; (measured) : C 77.22, H 7.89, N 3.31, O 3.83, S 7.67.

### Comparative Example II-2

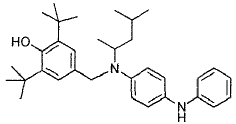

Under the protective atomosphere of nitrogen gas, to a 250ml four-necked flask equiped with a stirrer, a thermometer, a condensing tube and a dropping funnel, there were added 10.71g (52mmol) 2,6-di-tert-butyl-4-mercaptophenol, 1.89g (63mmol) formaldehyde, 17.42g (65mmol) N- (1,3-dimethyl butyl)-N'-phenyl p-phenylene diamine and 150mL methanol, rapidly stirred, reacted at 70 degrees Celsius for 4h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 0.80 (6H), 1.11 (3H), 1.29 (1H), 1.36 (18H), 3.45 (1H), 4.53 (2H), 5.32 (2H), 6.97-7.07 (9H), 7.26 (2H), 7.55 (1H);
¹³C NMR (75MHz, CDCl₃) : δ17.9, 22.40, 24.6, 30.4, 34.3, 45.7, 54.5, 56.7, 116.6, 120.4, 121.8, 125.9, 128.9, 129.5, 135.6, 146.1, 153.5, 154.8;
C₃₃H₄₆N₂O (calculated) : C 81.43, H 9.53, N 5.76, O 3.29; (measured): C 81.38, H 9.51, N 5.79, O 3.31.

### Comparative Example II-3

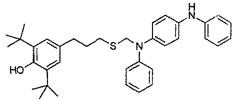

Under the protective atomosphere of nitrogen gas, to a 250ml four-necked flask equiped with a stirrer, a thermometer, a condensing tube and a dropping funnel, there were added 9.24g (33mmol) 2,6-ditert-butyl-4-(3-mercaptopropyl) phenol, 3.07g (37mmol) formaldehyde, 11.71g (45mmol) N,N'-diphenyl-1,4-phenylene diamine and 150mL benzene, rapidly stirred, reacted at 80 degrees Celsius for 4h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.40 (18H), 1.99 (1H), 2.61-2.80 (6H), 4.63 (2H), 5.32 (1H), 6.97-7.02 (12H), 7.26 (4H), 7.55 (1H);
¹³C NMR (75MHz, CDCl₃) : δ 28.7, 31.1, 34.3, 35.7, 54.1, 117.1, 119.4, 123.3, 124.8, 129.2, 131.8, 136.0, 142.8, 144.9, 146.1, 151.9;
C₃₆H₄₄N₂OS (calculated) : C 78.22, H 8.02, N 5.07, O 2.89, S 5.80; (measured) : C 78.27, H 7.96, N 4.98, O 2.92, S 5.83.

### Example II-19 to Example II-35 and Comparative Example II-4 to Comparative Example II-8

Each of the hindered phenol compounds of Example II-2 to Example II-18 and Comparative Example II-1 to Comparative Example II-3 was respectively mixed with a lubricant base oil in line with the composition and ratio specified in Table II-1 at 40 degrees Celsius for 2h, to obtain the lubricant oil compositions of Example II-19 to Example II-35 and the lubricant oil compositions of Comparative Example II-4 to Comparative Example II-6. Further, in line with the composition and ratio specified in Table II-2, the antioxidant was respectively mixed with a lubricant base oil at 40 degrees Celsius for 2h, to obtain the lubricant oil compositions of Comparative Example II-7 to Comparative Example II-10. Herein, based on the total weight of each lubricant oil composition, the amount of the antioxidant to be used in each case was 0.5wt%, the lubricant base oil used was a Group II hydrogenated base oil (produced by Sinopec Group,Gaoqiao Shanghai Branch). Further, the base oil without the antioxidant was taken as Control.

**Table II-1**

| lubricant oil composition | antioxidant | lubricant base oil | antioxidant amount |
|---|---|---|---|
| Example II-19 | Example II-2 | II | 0.50% |
| Example II-20 | Example II-3 | II | 0.50% |
| Example II-21 | Example II-4 | II | 0.50% |
| Example II-22 | Example II-5 | II | 0.50% |
| Example II-23 | Example II-6 | II | 0.50% |
| Example II-24 | Example II-7 | II | 0.50% |
| Example II-25 | Example II-8 | II | 0.50% |
| Example II-26 | Example II-9 | II | 0.50% |
| Example II-27 | Example II-10 | II | 0.50% |
| Example II-28 | Example II-11 | II | 0.50% |
| Example II-29 | Example 11-12 | II | 0.50% |
| Example II-30 | Example II-13 | II | 0.50% |
| Example II-31 | Example II-14 | II | 0.50% |
| Example II-32 | Example II-15 | II | 0.50% |
| Example II-33 | Example II-16 | II | 0.50% |
| Example II-34 | Example II-17 | II | 0.50% |
| Example II-35 | Example II-18 | II | 0.50% |
| Comparative Example II-4 | Comparative Example II-1 | II | 0.50% |
| Comparative Example II-5 | Comparative Example II-2 | II | 0.50% |
| Comparative Example II-6 | Comparative Example II-3 | II | 0.50% |
| Control | - | II | - |

**Table II-2**

| lubricant oil composition | hindered phenol compound | aromatic amine | hindered phenol compoun d amount | aromati c amine amount | lubricant t base oil |
|---|---|---|---|---|---|
| Comparativ e Example II-7 | 2,2'-thiobis[3-(3,5-ditert-butyl-4-hydrox yl phenyl)propionic acid ethyl ester] | - | 0.50% | - | II |
| Comparativ e Example II-8 | - | N-(4-tert-octyl phenyl)-1-naphthylamin e | - | 0.50% | II |
| Comparativ e Example II-9 | 2,6-ditert-butyl-4-amylthiophenol | 4,4'-ditert-octyl diphenyl amine | 0.25% | 0.25% | II |
| Comparativ e Example II-10 | 4,4'-[thiobismethylene]bis[2,6-ditert-but yl phenol] | 3,7-ditert-octyl phenothiazine | 0.30% | 0.20% | II |

The lubricant oil compositions of Example II-19 to Example II-35, the lubricant oil compositions of Comparative Example II-4 to Comparative Example II-10 and the Control were taken as test samples, to evaluate the oxidation resistance at elevated temperatures, with a PDSC temperature of 210 degrees Celsius, the results of were shown in Table II-3.

**Table II-3**

| lubricant oil composition | PDSC oxidative induction period (min) | lubricant oil composition | PDSC oxidative induction period (min) |
|---|---|---|---|
| Example II-19 | 17.5 | Example II-32 | 18.9 |
| Example II-20 | 21.3 | Example II-33 | 22.4 |
| Example II-21 | 19.7 | Example II-34 | 16.3 |
| Example II-22 | 20.1 | Example II-35 | 17.1 |
| Example II-23 | 22.6 | Comparative Example II-4 | 4.9 |
| Example II-24 | 18.7 | Comparative Example II-5 | 7.5 |
| Example II-25 | 17.6 | Comparative Example II-6 | 16.1 |
| Example II-26 | 26.1 | Comparative Example II-7 | 8.6 |
| Example II-27 | 21.3 | Comparative Example II-8 | 15.2 |
| Example II-28 | 22.6 | Comparative Example II-9 | 5.3 |
| Example II-29 | 23.7 | Comparative Example II-10 | 7.1 |
| Example II-30 | 22.5 | Control | 0.2 |
| Example II-31 | 20.3 | | |

The lubricant oil compositions of Example II-19 to Example II-35, the lubricant oil compositions of Comparative Example II-4 to Comparative Example II-10 and the Control were taken as test samples, to evaluate the deposite formation inhibition performance, the results were shown in Table II-4.

**Table II-4**

| lubricant oil composition | deposit amount (mg) |
|---|---|
| Example II-22 | 16.9 |
| Example II-25 | 15.1 |
| Example II-29 | 10.8 |
| Example II-31 | 13.5 |
| Example II-34 | 6.8 |
| Example II-35 | 7.1 |
| Comparative Example II-4 | 19.5 |
| Comparative Example II-5 | 23.4 |
| Comparative Example II-6 | 20.8 |
| Comparative Example II-7 | 21.6 |
| Comparative Example II-8 | 25.6 |
| Comparative Example II-9 | 19.2 |
| Comparative Example II-10 | 18.5 |
| Control | 32.8 |

The lubricant oil compositions of Example II-19 to Example II-35, the lubricant oil compositions of Comparative Example II-4 to Comparative Example II-10 and the Control were taken as test samples, to evaluate the anticorrosion performance. The results of BRT (ball rust test) were shown in Table II-5.

**Table II-5**

| lubricant oil composition | corrosion degree |
|---|---|
| Example II-22 | slight |
| Example II-25 | slight |
| Example II-29 | slight |
| Example II-31 | moderate |
| Example II-34 | slight |
| Example II-35 | slight |
| Comparative Example II-4 | moderate |
| Comparative Example II-5 | moderate |
| Comparative Example II-6 | moderate |
| Comparative Example II-7 | moderate |
| Comparative Example II-8 | moderate |
| Comparative Example II-9 | moderate |
| Comparative Example II-10 | moderate |
| Control | severe |

The lubricant oil compositions of Example II-19 to Example II-35, the lubricant oil compositions of Comparative Example II-4 to Comparative Example II-10 and the Control were taken as test samples, to evaluate the performances of inhibiting inrease in viscosity and inhibiting inrease in acid value, the results of were shown in Table II-6.

**Table II-6**

| lubricant oil composition | increase in viscosity (%) | increase in acid value (mgKOH·g⁻¹) |
|---|---|---|
| Example II-19 | 25.7 | 2.523 |
| Example II-20 | 21.3 | 1.967 |
| Example 11-21 | 20.9 | 2.031 |
| Example II-22 | 23.4 | 1.834 |
| Example II-23 | 21.6 | 2.007 |
| Example II-24 | 19.9 | 1.679 |
| Example II-25 | 21.5 | 1.372 |
| Example II-26 | 18.7 | 2.021 |
| Example II-27 | 19.3 | 2.318 |
| Example II-28 | 21.7 | 1.936 |
| Example 11-29 | 20.6 | 2.217 |
| Example 11-30 | 22.3 | 1.894 |
| Example II-31 | 19.3 | 2.334 |
| Example II-32 | 21.5 | 2.119 |
| Example II-33 | 24.3 | 1.793 |
| Example II-34 | 23.8 | 2.106 |
| Example II-35 | 21.5 | 1.984 |
| Comparative Example II-4 | 33.5 | 4.621 |
| Comparative Example II-5 | 27.5 | 3.482 |
| Comparative Example II-6 | 25.8 | 5.168 |
| Comparative Example II-7 | 27.6 | 2.953 |
| Comparative Example II-8 | 30.5 | 3.846 |
| Comparative Example II-9 | 28.1 | 5.429 |
| Comparative Example II-10 | 24.7 | 6.517 |
| Control | 67.2 | 24.525 |

### Example III-1

To a 500ml four-necked flask equiped with a stirrer, a thermometer, a condensing tube and a dropping funnel, there were added 58.79g (0.323mol) 2-tert-butyl-6-mercaptophenol, 6.88g (0.048mol) boron trifluoride diethyl ether (an alkylation catalyst), 100ml n-hexane (as the solvent) and 161.61g (0.162mol) polyisobutene (Mn=1000, from Jilin Chemical Group Fine Chemicals Co., Ltd.), reacted at 80 degrees Celsius for 2h. Upon completion of the reaction, the reaction mixture was washed once with a 5wt% KOH aqueous solution, and then washed with hot water till neutral to remove any catalyst, and then vacuum distillated to remove any solvent and unreacted phenols, to obtain a polyisobutenyl thiophenol, having a hydroxyl value of 53.49mgKOH/g. The hydroxyl value was determined by referring to the acetic anhydride method in GB/T7383-2007.

The reaction procedure can be illustrated as follows.

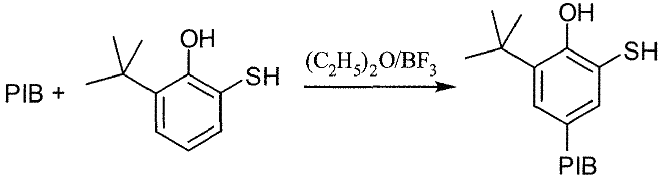

On the ¹H-NMR spectra of the produced polyisobutenyl thiophenol, the peak at the chemical shift of 1.40 was identified as the characteristic peak of the hydrogen of the tert-butyl on the benzene ring; the single peak at the chemical shift of 3.58 was identified as the characteristic peak of the hydrogen of the mercapto on the benzene ring; the single peak at the chemical shift of 4.84 was identified as the characteristic peak of the hydrogen of the hydroxyl on the benzene ring; the single peak at the chemical shift of 7.12 and that at 7.20 were respectively identified as the characteristic peak of the two hydrogens on the benzene ring. If the integral of the hydrogen of the hydroxyl on the benzene ring is defined as 1, the value of the integral ratio between the hydrogen on the benzene ring, the hydrogen of the mercapto and the hydrogen of the hydroxyl were caculated as 0.95 : 0.97 : 1.05 : 0.94, approximating to the theoretical value of 1 : 1 : 1 : 1 : 1. As can be seen from this NMR spectra analysis, the polyisobutenyl thiophenol was obtained as anticipated.

### Example III-2

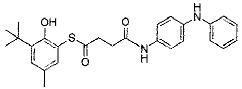

Under the protective atomosphere of nitrogen gas, to a 250ml four-necked flask equiped with a stirrer, a thermometer, a condensing tube and a dropping funnel, there were added 30.58g (156mmol) 2-mercapto-4-methyl-6-methyl phenol, 11.21g (112mmol) succinic anhydride, 32.38g (176mmol) N-phenyl p-phenylene diamine, 3.45g (25mmol) potassium carbonate and 100mL acetone, rapidly stirred, reacted at the room temperature for 2h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.40 (9H), 2.37 (3H), 2.86 (2H), 3.01 (2H), 4.84 (1H), 6.95-7.26 (11H), 7.45 (1H), 7.55 (1H);
¹³C NMR (75MHz, CDCl₃) : δ 21.2, 30.1, 34.5, 36.4, 39.3, 114.3, 119.4, 121.8, 125.8, 129.5, 132.3, 133.5, 137.5, 146.1, 153.5, 174.0, 196.9;
C₂₇H₃₀N₂O₃S (calculated) : C 70.10, H 6.54, N 6.06, O 10.38, S 6.93; (measured) : C 70.03, H 6.54, N 6.01, O 10.47, S 6.85.

### Example III-3

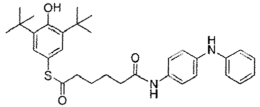

Under the protective atomosphere of nitrogen gas, to a 250ml four-necked flask equiped with a stirrer, a thermometer, a condensing tube and a dropping funnel, there were added 31.65g (133mmol) 2,6-di-tert-butyl-4-mercaptophenol, 7.94g (62mmol) adipic anhydride, 28.15g (153mmol) N-phenyl p-phenylene diamine, 0.79g (7.5mmol) sodium carbonate and 100mLisopropanol, rapidly stirred, reacted at the room temperature for 24h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.36 (18H), 1.57 (2H), 1.72 (2H), 2.23 (2H), 2.66 (2H), 5.32 (2H), 6.97-7.26 (9H), 7.45 (1H), 7.48 (2H), 7.55 (1H);
¹³C NMR (75MHz, CDCl₃) : δ 24.9, 25.4, 29.6, 34.6, 37.8, 43.5, 114.3, 119.3, 121.8, 123.8, 126.2, 132.3, 136.5, 137.5, 146.1, 153.4, 171.6, 196.9;
C₃₂H₄₀N₂O₃S (calculated) : C 72.14, H 7.57, N 5.26, O 9.01, S 6.02; (measured) : C 72.24, H 7.59, N 5.18, O 9.05, S 5.95.

### Example III-4

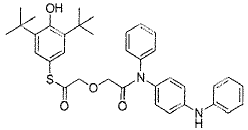

Under the protective atomosphere of nitrogen gas, to a 250ml four-necked flask equiped with a stirrer, a thermometer, a condensing tube and a dropping funnel, there were added 8.57g (36mmol) 2,6-di-tert-butyl-4-mercaptophenol, 2.56g (15mmol) 2,2'-oxydiacetyl chloride, 10.14g (39mmol) N,N'-diphenyl-1,4-phenylene diamine and 100mL toluene, rapidly stirred, reacted at 90 degrees Celsius for 4h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.36 (18H), 4.11 (2H), 4.33 (2H), 5.32 (1H), 6.97-7.48 (16H), 7.55 (1H);
¹³C NMR (75MHz, CDCl₃) : δ 29.6, 34.6, 69.2, 71.9, 116.6, 119.3, 121.8, 126.2, 127.3, 127.7, 129.1, 129.5, 136.5, 141.9, 142.8, 146.1, 153.5, 165.1, 189.9;
C₃₆H₄₀N₂O₄S (calculated) : C 72.45, H 6.76, N 4.69, O 10.72, S 5.37; (measured) : C 72.40, H 6.71, N 4.72, O 10.78, S 5.35.

### Example III-5

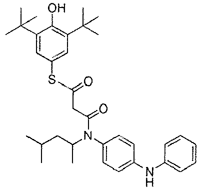

Under the protective atomosphere of nitrogen gas, to a 250ml four-necked flask equiped with a stirrer, a thermometer, a condensing tube and a dropping funnel, there were added 20.23g (85mmol) 2,6-di-tert-butyl-4-mercaptophenol, 16.22g (115mmol) malonyl dichloride, 18.49g (69mmol) N-(1,3-dimethylbutyl)-N'-phenyl p-phenylene diamine, 0.76g (7.2mmol) sodium carbonate and 150mL acetonitrile, rapidly stirred, reacted at the room temperature for 3h. Upon completion of the reaction, the solvent and water (generated during the reaction) were removed by vacuum distillation, the titled hindered phenol compound was obtained by using column chromatography. The compound was characterized as follows :
¹H NMR (300MHz, CDCl₃) : δ 0.80 (6H), 1.11 (3H), 1.36 (15H), 1.54-1.72 (6H), 3.67-3.91 (3H), 5.32 (1H), 6.80-7.17 (11H), 7.55 (1H);
¹³C NMR (75MHz, CDCl₃): δ 17.92,22.4, 24.6, 29.6, 31.9, 34.6, 44.6, 52.7, 53.1, 115.4, 116.6, 119.4, 121.8, 126.2, 129.5, 131.4, 136.6, 142.8, 146.1, 153.5, 161.7, 187.6;
C₃₅H₄₆N₂O₃S (calculated) : C 73.13, H 8.07, N 4.87, O 8.35, S 5.58; (measured) : C 73.08, H 8.01, N 4.84, O 8.42, S 5.63.

### Example III-6

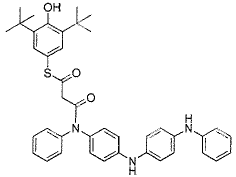

Under the protective atomosphere of nitrogen gas, to a 250ml four-necked flask equiped with a stirrer, a thermometer, a condensing tube and a dropping funnel, there were added 3.57g (15mmol) 2,6-di-tert-butyl-4-mercaptophenol, 8.74g (62mmol) malonyl dichloride, 21.76g (103mmol) N-phenyl-N'-[4-(phenylamino)phenyl]-1,4-phenylene diamine and 100mL toluene, rapidly stirred, reacted at 110 degrees Celsius for 4h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.36 (18H), 3.79 (2H), 5.32 (1H), 6.97-7.17 (14H), 7.20 (1H), 7.22-7.35 (6H), 7.55 (1H);
¹³C NMR (75MHz, CDCl₃): δ 29.6, 34.6, 52.8, 114.3, 116.3, 117.7, 119.3, 121.8, 126.2, 126.7, 127.3, 129.3, 132.4, 136.5, 141.9, 142.8, 143.3, 146.1, 153.5, 162.1, 186.2;
C₄₁H₄₃N₃O₃S (calculated) : C 74.85, H 6.59, N 6.39, O 7.30, S 4.87; (measured) : C 74.81, H 6.51, N 6.45, O 7.24, S 4.85.

### Example III-7

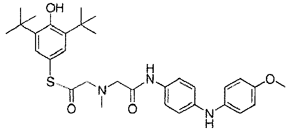

Under the protective atomosphere of nitrogen gas, to a 250ml four-necked flask equiped with a stirrer, a thermometer, a condensing tube and a dropping funnel, there were added 34.51g (145mmol) 2,6-di-tert-butyl-4-mercaptophenol, 5.11g (35mmol) N-methyl iminodiacetic acid, 4.49g (21mmol) 4-amino-4'-methoxy diphenyl amine, 0.28g (2.6mmol) sodium carbonate and 100mL toluene, rapidly stirred, reacted at 90 degrees Celsius for 4h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.36 (18H), 2.27 (3H), 3.73 (2H), 3.76 (3H), 4.06 (2H), 5.23 (1H), 6.97-7.17 (8H), 7.20 (1H), 7.48 (2H), 8.24 (1H);
¹³C NMR (75MHz, CDCl₃) : δ 29.6, 34.6, 45.2, 55.0, 55.5, 114.3, 119.3, 120.6, 121.5, 126.2, 132.3, 136.5, 137.5, 153.4, 154.5, 163.1, 192.6;
C₃₂H₄₁N₃O₄S (calculated) : C 68.18, H 7.33, N 7.45, O 11.35, S 5.69; (measured) : C 68.11, H 7.29, N 7.52, O 11.38, S 5.57.

### Example III-8

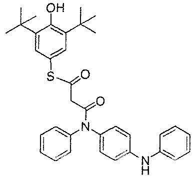

Under the protective atomosphere of nitrogen gas, to a 250ml four-necked flask equiped with a stirrer, a thermometer, a condensing tube and a dropping funnel, there were added 21.66g (91 mmol) 2,6-di-tert-butyl-4-mercaptophenol, 0.99g (9.51mmol) malonic acid, 6.51g (25mmol) N,N'-diphenyl-1,4-phenylene diamine, 0.65g (6.5mmol) potassium hydrogen carbonate and 150mL toluene, rapidly stirred, reacted at 150 degrees Celsius for 6h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.36 (18H), 3.85 (2H), 5.32 (1H), 6.80-7.17 (10H), 7.27 (4H), 7.35 (2H), 7.55 (1H); ¹³C NMR (75MHz, CDCl₃) : δ 29.6, 34.6, 52.6, 116.6, 119.4, 121.8, 122.6, 126.2, 127.7, 129.5, 136.6, 142.8, 146.1, 153.4, 161.8, 186.3;
C₃₅H₃₈N₂O₃S (calculated) : C 74.17, H 6.76, N 4.94, O 8.47, S 5.66; (measured) : C 74.25, H 6.81, N 4.83, O 8.41, S 5.59.

### Example III-9

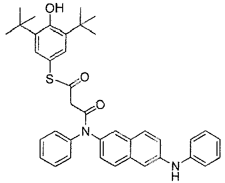

Under the protective atomosphere of nitrogen gas, to a 250ml four-necked flask equiped with a stirrer, a thermometer, a condensing tube and a dropping funnel, there were added 8.33g (35mmol) 2,6-di-tert-butyl-4-mercaptophenol, 7.19g (51 mmol) malonyl dichloride, 9.61g (31mmol) N,N'-diphenyl-2,6-naphthalene diamine, 0.68g (4.9mmol) potassium hydrogen carbonate and 150mL toluene, rapidly stirred, reacted at 110 degrees Celsius for 4h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.36 (18H), 3.84 (2H), 5.32 (1H), 5.80 (1H), 7.00-7.84 (18H);
¹³C NMR (75MHz, CDCl₃) : δ 29.6, 34.6, 52.8, 106.9, 111.0, 116.5, 119.9, 120.7, 121.8, 126.2, 127.7, 128.9, 129.1, 136.5, 140.8, 142.8, 153.5, 160.5, 186.2;
C₃₉H₄₀N₂O₃S (calculated) : C 75.94, H 6.54, N 4.54, O 7.78, S 5.20; (measured) : C 75.85, H 6.51, N 4.63, O 7.72, S 5.23.

### Example III-10

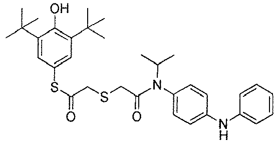

Under the protective atomosphere of nitrogen gas, to a 250ml four-necked flask equiped with a stirrer, a thermometer, a condensing tube and a dropping funnel, there were added 6.19g (26mmol) 2,6-di-tert-butyl-4-mercaptophenol, 16.38g (92mmol) thiodipropionic acid, 3.84g (17mmol) N-isopropyl-N'-phenyl p-phenylene diamine and 150mL toluene, rapidly stirred, reacted at 80 degrees Celsius for 2h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.19 (6H), 1.36 (18H), 3.74 (1H), 3.94 (2H), 3.96 (2H), 5.32 (1H), 6.80-7.26 (11H), 7.55 (1H);
¹³C NMR (75MHz, CDCl₃) : δ 20.6, 29.6, 34.6, 34.7, 41.9, 48.2, 116.6, 119.3, 121.8, 126.2, 129.5, 136.5, 139.6, 142.8, 146.1, 153.5, 166.2, 189.8;
C₃₃H₄₂N₂O₃S₂ (calculated) : C 68.48, H 7.31, N 4.84, O 8.29, S 11.08; (measured): C 68.41, H 7.28, N 4.91, O 8.30, S 11. 11.

### Example III-11

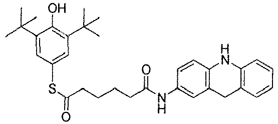

Under the protective atomosphere of nitrogen gas, to a 250ml four-necked flask equiped with a stirrer, a thermometer, a condensing tube and a dropping funnel, there were added 4.99g (21 mmol) 2,6-di-tert-butyl-4-mercaptophenol, 6.78g (53mmol) adipic anhydride, 5.70g (31 mmol) N-phenyl p-phenylene diamine and 150mL ethanol, rapidly stirred, reacted at 60 degrees Celsius for 2h, cooled to the room temperature, there were added 7.05g (85mmol) formaldehyde, heated to 85 degrees Celsius and reacted for 2.5h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.36 (18H), 1.57-2.66 (8H), 4.15 (2H), 5.32 (1H), 6.80-7.24 (9H), 8.73 (1H);
¹³C NMR (75MHz, CDCl₃) : δ 24.9, 25.3, 29.6, 32.6, 34.6, 37.8, 43.6, 113.0, 116.3, 118.9, 119.2, 123.3, 123.9, 124.6, 126.2, 127.9, 136.5, 139.5, 140.5, 153.4, 174.0, 196.9;
C₃₃H₄₀N₂O₃S (calculated) : C 72.76, H 7.40, N 5.14, O 8.81, S 5.89; (measured) : C 72.69, H 7.35, N 5.23, O 8.78, S 5.83.

### Example 111-12

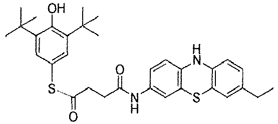

Under the protective atomosphere of nitrogen gas, to a 250ml four-necked flask equiped with a stirrer, a thermometer, a condensing tube and a dropping funnel, there were added 19.52g (82mmol) 2,6-di-tert-butyl-4-mercaptophenol, 17.06g (121 mmol) succinyl chloride, 19.93g (94mmol) 4-amino-4'-ethyl diphenyl amine, 2.22g (16.1 mmol) potassium carbonate and 150mL toluene, rapidly stirred, after reacted at the room temperature for 1h, to the toluene layer, there were added 10.11g (316mmol) sulfur and 0.04g (0.35mmol) iodine, heated to 150 degrees Celsius and reacted for 8h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.23 (3H), 1.36 (18H), 2.61 (2H), 2.94 (2H), 3.09 (2H), 5.32 (1H), 5.70 (1H), 6.98-7.17 (7H), 7.93 (1H);
¹³C NMR (75MHz, CDCl₃) : δ 15.60, 29.0, 29.6, 34.6, 36.1, 115.9, 116.1, 118.9, 124.9, 126.2, 127.9, 129.7, 136.6, 141.2, 146.2, 153.4, 174.0;
C₃₂H₃₈N₂O₃S (calculated) : C 68.29, H 6.81, N 4.98, O 8.53, S 11.40; (measured) : C 68.11, H 6.75, N 4.86, O 8.59, S 11.61.

### Example III-13

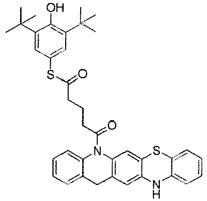

Under the protective atomosphere of nitrogen gas, to a 250ml four-necked flask equiped with a stirrer, a thermometer, a condensing tube and a dropping funnel, there were added 10.95g (46mmol) 2,6-di-tert-butyl-4-mercaptophenol, 8.11g (48mmol) glutaryl chloride, 13.78g (53mmol) N,N'-diphenyl-1,4-phenylene diamine and 150mL trimethylbenzene, rapidly stirred, after reacted at 85 degrees Celsius for 1h, there were added 3.82g (46mmol) formaldehyde, after reacted at 85 degrees Celsius for 1h, cooled to the room temperature, there were added 10.11g (316mmol) sulfur and 0.27g (2.11 mmol) iodine, heated to 180 degrees Celsius and reacted for 1h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.36 (18H), 2.11-2.81 (6H), 4.21 (2H), 5.32 (1H), 6.44 (1H), 6.50-7.37 (12H);
¹³C NMR (75MHz, CDCl₃) : δ 21.2,29.6, 32.2, 34.6, 35.4, 43.0, 114.4, 116.0, 119.1, 120.6, 121.6, 124.3, 126.6, 127.0, 130.2, 133.8, 136.5, 141.1, 153.5, 166.3, 196.9;
C₃₈H₄₀N₂O₃S₂ (calculated) : C 71.66, H 6.33, N 4.40, O 7.54, S 10.07; (measured) : C 71.59, H 6.28, N 4.49, O 7.51, S 10.10.

### Example III-14

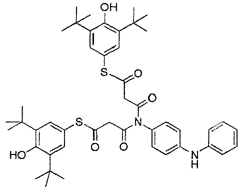

Under the protective atomosphere of nitrogen gas, to a 250ml four-necked flask equiped with a stirrer, a thermometer, a condensing tube and a dropping funnel, there were added 21.66g (91 mmol) 2,6-di-tert-butyl-4-mercaptophenol, 12.55g (89mmol) malonyl dichloride, 6.62g (36mmol) N-phenyl p-phenylene diamine, 0.62g (4.51 mmol) potassium carbonate and 150mL isopropanol, rapidly stirred, reacted at 80 degrees Celsius for 3h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.36 (36H), 3.98 (4H), 5.32 (1H), 6.80-7.26 (13H), 7.55 (1H);
¹³C NMR (75MHz, CDCl₃) : δ 29.6, 34.6, 52.1, 104.6, 116.6, 117.7, 119.3, 121.8, 124.6, 126.2, 129.45, 136.5, 142.8, 146.1, 153.5, 156.9, 186.8;
C₄₆H₅₆N₂O₆S₂ (calculated) : C 69.31, H 7.08, N 3.51, O 12.04, S 8.05; (measured): C 69.25, H 7.01, N 3.57, O 12.01, S 7.99.

### Example III-15

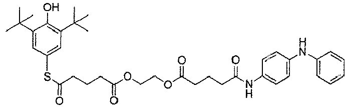

Under the protective atomosphere of nitrogen gas, to a 250ml four-necked flask equiped with a stirrer, a thermometer, a condensing tube and a dropping funnel, there were added 8.57g (36mmol) 2,6-di-tert-butyl-4-mercaptophenol, 26.68g (92mmol) glutaric acid 1,2-ethylene glycol ester, 13.06g (71 mmol) N-phenyl p-phenylene diamine and 150mL toluene, rapidly stirred, reacted at 90 degrees Celsius for 4h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.36 (12H), 1.54 (6H), 2.04 (4H), 2.29-2.67 (8H), 4.23 (4H), 5.32 (1H), 6.97-7.26 (9H), 7.45 (1H), 7.48 (2H), 7.55 (1H);
¹³C NMR (75MHz, CDCl₃) : δ 20.9, 21.3, 29.6, 31.98, 33.9, 34.6, 34.9, 43.0, 62.0, 114.3, 115.9, 119.3, 121.8, 126.2, 129.5, 132.3, 136.5, 137.5, 146.1, 153.5, 171.6, 173.5, 196.9;
C₃₈H₄₈N₂O₇S (calculated) : C 67.43, H 7.15, N 4.14, O 16.55, S 4.74; (measured) : C 67.53, H 7.19, N 4.08, O 16.47, S 4.76.

### Example III-16

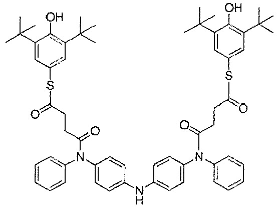

Under the protective atomosphere of nitrogen gas, to a 250ml four-necked flask equiped with a stirrer, a thermometer, a condensing tube and a dropping funnel, there were added 18.56g (78mmol) 2,6-di-tert-butyl-4-mercaptophenol, 11.21g (92mmol) succinic acid, 11.23g (32mmol) N-phenyl-N'-[4-(phenylamino)phenyl]-1,4-phenylene diamine and 150mL toluene, rapidly stirred, reacted at 100 degrees Celsius for 3h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.36-1.54 (36H), 2.89-3.19 (8H), 5.32 (2H), 6.80-7.35 (22H), 7.55 (1H);
¹³C NMR (75MHz, CDCl₃) : δ 29.6, 34.4, 35.5, 37.8, 93.8, 116.6, 122.5, 126.2, 127.6, 129.2, 141.9, 142.8, 143.3, 15.7, 173.2, 196.9;
C₆₀H₆₉N₃O₆S₂ (calculated) : C 72.62, H 7.01, N 4.23, O 9.67, S 6.46; (measured) : C 72.65, H 7.07, N 4.26, O 9.61, S 6.42.

### Example III-17

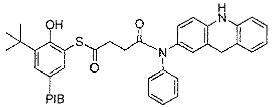

Under the protective atomosphere of nitrogen gas, to a 250ml four-necked flask equiped with a stirrer, a thermometer, a condensing tube and a dropping funnel, there were added 47.16g (45mmol) polyisobutenyl thiophenol produced by Example III-1, 5.11g (51mmol) succinic anhydride, 9.75g (53mmol) N-phenyl p-phenylene diamine, 0.86g (8.15mmol) sodium carbonate and 150mL benzene, rapidly stirred, reacted at 80 degrees Celsius for 2.5h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 0.88, 0.99, 1.02, 1.40, 1.41, 1.46, 1.52, 1.64, 1.68, 1.92,2.21-2.29, 2.57, 2.74, 4.86, 6.97, 7.00, 7.02, 7.26, 7.35, 7.55;
¹³C NMR (75MHz, CDCl₃) : δ 23.9, 24.9, 25.4, 28.2, 30.1, 34.5, 34.5, 35.4, 38.4, 43.6, 51.1, 116.0, 117.7, 119.4, 121.8, 126.7, 129.5, 133.6, 142.8, 146.1, 154.5, 173.3.

### Example III-18

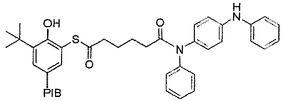

Under the protective atomosphere of nitrogen gas, to a 250ml four-necked flask equiped with a stirrer, a thermometer, a condensing tube and a dropping funnel, there were added 29.34g (28mmol) polyisobutenyl thiophenol produced by Example III-1, 2.91g (35mmol) adipoyl dichloride, 6.24g (24mmol) N,N'-diphenyl-1,4-phenylene diamine, 0.34g (3.2mmol) sodium carbonate and 150mL benzene, rapidly stirred, reacted at 60 degrees Celsius for 2h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 0.82, 0.99, 1.02, 1.40, 1.46, 1.52, 1.60, 1.68, 1.92,2.21-2.29, 2.59, 2.74, 4.86, 6.80, 6.97, 7.02, 7.17, 7.26, 7.55;
¹³C NMR (75MHz, CDCl₃) : δ 23.9, 25.0, 25.4, 28.2, 30.1, 34.5, 35.3, 38.4, 43.6, 48.6, 51.1, 116.6, 119.4, 121.8, 125.2, 126.8, 127.3, 129.5, 133.6, 142.8, 143.3, 146.1, 154.5, 173.3.

### Example III-19

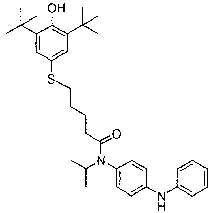

Under the protective atomosphere of nitrogen gas, to a 250ml four-necked flask equiped with a stirrer, a thermometer, a condensing tube and a dropping funnel, there were added 6.19g (26mmol) 2,6-di-tert-butyl-4-mercaptophenol, 13.64g (88mmol) 5-chloro-valeryl chloride, 3.84g (17mmol) N-isopropyl-N'-phenyl p-phenylene diamine, 1.23g (12.3mmol) potassium hydrogen carbonate and 150mL toluene, rapidly stirred, reacted at 80 degrees Celsius for 2h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.19 (6H), 1.36 (18H), 1.55-1.97 (4H), 2.03 (2H), 2.86 (2H), 3.74 (1H), 5.32 (1H), 6.80-7.26 (11H), 7.55 (1H);
¹³C NMR (75MHz, CDCl₃) : δ 20.6, 25.0, 28.3, 29.6, 34.6, 35.5, 48.2, 116.6, 119.3, 121.8, 126.2, 129.5, 136.5, 139.6, 142.8, 146.1, 153.5, 169.9;
C₃₄H₄₆N₂O₂S (calculated) : C 74.68, H 8.48, N 5.12, O 5.85, S 5.86; (measured) : C 74.61, H 8.42, N 5.19, O 5.83, S 5.91.

### Example III-20

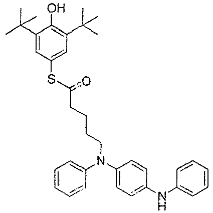

Under the protective atomosphere of nitrogen gas, to a 250ml four-necked flask equiped with a stirrer, a thermometer, a condensing tube and a dropping funnel, there were added 21.66g (91 mmol) 2,6-di-tert-butyl-4-mercaptophenol, 1.47g (9.51 mmol) 5-chloro-valeryl chloride, 6.51g (25mmol) N,N'-diphenyl-1,4-phenylene diamine, 0.65g (6.5mmol) potassium hydrogen carbonate and 150mL benzene, rapidly stirred, reacted at 75 degrees Celsius for 6h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.36 (18H), 1.70-1.97 (4H), 2.54 (2H), 3.47 (2H), 5.32 (1H), 6.80-7.26 (16H), 7.55 (1H);
¹³C NMR (75MHz, CDCl₃) : δ 24.7, 27.3, 29.6, 34.6, 43.3, 46.8, 117.7, 119.3, 121.8, 126.2, 129.5, 136.5, 142.8, 146.1, 148.7, 153.5, 169.9;
C₃₇H₄₄N₂O₂S (calculated) : C 76.51, H 7.64, N 4.82, O 5.51, S 5.52; (measured) : C 76.61, H 7.66, N 4.71, O 5.57, S 5.58.

### Example III-21

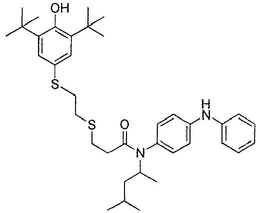

Under the protective atomosphere of nitrogen gas, to a 250ml four-necked flask equiped with a stirrer, a thermometer, a condensing tube and a dropping funnel, there were added 20.23g (85mmol) 2,6-di-tert-butyl-4-mercaptophenol, 16.22g (115mmol) malonyl dichloride, 18.49g (69mmol) N-(1,3-dimethyl butyl)-N'-phenyl p-phenylene diamine, 0.76g (7.2mmol) sodium carbonate and 150mL acetonitrile, rapidly stirred, reacted at the room temperature for 3h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 0.80 (6H), 1.11 (3H), 1.36 (18H), 1.54-1.73 (3H), 2.54-3.28 (8H), 3.65 (1H), 5.32 (1H), 6.80-7.26 (11H), 7.55 (1H);
¹³C NMR (75MHz, CDCl₃) : δ 17.9, 22.4, 24.6, 29.6, 31.85, 34.6, 40.1,44.9, 52.8, 111.8, 119.3, 121.8, 126.2, 129.5, 131.1, 136.5, 142.8, 146.1, 148.7, 153.5, 168.4;
C₃₇H₅₂N₂O₂S₂ (calculated): : C 71.57, H 8.44, N 4.51, O 5.15, S 10.33; (measured): C 71.51, H 8.55, N 4.66, O 5.05, S 10.25.

### Comparative Example III-1

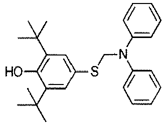

Under the protective atomosphere of nitrogen gas, to a 250ml four-necked flask equiped with a stirrer, a thermometer, a condensing tube and a dropping funnel, there were added 20.23g (85mmol) 2,6-di-tert-butyl-4-mercaptophenol, 7.22g (87mmol) formaldehyde, 14.37g (85mmol) diphenyl amine and 150mL methanol, rapidly stirred, reacted at 60 degrees Celsius for 2h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ1.36 (18H), 5.21 (2H), 5.32 (1H), 6.99 (6H), 7.17 (2H), 7.27 (4H);
¹³C NMR (75MHz, CDCl₃) : δ 29.6, 34.6, 55.2, 120.4, 123.3, 125.9, 126.2, 129.2, 136.6, 150.0, 153.5;
C₂₇H₃₃NOS (calculated) : C 77.28, H 7.93, N 3.34, O 3.81, S 7.64; (measured) : C 77.22, H 7.89, N 3.31, O 3.83, S 7.67.

### Comparative Example III-2

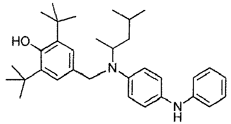

Under the protective atomosphere of nitrogen gas, to a 250ml four-necked flask equiped with a stirrer, a thermometer, a condensing tube and a dropping funnel, there were added 10.71g (52mmol) 2,6-di-tert-butyl-4-mercaptophenol, 1.89g (63mmol) formaldehyde, 17.42g (65mmol) N- (1,3-dimethyl butyl)-N'-phenyl p-phenylene diamine and 150mL methanol, rapidly stirred, reacted at 70 degrees Celsius for 4h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 0.80 (6H), 1.11 (3H), 1.29 (1H), 1.36 (18H), 3.45 (1H), 4.53 (2H), 5.32 (2H), 6.97-7.07 (9H), 7.26 (2H), 7.55 (1H);
¹³C NMR (75MHz, CDCl₃) : δ17.9, 22.40, 24.6, 30.4, 34.3, 45.7, 54.5, 56.7, 116.6, 120.4, 121.8, 125.9, 128.9, 129.5, 135.6, 146.1, 153.5, 154.8;
C₃₃H₄₆N₂O (calculated) : C 81.43, H 9.53, N 5.76, O 3.29; (measured): C 81.38, H 9.51, N 5.79, O 3.31.

### Comparative Example III-3

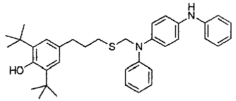

Under the protective atomosphere of nitrogen gas, to a 250ml four-necked flask equiped with a stirrer, a thermometer, a condensing tube and a dropping funnel, there were added 9.24g (33mmol) 2,6-ditert-butyl-4-(3-mercaptopropyl) phenol, 3.07g (37mmol) formaldehyde, 11.71g (45mmol) N,N'-diphenyl-1,4-phenylene diamine and 150mL benzene, rapidly stirred, reacted at 80 degrees Celsius for 4h. Upon completion of the reaction, the solvent and water (generated during the reaction) were removed by vacuum distillation, the titled hindered phenol compound was obtained by using column chromatography.

The compound was characterized as follows :
¹H NMR (300MHz, CDCl₃) : δ 1.40 (18H), 1.99 (1H), 2.61-2.80 (6H), 4.63 (2H), 5.32 (1H), 6.97-7.02 (12H), 7.26 (4H), 7.55 (1H);
¹³C NMR (75MHz, CDCl₃) : δ 28.7, 31.1, 34.3, 35.7, 54.1, 117.1, 119.4, 123.3, 124.8, 129.2, 131.8, 136.0, 142.8, 144.9, 146.1, 151.9;
C₃₆H₄₄N₂OS (calculated) : C 78.22, H 8.02, N 5.07, O 2.89, S 5.80; (measured) : C 78.27, H 7.96, N 4.98, O 2.92, S 5.83.

### Example III-19 to Example III-35 and Comparative Example III-4 to Comparative Example III-8

Each of the hindered phenol compounds of Example III-2 to Example III-21 and Comparative Example III-1 to Comparative Example III-3 was respectively mixed with a lubricant base oil in line with the composition and ratio specified in Table III-1 at 40 degrees Celsius for 2h, to obtain the lubricant oil compositions of Example III-22 to Example III-41 and the lubricant oil compositions of Comparative Example III-4 to Comparative Example III-6. Further, in line with the composition and ratio specified in Table III-2, the antioxidant was respectively mixed with a lubricant base oil at 40 degrees Celsius for 2h, to obtain the lubricant oil compositions of Comparative Example III-7 to Comparative Example III-10. Herein, based on the total weight of each lubricant oil composition, the amount of the antioxidant to be used in each case was 0.5wt%, the lubricant base oil used was a Group II hydrogenated base oil (produced by Sinopec Group,Gaoqiao Shanghai Branch). Further, the base oil without the antioxidant was taken as Control.

**Table III-1**

| lubricant oil composition | antioxidant | lubricant base oil | antioxidant amount |
|---|---|---|---|
| Example III-22 | Example III-2 | II | 0.50% |
| Example III-23 | Example III-3 | II | 0.50% |
| Example III-24 | Example III-4 | II | 0.50% |
| Example III-25 | Example III-5 | II | 0.50% |
| Example III-26 | Example III-6 | II | 0.50% |
| Example III-27 | Example III-7 | II | 0.50% |
| Example III-28 | Examples III-8 | II | 0.50% |
| Example III-29 | Example III-9 | II | 0.50% |
| Example III-30 | Example III-10 | II | 0.50% |
| Example III-31 | Example III-11 | II | 0.50% |
| Example III-32 | Example III-12 | II | 0.50% |
| Example III-33 | Example III-13 | II | 0.50% |
| Example III-34 | Example III-14 | II | 0.50% |
| Example III-35 | Example III-15 | II | 0.50% |
| Example III-36 | Example III-16 | II | 0.50% |
| Example III-37 | Example III-17 | II | 0.50% |
| Example III-38 | Example III-18 | II | 0.50% |
| Example III-39 | Example III-19 | II | 0.50% |
| Example III-40 | Example III-20 | II | 0.50% |
| Example III-41 | Example III-21 | II | 0.50% |
| Comparative Example III-4 | Comparative Example III-1 | II | 0.50% |
| Comparative Example III-5 | Comparative Example III-2 | II | 0.50% |
| Comparative Example III-6 | Comparative Example III-3 | II | 0.50% |
| Control | - | II | - |

**Table III-2**

| lubricant oil composition | hindered phenol compound | aromatic amine | hindered phenol compoun d amount | aromati c amine amount | lubricant t base oil |
|---|---|---|---|---|---|
| Comparative Example III-7 | 2,2'-thiobis[3-(3,5-ditert-butyl-4-hydrox ylphenyl)propionic acid ethyl ester] | - | 0.50% | - | II |
| Comparative Example III-8 | - | N-(4-tert-octyl phenyl)-1-naphthylamin e | - | 0.50% | II |
| Comparative Example III-9 | 2,6-ditert-butyl-4-amylthiophenol | 4,4'-ditert-octyl diphenyl amine | 0.25% | 0.25% | II |
| Comparative Example III-10 | 4,4'-[thiobismethylene]bis[2,6-ditert-but ylphenol] | 3,7-ditert-octyl phenothiazine | 0.30% | 0.20% | II |

The lubricant oil compositions of Example III-22 to Example III-41, the lubricant oil compositions of Comparative Example III-4 to Comparative Example III-10 and the Control were taken as test samples, to evaluate the oxidation resistance at elevated temperatures, with a PDSC temperature of 210 degrees Celsius, the results of were shown in Table III-3.

**Table III-3**

| lubricant oil composition | PDSC oxidative induction period (min) | lubricant oil composition | PDSC oxidative induction period (min) |
|---|---|---|---|
| Example III-22 | 16.7 | Example III-36 | 22.5 |
| Example III-23 | 18.9 | Example III-37 | 16.7 |
| Example III-24 | 19.7 | Example III-38 | 17.6 |
| Example III-25 | 21.3 | Example III-39 | 17.3 |
| Example III-26 | 18.9 | Example III-40 | 18.9 |
| Example III-27 | 22.3 | Example III-41 | 20.1 |
| Example III-28 | 20.7 | Comparative Example III-4 | 4.9 |
| Example III-29 | 26.3 | Comparative Example III-5 | 7.5 |
| Example III-30 | 21.5 | Comparative Example III-6 | 16.1 |
| Example III-31 | 19.3 | Comparative Example III-7 | 8.6 |
| Example III-32 | 22.4 | Comparative Example III-8 | 15.2 |
| Example III-33 | 20.7 | Comparative Example III-9 | 5.3 |
| Example III-34 | 20.3 | Comparative Example III-10 | 7.1 |
| Example III-35 | 19.6 | Control | 0.2 |

The lubricant oil compositions of Example III-22 to Example III-41, the lubricant oil compositions of Comparative Example III-4 to Comparative Example III-10 and the Control were taken as test samples, to evaluate the deposite formation inhibition performance, the results were shown in Table III-4.

**Table III-4**

| lubricant oil composition | deposit amount (mg) |
|---|---|
| Example III-25 | 13.6 |
| Example III-28 | 12.1 |
| Example III-32 | 11.2 |
| Example III-34 | 15.7 |
| Example III-37 | 6.3 |
| Example III-38 | 4.8 |
| Example III-39 | 10.7 |
| Example III-41 | 12.5 |
| Comparative Example III-4 | 19.5 |
| Comparative Example III-5 | 23.4 |
| Comparative Example III-6 | 20.8 |
| Comparative Example III-7 | 21.6 |
| Comparative Example III-8 | 25.6 |
| Comparative Example III-9 | 19.2 |
| Comparative Example III-10 | 18.5 |
| Control | 32.8 |

The lubricant oil compositions of Example III-22 to Example III-41, the lubricant oil compositions of Comparative Example III-4 to Comparative Example III-10 and the Control were taken as test samples, to evaluate the anticorrosion performance. The results of BRT (ball rust test) were shown in Table III-5.

**Table III-5**

| lubricant oil composition | corrosion degree |
|---|---|
| Example III-25 | slight |
| Example III-28 | slight |
| Example III-32 | slight |
| Example III-34 | moderate |
| Example III-37 | slight |
| Example III-38 | slight |
| Example III-39 | slight |
| Example III-41 | slight |
| Comparative Example III-4 | moderate |
| Comparative Example III-5 | moderate |
| Comparative Example III-6 | moderate |
| Comparative Example III-7 | moderate |
| Comparative Example III-8 | moderate |
| Comparative Example III-9 | moderate |
| Comparative Example III-10 | moderate |
| Control | severe |

The lubricant oil compositions of Example III-22 to Example III-41, the lubricant oil compositions of Comparative Example III-4 to Comparative Example III-10 and the Control were taken as test samples, to evaluate the performances of inhibiting inrease in viscosity and inhibiting inrease in acid value, the results of were shown in Table III-6.

**Table III-6**

| lubricant oil composition | increase in viscosity (%) | increase in acid value (mgKOH·g⁻¹) |
|---|---|---|
| Example III-22 | 25.6 | 2.631 |
| Example III-23 | 22.1 | 2.035 |
| Example III-24 | 20.4 | 1.967 |
| Example III-25 | 23.6 | 1.639 |
| Example III-26 | 18.4 | 2.004 |
| Example III-27 | 19.1 | 1.569 |
| Example III-28 | 24.7 | 1.812 |
| Example III-29 | 20.6 | 1.645 |
| Example III-30 | 19.3 | 1.996 |
| Example III-31 | 20.9 | 2.733 |
| Example III-32 | 25.2 | 2.317 |
| Example III-33 | 23.3 | 1.962 |
| Example III-34 | 20.8 | 2.775 |
| Example III-35 | 19.9 | 2.284 |
| Exampie III-36 | 24.1 | 1.965 |
| Example III-37 | 22.5 | 1.882 |
| Example III-38 | 20.7 | 2.337 |
| Example III-39 | 22.5 | 2.023 |
| Example III-40 | 21.9 | 1.864 |
| Example III-41 | 21.2 | 2.067 |
| Comparative Example III-4 | 33.5 | 4.621 |
| Comparative Example III-5 | 27.5 | 3.482 |
| Comparative Example III-6 | 25.8 | 5.168 |
| Comparative Example | 27.6 | 2.953 |
| Comparative Example III-8 | 30.5 | 3.846 |
| Comparative Example III-9 | 28.1 | 5.429 |
| Comparative Example III-10 | 24.7 | 6.517 |
| Control | 67.2 | 24.525 |

## Claims

1. A hindered phenol compound represented by Formula (I), 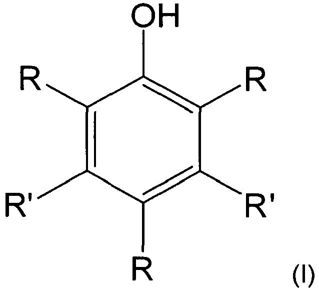 In Formula (I),
the plural Rs may be identical to or different from each other, each independently selected from the group consisting of a C₁₋₃₀₀ linear or branched alkyl, or a polyolefin group having a number-average molecular weight (Mn) of from 300 to 3000, a group represented by Formula (II) and a group represented by Formula (III), with the proviso that at least one R is a group represented by Formula (II);
the plural R's may be identical to or different from each other, each independently selected from the group consisting of hydrogen and a C₁₋₂₀ linear or branched alkyl,
-S-L(̵A)ₘ (II) 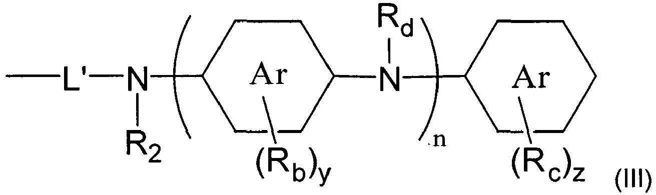 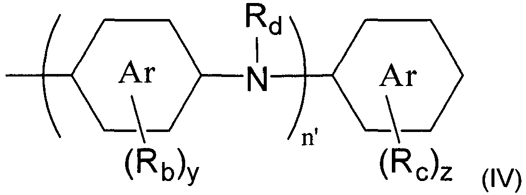
In each Formula, the group L is selected from the group consisting of a m+1 valent C₁₋₂₀ linear or branched alkyl optionally substituted by one or more substituent selected from the group consisting of oxo, a C₁₋₂₀ hydrocarbyl, and a C₃₋₂₀ linear or branched hetero-alkyl and a m+1 valent C₃₋₂₀ linear or branched hetero-alkyl optionally substituted by one or more substituent selected from the group consisting of oxo, a C₁₋₂₀ hydrocarbyl, and a C₃₋₂₀ linear or branched hetero-alkyl;
the group L' is a group represented by 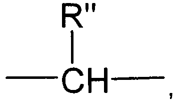 wherein the group R" is selected from the group consisting of hydrogen, a C₁₋₂₀ hydrocarbyl, and a C₃₋₂₀ linear or branched hetero-alkyl, the plural As may be identical to or different from each other, each independently selected from the group consisting of 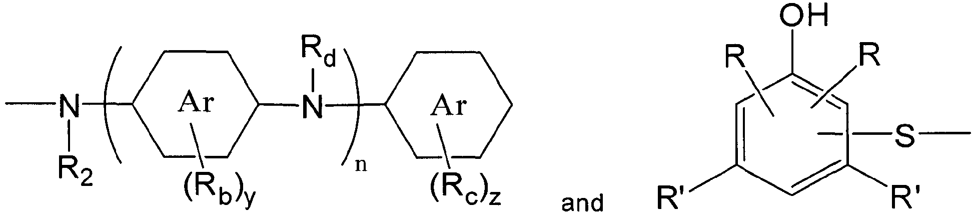 wherein, the plural Rs may be identical to or different from each other, each independently selected from the group consisting of hydrogen, a C₁₋₃₀₀ linear or branched alkyl, or a polyolefin group having a number-average molecular weight (Mn) of from 300 to 3000, a group represented by Formula (II) and a group represented by Formula (III), preferably each independently selected from the group consisting of hydrogen and a C₁₋₃₀₀ linear or branched alkyl; the plural R's may be identical to or different from each other, each independently selected from the group consisting of hydrogen and a C₁₋₂₀ linear or branched alkyl, two Rs and one group -S- occupy the rest three positions on the benzene ring respectively,
with the proviso that at least one A is 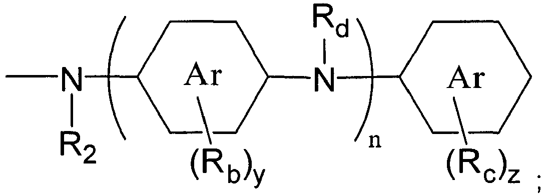 m is an integer in the range of from 1 to 4; the plural R₂s may be identical to or different from each other, each independently selected from the group consisting of hydrogen, a C₁₋₂₀ linear or branched alkyl, a group represented by Formula (IV) and a group represented by Formula (V),; the plural R_{b}s may be identical to or different from each other, each independently selected from the group consisting of hydrogen and a C₁₋₂₀ linear or branched alkyl,; the plural R_{c}s may be identical to or different from each other, each independently selected from the group consisting of hydrogen, a C₁₋₂₀ linear or branched alkyl and a C₁₋₂₀ linear or branched alkyloxy, ; y is an integer in the range of from 0 to 3,; z is an integer in the range of from 0 to 3; n is an integer in the range of from 1 to 8; n' is an integer in the range of from 0 to 7, with the proviso that n'+n≤8; the plural R_{d}s may be identical to or different from each other, each independently selected from the group consisting of hydrogen and a group represented by Formula (V); the plural rings 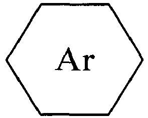 may be identical to or different from each other, each independently selected from the group consisting of benzene ring and naphthalene ring, wherein two adjacent rings 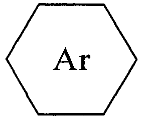 may optionally form a phenothiazine ring with the N atom bridging these two rings and an additional S atom, and/or, two adjacent rings 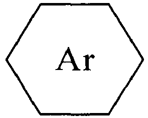 may optionally form a 9,10-dihydroacridine ring with the N atom bridging these two rings and an additional group 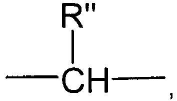 wherein the group R" is selected from the group consisting of hydrogen, a C₁₋₂₀ hydrocarbyl, and a C₃₋₂₀ linear or branched hetero-alkyl, or a 9,10-dihydroacridine ring,
-L(̵A)ₘ (V)
In Formula (V), the plural As may be identical to or different from each other, each independently selected from the group consisting of 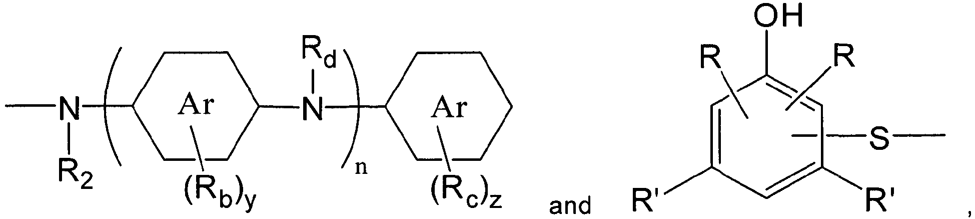 wherein the plural Rs may be identical to or different from each other, each independently selected from the group consisting of hydrogen, a C₁₋₃₀₀ linear or branched alkyl, or a polyolefin group having a number-average molecular weight (Mn) of from 300 to 3000, a group represented by Formula (II) and a group represented by Formula (III), the plural R's may be identical to or different from each other, each independently selected from the group consisting of hydrogen and a C₁₋₂₀ linear or branched alkyl,; two Rs and one group -S- occupy the rest three positions on the benzene ring respectively; the group L, the group R₂, the group R_{b}, the group R_{c}, the group R_{d}, the ring 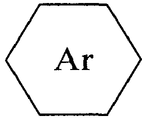 and y, n, z and m are as defined in Formula (II) respectively,
wherein the linear or branched hetero-alkyl is selected from the group consisting of a group obtained by directly replacing one or more group -CH₂- locating inside of a linear or branched alkyl by a corresponding number of replacing group selected from -O-, -Sand -NR'-, wherein R' is H or a C₁₋₄ linear or branched alkyl and a group obtained by directly replacing one or more group -CH< locating inside of a linear or branched alkyl by a corresponding number of replacing group -N<, wherein throughout the molecular structure of the hindered phenol compound, at least one R_{d} is hydrogen.

2. The hindered phenol compound according to Claim 1, wherein the group L is selected from the group consisting of a group 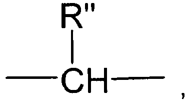 herein m=1, a m+1 valent C₂₋₂₀ linear or branched alkyl optionally substituted by one or more substituent selected from the group consisting of a C₁₋₂₀ hydrocarbyl, and a C₃₋₂₀ linear or branched hetero-alkyl, a m+1 valent C₃₋₂₀ linear or branched hetero-alkyl optionally substituted by one or more substituent selected from the group consisting of a C₁₋₂₀ hydrocarbyl, and a C₃₋₂₀ linear or branched hetero-alkyl and a group 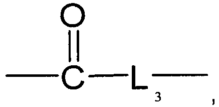 wherein the group R" is selected from the group consisting of hydrogen, a C₁₋₂₀ hydrocarbyl, and a C₃₋₂₀ linear or branched hetero-alkyl, , the group L₃ is selected from the group consisting of a m+1 valent C₂₋₁₉ linear or branched alkyl optionally substituted by one or more substituent selected from the group consisting of oxo, a C₁₋₂₀ hydrocarbyl, and a C₃₋₂₀ linear or branched hetero-alkyl and a m+1 valent C₃₋₁₉ linear or branched hetero-alkyl optionally substituted by one or more substituent selected from the group consisting of oxo, a C₁₋₂₀ hydrocarbyl, and a C₃₋₂₀ linear or branched hetero-alkyl.

3. The hindered phenol compound according to Claim 1, wherein the Formula (II) is selected from one of the following Formula (II_{X}), Formula (II_{XX}) and Formula (II_{XXX}), 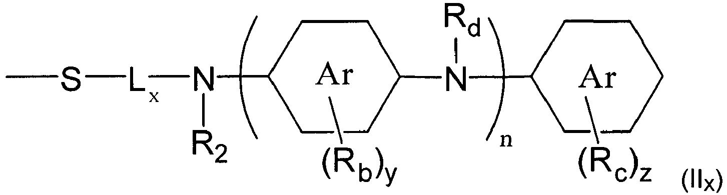 In Formula (II_{X}), the group L_{X} is a group represented by 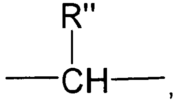 wherein the group R" is selected from the group consisting of hydrogen, a C₁₋₂₀ hydrocarbyl, and a C₃₋₂₀ linear or branched hetero-alkyl, , 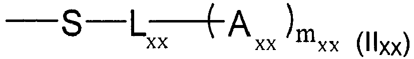 In Formula (II_{XX}), the group L_{XX} is selected from the group consisting of a m_{XX}+1 valent C₂₋₂₀ linear or branched alkyl optionally substituted by one or more substituent selected from the group consisting of a C₁₋₂₀ hydrocarbyl, and a C₃₋₂₀ linear or branched hetero-alkyl and a m_{XX}+1 valent C₃₋₂₀ linear or branched hetero-alkyl optionally substituted by one or more substituent selected from the group consisting of a C₁₋₂₀ hydrocarbyl, and a C₃₋₂₀ linear or branched hetero-alkyl; the plural A_{XX}s may be identical to or different from each other, each independently selected from the group consisting of 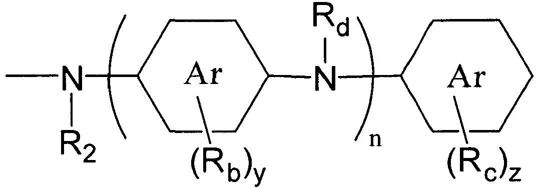 and 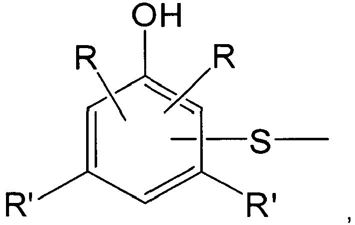 wherein, the plural Rs may be identical to or different from each other, each independently selected from the group consisting of hydrogen, a C₁₋₃₀₀ linear or branched alkyl, or a polyolefin group having a number-average molecular weight (Mn) of from 300 to 3000, a group represented by Formula (II_{XX}) and a group represented by Formula (III), the plural R's may be identical to or different from each other, each independently selected from the group consisting of hydrogen and a C₁₋₂₀ linear or branched alkyl, two Rs and one group -S- occupy the rest three positions on the benzene ring respectively, with the proviso that at least one A_{XX} is 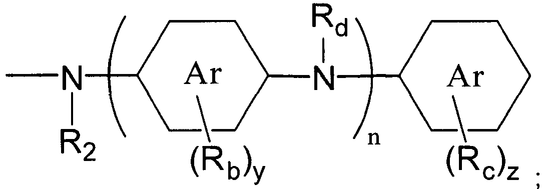 m_{XX} is an integer in the range of from 1 to 4, preferably 1, 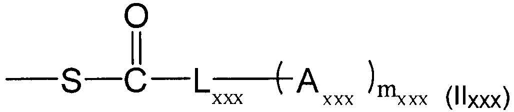 In Formula (II_{XXX}), the group L_{XXX} is selected from the group consisting of a m_{XXX}+1 valent C₂ to C19-m_{XXX} linear or branched alkyl optionally substituted by one or more substituent selected from the group consisting of a C₁₋₂₀ hydrocarbyl, and a C₃₋₂₀ linear or branched hetero-alkyl, a m_{XXX}+1 valent C₃ to C19-m_{XXX} linear or branched hetero-alkyl optionally substituted by one or more substituent selected from the group consisting of a C₁₋₂₀ hydrocarbyl, and a C₃₋₂₀ linear or branched hetero-alkyl and a group represented by 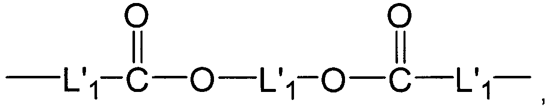 wherein the plural L'₁s may be identical to or different from each other, each independently selected from the group consisting of a C₁₋₁₀ linear or branched alkyl and a C₃₋₁₀ linear or branched hetero-alkyl, with the proviso that (1) the plural L'₁s each independently represents a 2 valent to m_{XXX}+1 valent group, so as to make the group 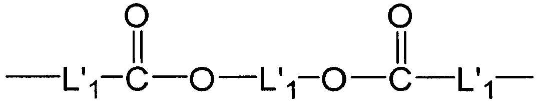 to present as, as a whole, m_{XXX}+1 valent, and (2) the total atom number of all groups L'₁ is no more than 14, wherein the plural L'₁s may each independently be optionally further substituted by one or more substituent selected from the group consisting of a C₁₋₂₀ hydrocarbyl, and a C₃₋₂₀ linear or branched hetero-alkyl; the plural A_{XXX}s may be identical to or different from each other, each independently selected from the group consisting of 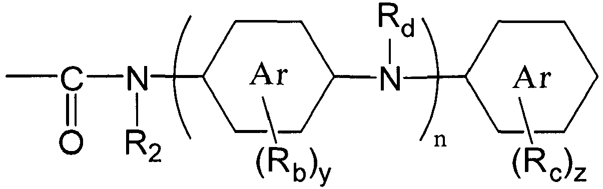 and 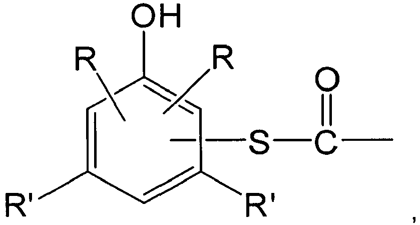 wherein, the plural Rs may be identical to or different from each other, each independently selected from the group consisting of hydrogen, a C₁₋₃₀₀ linear or branched alkyl, or a polyolefin group having a number-average molecular weight (Mn) of from 300 to 3000, a group represented by Formula (II_{XXX}) and a group represented by Formula (III), the plural R's may be identical to or different from each other, each independently selected from the group consisting of hydrogen and a C₁₋₂₀ linear or branched alkyl, two Rs and one group 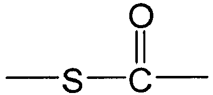 occupy the rest three positions on the benzene ring respectively, with the proviso that at least one A_{XXX} is 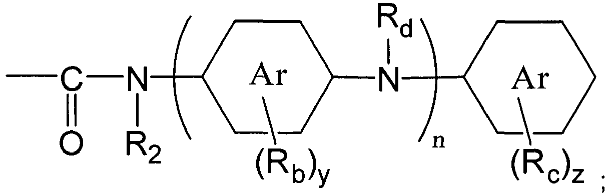 m_{XXX} is an integer in the range of from 1 to 4, preferably 1, and/or, the Formula (V) is selected from one of the following Formula (V_{X}), Formula (V_{XX}) and Formula (V_{XXX}), 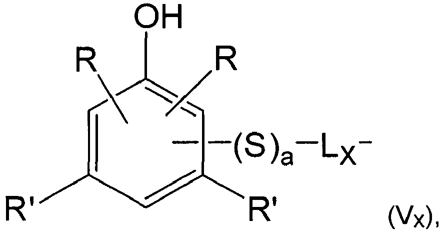 In Formula (V_{X}), the plural Rs may be identical to or different from each other, each independently selected from the group consisting of hydrogen, a C₁₋₃₀₀ linear or branched alkyl, or a polyolefin group having a number-average molecular weight (Mn) of from 300 to 3000, a group represented by Formula (II_{X}) and a group represented by Formula (III),; the plural R's may be identical to or different from each other, each independently selected from the group consisting of hydrogen and a C₁₋₂₀ linear or branched alkyl,; the group L_{X} is a group represented by 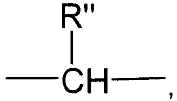 wherein the group R" is selected from the group consisting of hydrogen, a C₁₋₂₀ hydrocarbyl, and a C₃₋₂₀ linear or branched hetero-alkyl, a is 1, and two Rs and one group - (S)ₐ-L_{X}-occupy the rest three positions on the benzene ring respectively, 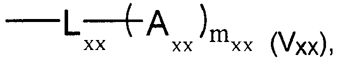 In Formula (V_{XX}), the plural A_{XX}s may be identical to or different from each other, each independently selected from the group consisting of 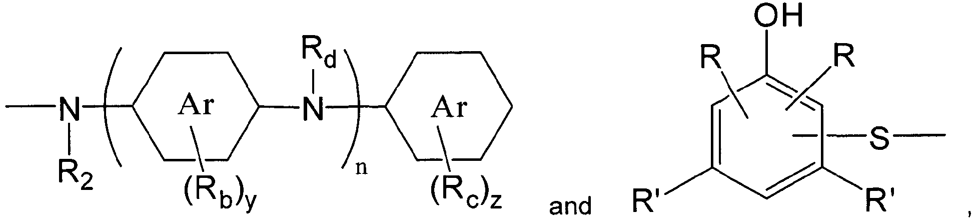 wherein the plural Rs may be identical to or different from each other, each independently selected from the group consisting of hydrogen, a C₁₋₃₀₀ linear or branched alkyl, or a polyolefin group having a number-average molecular weight (Mn) of from 300 to 3000, a group represented by Formula (II_{XX}) and a group represented by Formula (III),; the plural R's may be identical to or different from each other, each independently selected from the group consisting of hydrogen and a C₁₋₂₀ linear or branched alkyl,; two Rs and one group -S- occupy the rest three positions on the benzene ring respectively; the group L_{XX} and m_{XX} are as defined in Formula (II_{XX}) respectively, 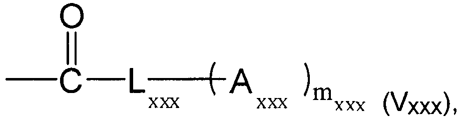 In Formula (V_{XXX}), the plural A_{XXX}s may be identical to or different from each other, each independently selected from the group consisting of 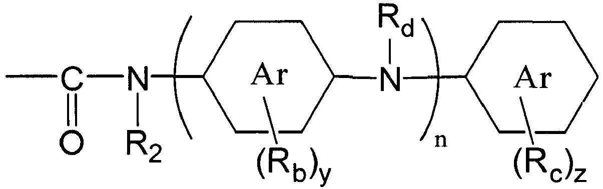 and 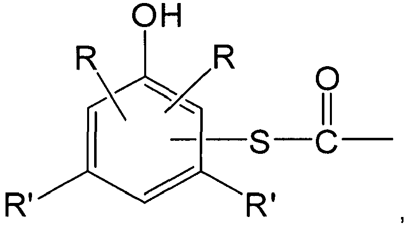 wherein the plural Rs may be identical to or different from each other, each independently selected from the group consisting of hydrogen, a C₁₋₃₀₀ linear or branched alkyl, or a polyolefin group having a number-average molecular weight (Mn) of from 300 to 3000, a group represented by Formula (II_{XXX}) and a group represented by Formula (III),; the plural R's may be identical to or different from each other, each independently selected from the group consisting of hydrogen and a C₁₋₂₀ linear or branched alkyl,; two Rs and one group 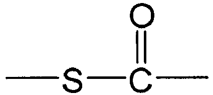 occupy the rest three positions on the benzene ring respectively; the group L_{XXX} and m_{XXX} are as defined in Formula (II_{XXX}) respectively, other groups and numerical values are as defined in Claim 1.

4. The hindered phenol compound according to Claim 1, selected from the group consisting of the following specific compounds or their mixture at any ratio therebetween :

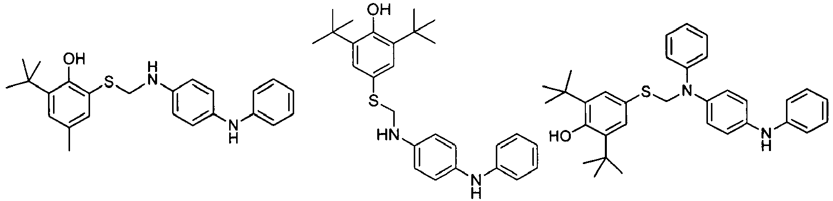

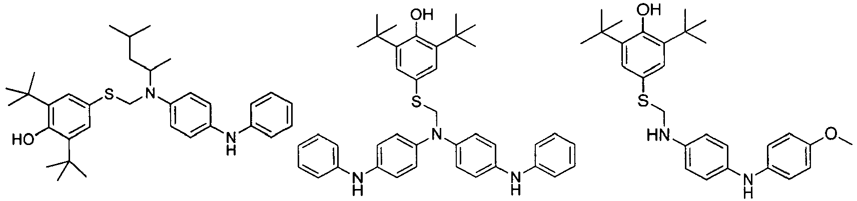

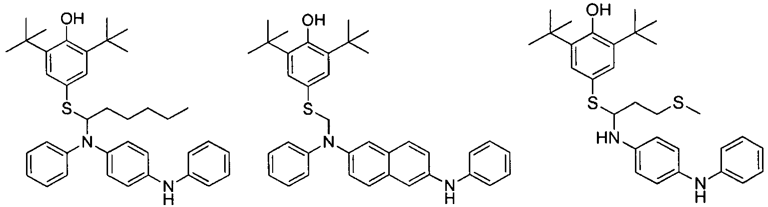

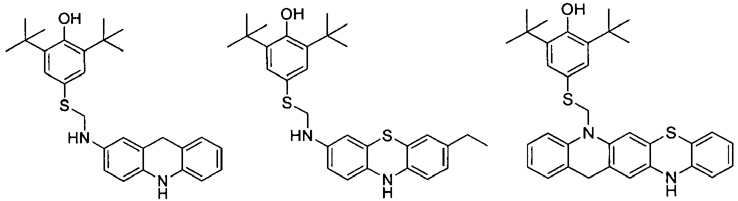

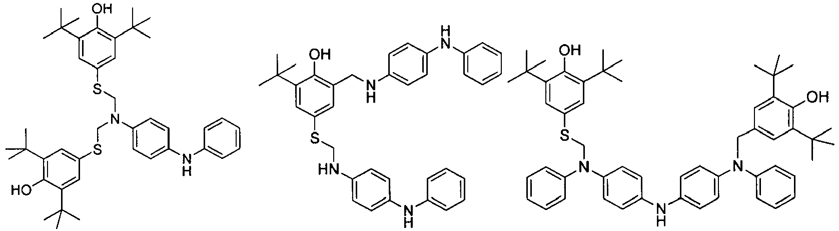

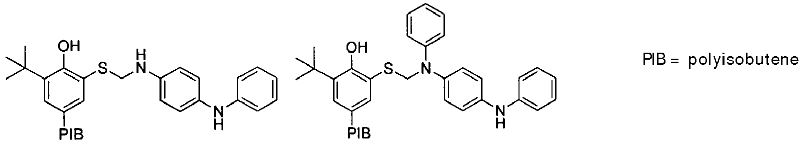

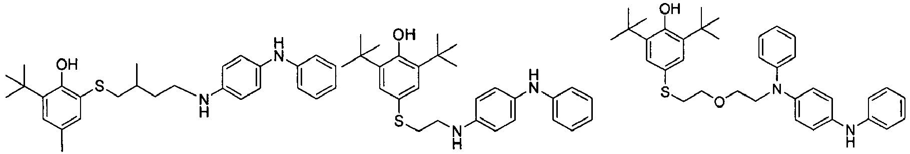

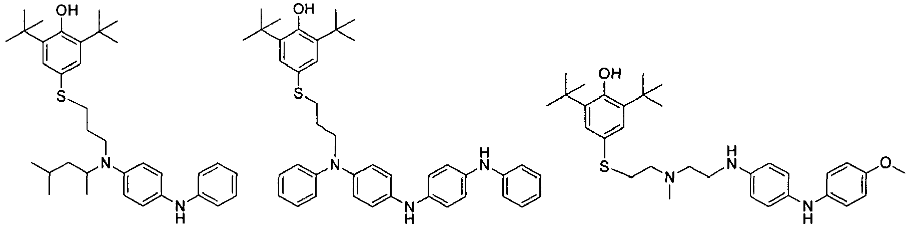

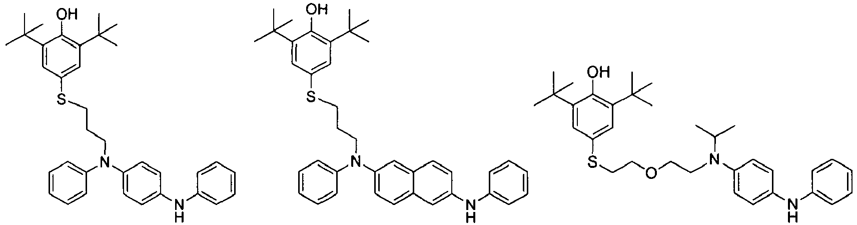

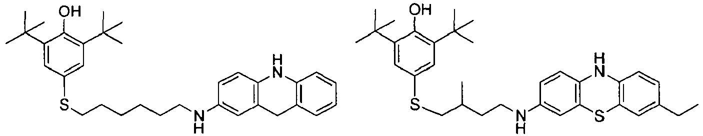

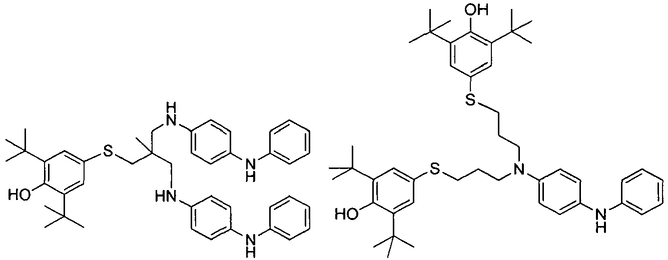

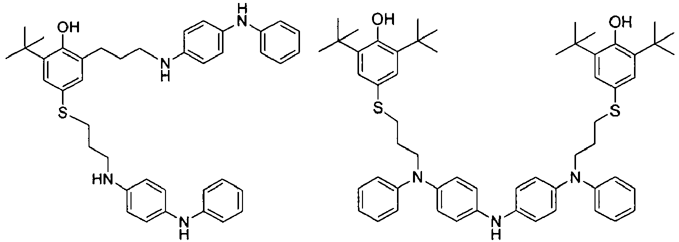

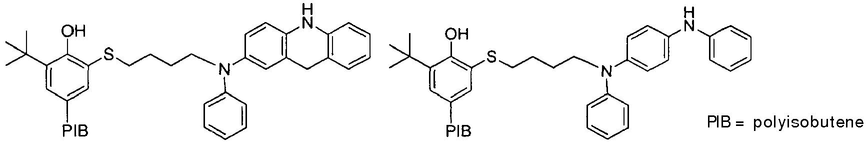

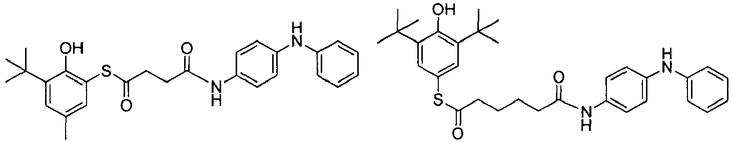

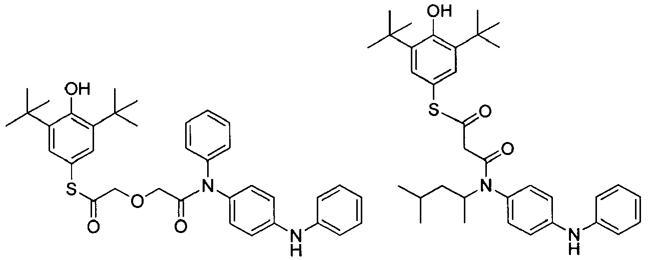

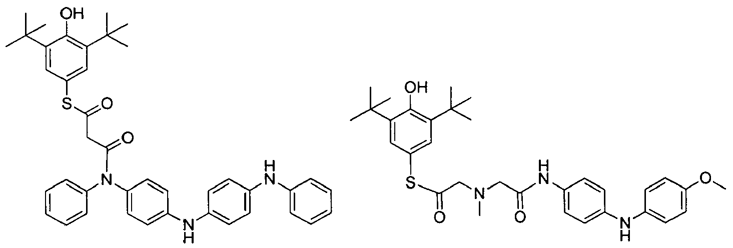

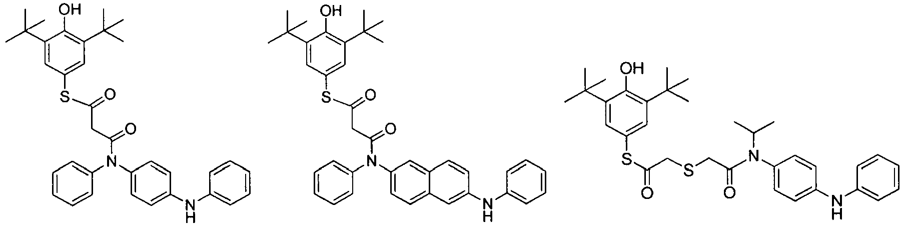

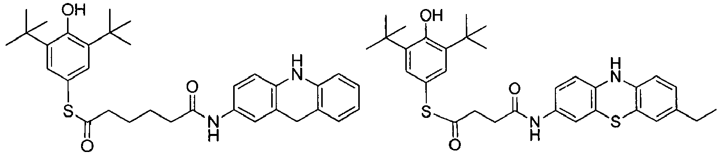

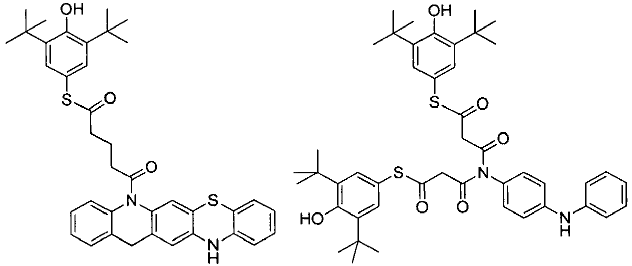

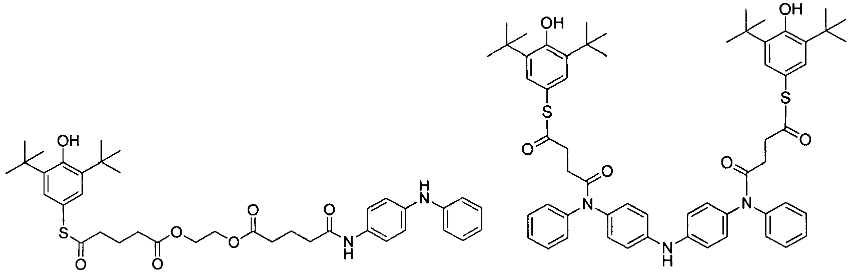

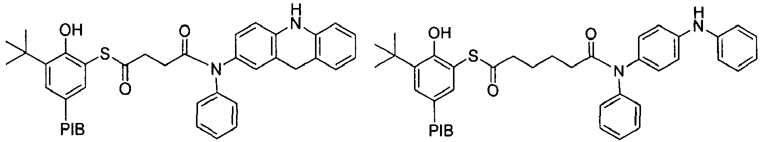

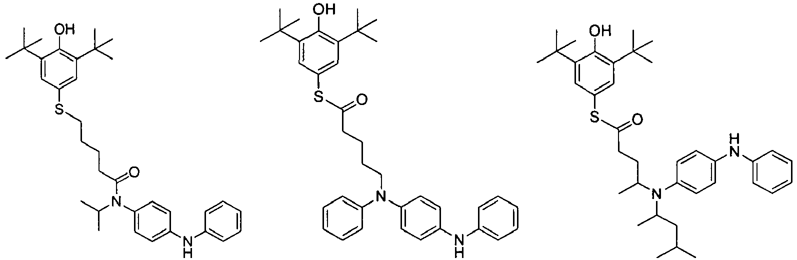

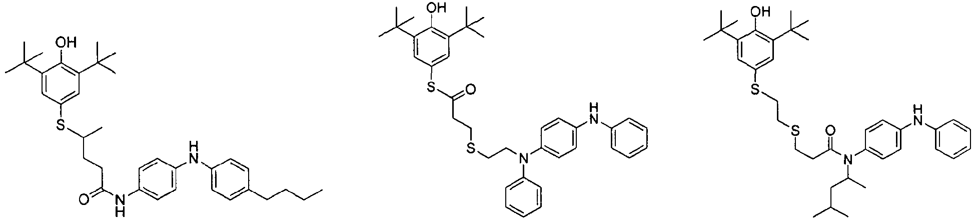

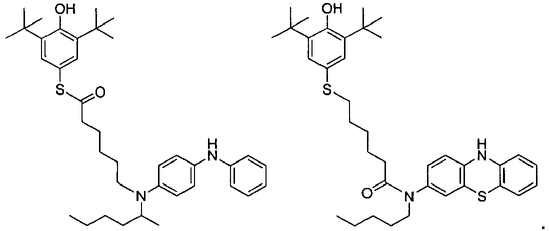

5. A process for producing a hindered phenol compound, including a first step of having a phenol compound represented by Formula (X) to react with an amine compound represented by Formula (Y) in the presence of at least one bridging compound selected from the group consisting of a compound represented by Formula (A) and formaldehyde, optionally further including an additional step of having the resultant of the first step to react with a sulfuring agent, preferably sulfur, and/or to react with an aldehyde compound represented by Formula (Z), 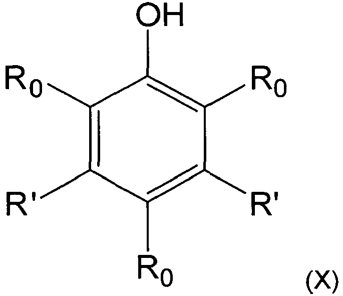 In Formula (X), the plural R₀s may be identical to or different from each other, each independently selected from the group consisting of -SH and a C₁₋₃₀₀ linear or branched alkyl, or a polyolefin group having a number-average molecular weight (Mn) of from 300 to 3000, with the proviso that at least one R₀ is -SH; the plural R's may be identical to or different from each other, each independently selected from the group consisting of hydrogen and a C₁₋₂₀ linear or branched alkyl, 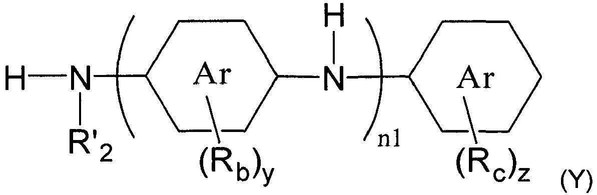 In Formula (Y), the group R'₂ is selected from the group consisting of hydrogen, a C₁₋₂₀ linear or branched alkyl and a group represented by 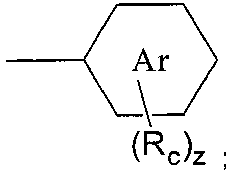 the plural R_{b}s may be identical to or different from each other, each independently selected from the group consisting of hydrogen and a C₁₋₂₀ linear or branched alkyl, the plural R_{c}s may be identical to or different from each other, each independently selected from the group consisting of hydrogen, a C₁₋₂₀ linear or branched alkyl and a C₁₋₂₀ linear or branched alkyloxy, a; y is an integer in the range of from 0 to 3; z is an integer in the range of from 0 to 3,; n1 is an integer in the range of from 1 to 8; the plural rings 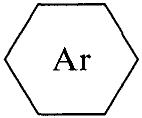 may be identical to or different from each other, each independently selected from the group consisting of benzene ring and naphthalene ring, ,
R_{f}(̵Fun )ₘ₁ (A)
In Formula (A), the group R_{f} is selected from the group consisting of a m1 valent C₁₋₂₀ hydrocarbyl, optionally substituted by one or more substituent selected from the group consisting of oxo, a C₁₋₂₀ hydrocarbyl, and a C₃₋₂₀ linear or branched hetero-alkyl and a m1 valent C₃₋₂₀ linear or branched hetero-alkyl optionally substituted by one or more substituent selected from the group consisting of oxo, a C₁₋₂₀ hydrocarbyl, and a C₃₋₂₀ linear or branched hetero-alkyl; m1 is an integer in the range of from 2 to 5, the plural Funs may be identical to or different from each other, each independently selected from the group consisting of a halogen atom, a carboxylic acid residue group, an anhydride residue group and an aldehyde residue group, while in case that m1 is 1, the group Fun is an aldehyde residue group, wherein by "carboxylic acid residue group " it refers to a group obtained by removing one carbonyl, i.e. 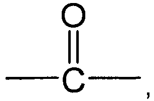 from carboxyl, i.e. -COOH, with the proviso that it is necessary for the carbon atom directly bonding to the group to take the form of carbonyl, i.e. 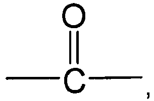 by "anhydride residue group " it refers to a group obtained by removing two carbonyls, i.e. 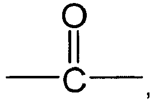 from anhydride, i.e. 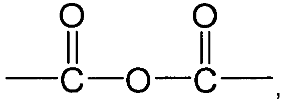 with the proviso that it is necessary for the two carbon atoms directly bonding to the group to take the form of carbonyl, i.e. 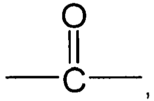 by "aldehyde residue group " it refers to a group obtained by removing one carbonyl, i.e. 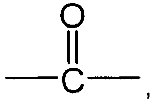 from formyl, i.e. 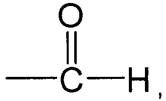 with the proviso that it is necessary for the carbon atom directly bonding to the group to take the form of carbonyl, i.e. 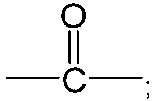 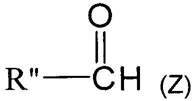 In Formula (Z), the group R" is selected from the group consisting of hydrogen, a C₁₋₂₀ hydrocarbyl, and a C₃₋₂₀ linear or branched hetero-alkyl,
wherein, the linear or branched hetero-alkyl is selected from the group consisting of a group obtained by directly replacing one or more group -CH₂- locating inside of a linear or branched alkyl by a corresponding number of replacing group selected from -O-, -S- or -NR'-, wherein R' is H or a C₁₋₄ linear or branched alkyl, and a group obtained by directly replacing one or more group -CH< locating inside of a linear or branched alkyl by a corresponding number of replacing group -N<.

6. The process according to Claim 5, wherein the bridging compound is one or more selected from the group consisting of an aldehyde compound represented by Formula (A_{Y}), a polyhalo-compound represented by Formula (A_{YY}) and a polybasic carboxylic acid represented by Formula (A_{YYY}) or derivatives thereof, selected from the group consisting of an anhydride of the polybasic carboxylic acid and an acyl halide of the polybasic carboxylic acid, 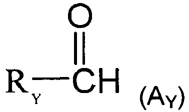 In Formula (A_{Y}), the group R_{Y} is selected from the group consisting of hydrogen, a C₁₋₂₀ hydrocarbyl, and a C₃₋₂₀ linear or branched hetero-alkyl,
Rₕₐₗₒ(̵Halo)_{m_{YY}} (A_{YY})
In Formula (A_{YY}), the group Rₕₐₗₒ is selected from the group consisting of a m_{YY} valent C₂₋₂₀ linear or branched alkyl optionally substituted by one or more substituent selected from the group consisting of a C₁₋₂₀ hydrocarbyl, and a C₃₋₂₀ linear or branched hetero-alkyl and a m_{YY} valent C₃₋₂₀ linear or branched hetero-alkyl optionally substituted by one or more substituent selected from the group consisting of a C₁₋₂₀ hydrocarbyl, and a C₃₋₂₀ linear or branched hetero-alkyl, the group Halo is a halogen atom, m_{YY} is an integer in the range of from 2 to 5,
R_{L}(̵COOH)_{m_{YYY}} (A_{YYY})
In Formula (A_{YYY}), the group R_{L} is selected from the group consisting of a m_{YYY} valent C₂ to C20-m_{YYY} linear or branched alkyl optionally substituted by one or more substituent selected from the group consisting of a C₁₋₂₀ hydrocarbyl, and a C₃₋₂₀ linear or branched hetero-alkyl, a m_{YYY} valent C₃ to C20-m_{YYY} linear or branched hetero-alkyl optionally substituted by one or more substituent selected from the group consisting of a C₁₋₂₀ hydrocarbyl, and a C₃₋₂₀ linear or branched hetero-alkyl and a group represented by 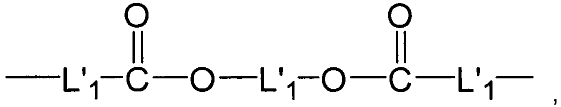 wherein, the plural L'₁s may be identical to or different from each other, each independently selected from the group consisting of a C₁₋₁₀ linear or branched alkyl and a C₃₋₁₀ linear or branched hetero-alkyl, with the proviso that (1) the plural L'₁s each independently represents a 2 valent to m_{YYY} valent group, so as to make the group 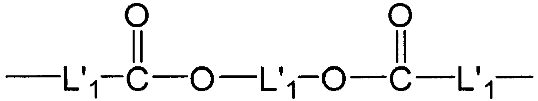 to present as, as a whole, m_{YYY} valent, and (2) the total atom number of all groups L'₁ is no more than 14, wherein the plural L'₁s may each independently be optionally further substituted by one or more substituent selected from the group consisting of a C₁₋₂₀ hydrocarbyl, m_{YYY} is an integer in the range of from 2 to 5, preferably 2.

7. The process according to Claim 5, wherein in the first step, molar ratio of the phenol compound represented by Formula (X) to the amine compound represented by Formula (Y) is 1 : 0.1-10, molar ratio of the phenol compound represented by Formula (X) to the bridging compound is 1 : 0.1-10, in the additional step, molar ratio of the amine compound represented by Formula (Y) to the sulfuring agent is 1 : 1-10, molar ratio of the amine compound represented by Formula (Y) to the aldehyde compound represented by Formula (Z) is 1 : 0.1-10.

8. Use of the hindered phenol compound according to any one of Claims 1 to 4 or a hindered phenol compound produced in line with the process according to any one of Claims 5 to 7 as an antioxidant.

9. A lubricant oil composition, comprising a lubricant base oil and a hindered phenol compound according to any one of Claims 1 to 4 or a hindered phenol compound produced in line with the process according to any one of Claims 5 to 7 as an antioxidant, wherein the antioxidant represents 0.001-30wt%, of the total weight of the lubricant oil composition.

10. The lubricant oil composition according to Claim 9, produced by mixing the hindered phenol compound with the lubricant base oil.

## Patentansprüche

1. Gehinderte Phenolverbindung, dargestellt durch Formel (I), 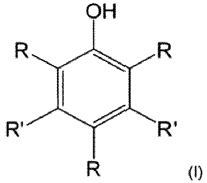 wobei in Formel (I),
die mehreren Rs identisch zueinander oder verschieden voneinander sein können, wobei jedes unabhängig ausgewählt ist aus der Gruppe, bestehend aus einem linearen oder verzweigten C₁₋₃₀₀-Alkyl oder einer Polyolefingruppe mit einem zahlengemittelten Molekulargewicht (Mn) von 300 bis 3000, einer Gruppe, die durch Formel (II) dargestellt ist und einer Gruppe, die durch Formel (III) dargestellt ist, mit der Maßgabe, dass zumindest ein R eine durch Formel (II) dargestellte Gruppe ist;
wobei die mehreren R identisch zueinander oder verschieden voneinander sein können, jeweils unabhängig ausgewählt aus der Gruppe, bestehend aus Wasserstoff und einem linearen oder verzweigten C₁₋₂₀-Alkyl,
-S-L(̵A)ₘ (II) 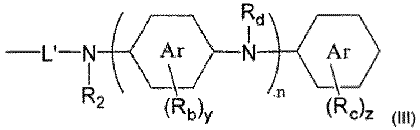 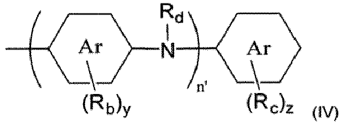
wobei in jeder Formel die Gruppe L ausgewählt ist aus der Gruppe, bestehend aus einem m+1-valenten linearen oder verzweigten C₁₋₂₀-Alkyl, das optional durch einen oder mehrere Substituenten substituiert ist, ausgewählt aus der Gruppe, bestehend aus Oxo, einem C₁₋₂₀-Hydrocarbyl und einem linearen oder verzweigten C₃₋₂₀-Heteroalkyl und einem m+1-valenten linearen oder verzweigten C₃₋₂₀-Heteroalkyl, das optional durch einen oder mehrere Substituenten substituiert ist, ausgewählt aus der Gruppe bestehend aus Oxo, einem C₁₋₂₀-Hydrocarbyl und einem linearen oder verzweigten C₃₋₂₀-Heteroalkyl;
wobei die Gruppe L' eine Gruppe ist, die dargestellt ist durch 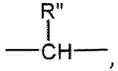 wobei die Gruppe R" ausgewählt ist aus der Gruppe, bestehend aus Wasserstoff, einem C₁₋₂₀-Hydrocarbyl und einem linearen oder verzweigten C₃₋₂₀-Heteroalkyl, wobei die mehreren A identisch zueinander oder verschieden voneinander sein können, jeweils unabhängig ausgewählt aus der Gruppe, bestehend aus 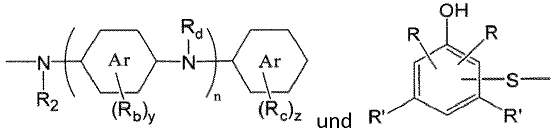 wobei die mehreren R identisch zueinander oder verschieden voneinander sein können, jeweils unabhängig ausgewählt aus der Gruppe, bestehend aus Wasserstoff, einem linearen oder verzweigten C₁₋₃₀₀-Alkyl oder einer Polyolefingruppe mit einem zahlengemittelten Molekulargewicht (Mn) von 300 bis 3000, einer Gruppe, die durch Formel (II) dargestellt ist und einer Gruppe, die durch Formel (III) dargestellt ist, wobei vorzugsweise jede unabhängig ausgewählt ist aus der Gruppe, bestehend aus Wasserstoff und einem linearen oder verzweigten C₁₋₃₀₀-Alkyl; wobei die mehreren R identisch zueinander oder verschieden voneinander sein können, jeweils unabhängig ausgewählt aus der Gruppe, bestehend aus Wasserstoff und einem linearen oder verzweigten C₁₋₂₀-Alkyl, wobei zwei R und eine -S--Gruppe jeweils die drei Restpositionen an dem Benzolring besetzen,
mit der Maßgabe, dass zumindest ein A 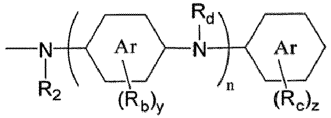 ist; wobei m eine ganze Zahl in dem Bereich von 1 bis 4 ist; wobei die mehreren R₂ identisch zueinander oder verschieden voneinander sein können, jeweils unabhängig ausgewählt sind aus der Gruppe, bestehend aus Wasserstoff, einem linearen oder verzweigten C₁₋₂₀-Alkyl, einer durch Formel (IV) dargestellten Gruppe und einer durch Formel (V) dargestellten Gruppe,; wobei die mehreren R_{b} identisch zueinander oder verschieden voneinander sein können, jeweils unabhängig ausgewählt aus der Gruppe, bestehend aus Wasserstoff und einen linearen oder verzweigten C₁₋₂₀-Alkyl,; wobei die mehreren R_{c}s identisch zueinander oder verschieden voneinander sein können, jeweils unabhängig ausgewählt sind aus der Gruppe, bestehend aus Wasserstoff, einem linearen oder verzweigten C₁₋₂₀-Alkyl und einem linearen oder verzweigten C₁₋₂₀-Alkyloxy, ; wobei y eine ganze Zahl in dem Bereich von 0 bis 3 ist,; z eine ganze Zahl in dem Bereich von 0 bis 3 ist; n eine ganze Zahl in dem Bereich von 1 bis 8 ist; n' eine ganze Zahl in dem Bereich von 0 bis 7 ist, mit der Maßgabe, dass n'+n ≤ 8 ist; wobei die mehreren R_{d} identisch zueinander oder verschieden voneinander sein können, jeweils unabhängig ausgewählt aus der Gruppe, bestehend aus Wasserstoff und einer durch Formel (V) dargestellten Gruppe; wobei die mehreren Ringe 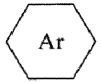 identisch zueinander oder verschieden voneinander sein können, jeweils unabhängig ausgewählt aus der Gruppe, bestehend aus einem Benzolring und einem Naphthalinring, wobei zwei aneinander angrenzende Ringe 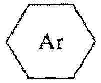 optional einen Phenothiazinring bilden können, wobei das N-Atom diese zwei Ringe und ein zusätzliches S-Atom überbrückt und/oder wobei zwei aneinander angrenzende Ringe 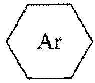 optional einen 9,10-Dihydroacridinring bilden können, wobei das N-Atom diese zwei Ringe und eine zusätzliche 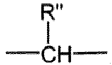 Gruppe überbrückt, wobei die Gruppe R" ausgewählt ist aus der Gruppe, bestehend aus Wasserstoff, einem C₁₋₂₀-Hydrocarbyl, und einem linearen oder verzweigten C₃₋₂₀-Heteroalkyl, oder einem 9,10-Dihydroacridinring,
-L(̵A)ₘ (V)
wobei in Formel (V) die mehreren A identisch zueinander oder verschieden voneinander sein können, jeweils unabhängig voneinander ausgewählt aus der Gruppe, bestehend aus 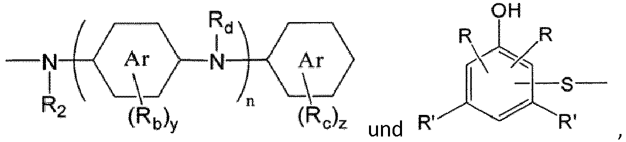 wobei die mehreren R identisch zueinander oder verschieden voneinander sein können, jeweils unabhängig ausgewählt aus der Gruppe, bestehend aus Wasserstoff, einem linearen oder verzweigten C₁₋₃₀₀-Alkyl, oder einer Polyolefingruppe mit einem zahlengemittelten Molekulargewicht (Mn) von 300 bis 3000, einer durch Formel (II) dargestellten Gruppe und einer durch Formel (III) dargestellten Gruppe, wobei die mehreren R identisch zueinander oder verschieden voneinander sein können, jeweils unabhängig ausgewählt sind aus der Gruppe, bestehend aus Wasserstoff und einem C₁₋₂₀ linearen oder verzweigten Alkyl,; wobei zwei R und eine -S--Gruppe jeweils die drei Restpositionen an dem Benzolring besetzen; wobei die Gruppe L, die Gruppe R₂, die Gruppe R_{b}, die Gruppe R_{c}, die Gruppe R_{d}, der Ring 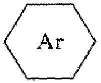 und y, n, z und m entsprechend wie in Formel (II) definiert sind,
wobei das lineare oder verzweigte Heteroalkyl ausgewählt ist aus der Gruppe, bestehend aus einer Gruppe, die durch direktes Ersetzen einer oder mehrerer Gruppen - CH₂-, die sich innerhalb eines linearen oder verzweigten Alkyls befinden, durch eine korrespondierende Anzahl von Ersatzgruppen, ausgewählt aus -O-, -S- und -NR'-erhalten wird, wobei R' H oder ein lineares oder verzweigtes C₁₋₄-Alkyl ist, und eine Gruppe, die durch direktes Ersetzen einer oder mehrerer -CH<-Gruppen, die sich innerhalb eines linearen oder verzweigten Alkyls befinden, durch eine korrespondierende Anzahl von -N<-Ersatzgruppen erhalten wird, wobei durch die Molekülstruktur der gehinderten Phenolverbindung hinweg zumindest ein R_{d} Wasserstoff ist.

2. Gehinderte Phenolverbindung nach Anspruch 1, wobei die Gruppe L ausgewählt ist aus der Gruppe, bestehend aus einer 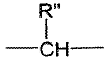 -Gruppe, wobei hierin m=1 ist, a ein m+1-valentes lineares oder verzweigtes C₂₋₂₀-Alkyl ist, das optional durch einen oder mehrere Substituenten substituiert ist, ausgewählt aus der Gruppe, bestehend aus einem C₁₋₂₀-Hydrocarbyl und einem linearen oder verzweigten C₃₋₂₀-Heteroalkyl, einem m+1-valenten linearen oder verzweigten C₃₋₂₀-Heteroalkyl, optional substituiert durch einen oder mehrere Substituenten, ausgewählt aus der Gruppe, bestehend aus einem C₁₋₂₀-Hydrocarbyl und einem linearen oder verzweigten C₃₋₂₀-Heteroalkyl und einer 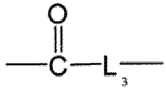 -Gruppe, wobei die Gruppe R" ausgewählt ist aus der Gruppe, bestehend aus Wasserstoff, einem C₁₋₂₀-Hydrocarbyl und einem linearen oder verzweigten C₃₋₂₀-Heteroalkyl, , wobei die Gruppe L₃ ausgewählt ist aus der Gruppe, bestehend aus einem m+1-valenten linearen oder verzweigten C₂₋₁₉-Alkyl, optional substituiert durch einen oder mehrere Substituenten, ausgewählt aus der Gruppe bestehend aus Oxo, einem C₁₋₂₀-Hydrocarbyl und einem linearen oder verzweigten C₃₋₂₀-Heteroalkyl und einem m+1-valenten linearen oder verzweigten C₃₋₁₉-Heteroalkyl, optional substituiert durch einen oder mehrere Substituenten, ausgewählt aus der Gruppe bestehend aus Oxo, einem C₁₋₂₀-Hydrocarbyl und einem linearen oder verzweigten C₃₋₂₀-Heteroalkyl.

3. Gehinderte Phenolverbindung nach Anspruch 1, wobei die Formel (II) ausgewählt ist aus einer der folgenden Formel (IIₓ), Formel (IIₓₓ) und Formel (IIₓₓₓ), 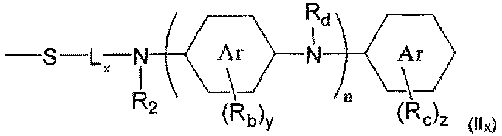 wobei in Formel (IIₓ) die Gruppe Lₓ eine durch 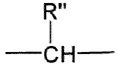 dargestellte Gruppe ist, wobei die Gruppe R" ausgewählt ist aus der Gruppe, bestehend aus Wasserstoff, einem C₁₋₂₀-Hydrocarbyl und einem linearen oder verzweigten C₃₋₂₀-Heteroalkyl, 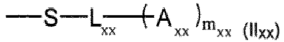 wobei in Formel (IIₓₓ), die Gruppe Lₓₓ ausgewählt ist aus der Gruppe, bestehend aus einem mₓₓ+1-valenten linearen oder verzweigten C₂₋₂₀-Alkyl, optional substituiert durch einen oder mehrere Substituenten, ausgewählt aus der Gruppe, bestehend aus einem C₁₋₂₀-Hydrocarbyl und einem linearen oder verzweigten C₃₋₂₀-Heteroalkyl und einem mₓₓ+1-valenten linearen oder verzweigten C₃₋₂₀-Heteroalkyl, optional substituiert durch einen oder mehrere Substituenten, ausgewählt aus der Gruppe, bestehend aus einem C₁₋₂₀-Hydrocarbyl und einem linearen oder verzweigten C₃₋₂₀-Heteroalkyl; wobei die mehreren Aₓₓ identisch zueinander oder verschieden voneinander sein können, jeweils unabhängig ausgewählt aus der Gruppe, bestehend aus 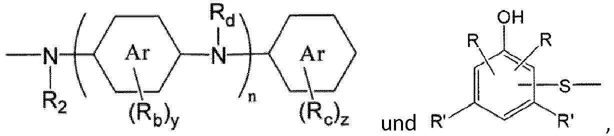 wobei die mehreren R identisch zueinander oder verschieden voneinander sein können, jeweils unabhängig voneinander ausgewählt aus der Gruppe, bestehend aus Wasserstoff, einem linearen oder verzweigten C₁₋₃₀₀-Alkyl, oder einer Polyolefingruppe mit einem zahlengemittelten Molekulargewicht (Mn) von 300 bis 3000, einer durch Formel (IIₓₓ) dargestellten Gruppe und einer durch Formel (III) dargestellten Gruppe, wobei die mehreren R' identisch zueinander oder verschieden voneinander sein können, jeweils unabhängig ausgewählt aus der, bestehend aus Wasserstoff und einem linearen oder verzweigten C₁₋₂₀-Alkyl, wobei zwei R und eine -S--Gruppe jeweils die drei Restpositionen an dem Benzolring besetzen,
unter der Maßgabe, dass zumindest ein Aₓₓ 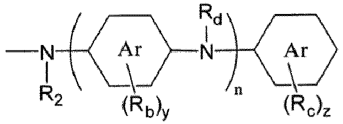 ist; ist; wobei mₓₓ eine ganze Zahl in dem Bereich von 1 bis 4 ist, vorzugsweise 1, 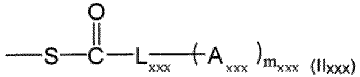 wobei in Formel (IIₓₓₓ) die Gruppe Lₓₓₓ ausgewählt ist aus der Gruppe, bestehend aus einem mₓₓₓ+1-valenten linearen oder verzweigten C₂ bis C19-mₓₓₓ Alkyl, optional substituiert durch einen oder mehrere Substituenten, ausgewählt aus der Gruppe, bestehend aus einem C₁₋₂₀-Hydrocarbyl und einem linearen oder verzweigten C₃₋₂₀-Heteroalkyl und einer durch 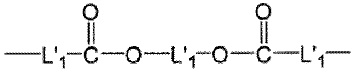 dargestellten Gruppe, wobei die mehreren L'₁ identisch zueinander oder verschieden voneinander sein können, jeweils unabhängig voneinander ausgewählt aus der Gruppe, bestehend aus einem linearen oder verzweigten C₁₋₁₀-Alkyl, und einem linearen oder verzweigten C₃₋₁₀-Heteroalkyl, mit der Maßgabe, dass (1) die mehreren L'₁ jeweils unabhängig eine 2-valente bis mₓₓₓ+1-valente Gruppe darstellen, um so die Gruppe 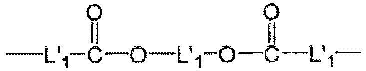 zu schaffen, um in einer Gesamtheit mₓₓₓ+1-valentzu machen, und (2) die Gesamtatomanzahl aller L'₁-Gruppen nicht größer als 14 ist, wobei die mehreren L'₁ jeweils unabhängig optional ferner substituiert sein können durch einen oder mehrere Substituenten, ausgewählt aus der Gruppe, bestehend aus einem C₁₋₂₀-Hydrocarbyl und einem linearen oder verzweigten C₃₋₂₀-Heteroalkyl; wobei die mehreren Aₓₓₓ identisch zueinander oder verschieden voneinander sein können, jeweils unabhängig ausgewählt aus der Gruppe, bestehend aus 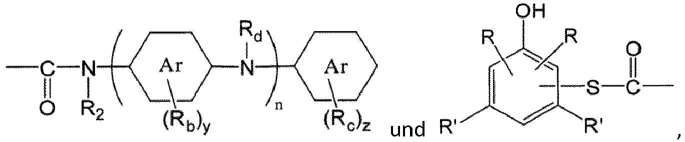 wobei die mehreren R identisch zueinander oder verschieden voneinander sein können, jeweils unabhängig ausgewählt aus der Gruppe, bestehend aus Wasserstoff, einem linearen oder verzweigten C₁₋₃₀₀-, oder einer Polyolefingruppe mit einem zahlengemittelten Molekulargewicht (Mn) von 300 bis 3000, einer durch Formel (IIₓₓₓ) dargestellten Gruppe und einer durch Formel (III) dargestellten Gruppe, wobei die mehreren R' identisch zueinander oder verschieden voneinander sein können, jeweils unabhängig ausgewählt aus der Gruppe, bestehend aus Wasserstoff und einem linearen oder verzweigten C₁₋₂₀-Alkyl, wobei zwei R und eine 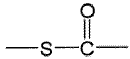 -Gruppe jeweils die drei Restpositionen an dem Benzolring besetzen, mit der Maßgabe, dass zumindest ein Axxx 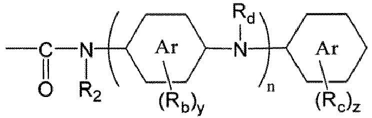 ist; wobei mₓₓₓ eine ganze Zahl in dem Bereich von 1 bis 4 ist, vorzugsweise 1, und/oder
wobei die Formel (V) ausgewählt ist aus einer der folgenden Formel (Vₓ), Formeln (Vₓₓ) und Formel (Vₓₓₓ), 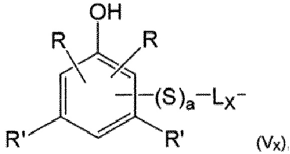 wobei in Formel (Vₓ) die mehreren R identisch zueinander oder verschieden voneinander sein können, jeweils unabhängig ausgewählt aus der Gruppe, bestehend aus Wasserstoff, einem linearen oder verzweigten C₁₋₃₀₀-Alkyl, oder einer Polyolefingruppe mit einem zahlengemittelten Molekulargewicht (Mn) von 300 bis 3000, einer durch Formel (IIₓ) dargestellten Gruppe und einer durch Formel (III) dargestellten Gruppe,; wobei die mehreren R' identisch zueinander oder verschieden sein können, jeweils ausgewählt aus der Gruppe, bestehend aus Wasserstoff und einem linearen oder verzweigten C₁₋₂₀-Alkyl,; wobei die Gruppe Lₓ eine durch 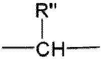 dargestellte Gruppe ist, wobei die Gruppe R" ausgewählt ist aus der Gruppe, bestehend aus Wasserstoff, einem C₁₋₂₀-Hydrocarbyl und einem linearen oder verzweigten C₃₋₂₀-Heteroalkyl, wobei a 1 ist, und wobei zwei R und eine Gruppe -(S)ₐ-Lₓ- jeweils die drei Restpositionen an dem Benzolring besetzen 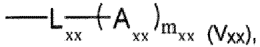 wobei in Formel (Vₓₓ) die mehreren Aₓₓ identisch oder verschieden sein können, jeweils unabhängig ausgewählt aus der Gruppe, bestehend aus 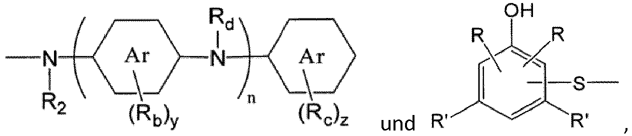 wobei die mehreren R identisch oder verschieden voneinander sein können, jeweils unabhängig ausgewählt aus der Gruppe, bestehend aus Wasserstoff, einem linearen oder verzweigten C₁₋₃₀₀-Alkyl, oder einer Polyolefingruppe mit einem zahlengemittelten Molekulargewicht (Mn) von 300 bis 3000, einer durch Formel (IIₓₓ) dargestellten Gruppe und einer durch Formel (III) dargestellten Gruppe,; wobei die mehreren R' identisch oder verschieden voneinander sein können, jeweils unabhängig ausgewählt aus der Gruppe, bestehend aus Wasserstoff und einem linearen oder verzweigten C₁₋₂₀-Alkyl,; wobei zwei R und eine -S--Gruppe jeweils die drei Restpositionen an dem Benzolring besetzen; wobei die Gruppe Lₓₓ und mₓₓ entsprechend als die in Formel (IIₓₓ) definierten sind, 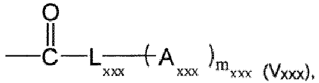 wobei in Formel (Vₓₓₓ), die mehreren Aₓₓₓ identisch zueinander oder verschieden voneinander sein können, jeweils unabhängig ausgewählt aus der Gruppe, bestehend aus 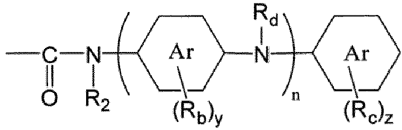 und 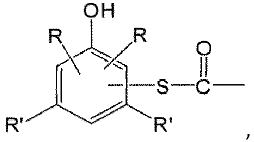 wobei die mehreren R identisch zueinander oder verschieden voneinander sein können, jeweils unabhängig ausgewählt aus der Gruppe, bestehend aus Wasserstoff, einem linearen oder verzweigten C₁₋₃₀₀- oder einer Polyolefingruppe mit einem zahlengemittelten Molekulargewicht (Mn) von 300 bis 3000, einer durch Formel (IIₓₓₓ) dargestellten Gruppe und einer durch Formel (III) dargestellten Gruppe,; wobei die mehreren R' identisch zueinander oder verschieden voneinander sein können, jeweils unabhängig ausgewählt aus der Gruppe, bestehend aus Wasserstoff und einem C₁₋₂₀ linearen oder verzweigten Alkyl,; wobei zwei R und eine 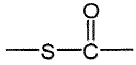 -Gruppe jeweils die drei Restpositionen an dem Benzolring besetzen; wobei die Gruppe Lₓₓₓ und mₓₓₓ entsprechend wie in Formel (IIₓₓₓ) definiert sind, andere Gruppen und numerische Werte wie in Anspruch 1 definiert sind.

4. Gehinderte Phenolverbindung nach Anspruch 1, ausgewählt aus der Gruppe, bestehend aus den folgenden spezifischen Verbindungen und deren Mischung bei beliebigem Verhältnis dazwischen : 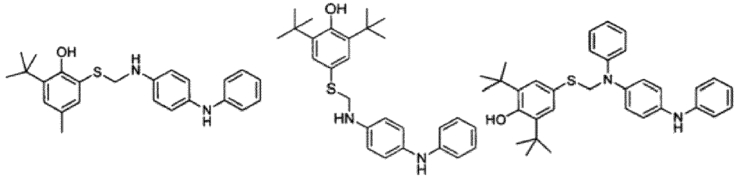 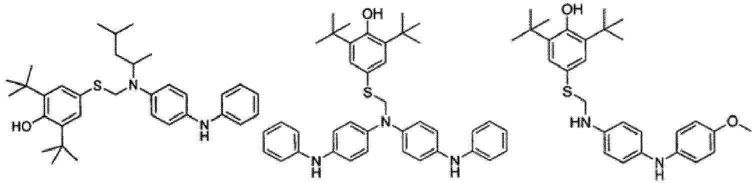 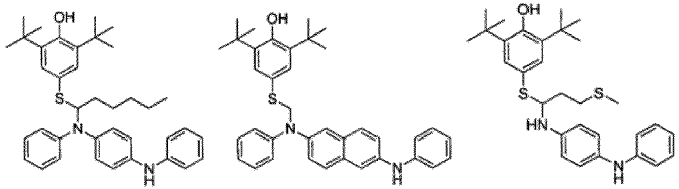 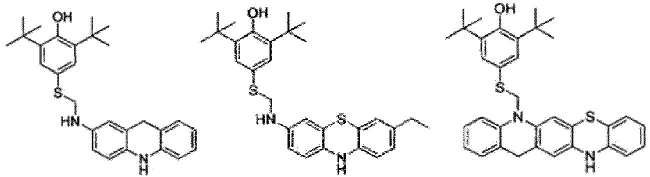 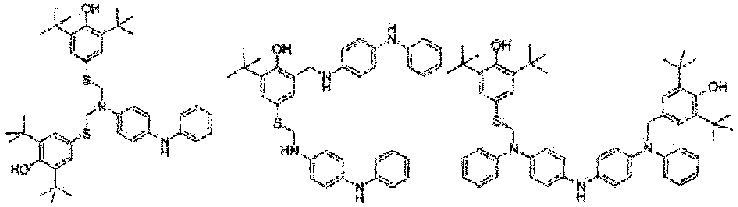 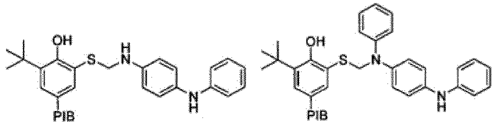 PIB = Polyisobuten

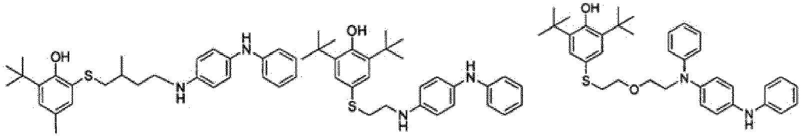

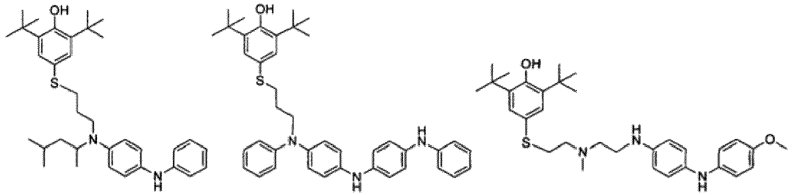

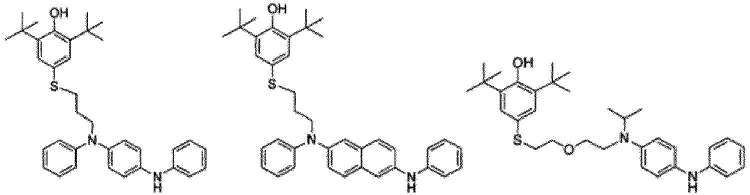

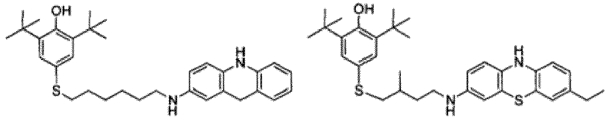

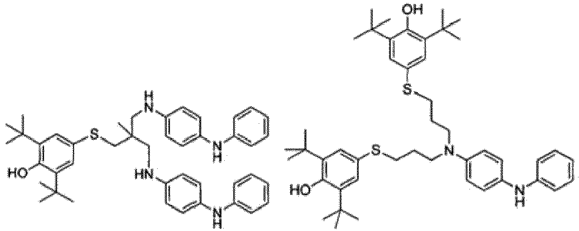

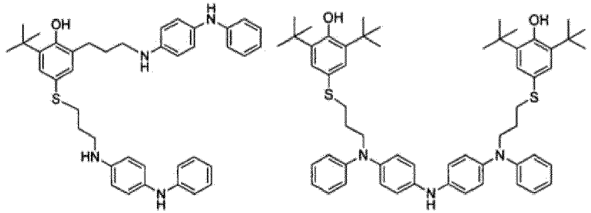

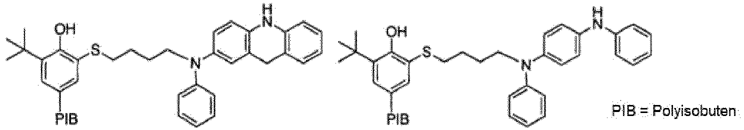

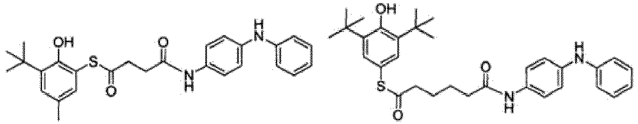

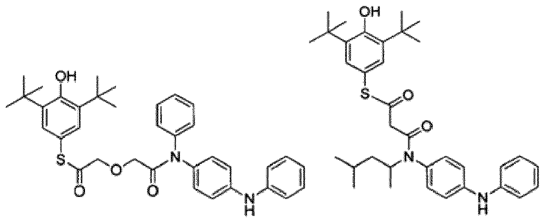

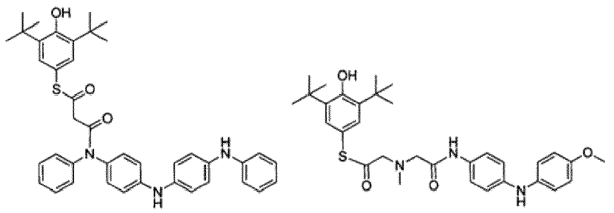

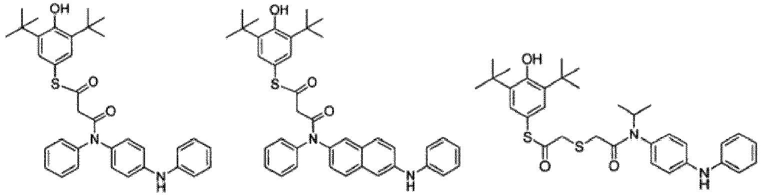

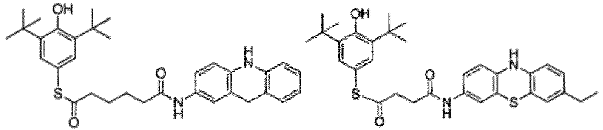

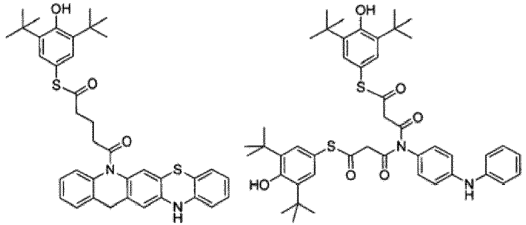

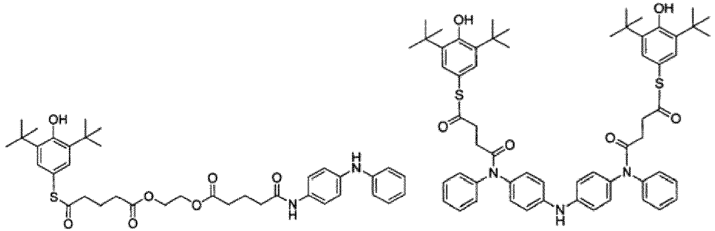

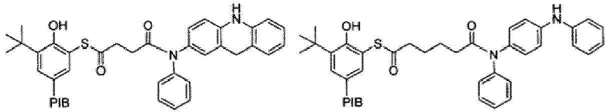

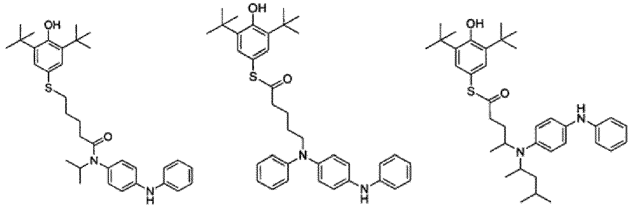

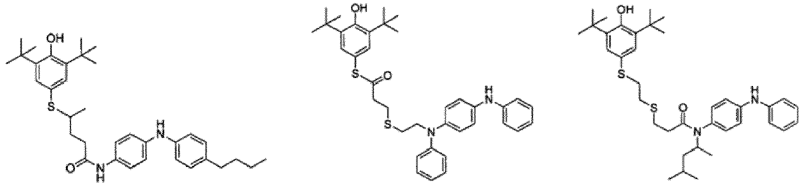

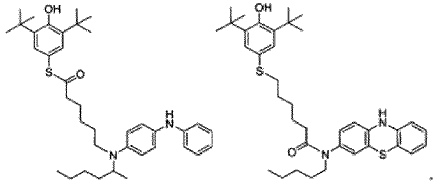

5. Verfahren zum Herstellen einer gehinderten Phenolverbindung, enthaltend einen ersten Schritt des Reagierenlassens einer durch Formel (X) dargestellten Phenolverbindungmit einer durch Formel (Y) dargestellten Aminverbindung in der Anwesenheit von zumindest einer brückenbildenden Verbindung, ausgewählt aus der Gruppe, bestehend aus einer durch Formel (A) dargestellten Verbindung und Formaldehyd, optional ferner enthaltend einen zusätzlichen Schritt des Reagierenlassens des Resultats des ersten Schritts mit einem Schwefelungsmittel, vorzugsweise Schwefel, und/oder mit einer durch Formel (Z) dargestellten Aldehydverbindung reagieren zu lassen, 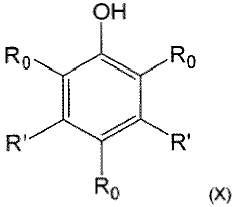 wobei in Formel (X) die mehreren R₀ identisch zueinander oder verschieden voneinander sein können, jeweils unabhängig ausgewählt aus der Gruppe, bestehend aus -SH und einem linearen oder verzweigten C₁₋₃₀₀-Alkyl oder einer Polyolefingruppe mit einem zahlengemittelten Molekulargewicht (Mn) von 300 bis 3000, mit der Maßgabe, dass zumindest ein R₀ -SH ist; wobei die mehreren R' identisch zueinander oder verschieden voneinander sein können, jeweils unabhängig ausgewählt aus der Gruppe, bestehend aus Wasserstoff und einem linearen oder verzweigten C₁₋₂₀-Alkyl, 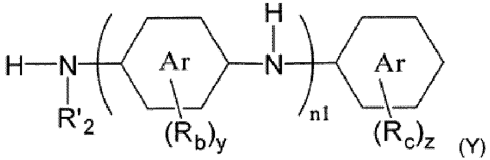 wobei in Formel (Y), die Gruppe R'₂ ausgewählt ist aus der Gruppe, bestehend aus Wasserstoff, einem linearen oder verzweigten C₁₋₂₀-Alkyl und einer durch 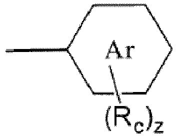 dargestellten Gruppe; wobei die mehreren R_{b} identisch zueinander oder verschieden voneinander sein können, jeweils unabhängig ausgewählt aus der Gruppe, bestehend aus Wasserstoff und einem linearen oder verzweigten C₁₋₂₀-Alkyl, wobei die mehreren R_{c} identisch zueinander oder verschieden voneinander sein können, jeweils unabhängig ausgewählt aus der Gruppe, bestehend aus Wasserstoff, einem linearen oder verzweigten C₁₋₂₀-Alkyl und einem linearen oder verzweigten C₁₋₂₀-Alkyloxy, a; wobei y eine ganze Zahl im Bereich von 0 bis 3 ist; wobei z eine ganze Zahl im Bereich von 0 bis 3 ist,; wobei n1 eine ganze Zahl im Bereich von 1 bis 8 ist; wobei die mehreren 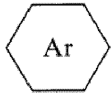 -Ringe identisch zueinander oder verschieden voneinander sein können, jeweils unabhängig ausgewählt aus der Gruppe, bestehend aus einem Benzolring und einem Naphthalinring,
R_{f}(̵Fun)ₘ₁ (A)
wobei in Formel (A) die Gruppe R_{f} ausgewählt ist aus der Gruppe, bestehend aus einem m1-valenten C₁₋₂₀-Hydrocarbyl, optional substituiert ist durch einen oder mehrere Substituenten, ausgewählt aus der Gruppe, bestehend aus Oxo, einem C₁₋₂₀-Hydrocarbyl und einem C₃₋₂₀ linearen oder verzweigten Heteroalkyl und einem m1-valenten linearen oder verzweigten C₃₋₂₀-Heteroalkyl, optional substituiert durch einen oder mehrere Substituenten, ausgewählt aus der Gruppe, bestehend aus Oxo, einem C₁₋₂₀-Hydrocarbyl, und einem linearen oder verzweigten C₃₋₂₀-Heteroalkyl; wobei m1 eine ganze Zahl im Bereich von 2 bis 5 ist, wobei die mehreren Fun identisch zueinander oder verschieden voneinander sein können, jeweils unabhängig ausgewählt aus der Gruppe, bestehend aus einem Halogenatom, einer Carbonsäurerestgruppe, einer Anhydridrestgruppe und einer Aldehydrestgruppe, wobei in dem Fall, dass m1 1 ist, die Fun-Gruppe eine Aldehydrestgruppe ist, wobei mit "Carbonsäurerestgruppe" auf eine Gruppe verwiesen wird, die durch Entfernen eines Carbonyls, d. h. 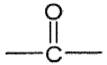 erhalten wurde, von Carboxyl, d. h. -COOH, mit der Maßgabe, dass es für das Kohlenstoffatom, das direkt an die Gruppe bindet, notwendig ist, um die Carbonylform anzunehmen, d. h. 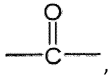 wobei mit "Anhydridrestgruppe" auf eine Gruppe verwiesen wird, die durch Entfernen zweier Carbonyle, d. h. 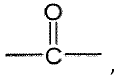 aus Anhydrid, d. h. 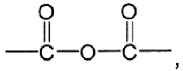 erhalten wird, mit der Maßgabe, dass es für die zwei Kohlenstoffatome, die direkt an die Gruppe binden, notwendig ist, um die Carbonylform anzunehmen, d. h. 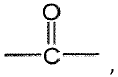 wobei mit "Aldehydrestgruppe" auf eine Gruppe verwiesen wird, die durch Entfernen eines Carbonyls, d. h. 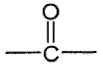 aus Formyl, d. h. 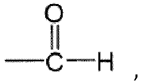 erhalten wird, mit der Maßgabe, dass es für das Kohlenstoffatom, das direkt an die Gruppe bindet, notwendig ist, um die Carbonylform anzunehmen, d. h. 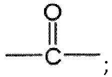 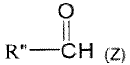 wobei in Formel (Z) die Gruppe R" ausgewählt ist aus der Gruppe, bestehend aus Wasserstoff, einem C₁₋₂₀-Hydrocarbyl, und einem linearen oder verzweigten C₃₋₂₀-Heteroalkyl,
wobei das lineare oder verzweigte Heteroalkyl ausgewählt ist aus der Gruppe, bestehend aus einer Gruppe, die durch direktes Ersetzen einer oder mehrerer -CH₂-Gruppen, die sich innerhalb eines linearen oder verzweigten Alkyls befinden, durch eine entsprechende Anzahl ersetzender Gruppen, ausgewählt aus -O-, -S- oder -NR'-, wobei R' H oder ein lineares oder verzweigtes C₁₋₄-Alkyl ist, und eine Gruppe, die durch direktes Ersetzen einer oder mehrerer -CH<-Gruppen erhalten wird, die sich innerhalb eines linearen oder verzweigten Alkyls befinden, durch eine entsprechende Anzahl von ersetzenden -N<-Gruppen.

6. Verfahren nach Anspruch 5, wobei die überbrückende Verbindung eine oder mehrere ist aus der Gruppe, bestehend aus einer durch Formel (A_{Y}) dargestellten Aldehydverbindung, einer durch Formel (A_{YY}) dargestellten Polyhalogenverbindung und einer durch Formel (A_{YYY}) dargestellten polybasischen Carbonsäure oder Ableitungen davon, ausgewählt aus der Gruppe, bestehend aus einem Anhydrid der polybasischen Carbonsäure und einem Acylhalogenid der polybasischen Carbonsäure, 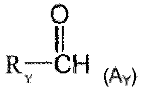 wobei in Formel (A_{Y}) die Gruppe R_{Y} ausgewählt ist aus der Gruppe, bestehend aus Wasserstoff, einem C₁₋₂₀-Hydrocarbyl und einem linearen oder verzweigten C₃₋₂₀-Heteroalkyl,
Rₕₐₗₒ(̵Halo)_{m_{YY}} (A_{YY})
wobei in Formel (A_{YY}) die Gruppe R_{Halo} ausgewählt ist aus der Gruppe, bestehend aus einem m_{YY}-valenten linearen oder verzweigten C₂₋₂₀-Alkyl, optional substituiert durch einen oder mehrere Substituenten, ausgewählt aus der Gruppe, bestehend aus einem C₁₋₂₀-Hydrocarbyl und einem linearen oder verzweigten C₃₋₂₀-Heteroalkyl und einem m_{YY}-valenten linearen oder verzweigten C₃₋₂₀-Heteroalkyl, optional substituiert durch einen oder mehrere Substituenten, ausgewählt aus der Gruppe, bestehend aus einem C₁₋₂₀-Hydrocarbyl und einem linearen oder verzweigten C₃₋₂₀-Heteroalkyl, wobei die Halo-Gruppe ein Halogenatom ist, wobei m_{YY} eine ganze Zahl im Bereich von 2 bis 5 ist,
R_{L}(̵COOH)_{m_{YYY}} (A_{YYY})
wobei in Formel (A_{YYY}) die Gruppe R_{L} ausgewählt ist aus der Gruppe, bestehend aus einem m_{YYY}-valenten linearen oder verzweigten C₂ bis C20-m_{YYY}-Alkyl, optional substituiert durch einen oder mehrere Substituenten, ausgewählt aus der Gruppe, bestehend aus einem C₁₋₂₀-Hydrocarbyl und einem linearen oder verzweigten C₃₋₂₀-Heteroalkyl, einem m_{YYY}-valenten linearen oder verzweigten C₃ bis C20-m_{YYY}-Heteroalkyl, optional substituiert durch einen oder mehrere Substituenten, ausgewählt aus der Gruppe, bestehend aus einem C₁₋₂₀-Hydrocarbyl und einem linearen oder verzweigten C₃₋₂₀-Heteroalkyl und einer durch 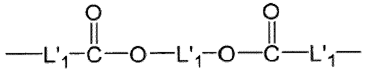 dargestellten Gruppe, wobei die mehreren L'₁ identisch zueinander oder verschieden voneinander sein können, jeweils unabhängig ausgewählt aus der Gruppe, bestehend aus einem linearen oder verzweigten C₁₋₁₀-Alkyl und einem linearen oder verzweigten C₃₋₁₀-Heteroalkyl, mit der Maßgabe, dass (1) die mehreren L'₁ jeweils unabhängig eine 2-valente bis m_{YYY}-valente Gruppe darstellen, um so die Gruppe 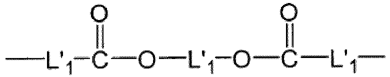 zu schaffen, um in einer Gesamtheit m_{YYY}-Valenz abzubilden, und dass (2) die Gesamtatomanzahl aller L'₁-Guppen nicht größer ist als 14, wobei die mehreren L'₁ jeweils unabhängig optional ferner substituiert sein können durch einen oder mehrere Substituenten, ausgewählt aus der Gruppe, bestehend aus einem C₁₋₂₀-Hydrocarbyl, wobei m_{YYY} eine ganze Zahl im Bereich von 2 bis 5 ist, vorzugsweise 2.

7. Verfahren nach Anspruch 5, wobei in dem ersten Schritt das Molverhältnis der Phenolverbindung, dargestellt durch Formel (X), zu der Aminverbindung, dargestellt durch Formel (Y), 1 : 0,1-10 beträgt, wobei das Molverhältnis der Phenolverbindung, dargestellt durch Formel (X), zu der überbrückenden Verbindung 1 : 0,1-10 beträgt, wobei in dem zusätzlichen Schritt ein Molverhältnis der Aminverbindung, dargestellt durch Formel (Y), zu dem Schwefelungsmittel 1 : 1-10 beträgt, wobei das Molverhältnis der Aminverbindung, dargestellt durch Formel (Y), zu der Aldehydverbindung, dargestellt durch Formel (Z), 1 : 0,1- 10 beträgt.

8. Verwendung der gehinderten Phenolverbindung nach einem der Ansprüche 1 bis 4 oder eine im Einklang mit dem Verfahren nach einem der Ansprüche 5 bis 7 hergestellte gehinderte Phenolverbindung als ein Antioxidationsmittel.

9. Schmierölzusammensetzung, umfassend ein Basisschmieröl und eine gehinderte Phenolverbindung nach einem der Ansprüche 1 bis 4 oder eine im Einklang mit dem Verfahren nach einem der Ansprüche 5 bis 7 hergestellte gehinderte Phenolverbindung als ein Antioxidationsmittel, wobei das Antioxidationsmittel 0,001-30 Gew.-% des Gesamtgewichts der Schmierölzusammensetzung darstellt.

10. Schmierölzusammensetzung nach Anspruch 9, hergestellt durch Mischen der gehinderten Phenolverbindung mit dem Basisschmieröl.

## Revendications

1. Composé de phénol encombré représenté par la formule (I), 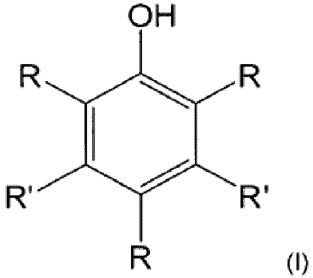 dans la formule (I),
les R peuvent être identiques les uns aux autres ou différents les uns des autres, sélectionnés chacun indépendamment dans le groupe constitué d'un alkyle linéaire ou ramifié en C₁ à C₃₀₀, ou d'un groupe polyoléfine présentant un poids moléculaire moyen en nombre (Mn) de 300 à 3000, d'un groupe représenté par la formule (II) et d'un groupe représenté par la formule (III), à condition qu'au moins un R soit un groupe représenté par la formule (II) ;
les R' peuvent être identiques les uns aux autres ou différents les uns des autres, sélectionnés chacun indépendamment dans le groupe constitué d'un hydrogène et d'un alkyle linéaire ou ramifié en C₁ à C₂₀,
-S-L(̵A)ₘ (II) 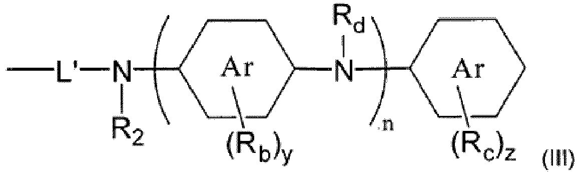 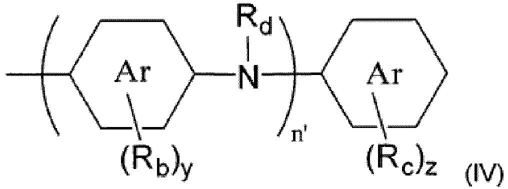
dans chaque formule, le groupe L est sélectionné dans le groupe constitué d'un alkyle linéaire ou ramifié de valence m+1 en C₁ à C₂₀ facultativement substitué avec un ou plusieurs substituants sélectionnés dans le groupe constitué d'un oxo, d'un hydrocarbyle en C₁ à C₂₀, et d'un hétéro-alkyle linéaire ou ramifié en C₃ à C₂₀ et d'un hétéro-alkyle linéaire ou ramifié de valence m+1 en C₃ à C₂₀ facultativement substitué avec un ou plusieurs substituants sélectionnés dans le groupe constitué d'un oxo, d'un hydrocarbyle en C₁ à C₂₀, et d'un hétéro-alkyle linéaire ou ramifié en C₃ à C₂₀ ; le groupe L' est un groupe représenté par 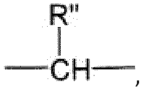 dans lequel le groupe R" est sélectionné dans le groupe constitué d'un hydrogène, d'un hydrocarbyle en C₁ à C₂₀, et d'un hétéro-alkyle linéaire ou ramifié en C₃ à C₂₀, les A peuvent être identiques les uns aux autres ou différents les uns des autres, sélectionnés chacun indépendamment dans le groupe constitué de 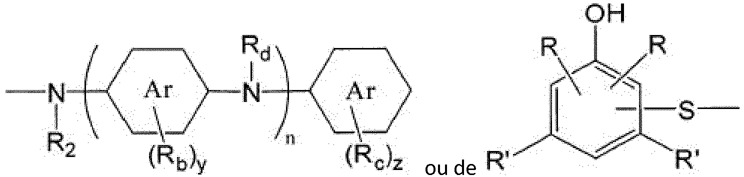 dans lesquels les R peuvent être identiques les uns aux autres ou différents les uns des autres, sélectionnés chacun indépendamment dans le groupe constitué d'un hydrogène, d'un alkyle linéaire ou ramifié en C₁ à C₃₀₀, ou d'un groupe polyoléfine présentant un poids moléculaire moyen en nombre (Mn) de 300 à 3000, d'un groupe représenté par la formule (II) et d'un groupe représenté par la formule (III), de préférence sélectionnés chacun indépendamment dans le groupe constitué d'un hydrogène et d'un alkyle linéaire ou ramifié en C₁ à C₃₀₀ ; les R' peuvent être identiques les uns aux autres ou différents les uns des autres, sélectionnés chacun indépendamment dans le groupe constitué d'un hydrogène et d'un alkyle linéaire ou ramifié en C₁ à C₂₀, deux R et un groupe -S- occupent les trois positions restantes sur le cycle benzène, respectivement,
à condition qu'au moins un A soit 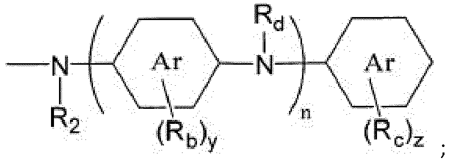 m est un nombre entier situé dans la plage allant de 1 à 4; les R₂ peuvent être identiques les uns aux autres ou différents les uns des autres, sélectionnés chacun indépendamment dans le groupe constitué d'un hydrogène, d'un alkyle linéaire ou ramifié en C₁ à C₂₀, d'un groupe représenté par la formule (IV) et d'un groupe représenté par la formule (V) ; les R_{b} peuvent être identiques les uns aux autres ou différents les uns des autres, sélectionnés chacun indépendamment dans le groupe constitué d'un hydrogène et d'un alkyle linéaire ou ramifié en C₁ à C₂₀ ; les R_{c} peuvent être identiques les uns aux autres ou différents les uns des autres, sélectionnés chacun indépendamment dans le groupe constitué d'un hydrogène, d'un alkyle linéaire ou ramifié en C₁ à C₂₀ et d'un alkyloxy linéaire ou ramifié en C₁ à C₂₀; y est un nombre entier situé dans la plage allant de 0 à 3 ; z est un nombre entier situé dans la plage allant de 0 à 3 ; n est un nombre entier situé dans la plage allant de 1 à 8 ; n' est un nombre entier situé dans la plage allant de 0 à 7, à condition que n' + n ≤ 8 ; les R_{d} peuvent être identiques les uns aux autres ou différents les uns des autres, sélectionnés chacun indépendamment dans le groupe constitué d'un hydrogène et d'un groupe représenté par la formule (V) ; les cycles 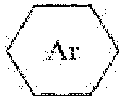 peuvent être identiques les uns aux autres ou différents les uns des autres, sélectionnés chacun indépendamment dans le groupe constitué d'un cycle benzène et d'un cycle naphtalène, dans lequel deux cycles 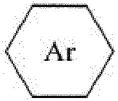 adjacents peuvent former facultativement un cycle phénothiazine avec l'atome N reliant ces deux cycles et un atome S supplémentaire, et/ou, deux cycles 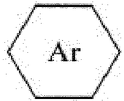 adjacents peuvent former facultativement un cycle 9,10-dihydroacridine avec l'atome N reliant ces deux cycles et un groupe 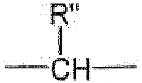 supplémentaire, dans lequel le groupe R" est sélectionné dans le groupe constitué d'un hydrogène, d'un hydrocarbyle en C₁ à C₂₀, et d'un hétéro-alkyle linéaire ou ramifié en C₃ à C₂₀, ou d'un cycle 9,10-dihydroacridine,
-L(̵A)ₘ (V)
dans la formule (V), les A peuvent être identiques les uns aux autres ou différents les uns des autres, sélectionnés chacun indépendamment dans le groupe constitué de 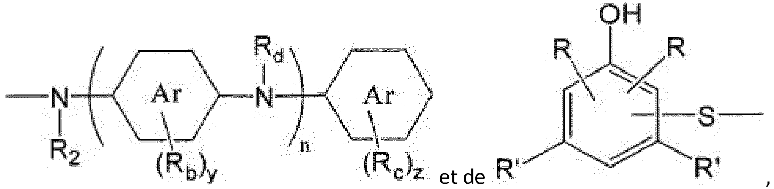 dans lesquels les R peuvent être identiques les uns aux autres ou différents les uns des autres, sélectionnés chacun indépendamment dans le groupe constitué d'un hydrogène, d'un alkyle linéaire ou ramifié en C₁ à C₃₀₀, ou d'un groupe polyoléfine présentant un poids moléculaire moyen en nombre (Mn) de 300 à 3000, d'un groupe représenté par la formule (II) et d'un groupe représenté par la formule (III), les R' peuvent être identiques les uns aux autres ou différents les uns des autres, sélectionnés chacun indépendamment dans le groupe constitué d'un hydrogène et d'un alkyle linéaire ou ramifié en C₁ à C₂₀ ; deux R et un groupe -S- occupent les trois positions restantes sur le cycle benzène, respectivement ; le groupe L, le groupe R₂, le groupe R_{b}, le groupe R_{c}, le groupe R_{d}, le cycle 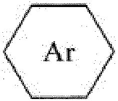 et y, n, z et m sont tels que définis dans la formule (II), respectivement,
dans lequel l'hétéro-alkyle linéaire ou ramifié est sélectionné dans le groupe constitué d'un groupe obtenu par le remplacement direct d'un ou de plusieurs groupes -CH₂- se situant à l'intérieur d'un alkyle linéaire ou ramifié par un nombre correspondant de groupes de remplacement sélectionnés parmi -O-, -S- et -NR'-, dans lequel R' est H ou un alkyle linéaire ou ramifié en C₁ à C₄ et un groupe obtenu par le remplacement direct d'un ou de plusieurs groupes -CH< se situant à l'intérieur d'un alkyle linéaire ou ramifié par un nombre correspondant de groupes de remplacement -N<, dans lequel tout au long de la structure moléculaire du composé de phénol encombré, au moins un R_{d} est un hydrogène.

2. Composé de phénol encombré selon la revendication 1, dans lequel le groupe L est sélectionné dans le groupe constitué d'un groupe 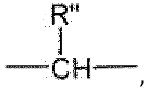 ici m = 1, d'un alkyle linéaire ou ramifié de valence m+1 en C₂ à C₂₀ facultativement substitué avec un ou plusieurs substituants sélectionnés dans le groupe constitué d'un hydrocarbyle en C₁ à C₂₀, et d'un hétéro-alkyle linéaire ou ramifié en C₃ à C₂₀, d'un hétéro-alkyle linéaire ou ramifié de valence m+1 en C₃ à C₂₀ facultativement substitué avec un ou plusieurs substituants sélectionnés dans le groupe constitué d'un hydrocarbyle en C₁ à C₂₀, et d'un hétéro-alkyle linéaire ou ramifié en C₃ à C₂₀ et d'un groupe 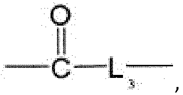 dans lequel le groupe R" est sélectionné dans le groupe constitué d'un hydrogène, d'un hydrocarbyle en C₁ à C₂₀, et d'un hétéro-alkyle linéaire ou ramifié en C₃ à C₂₀, le groupe L₃ est sélectionné dans le groupe constitué d'un alkyle linéaire ou ramifié de valence m+1 en C₂ à C₁₉ facultativement substitué avec un ou plusieurs substituants sélectionnés dans le groupe constitué d'un oxo, d'un hydrocarbyle en C₁ à C₂₀, et d'un hétéro-alkyle linéaire ou ramifié en C₃ à C₂₀ et d'un hétéro-alkyle linéaire ou ramifié de valence m+1 en C₃ à C₁₉ facultativement substitué avec un ou plusieurs substituants sélectionnés dans le groupe constitué d'un oxo, d'un hydrocarbyle en C₁ à C₂₀, et d'un hétéro-alkyle linéaire ou ramifié en C₃ à C₂₀.

3. Composé de phénol encombré selon la revendication 1, dans lequel la formule (II) est sélectionnée parmi l'une de la formule (IIₓ), la formule (IIₓₓ) et la formule (IIₓₓₓ) suivantes, 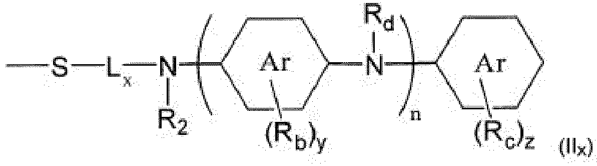 dans la formule (IIₓ), le groupe Lₓ est un groupe représenté par 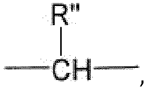 dans lequel le groupe R" est sélectionné dans le groupe constitué d'un hydrogène, d'un hydrocarbyle en C₁ à C₂₀, et d'un hétéro-alkyle linéaire ou ramifié en C₃ à C₂₀, 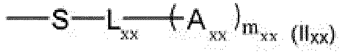 dans la formule (IIₓₓ), le groupe Lₓₓ est sélectionné dans le groupe constitué d'un alkyle linéaire ou ramifié de valence mₓₓ+1 en C₂ à C₂₀ facultativement substitué avec un ou plusieurs substituants sélectionnés dans le groupe constitué d'un hydrocarbyle en C₁ à C₂₀, et d'un hétéro-alkyle linéaire ou ramifié en C₃ à C₂₀ et d'un hétéro-alkyle linéaire ou ramifié de valence mₓₓ+1 en C₃ à C₂₀ facultativement substitué avec un ou plusieurs substituants sélectionnés dans le groupe constitué d'un hydrocarbyle en C₁ à C₂₀, et d'un hétéro-alkyle linéaire ou ramifié en C₃ à C₂₀ ; les Aₓₓ peuvent être identiques les uns aux autres ou différents les uns des autres, sélectionnés chacun indépendamment dans le groupe constitué de 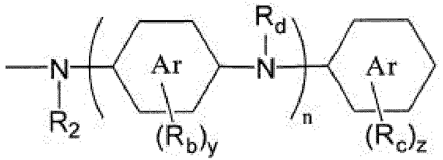 et de 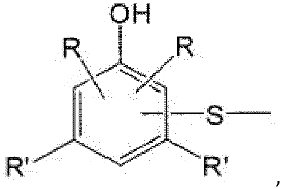 dans lesquels les R peuvent être identiques les uns aux autres ou différents les uns des autres, sélectionnés chacun indépendamment dans le groupe constitué d'un hydrogène, d'un alkyle linéaire ou ramifié en C₁ à C₃₀₀, ou d'un groupe polyoléfine présentant un poids moléculaire moyen en nombre (Mn) de 300 à 3000, d'un groupe représenté par la formule (IIₓₓ) et d'un groupe représenté par la formule (III), les R' peuvent être identiques les uns aux autres ou différents les uns des autres, sélectionnés chacun indépendamment dans le groupe constitué d'un hydrogène et d'un alkyle linéaire ou ramifié en C₁ à C₂₀, deux R et un groupe -S- occupent les trois positions restantes sur le cycle benzène, respectivement, à condition qu'au moins un Aₓₓ soit 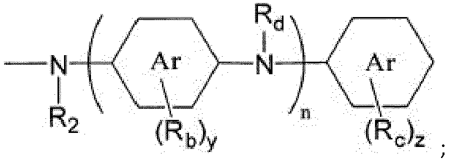 mₓₓ est un nombre entier situé dans la plage allant de 1 à 4, de préférence 1, 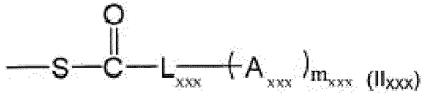 dans la formule (IIₓₓₓ), le groupe Lₓₓₓ est sélectionné dans le groupe constitué d'un alkyle linéaire ou ramifié de valence mₓₓₓ+1 en C₂ à C19-mₓₓₓ facultativement substitué avec un ou plusieurs substituants sélectionnés dans le groupe constitué d'un hydrocarbyle en C₁ à C₂₀, et d'un hétéro-alkyle linéaire ou ramifié en C₃ à C₂₀, et d'un hétéro-alkyle linéaire ou ramifié mₓₓₓ+1 valent en C₃ à C19-mₓₓₓ facultativement substitué avec un ou plusieurs substituants sélectionnés dans le groupe constitué d'un hydrocarbyle en C₁ à C₂₀, et d'un hétéro-alkyle linéaire ou ramifié en C₃ à C₂₀ et d'un groupe représenté par 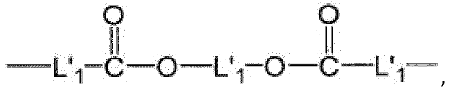 dans lequel les L'₁ peuvent être identiques les uns aux autres ou différents les uns des autres, sélectionnés chacun indépendamment dans le groupe constitué d'un alkyle linéaire ou ramifié en C₁ à C₁₀ et d'un hétéro-alkyle linéaire ou ramifié en C₃ à C₁₀, à condition que (1) les L'₁ représentent chacun indépendamment un groupe de valence 2 à valence mₓₓₓ+1, de façon que le groupe 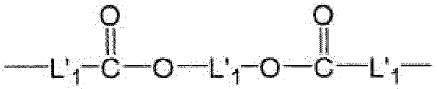 soit, dans son ensemble, de valence mₓₓₓ+1, et (2) le nombre total d'atomes de tous les groupes L'1 ne soit pas supérieur à 14, dans lequel les L'₁ peuvent être chacun indépendamment facultativement davantage substitués avec un ou plusieurs substituants sélectionnés dans le groupe constitué d'un hydrocarbyle en C₁ à C₂₀, et d'un hétéro-alkyle linéaire ou ramifié en C₃ à C₂₀ ; les Aₓₓₓ peuvent être identiques les uns aux autres ou différents les uns des autres, sélectionnés chacun indépendamment dans le groupe constitué de 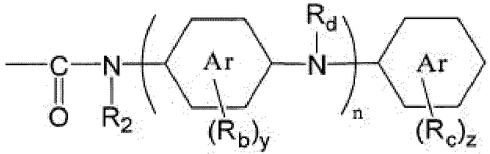 et de 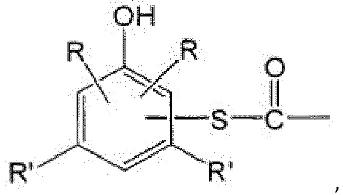 dans lesquels les R peuvent être identiques les uns aux autres ou différents les uns des autres, sélectionnés chacun indépendamment dans le groupe constitué d'un hydrogène, d'un alkyle linéaire ou ramifié en C₁ à C₃₀₀, ou d'un groupe polyoléfine présentant un poids moléculaire moyen en nombre (Mn) de 300 à 3000, d'un groupe représenté par la formule (IIₓₓₓ) et d'un groupe représenté par la formule (III), les R' peuvent être identiques les uns aux autres ou différents les uns des autres, sélectionnés chacun indépendamment dans le groupe constitué d'un hydrogène et d'un alkyle linéaire ou ramifié en C₁ à C₂₀, deux R et un groupe 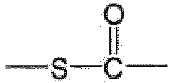 occupent les trois positions restantes sur le cycle benzène, respectivement, à condition qu'au moins un Aₓₓₓ soit 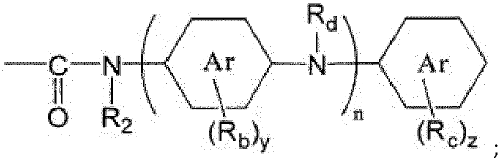 mₓₓₓ est un nombre entier situé dans la plage allant de 1 à 4, de préférence 1,
et/ou,
la formule (V) est sélectionnée parmi l'une de la formule (Vₓ), la formule (Vₓₓ) et la formule (Vₓₓₓ) suivantes, 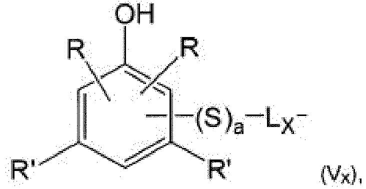 dans la formule (Vₓ), les R peuvent être identiques les uns aux autres ou différents les uns des autres, sélectionnés chacun indépendamment dans le groupe constitué d'un hydrogène, d'un alkyle linéaire ou ramifié en C₁ à C₃₀₀, ou d'un groupe polyoléfine présentant un poids moléculaire moyen en nombre (Mn) de 300 à 3000, d'un groupe représenté par la formule (IIₓ) et d'un groupe représenté par la formule (III) ; les R' peuvent être identiques les uns aux autres ou différents les uns des autres, sélectionnés chacun indépendamment dans le groupe constitué d'un hydrogène et d'un alkyle linéaire ou ramifié en C₁ à C₂₀ ; le groupe Lₓ est un groupe représenté par 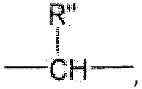 dans lequel le groupe R" est sélectionné dans le groupe constitué d'un hydrogène, d'un hydrocarbyle en C₁ à C₂₀, et d'un hétéro-alkyle linéaire ou ramifié en C₃ à C₂₀, a est 1, et deux R et un groupe -(S)ₐ-Lₓ- occupent les trois positions restantes sur le cycle benzène, respectivement, 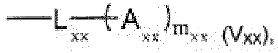 dans la formule (Vₓₓ), les Aₓₓ peuvent être identiques les uns aux autres ou différents les uns des autres, sélectionnés chacun indépendamment dans le groupe constitué de 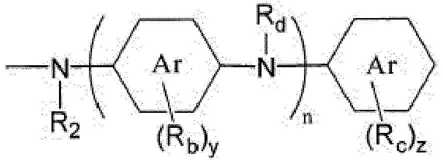 et de 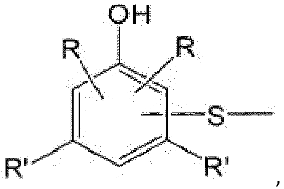 dans lesquels les R peuvent être identiques les uns aux autres ou différents les uns des autres, sélectionnés chacun indépendamment dans le groupe constitué d'un hydrogène, d'un alkyle linéaire ou ramifié en C₁ à C₃₀₀, ou d'un groupe polyoléfine présentant un poids moléculaire moyen en nombre (Mn) de 300 à 3000, d'un groupe représenté par la formule (IIₓₓ) et d'un groupe représenté par la formule (III) ; les R' peuvent être identiques les uns aux autres ou différents les uns des autres, sélectionnés chacun indépendamment dans le groupe constitué d'un hydrogène et d'un alkyle linéaire ou ramifié en C₁ à C₂₀ ; deux R et un groupe -S- occupent les trois positions restantes sur le cycle benzène, respectivement; le groupe Lₓₓ et mₓₓ sont tels que définis dans la formule (IIₓₓ) respectivement, 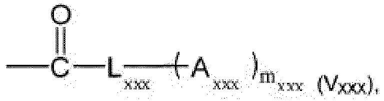 dans la formule (Vₓₓₓ), les Aₓₓₓ peuvent être identiques les uns aux autres ou différents les uns des autres, sélectionnés chacun indépendamment dans le groupe 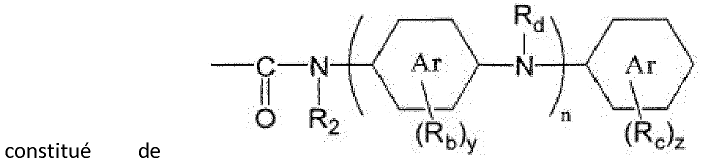 et de 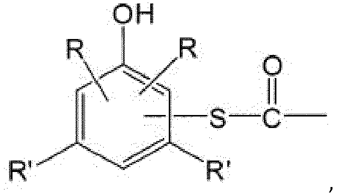 dans lesquels les R peuvent être identiques les uns aux autres ou différents les uns des autres, sélectionnés chacun indépendamment dans le groupe constitué d'un hydrogène, d'un alkyle linéaire ou ramifié en C₁ à C₃₀₀, ou d'un groupe polyoléfine présentant un poids moléculaire moyen en nombre (Mn) de 300 à 3000, d'un groupe représenté par la formule (IIₓₓₓ) et d'un groupe représenté par la formule (III) ; les R' peuvent être identiques les uns aux autres ou différents les uns des autres, sélectionnés chacun indépendamment dans le groupe constitué d'un hydrogène et d'un alkyle linéaire ou ramifié en C₁ à C₂₀ ; deux R et un groupe 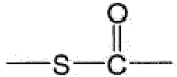 occupent les trois positions restantes sur le cycle benzène, respectivement ; les groupes Lₓₓₓ et mₓₓₓ sont tels que définis dans la formule (IIₓₓₓ) respectivement,
les autres groupes et valeurs numériques sont tels que définis dans la revendication 1.

4. Composé de phénol encombré selon la revendication 1, sélectionné dans le groupe constitué des composés spécifiques suivants ou de leurs mélanges à n'importe quel rapport entre eux : 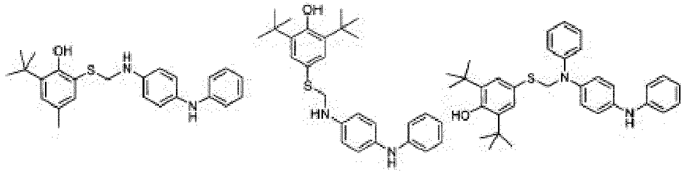 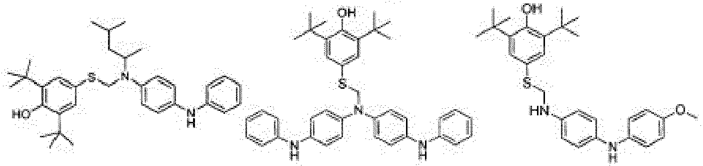 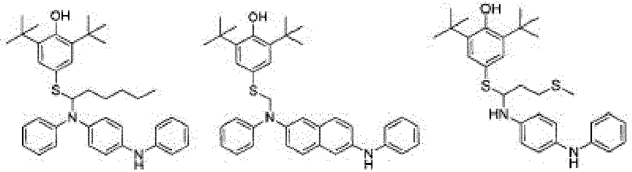 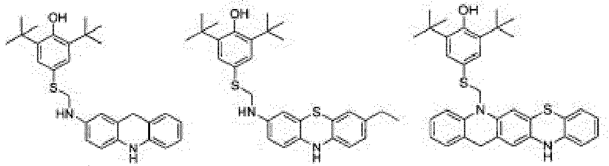 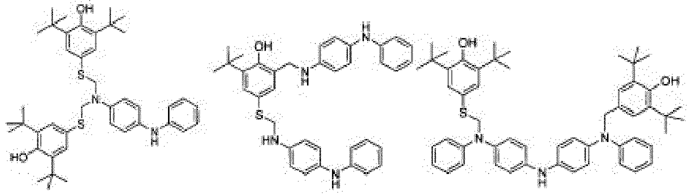 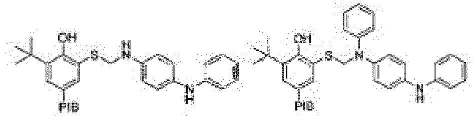 PIB = polyisobutène 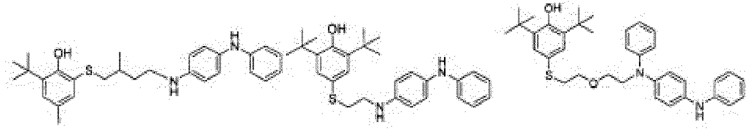 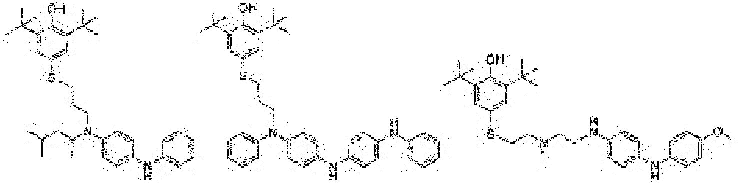 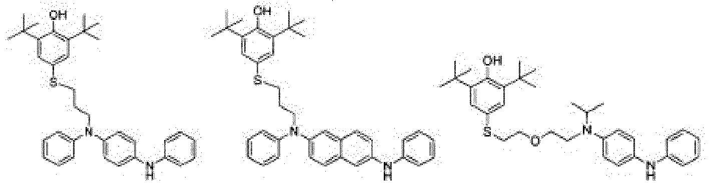 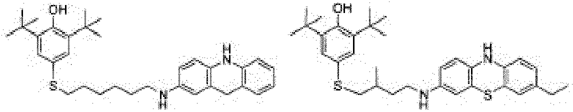 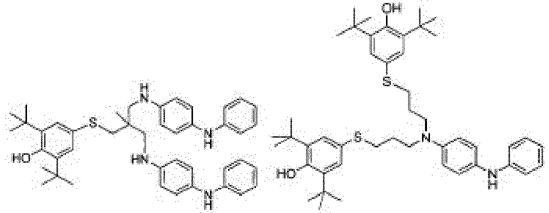 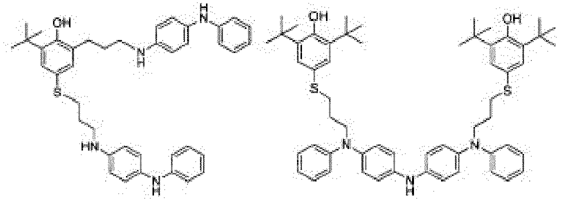 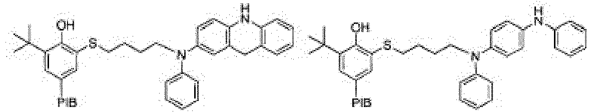 PIB = polyisobutène

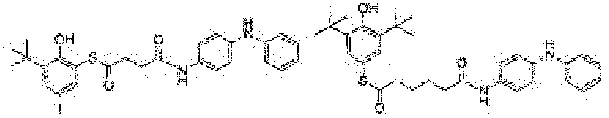

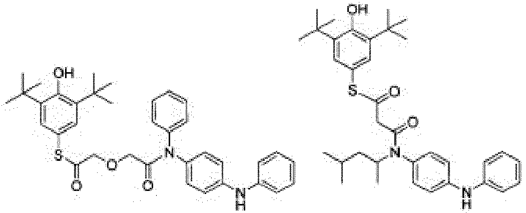

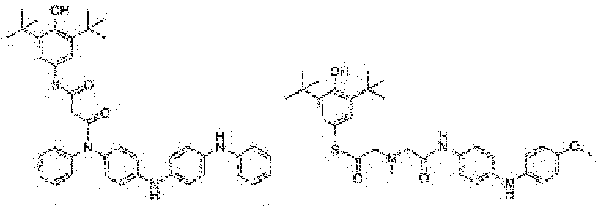

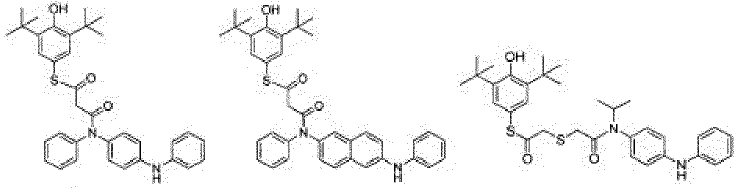

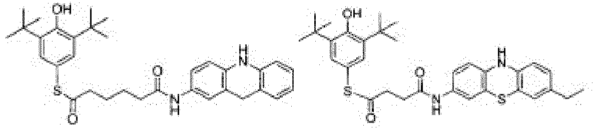

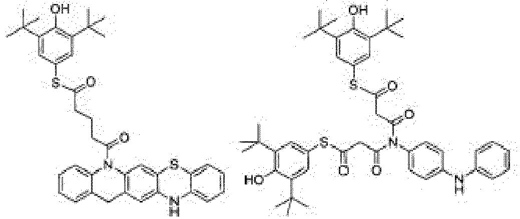

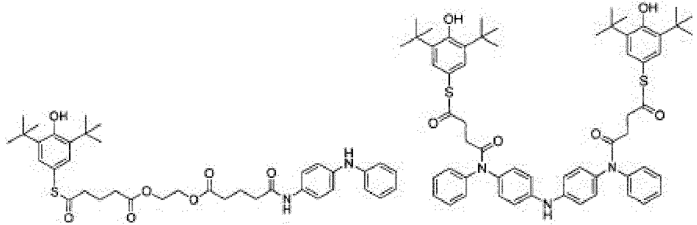

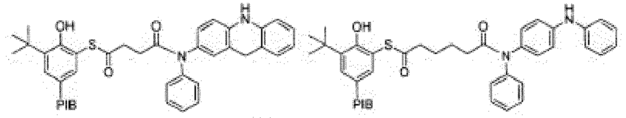

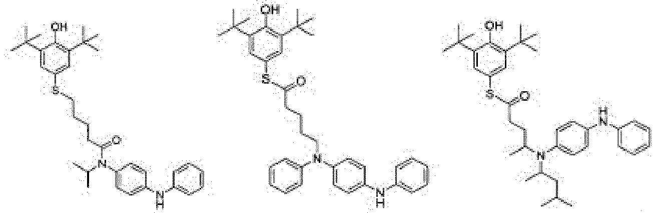

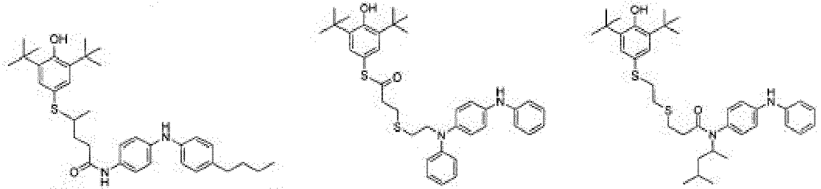

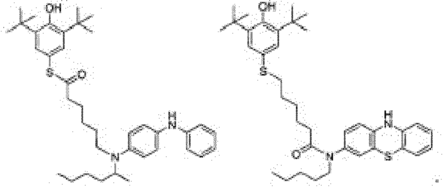

5. Procédé de production d'un composé de phénol encombré, comprenant une première étape de réaction d'un composé de phénol représenté par la formule (X) avec un composé d'amine représenté par la formule (Y) en présence d'au moins un composé de liaison sélectionné dans le groupe constitué d'un composé représenté par la formule (A) et du formaldéhyde, comprenant en outre facultativement une étape supplémentaire de réaction de ce qui résulte de la première étape avec un agent de sulfuration, de préférence le soufre, et/ou avec un composé d'aldéhyde représenté par la formule (Z), 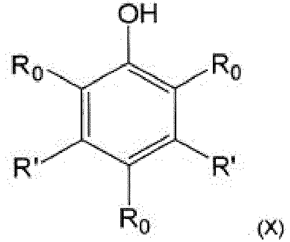 dans la formule (X), les R₀ peuvent être identiques les uns aux autres ou différents les uns des autres, sélectionnés chacun indépendamment dans le groupe constitué de -SH et d'un alkyle linéaire ou ramifié en C₁ à C₃₀₀, ou d'un groupe polyoléfine présentant un poids moléculaire moyen en nombre (Mn) de 300 à 3000, à condition qu'au moins un R₀ soit -SH ; les R' peuvent être identiques les uns aux autres ou différents les uns des autres, sélectionnés chacun indépendamment dans le groupe constitué d'un hydrogène et d'un alkyle linéaire ou ramifié en C₁ à C₂₀, 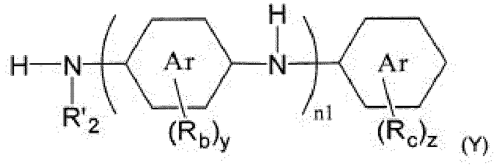 dans la formule (Y), le groupe R'₂ est sélectionné dans le groupe constitué d'un hydrogène, d'un alkyle linéaire ou ramifié en C₁ à C₂₀ et d'un groupe représenté par 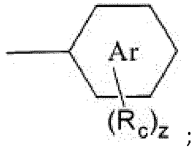 les R_{b} peuvent être identiques les uns aux autres ou différents les uns des autres, sélectionnés chacun indépendamment dans le groupe constitué d'un hydrogène et d'un alkyle linéaire ou ramifié en C₁ à C₂₀, les R_{c} peuvent être identiques les uns aux autres ou différents les uns des autres, sélectionnés chacun indépendamment dans le groupe constitué d'un hydrogène, d'un alkyle linéaire ou ramifié en C₁ à C₂₀ et d'un alkyloxy linéaire ou ramifié en C₁ à C₂₀, a ; y est un nombre entier situé dans la plage allant de 0 à 3 ; z est un nombre entier situé dans la plage allant de 0 à 3 ; n1 est un nombre entier situé dans la plage allant de 1 à 8 ; les cycles 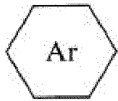 peuvent être identiques les uns aux autres ou différents les uns des autres, sélectionnés chacun indépendamment dans le groupe constitué d'un cycle benzène et d'un cycle naphtalène,
R_{f}(̵Fun )ₘ₁ (A)
dans la formule (A), le groupe R_{f} est sélectionné dans le groupe constitué d'un hydrocarbyle de valence m1 en C₁ à C₂₀, facultativement substitué avec un ou plusieurs substituants sélectionnés dans le groupe constitué d'un oxo, d'un hydrocarbyle en C₁ à C₂₀, et d'un hétéro-alkyle linéaire ou ramifié en C₃ à C₂₀ et d'un hétéro-alkyle linéaire ou ramifié de valence m1 en C₃ à C₂₀ facultativement substitué avec un ou plusieurs substituants sélectionnés dans le groupe constitué d'un oxo, d'un hydrocarbyle en C₁ à C₂₀, et d'un hétéro-alkyle linéaire ou ramifié en C₃ à C₂₀ ; m1 est un nombre entier situé dans la plage allant de 2 à 5, les Fun peuvent être identiques les uns aux autres ou différents les uns des autres, sélectionnés chacun indépendamment dans le groupe constitué d'un atome d'halogène, d'un groupe résidu d'acide carboxylique, d'un groupe résidu d'anhydride et d'un groupe résidu d'aldéhyde, tandis que dans le cas où m1 est 1, le groupe Fun est un groupe résidu d'aldéhyde, dans lequel par « groupe résidu d'acide carboxylique », il est fait référence à un groupe obtenu par l'élimination d'un carbonyle, c'est-à-dire 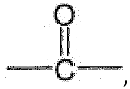 à partir d'un carboxyle, c'est-à-dire -COOH, à condition qu'il soit nécessaire pour l'atome de carbone se liant directement au groupe de prendre la forme d'un carbonyle, c'est-à-dire 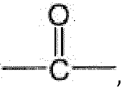 par « groupe résidu d'anhydride », il est fait référence à un groupe obtenu par l'élimination de deux carbonyles, c'est-à-dire 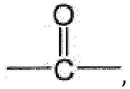 à partir d'un anhydride, c'est-à-dire 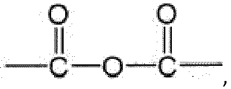 à condition qu'il soit nécessaire pour les deux atomes de carbone se liant directement au groupe de prendre la forme d'un carbonyle, c'est-à-dire 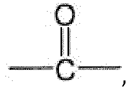 par « groupe résidu d'aldéhyde », il est fait référence à un groupe obtenu par l'élimination d'un carbonyle, c'est-à-dire 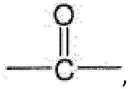 à partir d'un formyle, c'est-à-dire 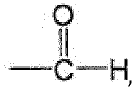 à condition qu'il soit nécessaire pour l'atome de carbone se liant directement au groupe de prendre la forme d'un carbonyle, c'est-à-dire 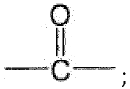 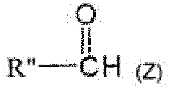 dans la formule (Z), le groupe R" est sélectionné dans le groupe constitué d'un hydrogène, d'un hydrocarbyle en C₁ à C₂₀, ou d'un hétéro-alkyle linéaire ou ramifié en C₃ à C₂₀ ;
dans lequel, l'hétéro-alkyle linéaire ou ramifié est sélectionné dans le groupe constitué d'un groupe obtenu par le remplacement direct d'un ou de plusieurs groupes -CH₂- se situant à l'intérieur d'un alkyle linéaire ou ramifié par un nombre correspondant de groupes de remplacement sélectionnés parmi -O-, -S- ou -NR'-, dans lequel R' est H ou un alkyle linéaire ou ramifié en C₁ à C₄, et d'un groupe obtenu par le remplacement direct d'un ou de plusieurs groupes -CH< se situant à l'intérieur d'un alkyle linéaire ou ramifié par un nombre correspondant de groupes de remplacement -N<.

6. Procédé selon la revendication 5, dans lequel le composé de liaison est un ou plusieurs sélectionnés dans le groupe constitué d'un composé d'aldéhyde représenté par la formule (A_{Y}), d'un composé polyhalo représenté par la formule (A_{YY}) et d'un acide carboxylique polybasique représenté par la formule (A_{YYY}) ou de dérivés de ceux-ci, sélectionnés dans le groupe constitué d'un anhydride de l'acide carboxylique polybasique et d'un halogénure d'acyle de l'acide carboxylique polybasique, 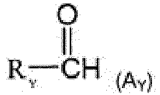 dans la formule (A_{Y}), le groupe R_{Y} est sélectionné dans le groupe constitué d'un hydrogène, d'un hydrocarbyle en C₁ à C₂₀, et d'un hétéro-alkyle linéaire ou ramifié en C₃ à C_{20,}
Rₕₐₗₒ(̵Halo)_{m_{YY}} (A_{YY})
dans la formule (A_{YY}), le groupe Rₕₐₗₒ est sélectionné dans le groupe constitué d'un alkyle linéaire ou ramifié de valence m_{YY} en C₂ à C₂₀ facultativement substitué avec un ou plusieurs substituants sélectionnés dans le groupe constitué d'un hydrocarbyle en C₁ à C₂₀, et d'un hétéro-alkyle linéaire ou ramifié en C₃ à C₂₀ et d'un hétéro-alkyle linéaire ou ramifié de valence m_{YY} en C₃ à C₂₀ facultativement substitué avec un ou plusieurs substituants sélectionnés dans le groupe constitué d'un hydrocarbyle en C₁ à C₂₀, et d'un hétéro-alkyle linéaire ou ramifié en C₃ à C₂₀, le groupe Halo est un atome d'halogène, m_{YY} est un nombre entier situé dans la plage allant de 2 à 5,
R_{L}(̵COOH)_{m_{YYY}} (A_{YYY})
dans la formule (A_{YYY}), le groupe R_{L} est sélectionné dans le groupe constitué d'un alkyle linéaire ou ramifié de valence m_{YYY} en C₂ à C_{20-mYYY} facultativement substitué avec un ou plusieurs substituants sélectionnés dans le groupe constitué d'un hydrocarbyle en C₁ à C₂₀, et d'un hétéro-alkyle linéaire ou ramifié en C₃ à C₂₀, d'un hétéro-alkyle linéaire ou ramifié de valence m_{YYY} en C₃ à C_{20-mYYY} facultativement substitué avec un ou plusieurs substituants sélectionnés dans le groupe constitué d'un hydrocarbyle en C₁ à C₂₀, et d'un hétéro-alkyle linéaire ou ramifié en C₃ à C₂₀ et d'un groupe représenté par 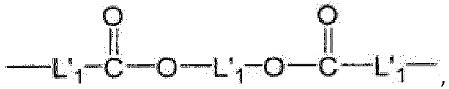 dans lequel, les L'₁ peuvent être identiques les uns aux autres ou différents les uns des autres, sélectionnés chacun indépendamment dans le groupe constitué d'un alkyle linéaire ou ramifié en C₁ à C₁₀ et d'un hétéro-alkyle linéaire ou ramifié en C₃ à C₁₀, à condition que (1) les L'₁ représentent chacun indépendamment un groupe de valence 2 à valence m_{YYY}, de façon que le groupe 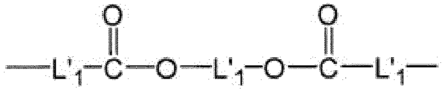 soit, dans son ensemble, de valence m_{YYY}, et (2) le nombre total d'atomes de tous les groupes L'1 ne soit pas supérieur à 14, dans lequel les L'₁ peuvent être chacun indépendamment facultativement davantage substitués avec un ou plusieurs substituants sélectionnés dans le groupe constitué d'un hydrocarbyle en C₁ à C₂₀, m_{YYY} est un nombre entier situé dans la plage allant de 2 à 5, de préférence 2.

7. Procédé selon la revendication 5, dans lequel dans la première étape, le rapport molaire entre le composé de phénol représenté par la formule (X) et le composé d'amine représenté par la formule (Y) est de 1:0,1 à 10, le rapport molaire entre le composé de phénol représenté par la formule (X) et le composé de liaison est de 1 : 0,1 à 10, dans l'étape supplémentaire, le rapport molaire entre le composé d'amine représenté par la formule (Y) et l'agent de sulfuration est de 1 : 1 à 10, le rapport molaire entre le composé d'amine représenté par la formule (Y) et le composé d'aldéhyde représenté par la formule (Z) est de 1 : 0,1 à 10.

8. Utilisation du composé de phénol encombré selon l'une quelconque des revendications 1 à 4 ou d'un composé de phénol encombré produit en ligne avec le procédé selon l'une quelconque des revendications 5 à 7 comme antioxydant.

9. Composition d'huile lubrifiante, comprenant une huile de base lubrifiante et un composé de phénol encombré selon l'une quelconque des revendications 1 à 4 ou un composé de phénol encombré produit en ligne avec le procédé selon l'une quelconque des revendications 5 à 7 en tant qu'antioxydant, dans laquelle l'antioxydant représente 0,001 à 30 % en poids, du poids total de la composition d'huile lubrifiante.

10. Composition d'huile lubrifiante selon la revendication 9, produite par le mélange du composé de phénol encombré avec l'huile de base lubrifiante.
